# EUROPEAN PATENT SPECIFICATION

(11) **EP 1 458 378 B1**
(45) Date of publication and mention of the grant of the patent: **24.05.2006**
(21) Application number: 02804013.7
(22) Date of filing: 21.11.2002
(51) Int. Cl.: A61K 31/405, A61K 31/18, A61K 31/198, A61P 25/28

(54) **AMINO ACID DERIVATIVES USEFUL FOR THE TREATMENT OF ALZHEIMER'S DISEASE**
AMINOSÄUREDERIVATE ZUR BEHANDLUNG DER ALZHEIMER-KRANKHEIT
DERIVES D'ACIDE AMINE UTILES POUR LE TRAITEMENT DE LA MALADIE D'ALZHEIMER

(30) Priority: 21.11.2001 US 334692 P
(43) Date of publication of application: 22.09.2004
(73) Proprietor: ELAN PHARMACEUTICALS, INC., South San Francisco, CA 94080 (US); Pharmacia & Upjohn Company LLC, Kalamazoo, MI 49001 (US)
(72) Inventor: JOHN, Varghese, San Francisco, CA 94122 (US)
(74) Representative: Adam, Holger
(86) International application number: PCT/US2002/037360
(87) International publication number: WO 2003/045378

(56) References cited:
- EP-A- 0 677 517
- WO-A-00/17369
- WO-A-01/68593
- WO-A-99/55687
- YAN RIQIANG ET AL: "Membrane-anchored aspartyl protease with Alzheimer's disease beta-secretase activity" NATURE, MACMILLAN JOURNALS LTD. LONDON, GB, vol. 402, 1999, pages 533-537, XP002136300 ISSN: 0028-0836
- WANG, GARY T. ET AL: "Automated synthesis of fluorogenic protease substrates: design of probes for Alzheimer's disease-associated proteases" BIOORGANIC & MEDICINAL CHEMISTRY LETTERS, vol. 2, no. 12, 1992, pages 1665-1668, XP009008042

## Description

### Field of the Invention

The present invention relates to the treatment of Alzheimer's disease and other similar diseases, and more specifically to the use of compounds that inhibit beta-secretase, an enzyme that cleaves amyloid precursor protein to produce A beta peptide, a major component of the amyloid plaques found in the brains of Alzheimer's sufferers, in such methods.

### Background of the Invention

Alzheimer's disease (AD) is a progressive degenerative disease of the brain primarily associated with aging. Clinical presentation of AD is characterized by loss of memory, cognition, reasoning, judgment, and orientation. As the disease progresses, motor, sensory, and linguistic abilities are also affected until there is global impairment of multiple cognitive functions. These cognitive losses occur gradually, but typically lead to severe impairment and eventual death in the range of four to twelve years.

Alzheimer's disease is characterized by two major pathologic observations in the brain: neurofibrillary tangles and beta amyloid (or neuritic) plaques, comprised predominantly of an aggregate of a peptide fragment know as A beta. Individuals with AD exhibit characteristic beta-amyloid deposits in the brain (beta amyloid plaques) and in cerebral blood vessels (beta amyloid angiopathy) as well as neurofibrillary tangles. Neurofibrillary tangles occur not only in Alzheimer's disease but also in other dementia-inducing disorders. On autopsy, large numbers of these lesions are generally found in areas of the human brain important for memory and cognition.

Smaller numbers of these lesions in a more restricted anatomical distribution are found in the brains of most aged humans who do not have clinical AD. Amyloidogenic plaques and vascular amyloid angiopathy also characterize the brains of individuals with Trisomy 21 (Down's Syndrome), Hereditary Cerebral Hemorrhage with Amyloidosis of the Dutch-Type (HCHWA-D), and other neurodegenerative disorders. Beta-amyloid is a defining feature of AD, now believed to be a causative precursor or factor in the development of disease. Deposition of A beta in areas of the brain responsible for cognitive activities is a major factor in the development of AD. Beta-amyloid plaques are predominantly composed of amyloid beta peptide (A beta, also sometimes designated betaA4). A beta peptide is derived by proteolysis of the amyloid precursor protein (APP) and is comprised of 39-42 amino acids. Several proteases called secretases are involved in the processing of APP.

Cleavage of APP at the N-terminus of the A beta peptide by beta-secretase and at the C-terminus by one or more gamma- secretases constitutes the beta-amyloidogenic pathway, i.e. the pathway by which A beta is formed. Cleavage of APP by alpha- secretase produces alpha-sAPP, a secreted form of APP that does not result in beta-amyloid plaque formation. This alternate pathway precludes the formation of A beta peptide. A description of the proteolytic processing fragments of APP is found, for example, in U.S. Patent Nos. 5,441,870; 5,721,130; and 5,942,400.

An aspartyl protease has been identified as the enzyme responsible for processing of APP at the beta-secretase cleavage site. The beta-secretase enzyme has been disclosed using varied nomenclature, including BACE, Asp, and Memapsin. See, for example, Sindha et al., 1999, Nature 402:537-554 (p501) and published PCT application WO00/17369.

Several lines of evidence indicate that progressive cerebral deposition of beta-amyloid peptide (A beta) plays a seminal role in the pathogenesis of AD and can precede cognitive symptoms by years or decades. See, for example, Selkoe, 1991, Neuron 6:487. Release of A beta from neuronal cells grown in culture and the presence of A beta in cerebrospinal fluid (CSF) of both normal individuals and AD subjects has been demonstrated. See, for example, Seubert et al., 1992, Nature 359:325-327.

It has been proposed that A beta peptide accumulates as a result of APP processing by beta-secretase, thus inhibition of this enzyme's activity is desirable for the treatment of AD. In vivo processing of APP at the beta-secretase cleavage site is thought to be a rate-limiting step in A beta production, and is thus a therapeutic target for the treatment of AD. See for example, Sabbagh, M., et al., 1997, *Alz. Dis. Rev.* 3, 1-19.

BACE1 knockout mice fail to produce A beta, and present a normal phenotype. When crossed with transgenic mice that over express APP, the progeny show reduced amounts of A beta in brain extracts as compared with control animals (Luo et al., 2001 *Nature Neuroscience* 4:231-232). This evidence further supports the proposal that inhibition of beta-secretase activity and reduction of A beta in the brain provides a therapeutic method for the treatment of AD and other beta amyloid disorders. At present there are no effective treatments for halting, preventing, or reversing the progression of Alzheimer's disease. Therefore, there is an urgent need for pharmaceutical agents capable of slowing the progression of Alzheimer's disease and/or preventing it in the first place.

Compounds that are effective inhibitors of beta-secretase, that inhibit beta-secretase-mediated cleavage of APP, that are effective inhibitors of A beta production, and/or are effective to reduce amyloid beta deposits or plaques, are needed for the treatment and prevention of disease characterized by amyloid beta deposits or plaques, such as AD.

At present there are no effective treatments for halting, preventing, or reversing the progression of Alzheimer's disease. Therefore, there is an urgent need for pharmaceutical agents capable of slowing the progression of Alzheimer's disease and/or preventing it in the first place.

Compounds that are effective inhibitors of beta-secretase, that inhibit beta-secretase-mediated cleavage of APP, that are effective inhibitors of A beta production, and/or are effective to reduce amyloid beta deposits or plaques, are needed for the treatment and prevention of disease characterized by amyloid beta deposits or plaques, such as AD.

### SUMMARY OF INVENTION

The present invention relates to methods of treating a subject who has, or in preventing a subject from developing, a disease or condition selected from the group consisting of Alzheimer's disease, for helping prevent or delay the onset of Alzheimer's disease, for helping to slow the progression of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease and who is in need of such treatment which comprises administration of a therapeutically effective amount of a compound described in U.S. Patent No. 6,455,587 and published International Patent Application No. WO 01/68593, i.e., a compound of formula (I) 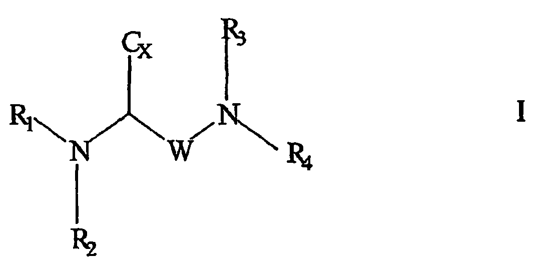 (as well as pharmaceutically acceptable derivatives thereof) and when the compound of formula I comprises an amino group or pharmaceutically acceptable ammonium salts thereof, wherein W is selected from the group consisting of -(CH₂)ₙ-, and -CH₂-XX-CH₂₋CH₂- wherein n is 1, 2, 3, 4 or 5, wherein XX is selected from the group consisting of 0, NR₅, S, SO andSO₂ wherein Cx is selected from the group consisting of -COOM, -COO R₅, -CH₂OH, - CONR₅R₆, -CONHOH, 9-fluorenylmethoxycarbonyl-lysyl-NH-CO, benzyloxycarbonyl, and tetrazolyl, wherein M is an alkali metal (e.g. Na, K, Cs, etc.) or an alkaline earth metal, wherein R₁ and R₃, the same or different, are selected (i.e. independently) from the group consisting of H, tert-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof (e.g. cyclopropylmethyl, cyclopentylmethyl, cyclohexylmethyl, cycloheptylmethyl, etc.) an arylalkyl group of formula (2) 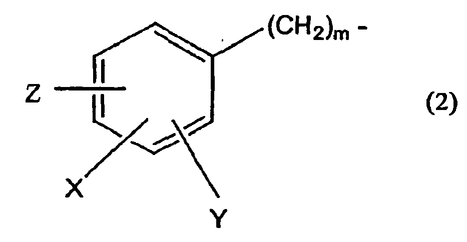 and a heterocycle-alkyl group of formula heterocycle-(CH2)ₘ- wherein R₂ and R₄ the same or different are selected (i.e. independently) from the group consisting of H, CHO-, CF₃-,CH₃CO-, benzoyl, 9-fluorenylmethoxycarbonyl, tert-butoxycarbonyl, benzyloxycarbonyl, 2-chlorobenzyloxycarbonyl, 4-OH-7-CF₃₋quinoline-3-CO-, 3-indole-CH₂CH₂CO-,3-indole-CH₂CO-,3-indole-CO-, 2-indole-CO-,C₆H₅OCH₂CO-, (C₆H₅)₂COHCO-,C₆H₅SCH₂CO-, C₆HCH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄₋SO₂-, C₆H₅CHCH-SO₂-, 4-NO₂C₆H₄-SO₂-, an aryalkyl group of formula (2) as defined above, a sulfonyl group of formula (3) 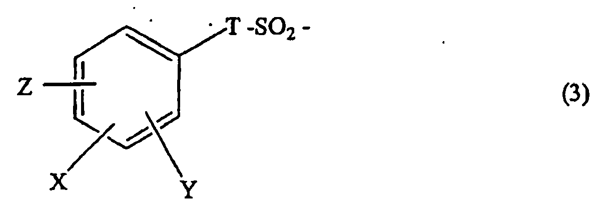 a heterocycle-alkylsulfonyl group of formula heterocycle-(CH₂)ₘ₋SO₂- and a carbonyl group of formula (4) 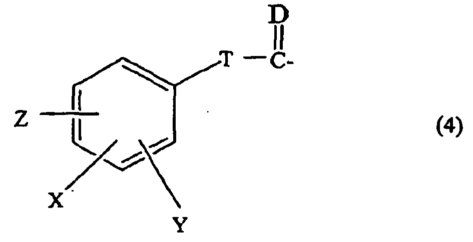 wherein T is selected from the group consisting of -(CH₂)ₘₘ-, -CH=CH-, and -CH₂-CH=CH-;
wherein D is selected from the group consisting of O, NR₇ and S;
wherein m is 1, 2, 3 or 4,
wherein mm is 0, 1, 2, 3 or 4
wherein X, Y and Z, the same or different, are selected (i.e. independently) from the group consisting of H, a straight or branched alkyl group of 1 to 6 carbon atoms, F, Cl, Br, I, - CF₃, -NO₂, -NH₂ -NHR₅, -NR₅R₆, -NHCOR₅, -NHCOheterocycle, heterocycle being as defined above, -OR₅, -SR₅, -SOR₅, -SO₂R₅, - COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, -NO₂, -NH₂ -NHR₅, -NR₅R₆, -NHCOR₅-OR₅, -SR₅, -SOR₅, -SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

U.S. Patent No. 6,455,587 and published International Patent Application No. WO 01/68593 disclose compounds of the general formula (I) and their use as antivirals. The reader is directed to U.S. Patent Nos. 6,455,587 and published International Patent Application No. WO 01/68593 for methods of preparing the compounds of the invention. The disclosure of each of these two documents is incorporated herein by reference, in its entirety.

The present invention provides methods comprising compounds, compositions, and kits for inhibiting beta-secretase-mediated cleavage of amyloid precursor protein (APP). More particularly, the methods comprising compounds, compositions, and kits are effective to inhibit the production of A beta peptide and to treat or prevent any human or veterinary disease or condition associated with a pathological form of A beta peptide.

### Detailed Description of the Invention

U.S. Patent No. 6,455,587 and published International Patent Application No. WO 01/68593 disclose various amino acid derivatives of the formula I 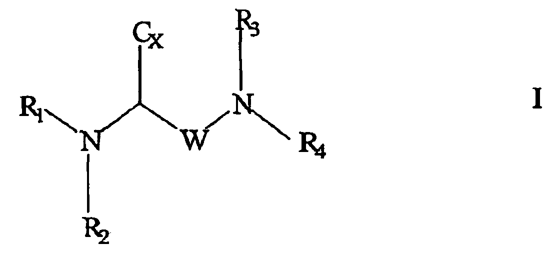 wherein R₁, R₂, R₃, R₄, W, and Cₓ are as defined above, and which are useful for the inhibition of the HIV protease enzyme. This patent does not have any disclosure with regard to Alzheimer's disease.

U.S. Patent No. 6,455,587 and published International Patent Application No. WO 01/68593 disclose how to make the above compounds and how to use them for the inhibition of the HIV protease enzyme.

In one aspect, the present invention relates to methods of treating a subject who has, or in preventing a subject from developing, a disease or condition selected from the group consisting of Alzheimer's disease, for helping prevent or delay the onset of Alzheimer's disease, for helping to slow the progression of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease and who is in need of such treatment which comprises administration of a therapeutically effective amount of a compound of formula (I), or pharmaceutically acceptable salts thereof: 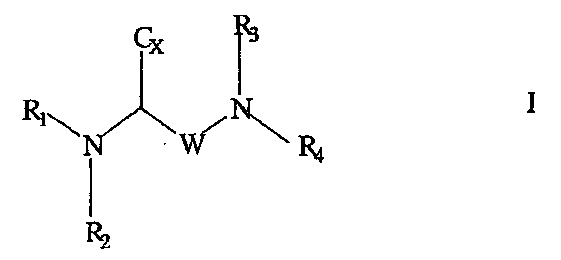 (as well as pharmaceutically acceptable derivatives thereof) and when the compound of formula I comprises an amino group or pharmaceutically acceptable ammonium salts thereof, wherein W is selected from the group consisting of -(CH₂)ₙ-, and -CH₂-XX-CH₂₋CH₂- wherein n is 1, 2, 3, 4 or 5, wherein XX is selected from the group consisting of 0, NR₅, S, SO andSO₂ wherein Cx is selected from the group consisting of -COOM, -COO R₅, -CH₂OH, - CONR₅R₆, -CONHOH, 9-fluorenylmethoxycarbonyl-lysyl-NH-CO, benzyloxycarbonyl, and tetrazolyl, wherein M is an alkali metal (e.g. Na, K, Cs, etc.) or an alkaline earth metal, wherein R₁ and R₃, the same or different, are selected (i.e. independently) from the group consisting of H, tert-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof (e.g. cyclopropylmethyl, cyclopentylmethyl, cyclohexylmethyl, cycloheptylmethyl, etc.) an arylalkyl group of formula (2) 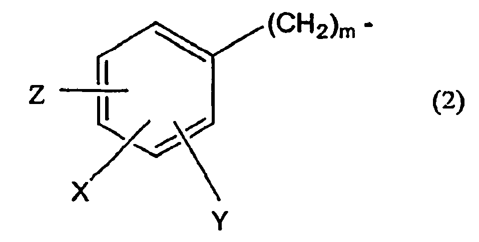 and a heterocycle-alkyl group of formula heterocycle-(CH2)ₘ- wherein R₂ and R₄ the same or different are selected (i.e. independently) from the group consisting of H, CHO-, CF₃-,CH₃CO-, benzoyl, 9-fluorenylmethoxycarbonyl, *tert*-butoxycarbonyl, benzyloxycarbonyl, 2-chlorobenzyloxycarbonyl, 4-OH-7-CF₃₋quinoline-3-CO-, 3-indole-CH₂CH₂CO-,3-indole-CH₂CO-,3-indole-CO-, 2-indole-CO-, C₆H₅OCH₂CO-, (C₆H₅)₂COHCO-, C₆H₅SCH₂CO-, C₆HCH₂CH₂CS-, cholesteryl-OCO-, 2-quinoline-CO-, xanthene-9-CO-, 4-C₆H₅CH₂CH₂CONHC₆H₄SO₂-, 2 -NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄₋SO₂-, C₆H₅CHCH-SO₂-, 4-NO₂C₆H₄-SO₂-, an aryalkyl group of formula (2) as defined above, a sulfonyl group of formula (3) 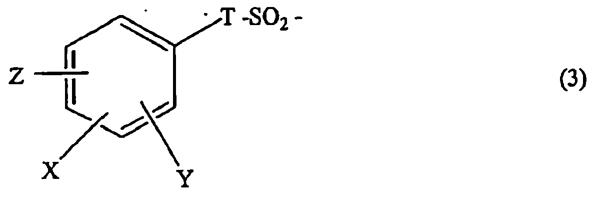 a heterocycle-alkylsulfonyl group of formula heterocycle-(CH₂)ₘ₋SO₂- and a carbonyl group of formula (4) 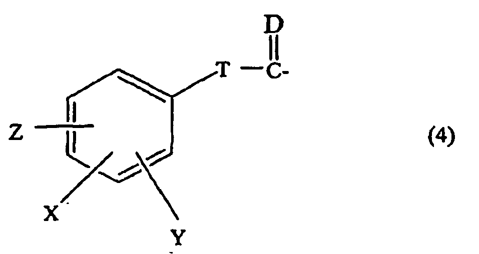 wherein T is selected from the group consisting of -(CH₂)ₘₘ-, -CH=CH-, and -CH₂-CH=CH-;
wherein D is selected from the group consisting of O, NR₇ and S;
wherein m is 1, 2, 3 or 4,
wherein mm is 0, 1, 2, 3 or 4
wherein X, Y and Z, the same or different, are selected (i.e. independently) from the group consisting of H, a straight or branched alkyl group of 1 to 6 carbon atoms, F, Cl, Br, I, -CF₃, -NO₂, -NH₂ -NHR₅, -NR₅R₆, -NHCOR₅, -NHCOheterocycle, heterocycle being as defined above, -OR₅, -SR₅, -SOR₅, -SO₂R₅, -COOR₅, -CH₂OH, - COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, -NO₂, -NH₂ -NHR₅, -NR₅R₆, -NHCOR₅-OR₅, - SR₅, -SOR₅,-SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

### Definitions

The compounds employed in the methods of this invention are identified in two ways: by descriptive names and by reference to structures having various chemical moieties. The following terms may also be used and are defined below.

The term "modulating" refers to the ability of a compound to at least partially block the active site of the beta amyloid converting enzyme, thereby decreasing, or inhibiting the turnover rate of the enzyme.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "cycloalkyl" is intended to include saturated ring groups, such as cyclopropyl, cyclobutyl, cyclopentyl, cyclohexyl (Cyh) and cycloheptyl.

"Halo", as used herein, means fluoro, chloro, bromo and iodo; and "counterion" is used to represent a small, single negatively-charged species, such as chloride, bromide, hydroxide, acetate, trifluroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl.

The term heterocycle or heterocyclic, as used herein except where noted, represents a stable 5- to 7-membered mono- or bicyclic or stable 7- to 10-membered bicyclic heterocyclic ring system, any ring of which may be saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl.

The pharmaceutically-acceptable salts of the compounds of Formula I (in the form of water- or oil-soluble or dispersible products) include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Other pharmaceutically acceptable salts include the sulfate salt ethanolate and sulfate salts.

In one aspect, this method of treatment can be used where the disease is Alzheimer's disease.

In another aspect, this method of treatment can help prevent or delay the onset of Alzheimer's disease.

In another aspect, this method of treatment can help slow the progression of Alzheimer's disease.

In another aspect, this method of treatment can be used where the disease is mild cognitive impairment.

In another aspect, this method of treatment can be used where the disease is Down's syndrome.

In another aspect, this method of treatment can be used where the disease is Hereditary Cerebral Hemorrhage with Amyloidosis of the Dutch-Type.

In another aspect, this method of treatment can be used where the disease is cerebral amyloid angiopathy.

In another aspect, this method of treatment can be used where the disease is degenerative dementias.

In another aspect, this method of treatment can be used where the disease is diffuse Lewy body type of Alzheimer's disease.

In another aspect, this method of treatment can treat an existing disease, such as those listed above.

In another aspect, this method of treatment can prevent a disease, such as those listed above, from developing or progressing.

The methods of the invention employ therapeutically effective amounts: for oral administration from about 0.1 mg/day to about 1,000 mg/day; for parenteral, sublingual, intranasal, intrathecal administration from about 0.5 to about 100 mg/day; for depo administration and implants from about 0.5 mg/day to about 50 mg/day; for topical administration from about 0.5 mg/day to about 200 mg/day; for rectal administration from about 0.5 mg to about 500 mg.

In a preferred aspect, the therapeutically effective amounts for oral administration is from about 1 mg/day to about 100 mg/day; and for parenteral administration from about 5 to about 50 mg daily.

In a more preferred aspect, the therapeutically effective amounts for oral administration is from about 5 mg/day to about 50 mg/day.

The present invention also includes the use of a compound of formula (I), or a pharmaceutically acceptable salt thereof for the manufacture of a medicament for use in treating a subject who has, or in preventing a subject from developing, a disease or condition selected from the group consisting of Alzheimer's disease, for helping prevent or delay the onset of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, diffuse Lewy body type of Alzheimer's disease and who is in need of such treatment.

In one aspect, this use of a compound of formula (I) can be employed where the disease is Alzheimer's disease.

In another aspect, this use of a compound of formula (I) can help prevent or delay the onset of Alzheimer's disease.

In another aspect, this use of a compound of formula (I) can help slow the progression of Alzheimer's disease.

In another aspect, this use of a compound of formula (I) can be employed where the disease is mild cognitive impairment.

In another aspect, this use of a compound of formula (I) can be employed where the disease is Down's syndrome.

In another aspect, this use of a compound of formula (I) can be employed where the disease is Hereditary Cerebral Hemorrhage with Amyloidosis of the Dutch-Type.

In another aspect, this use of a compound of formula (I) can be employed where the disease is cerebral amyloid angiopathy.

In another aspect, this use of a compound of formula (I) can be employed where the disease is degenerative dementias.

In another aspect, this use of a compound of formula (I) can be employed where the disease is diffuse Lewy body type of Alzheimer's disease.

In a preferred aspect, this use of a compound of formula (I) is a pharmaceutically acceptable salt of an acid selected from the group consisting of acids hydrochloric, hydrobromic, hydroiodic, nitric, sulfuric, phosphoric, citric, methanesulfonic, CH₃-(CH₂)ₙ-COOH where n is 0 thru 4, HOOC-(CH₂)ₙ₋COOH where n is as defined above, HOOC-CH=CH-COOH, and phenyl-COOH.

In another preferred aspect of the invention, the subject or patient is preferably a human subject or patient.

The present invention also includes methods for inhibiting beta-secretase activity, for inhibiting cleavage of amyloid precursor protein (APP), in a reaction mixture, at a site between Met596 and Asp597, numbered for the APP-695 amino acid isotype, or at a corresponding site of an isotype or mutant thereof; for inhibiting production of amyloid beta peptide (A beta) in a cell; for inhibiting the production of beta-amyloid plaque in an animal; and for treating or preventing a disease characterized by beta-amyloid deposits in the brain. These methods each include administration of a therapeutically effective amount of a compound of formula (I), or a pharmaceutically acceptable salt thereof.

The present invention also includes a method for inhibiting beta-secretase activity, including exposing said beta-secretase to an effective inhibitory amount of a compound of formula (I), or a pharmaceutically acceptable salt thereof.

In one aspect, this method includes exposing said beta-secretase to said compound in vitro.

In another aspect, this method includes exposing said beta-secretase to said compound in a cell.

In another aspect, this method includes exposing said beta-secretase to said compound in a cell in an animal.

In another aspect, this method includes exposing said beta-secretase to said compound in a human.

The present invention also includes a method for inhibiting cleavage of amyloid precursor protein (APP), in a reaction mixture, at a site between Met596 and Asp597, numbered for the APP-695 amino acid isotype; or at a corresponding site of an isotype or mutant thereof, including exposing said reaction mixture to an effective inhibitory amount of a compound of formula (I), or a pharmaceutically acceptable salt thereof.

In one aspect, this method employs a cleavage site: between Met652 and Asp653, numbered for the APP-751 isotype; between Met 671 and Asp 672, numbered for the APP-770 isotype; between Leu596 and Asp597 of the APP-695 Swedish Mutation; between Leu652 and Asp653 of the APP-751 Swedish Mutation; or between Leu671 and Asp672 of the APP-770 Swedish Mutation.

In another aspect, this method exposes said reaction mixture in vitro.

In another aspect, this method exposes said reaction mixture in a cell.

In another aspect, this method exposes said reaction mixture in an animal cell.

In another aspect, this method exposes said reaction mixture in a human cell.

The present invention also includes a method for inhibiting production of amyloid beta peptide (A beta) in a cell, including administering to said cell an effective inhibitory amount of a compound of formula (I), or a pharmaceutically acceptable salt thereof.

In an embodiment, this method includes administering to an animal.

In an embodiment, this method includes administering to a human.

The present invention also includes a method for inhibiting the production of beta-amyloid plaque in an animal, including administering to said animal an effective inhibitory amount of a compound of formula (I), or a pharmaceutically acceptable salt thereof.

In one embodiment of this aspect, this method includes administering to a human.

The present invention also includes a method for treating or preventing a disease characterized by beta-amyloid deposits in the brain including administering to a subject an effective therapeutic amount of a compound of formula (I) , or a pharmaceutically acceptable salt thereof.

In one aspect, this method employs a compound at a therapeutic amount in the range of from about 0.1 to about 1000 mg/day.

In another aspect, this method employs a compound at a therapeutic amount in the range of from about 15 to about 1500 mg/day.

In another aspect, this method employs a compound at a therapeutic amount in the range of from about 1 to about 100 mg/day.

In another aspect, this method employs a compound at a therapeutic amount in the range of from about 5 to about 50 mg/day.

In another aspect, this method can be used where said disease is Alzheimer's disease.

In another aspect, this method can be used where said disease is Mild Cognitive Impairment, Down's Syndrome, or Hereditary Cerebral Hemorrhage with Amyloidosis of the Dutch Type.

The present invention also includes a composition including beta-secretase complexed with a compound of formula (I), or a pharmaceutically acceptable salt thereof.

The present invention also includes a method for producing a beta-secretase complex including exposing beta-secretase to a compound of formula (I), or a pharmaceutically acceptable salt thereof, in a reaction mixture under conditions suitable for the production of said complex.

In an embodiment, this method employs exposing in vitro.

In an embodiment, this method employs a reaction mixture that is a cell.

The present invention also includes a component kit including component parts capable of being assembled, in which at least one component part includes a compound of formula (I) enclosed in a container.

In an embodiment, this component kit includes lyophilized compound, and at least one further component part includes a diluent.

The present invention also includes a container kit including a plurality of containers, each container including one or more unit dose of a compound of formula (I), or a pharmaceutically acceptable salt thereof.

In an embodiment, this container kit includes each container adapted for oral delivery and includes a tablet, gel, or capsule.

In an embodiment, this container kit includes each container adapted for parenteral delivery and includes a depot product, syringe, ampoule, or vial.

In an embodiment, this container kit includes each container adapted for topical delivery and includes a patch, medipad, ointment, or cream.

The present invention also includes an agent kit including a compound of formula (I), or a pharmaceutically acceptable salt thereof; and one or more therapeutic agents selected from the group consisting of an antioxidant, an anti-inflammatory, a gamma secretase inhibitor, a neurotrophic agent, an acetyl cholinesterase inhibitor, a statin, an A beta peptide, and an anti-A beta antibody.

The present invention provides compounds, compositions, kits, and methods for inhibiting beta-secretase-mediated cleavage of amyloid precursor protein (APP). More particularly, the compounds, compositions, and methods of the invention are effective to inhibit the production of A beta peptide and to treat or prevent any human or veterinary disease or condition associated with a pathological form of A beta peptide.

The compounds, compositions, and methods of the invention are useful for treating humans who have Alzheimer's Disease (AD), for helping prevent or delay the onset of AD, for treating subjects with mild cognitive impairment (MCI), and preventing or delaying the onset of AD in those subjects who would otherwise be expected to progress from MCI to AD, for treating Down's syndrome, for treating Hereditary Cerebral Hemorrhage with Amyloidosis of the Dutch Type, for treating cerebral beta-amyloid angiopathy and preventing its potential consequences such as single and recurrent lobar hemorrhages, for treating other degenerative dementias, including dementias of mixed vascular and degenerative origin, for treating dementia associated with Parkinson's disease, frontotemporal dementias with parkinsonism (FTDP), dementia associated with progressive supranuclear palsy, dementia associated with cortical basal degeneration, and diffuse Lewy body type AD.

The compounds of the invention possess beta-secretase inhibitory activity. The inhibitory activities of the compounds of the invention are readily demonstrated, for example, using one or more of the assays described herein or known in the art.

The compounds of formula (I) can form salts when reacted with acids. Pharmaceutically acceptable salts are generally preferred over the corresponding compounds of formula (I) since they frequently produce compounds which are usually more water soluble, stable and/or more crystalline. Pharmaceutically acceptable salts are any salt which retains the activity of the parent compound and does not impart any deleterious or undesirable effect on the subject to whom it is administered and in the context in which it is administered. Pharmaceutically acceptable salts include acid addition salts of both inorganic and organic acids. The preferred pharmaceutically acceptable salts include salts of the following acids acetic, aspartic, benzenesulfonic, benzoic, bicarbonic, bisulfuric, bitartaric, butyric, calcium edetate, camsylic, carbonic, chlorobenzoic, citric, edetic, edisylic, estolic, esyl, esylic, formic, fumaric, gluceptic, gluconic, glutamic, glycollylarsanilic, hexamic, hexylresorcinoic, hydrabamic, hydrobromic, hydrochloric, hydroiodic, hydroxynaphthoic, isethionic, lactic, lactobionic, maleic, malic, malonic, mandelic, methanesulfonic, methylnitric, methylsulfuric, mucic, muconic, napsylic, nitric, oxalic, p-nitromethanesulfonic, pamoic, pantothenic, phosphoric, monohydrogen phosphoric, dihydrogen phosphoric, phthalic, polygalactouronic, propionic, salicylic, stearic, succinic, succinic, sulfamic, sulfanilic, sulfonic, sulfuric, tannic, tartaric, teoclic and toluenesulfonic. For other acceptable salts, see *Int. J. Pharm*., 33, 201-217 (1986) and *J. Pharm. Sci*., 66(1), 1, (1977).

The present invention provides kits, and methods for inhibiting beta-secretase enzyme activity and A beta peptide production. Inhibition of beta-secretase enzyme activity halts or reduces the production of A beta from APP and reduces or eliminates the formation of beta-amyloid deposits in the brain.

### Methods of the Invention

The compounds of the invention, and pharmaceutically acceptable salts thereof, are useful for treating humans or animals suffering from a condition characterized by a pathological form of beta-amyloid peptide, such as beta-amyloid plaques, and for helping to prevent or delay the onset of such a condition. For example, the compounds are useful for treating Alzheimer's disease, for helping prevent or delay the onset of Alzheimer's disease, for treating subjects with MCI (mild cognitive impairment) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobal hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, and diffuse Lewy body type Alzheimer's disease. The compounds and compositions of the invention are particularly useful for treating, preventing, or slowing the progression of Alzheimer's disease. When treating or preventing these diseases, the compounds of the invention can either be used individually or in combination, as is best for the subject or subject.

With regard to these diseases, the term "treating" means that compounds of the invention can be used in humans with existing disease. The compounds of the invention will not necessarily cure the subject who has the disease but will delay or slow the progression or prevent further progression of the disease thereby giving the individual a more useful life span.

The term "preventing" means that that if the compounds of the invention are administered to those who do not now have the disease but who would normally develop the disease or be at increased risk for the disease, they will not develop the disease. In addition, "preventing" also includes delaying the development of the disease in an individual who will ultimately develop the disease or would be at risk for the disease due to age, familial history, genetic or chromosomal abnormalities, and/or due to the presence of one or more biological markers for the disease, such as a known genetic mutation of APP or APP cleavage products in brain tissues or fluids. By delaying the onset of the disease, compounds of the invention have prevented the individual from getting the disease during the period in which the individual would normally have gotten the disease or reduce the rate of development of the disease or some of its effects but for the administration of compounds of the invention up to the time the individual ultimately gets the disease. Preventing also includes administration of the compounds of the invention to those individuals thought to be predisposed to the disease.

In a preferred aspect, the compounds of the invention are useful for slowing the progression of disease symptoms.

In another preferred aspect, the compounds of the invention are useful for preventing the further progression of disease symptoms.

In treating or preventing the above diseases, the compounds of the invention are administered in a therapeutically effective amount. The therapeutically effective amount will vary depending on the particular compound used and the route of administration, as is known to those skilled in the art.

In treating a subject displaying any of the diagnosed above conditions a physician may administer a compound of the invention immediately and continue administration indefinitely, as needed. In treating subjects who are not diagnosed as having Alzheimer's disease, but who are believed to be at substantial risk for Alzheimer's disease, the physician should preferably start treatment when the subject first experiences early pre-Alzheimer's symptoms such as, memory or cognitive problems associated with aging. In addition, there are some subjects who may be determined to be at risk for developing Alzheimer's through the detection of a genetic marker such as APOE4 or other biological indicators that are predictive for Alzheimer's disease. In these situations, even though the subject does not have symptoms of the disease, administration of the compounds of the invention may be started before symptoms appear, and treatment may be continued indefinitely to prevent or delay the onset of the disease.

### Dosage Forms and Amounts

The compounds of the invention can be administered orally, parenterally, (IV, IM, depo-IM, SQ, and depo SQ), sublingually, intranasally (inhalation), intrathecally, topically, or rectally. Dosage forms known to those of skill in the art are suitable for delivery of the compounds of the invention.

Compositions are provided that contain therapeutically effective amounts of the compounds of the invention. The compounds are preferably formulated into suitable pharmaceutical preparations such as tablets, capsules, or elixirs for oral administration or in sterile solutions or suspensions for parenteral administration. Typically the compounds described above are formulated into pharmaceutical compositions using techniques and procedures well known in the art.

About 1 to 500 mg of a compound or mixture of compounds of the invention or a physiologically acceptable salt or ester is compounded with a physiologically acceptable vehicle, carrier, excipient, binder, preservative, stabilizer, flavor, etc., in a unit dosage form as called for by accepted pharmaceutical practice. The amount of active substance in those compositions or preparations is such that a suitable dosage in the range indicated is obtained. The compositions are preferably formulated in a unit dosage form, each dosage containing from about 2 to about 100 mg, more preferably about 10 to about 30 mg of the active ingredient. The term "unit dosage from" refers to physically discrete units suitable as unitary dosages for human subjects and other mammals, each unit containing a predetermined quantity of active material calculated to produce the desired therapeutic effect, in association with a suitable pharmaceutical excipient.

To prepare compositions, one or more compounds of the invention are mixed with a suitable pharmaceutically acceptable carrier. Upon mixing or addition of the compound(s), the resulting mixture may be a solution, suspension, emulsion, or the like. Liposomal suspensions may also be suitable as pharmaceutically acceptable carriers. These may be prepared according to methods known to those skilled in the art. The form of the resulting mixture depends upon a number of factors, including the intended mode of administration and the solubility of the compound in the selected carrier or vehicle. The effective concentration is sufficient for lessening or ameliorating at least one symptom of the disease, disorder, or condition treated and may be empirically determined.

Pharmaceutical carriers or vehicles suitable for administration of the compounds provided herein include any such carriers known to those skilled in the art to be suitable for the particular mode of administration. In addition, the active materials can also be mixed with other active materials that do not impair the desired action, or with materials that supplement the desired action, or have another action. The compounds may be formulated as the sole pharmaceutically active ingredient in the composition or may be combined with other active ingredients.

Where the compounds exhibit insufficient solubility, methods for solubilizing may be used. Such methods are known and include, but are not limited to, using cosolvents such as dimethylsulfoxide (DMSO), using surfactants such as Tween®, and dissolution in aqueous sodium bicarbonate. Derivatives of the compounds, such as salts or prodrugs may also be used in formulating effective pharmaceutical compositions.

The concentration of the compound is effective for delivery of an amount upon administration that lessens or ameliorates at least one symptom of the disorder for which the compound is administered. Typically, the compositions are formulated for single dosage administration.

The compounds of the invention may be prepared with carriers that protect them against rapid elimination from the body, such as time-release formulations or coatings. Such carriers include controlled release formulations, such as, but not limited to, microencapsulated delivery systems. The active compound is included in the pharmaceutically acceptable carrier in an amount sufficient to exert a therapeutically useful effect in the absence of undesirable side effects on the subject treated. The therapeutically effective concentration may be determined empirically by testing the compounds in known *in vitro* and *in vivo* model systems for the treated disorder.

The compounds and compositions of the invention can be enclosed in multiple or single dose containers. The enclosed compounds and compositions can be provided in kits, for example, including component parts that can be assembled for use. For example, a compound inhibitor in lyophilized form and a suitable diluent may be provided as separated components for combination prior to use. A kit may include a compound inhibitor and a second therapeutic agent for co-administration. The inhibitor and second therapeutic agent may be provided as separate component parts. A kit may include a plurality of containers, each container holding one or more unit dose of the compound of the invention. The containers are preferably adapted for the desired mode of administration, including, but not limited to tablets, gel capsules, sustained-release capsules, and the like for oral administration; depot products, pre-filled syringes, ampoules, vials, and the like for parenteral administration; and patches, medipads, creams, and the like for topical administration.

The concentration of active compound in the drug composition will depend on absorption, inactivation, and excretion rates of the active compound, the dosage schedule, and amount administered as well as other factors known to those of skill in the art.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from in vivo or in vitro test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

If oral administration is desired, the compound should be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

Oral compositions will generally include an inert diluent or an edible carrier and may be compressed into tablets or enclosed in gelatin capsules. For the purpose of oral therapeutic administration, the active compound or compounds can be incorporated with excipients and used in the form of tablets, capsules, or troches. Pharmaceutically compatible binding agents and adjuvant materials can be included as part of the composition.

The tablets, pills, capsules, troches, and the like can contain any of the following ingredients or compounds of a similar nature: a binder such as, but not limited to, gum tragacanth, acacia, corn starch, or gelatin; an excipient such as microcrystalline cellulose, starch, or lactose; a disintegrating agent such as, but not limited to, alginic acid and corn starch; a lubricant such as, but not limited to, magnesium stearate; a gildant, such as, but not limited to, colloidal silicon dioxide; a sweetening agent such as sucrose or saccharin; and a flavoring agent such as peppermint, methyl salicylate, or fruit flavoring.

When the dosage unit form is a capsule, it can contain, in addition to material of the above type, a liquid carrier such as a fatty oil. In addition, dosage unit forms can contain various other materials, which modify the physical form of the dosage unit, for example, coatings of sugar and other enteric agents. The compounds can also be administered as a component of an elixir, suspension, syrup, wafer, chewing gum or the like. A syrup may contain, in addition to the active compounds, sucrose as a sweetening agent and certain preservatives, dyes and colorings, and flavors.

The active materials can also be mixed with other active materials that do not impair the desired action, or with materials that supplement the desired action.

Solutions or suspensions used for parenteral, intradermal, subcutaneous, or topical application can include any of the following components: a sterile diluent such as water for injection, saline solution, fixed oil, a naturally occurring vegetable oil such as sesame oil, coconut oil, peanut oil, cottonseed oil, and the like, or a synthetic fatty vehicle such as ethyl oleate, and the like, polyethylene glycol, glycerine, propylene glycol, or other synthetic solvent; antimicrobial agents such as benzyl alcohol and methyl parabens; antioxidants such as ascorbic acid and sodium bisulfite; chelating agents such as ethylenediaminetetraacetic acid (EDTA) ; buffers such as acetates, citrates, and phosphates; and agents for the adjustment of tonicity such as sodium chloride and dextrose. Parenteral preparations can be enclosed in ampoules, disposable syringes, or multiple dose vials made of glass, plastic, or other suitable material. Buffers, preservatives, antioxidants, and the like can be incorporated as required.

Where administered intravenously, suitable carriers include physiological saline, phosphate buffered saline (PBS), and solutions containing thickening and solubilizing agents such as glucose, polyethylene glycol, polypropyleneglycol, and mixtures thereof. Liposomal suspensions including tissue-targeted liposomes may also be suitable as pharmaceutically acceptable carriers. These may be prepared according to methods known for example, as described in U.S. Patent No. 4,522,811.

The active compounds may be prepared with carriers that protect the compound against rapid elimination from the body, such as time-release formulations or coatings. Such carriers include controlled release formulations, such as, but not limited to, implants and microencapsulated delivery systems, and biodegradable, biocompatible polymers such as collagen, ethylene vinyl acetate, polyanhydrides, polyglycolic acid, polyorthoesters, polylactic acid, and the like. Methods for preparation of such formulations are known to those skilled in the art.

The compounds of the invention can be administered orally, parenterally (IV, IM, depo-IM, SQ, and depo-SQ), sublingually, intranasally (inhalation), intrathecally, topically, or rectally. Dosage forms known to those skilled in the art are suitable for delivery of the compounds of the invention.

Compounds of the invention may be administered enterally or parenterally. When administered orally, compounds of the invention can be administered in usual dosage forms for oral administration as is well known to those skilled in the art. These dosage forms include the usual solid unit dosage forms of tablets and capsules as well as liquid dosage forms such as solutions, suspensions, and elixirs. When the solid dosage forms are used, it is preferred that they be of the sustained release type so that the compounds of the invention need to be administered only once or twice daily.

The oral dosage forms are administered to the subject 1, 2, 3, or 4 times daily. It is preferred that the compounds of the invention be administered either three or fewer times, more preferably once or twice daily. Hence, it is preferred that the compounds of the invention be administered in oral dosage form. It is preferred that whatever oral dosage form is used, that it be designed so as to protect the compounds of the invention from the acidic environment of the stomach. Enteric coated tablets are well known to those skilled in the art. In addition, capsules filled with small spheres each coated to protect from the acidic stomach, are also well known to those skilled in the art.

When administered orally, an administered amount therapeutically effective to inhibit beta-secretase activity, to inhibit A beta production, to inhibit A beta deposition, or to treat or prevent AD is from about 0.1 mg/day to about 1,000 mg/day. It is preferred that the oral dosage is from about 1 mg/day to about 100 mg/day. It is more preferred that the oral dosage is from about 5 mg/day to about 50 mg/day. It is understood that while a subject may be started at one dose, that dose may be varied over time as the subject's condition changes.

Compounds of the invention may also be advantageously delivered in a nano crystal dispersion formulation. Preparation of such formulations is described, for example, in U.S. Patent 5,145,684. Nano crystalline dispersions of HIV protease inhibitors and their method of use are described in U.S. Patent No. 6,045,829. The nano crystalline formulations typically afford greater bioavailability of drug compounds.

The compounds of the invention can be administered parenterally, for example, by IV, IM, depo-IM, SC, or depo-SC. When administered parenterally, a therapeutically effective amount of about 0.5 to about 100 mg/day, preferably from about 5 to about 50 mg daily should be delivered. When a depot formulation is used for injection once a month or once every two weeks, the dose should be about 0.5 mg/day to about 50 mg/day, or a monthly dose of from about 15 mg to about 1,500 mg. In part because of the forgetfulness of the subjects with Alzheimer's disease, it is preferred that the parenteral dosage form be a depo formulation.

The compounds of the invention can be administered sublingually. When given sublingually, the compounds of the invention should be given one to four times daily in the amounts described above for IM administration.

The compounds of the invention can be administered intranasally. When given by this route, the appropriate dosage forms are a nasal spray or dry powder, as is known to those skilled in the art. The dosage of the compounds of the invention for intranasal administration is the amount described above for IM administration.

The compounds of the invention can be administered intrathecally. When given by this route the appropriate dosage form can be a parenteral dosage form as is known to those skilled in the art. The dosage of the compounds of the invention for intrathecal administration is the amount described above for IM administration.

The compounds of the invention can be administered topically. When given by this route, the appropriate dosage form is a cream, ointment, or patch. Because of the amount of the compounds of the invention to be administered, the patch is preferred. When administered topically, the dosage is from about 0.5 mg/day to about 200 mg/day. Because the amount that can be delivered by a patch is limited, two or more patches may be used. The number and size of the patch is not important, what is important is that a therapeutically effective amount of the compounds of the invention be delivered as is known to those skilled in the art. The compounds of the invention can be administered rectally by suppository as is known to those skilled in the art. When administered by suppository, the therapeutically effective amount is from about 0.5 mg to about 500 mg.

The compounds of the invention can be administered by implants as is known to those skilled in the art. When administering a compound of the invention by implant, the therapeutically effective amount is the amount described above for depot administration.

The invention here is the new compounds of the invention and new methods of using the compounds of the invention. Given a particular compound of the invention and a desired dosage form, one skilled in the art would know how to prepare and administer the appropriate dosage form.

The compounds of the invention are used in the same manner, by the same routes of administration, using the same pharmaceutical dosage forms, and at the same dosing schedule as described above, for preventing disease or treating subjects with MCI (mild cognitive impairment) and preventing or delaying the onset of Alzheimer's disease in those who would progress from MCI to AD, for treating or preventing Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, and diffuse Lewy body type of Alzheimer's disease.

The compounds of the invention can be used with each other or with other agents used to treat or prevent the conditions listed above. Such agents include gamma-secretase inhibitors, anti-amyloid vaccines and pharmaceutical agents such as donepezil hydrochloride (ARICEPT Tablets), tacrine hydrochloride (COGNEX Capsules) or other acetylcholine esterase inhibitors and with direct or indirectneurotropic agents of the future.

In addition, the compounds of the invention can also be used with inhibitors of P-glycoproten (P-gp). The use of P-gp inhibitors is known to those skilled in the art. See for example, *Cancer Research,* 53, 4595-4602 (1993), *Clin. Cancer Res.,* 2, 7-12 (1996), *Cancer Research,* 56, 4171-4179 (1996), International Publications WO99/64001 and WO01/10387. The important thing is that the blood level of the P-gp inhibitor be such that it exerts its effect in inhibiting P-gp from decreasing brain blood levels of the compounds of the invention. To that end the P-gp inhibitor and the compounds of the invention can be administered at the same time, by the same or different route of administration, or at different times. The important thing is not the time of administration but having an effective blood level of the P-gp inhibitor.

Suitable P-gp inhibitors include cyclosporin A, verapamil, tamoxifen, quinidine, Vitamin E-TGPS, ritonavir, megestrol acetate, progesterone, rapamycin, 10,11-methanodibenzosuberane, phenothiazines, acridine derivatives such as GF120918, FK506, VX-710, LY335979, PSC-833, GF-102,918 and other steroids. It is to be understood that additional agents will be found that do the same function and are also considered to be useful.

The P-gp inhibitors can be administered orally, parenterally, (IV, IM, IM-depo, SQ, SQ-depo), topically, sublingually, rectally, intranasally, intrathecally and by implant.

The therapeutically effective amount of the P-gp inhibitors is from about 0.1 to about 300 mg/kg/day, preferably about 0.1 to about 150 mg/kg daily. It is understood that while a subject may be started on one dose, that dose may have to be varied over time as the subject's condition changes.

When administered orally, the P-gp inhibitors can be administered in usual dosage forms for oral administration as is known to those skilled in the art. These dosage forms include the usual solid unit dosage forms of tablets and capsules as well as liquid dosage forms such as solutions, suspensions and elixirs. When the solid dosage forms are used, it is preferred that they be of the sustained release type so that the P-gp inhibitors need to be administered only once or twice daily. The oral dosage forms are administered to the subject one through four times daily. It is preferred that the P-gp inhibitors be administered either three or fewer times a day, more preferably once or twice daily. Hence, it is preferred that the P-gp inhibitors be administered in solid dosage form and further it is preferred that the solid dosage form be a sustained release form which permits once or twice daily dosing. It is preferred that what ever dosage form is used, that it be designed so as to protect the P-gp inhibitors from the acidic environment of the stomach. Enteric coated tablets are well known to those skilled in the art. In addition, capsules filled with small spheres each coated to protect from the acidic stomach, are also well known to those skilled in the art.

In addition, the P-gp inhibitors can be administered parenterally. When administered parenterally they can be administered IV, IM, depo-IM, SQ or depo-SQ. The P-gp inhibitors can be given sublingually. When given sublingually, the P-gp inhibitors should be given one thru four times daily in the same amount as for IM administration.

The P-gp inhibitors can be given intranasally. When given by this route of administration, the appropriate dosage forms are a nasal spray or dry powder as is known to those skilled in the art. The dosage of the P-gp inhibitors for intranasal administration is the same as for IM administration.

The P-gp inhibitors can be given intrathecally. When given by this route of administration the appropriate dosage form can be a parenteral dosage form as is known to those skilled in the art.

The P-gp inhibitors can be given topically. When given by this route of administration, the appropriate dosage form is a cream, ointment or patch. Because of the amount of the P-gp inhibitors needed to be administered the path is preferred. However, the amount that can be delivered by a patch is limited. Therefore, two or more patches may be required. The number and size of the patch is not important, what is important is that a therapeutically effective amount of the P-gp inhibitors be delivered as is known to those skilled in the art. The P-gp inhibitors can be administered rectally by suppository as is known to those skilled in the art.

The P-gp inhibitors can be administered by implants as is known to those skilled in the art.

There is nothing novel about the route of administration nor the dosage forms for administering the P-gp inhibitors. Given a particular P-gp inhibitor, and a desired dosage form, one skilled in the art would know how to prepare the appropriate dosage form for the P-gp inhibitor.

The compounds employed in the methods of the invention can be used in combination, with each other or with other therapeutic agents or approaches used to treat or prevent the conditions listed above. Such agents or approaches include: acetylcholine esterase inhibitors such as tacrine (tetrahydroaminoacridine, marketed as COGNEX®), donepezil hydrochloride, (marketed as Aricept® and rivastigmine (marketed as Exelon®); gamma-secretase inhibitors; anti-inflammatory agents such as cyclooxygenase II inhibitors; anti-oxidants such as Vitamin E and ginkolides; immunological approaches, such as, for example, immunization with A beta peptide or administration of anti-A beta peptide antibodies; statins; and direct or indirect neurotropic agents such as Cerebrolysin®, AIT-082 (Emilieu, 2000, *Arch. Neurol.* 57:454), and other neurotropic agents of the future.

It should be apparent to one skilled in the art that the exact dosage and frequency of administration will depend on the particular compounds employed in the methods of the invention administered, the particular condition being treated, the severity of the condition being treated, the age, weight, general physical condition of the particular subject, and other medication the individual may be taking as is well known to administering physicians who are skilled in this art.

### Inhibition of APP Cleavage

The compounds of the invention inhibit cleavage of APP between Met595 and Asp596 numbered for the APP695 isoform, or a mutant thereof, or at a corresponding site of a different isoform, such as APP751 or APP770, or a mutant thereof (sometimes referred to as the "beta secretase site"). While not wishing to be bound by a particular theory, inhibition of beta-secretase activity is thought to inhibit production of beta amyloid peptide (A beta). Inhibitory activity is demonstrated in one of a variety of inhibition assays, whereby cleavage of an APP substrate in the presence of a beta-secretase enzyme is analyzed in the presence of the inhibitory compound, under conditions normally sufficient to result in cleavage at the beta-secretase cleavage site. Reduction of APP cleavage at the beta-secretase cleavage site compared with an untreated or inactive control is correlated with inhibitory activity. Assay systems that can be used to demonstrate efficacy of the compound inhibitors of the invention are known. Representative assay systems are described, for example, in U.S. Patents No. 5,942,400, 5,744,346, as well as in the Examples below.

The enzymatic activity of beta-secretase and the production of A beta can be analyzed in vitro or in vivo, using natural, mutated, and/or synthetic APP substrates, natural, mutated, and/or synthetic enzyme, and the test compound. The analysis may involve primary or secondary cells expressing native, mutant, and/or synthetic APP and enzyme, animal models expressing native APP and enzyme, or may utilize transgenic animal models expressing the substrate and enzyme. Detection of enzymatic activity can be by analysis of one or more of the cleavage products, for example, by immunoassay, fluorometric or chromogenic assay, HPLC, or other means of detection. Inhibitory compounds are determined as those having the ability to decrease the amount of beta-secretase cleavage product produced in comparison to a control, where beta-secretase mediated cleavage in the reaction system is observed and measured in the absence of inhibitory compounds.

### Beta-Secretase

Various forms of beta-secretase enzyme are known, and are available and useful for assay of enzyme activity and inhibition of enzyme activity. These include native, recombinant, and synthetic forms of the enzyme. Human beta-secretase is known as Beta Site APP Cleaving Enzyme (BACE), Asp2, and memapsin 2, and has been characterized, for example, in U.S. Patent No. 5,744,346 and published PCT patent applications WO98/22597, WO00/03819, WO01/23533, and WO00/17369, as well as in literature publications (Hussain et al., 1999, *Mol. Cell. Neurosci.* 14:419-427; Vassar et al., 1999, *Science* 286:735-741; Yan et al., 1999, Nature 402:533-537; Sinha et al., 1999, *Nature* 40:537-540; and Lin et al., 2000, *PNAS USA* 97:1456-1460). Synthetic forms of the enzyme have also been described (WO98/22597 and WO00/17369). Beta-secretase can be extracted and purified from human brain tissue and can be produced in cells, for example mammalian cells expressing recombinant enzyme.

Preferred methods employ compounds that are effective to inhibit 50% of beta-secretase enzymatic activity at a concentration of less than about 50 micromolar, preferably at a concentration of less than about 10 micromolar, more preferably less than about 1 micromolar, and most preferably less than about 10 nanomolar.

### APP Substrate

Assays that demonstrate inhibition of beta-secretase-mediated cleavage of APP can utilize any of the known forms of APP, including the 695 amino acid "normal" isotype described by Kang et al., 1987, Nature 325:733-6, the 770 amino acid isotype described by Kitaguchi et. al., 1981, Nature 331:530-532, and variants such as the Swedish Mutation (KM670-1NL) (APP-SW), the London Mutation (V7176F), and others. See, for example, U.S. Patent No. 5,766,846 and also Hardy, 1992, Nature Genet. 1:233-234, for a review of known variant mutations. Additional useful substrates include the dibasic amino acid modification, APP-KK disclosed, for example, in WO 00/17369, fragments of APP, and synthetic peptides containing the beta-secretase cleavage site, wild type (WT) or mutated form, e.g., SW, as described, for example, in U.S. Patent No 5,942,400 and WO00/03819.

The APP substrate contains the beta-secretase cleavage site of APP (KM-DA or NL-DA) for example, a complete APP peptide or variant, an APP fragment, a recombinant or synthetic APP, or a fusion peptide. Preferably, the fusion peptide includes the beta-secretase cleavage site fused to a peptide having a moiety useful for enzymatic assay, for example, having isolation and/or detection properties. A useful moiety may be an antigenic epitope for antibody binding, a label or other detection moiety, a binding substrate, and the like.

### Antibodies

Products characteristic of APP cleavage can be measured by immunoassay using various antibodies, as described, for example, in Pirttila et al., 1999, Neuro. Lett. 249:21-4, and in U.S. Patent No. 5,612,486. Useful antibodies to detect A beta include, for example, the monoclonal antibody 6E10 (Senetek, St. Louis, MO) that specifically recognizes an epitope on amino acids 1-16 of the A beta peptide; antibodies 162 and 164 (New York State Institute for Basic Research, Staten Island, NY) that are specific for human A beta 1-40 and 1-42, respectively; and antibodies that recognize the junction region of beta-amyloid peptide, the site between residues 16 and 17, as described in U.S. Patent No. 5,593,846. Antibodies raised against a synthetic peptide of residues 591 to 596 of APP and SW192 antibody raised against 590-596 of the Swedish mutation are also useful in immunoassay of APP and its cleavage products, as described in U.S. Patent Nos. 5,604,102 and 5,721,130.

### Assay Systems

Assays for determining APP cleavage at the beta-secretase cleavage site are well known in the art. Exemplary assays, are described, for example, in U.S. Patent Nos. 5,744,346 and 5,942,400, and described in the Examples below.

### Cell Free Assays

Exemplary assays that can be used to demonstrate the inhibitory activity of the compounds of the invention are described, for example, in WO00/17369, WO 00/03819, and U.S. Patents No. 5,942,400 and 5,744,346. Such assays can be performed in cell-free incubations or in cellular incubations using cells expressing a beta-secretase and an APP substrate having a beta-secretase cleavage site.

An APP substrate containing the beta-secretase cleavage site of APP, for example, a complete APP or variant, an APP fragment, or a recombinant or synthetic APP substrate containing the amino acid sequence: KM-DA or NL-DA, is incubated in the presence of beta-secretase enzyme, a fragment thereof, or a synthetic or recombinant polypeptide variant having beta-secretase activity and effective to cleave the beta-secretase cleavage site of APP, under incubation conditions suitable for the cleavage activity of the enzyme. Suitable substrates optionally include derivatives that may be fusion proteins or peptides that contain the substrate peptide and a modification useful to facilitate the purification or detection of the peptide or its beta-secretase cleavage products. Useful modifications include the insertion of a known antigenic epitope for antibody binding; the linking of a label or detectable moiety, the linking of a binding substrate, and the like.

Suitable incubation conditions for a cell-free in vitro assay include, for example: approximately 200 nanomolar to 10 micromolar substrate, approximately 10 to 200 picomolar enzyme, and approximately 0.1 nanomolar to 10 micromolar inhibitor compound, in aqueous solution, at an approximate pH of 4 -7, at approximately 37 degrees C, for a time period of approximately 10 minutes to 3 hours. These incubation conditions are exemplary only, and can be varied as required for the particular assay components and/or desired measurement system. Optimization of the incubation conditions for the particular assay components should account for the specific beta-secretase enzyme used and its pH optimum, any additional enzymes and/or markers that might be used in the assay, and the like. Such optimization is routine and will not require undue experimentation.

One useful assay utilizes a fusion peptide having maltose binding protein (MBP) fused to the C-terminal 125 amino acids of APP-SW. The MBP portion is captured on an assay substrate by anti-MBP capture antibody. Incubation of the captured fusion protein in the presence of beta-secretase results in cleavage of the substrate at the beta-secretase cleavage site. Analysis of the cleavage activity can be, for example, by immunoassay of cleavage products. One such immunoassay detects a unique epitope exposed at the carboxy terminus of the cleaved fusion protein, for example, using the antibody SW192. This assay is described, for example, in U.S. Patent No 5,942,400.

### Cellular Assay

Numerous cell-based assays can be used to analyze beta-secretase activity and/or processing of APP to release A beta. Contact of an APP substrate with a beta-secretase enzyme within the cell and in the presence or absence of a compound inhibitor of the invention can be used to demonstrate beta-secretase inhibitory activity of the compound. Preferably, assay in the presence of a useful inhibitory compound provides at least about 30%, most preferably at least about 50% inhibition of the enzymatic activity, as compared with a non-inhibited control.

In one embodiment, cells that naturally express beta-secretase are used. Alternatively, cells are modified to express a recombinant beta-secretase or synthetic variant enzyme as discussed above. The APP substrate may be added to the culture medium and is preferably expressed in the cells. Cells that naturally express APP, variant or mutant forms of APP, or cells transformed to express an isoform of APP, mutant or variant APP, recombinant or synthetic APP, APP fragment, or synthetic APP peptide or fusion protein containing the beta-secretase APP cleavage site can be used, provided that the expressed APP is permitted to contact the enzyme and enzymatic cleavage activity can be analyzed.

Human cell lines that normally process A beta from APP provide a useful means to assay inhibitory activities of the compounds of the invention. Production and release of A beta and/or other cleavage products into the culture medium can be measured, for example by immunoassay, such as Western blot or enzyme-linked immunoassay (EIA) such as by ELISA.

Cells expressing an APP substrate and an active beta-secretase can be incubated in the presence of a compound inhibitor to demonstrate inhibition of enzymatic activity as compared with a control. Activity of beta-secretase can be measured by analysis of one or more cleavage products of the APP substrate. For example, inhibition of beta-secretase activity against the substrate APP would be expected to decrease release of specific beta-secretase induced APP cleavage products such as A beta.

Although both neural and non-neural cells process and release A beta, levels of endogenous beta-secretase activity are low and often difficult to detect by EIA. The use of cell types known to have enhanced beta-secretase activity, enhanced processing of APP to A beta, and/or enhanced production of A beta are therefore preferred. For example, transfection of cells with the Swedish Mutant form of APP (APP-SW); with APP-KK; or with APP-SW-KK provides cells having enhanced beta-secretase activity and producing amounts of A beta that can be readily measured.

In such assays, for example, the cells expressing APP and beta-secretase are incubated in a culture medium under conditions suitable for beta-secretase enzymatic activity at its cleavage site on the APP substrate. On exposure of the cells to the compound inhibitor, the amount of A beta released into the medium and/or the amount of CTF99 fragments of APP in the cell lysates is reduced as compared with the control. The cleavage products of APP can be analyzed, for example, by immune reactions with specific antibodies, as discussed above.

Preferred cells for analysis of beta-secretase activity include primary human neuronal cells, primary transgenic animal neuronal cells where the transgene is APP, and other cells such as those of a stable 293 cell line expressing APP, for example, APP-SW.

### In vivo Assays: Animal Models

Various animal models can be used to analyze beta-secretase activity and /or processing of APP to release A beta, as described above. For example, transgenic animals expressing APP substrate and beta-secretase enzyme can be used to demonstrate inhibitory activity of the compounds of the invention. Certain transgenic animal models have been described, for example, in U.S. Patent Nos.: 5,877,399; 5,612,486; 5,387,742; 5,720,936; 5,850,003; 5,877,015,, and 5,811,633, and in Ganes et al., 1995, Nature 373:523. Preferred are animals that exhibit characteristics associated with the pathophysiology of AD. Administration of the compound inhibitors of the invention to the transgenic mice described herein provides an alternative method for demonstrating the inhibitory activity of the compounds. Administration of the compounds in a pharmaceutically effective carrier and via an administrative route that reaches the target tissue in an appropriate therapeutic amount is also preferred.

Inhibition of beta-secretase mediated cleavage of APP at the beta-secretase cleavage site and of A beta release can be analyzed in these animals by measure of cleavage fragments in the animal's body fluids such as cerebral fluid or tissues. Analysis of brain tissues for A beta deposits or plaques is preferred.

On contacting an APP substrate with a beta-secretase enzyme in the presence of an inhibitory compound of the invention and under conditions sufficient to permit enzymatic mediated cleavage of APP and/or release of A beta from the substrate, the compounds of the invention are effective to reduce beta-secretase-mediated cleavage of APP at the beta-secretase cleavage site and/or effective to reduce released amounts of A beta. Where such contacting is the administration of the inhibitory compounds of the invention to an animal model, for example, as described above, the compounds are effective to reduce A beta deposition in brain tissues of the animal, and to reduce the number and/or size of beta amyloid plaques. Where such administration is to a human subject, the compounds are effective to inhibit or slow the progression of disease characterized by enhanced amounts of A beta, to slow the progression of AD in the, and/or to prevent onset or development of AD in a subject at risk for the disease.

Unless defined otherwise, all scientific and technical terms used herein have the same meaning as commonly understood by one of skill in the art to which this invention belongs. All patents and publications referred to herein are hereby incorporated by reference for all purposes.

APP, amyloid precursor protein, is defined as any APP polypeptide, including APP variants, mutations, and isoforms, for example, as disclosed in U.S. Patent No. 5,766,846.

A beta, amyloid beta peptide, is defined as any peptide resulting from beta-secretase mediated cleavage of APP, including peptides of 39, 40, 41, 42, and 43 amino acids, and extending from the beta-secretase cleavage site to amino acids 39, 40, 41, 42, or 43.

Beta-secretase (BACE1, Asp2, Memapsin 2) is an aspartyl protease that mediates cleavage of APP at the amino-terminal edge of A beta. Human beta-secretase is described, for example, in WO00/17369.

Pharmaceutically acceptable refers to those properties and/or substances that are acceptable to the subject from a pharmacological/toxicological point of view and to the manufacturing pharmaceutical chemist from a physical/chemical point of view regarding composition, formulation, stability, subject's acceptance and bioavailability.

A therapeutically effective amount is defined as an amount effective to reduce or lessen at least one symptom of the disease being treated or to reduce or delay onset of one or more clinical markers or symptoms of the disease.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

As noted above, depending on whether asymmetric carbon atoms are present, the compounds of the invention can be present as mixtures of isomers, especially as racemates, or in the form of pure isomers, especially optical antipodes.

Salts of compounds having salt-forming groups are especially acid addition salts, salts with bases or, where several salt-forming groups are present, can also be mixed salts or internal salts.

Salts are especially the pharmaceutically acceptable or non-toxic salts of compounds of formula I.

Such salts are formed, for example, by compounds of formula I having an acid group, for example a carboxy group or a sulfo group, and are, for example, salts thereof with suitable bases, such as non-toxic metal salts derived from metals of groups Ia, Ib, IIa and IIb of the Periodic Table of the Elements, for example alkali metal salts, especially lithium, sodium or potassium salts, or alkaline earth metal salts, for example magnesium or calcium salts, also zinc salts or ammonium salts, as well as salts formed with organic amines, such as unsubstituted or hydroxy-substituted mono-, di- or trialkylamines, especially mono-, di- or tri-lower alkylamines, or with quaternary ammonium bases, for example with methyl-, ethyl-, diethyl- or triethyl-amine, mono-, bis- or tris-(2-hydroxy-lower alkyl)-amines, such as ethanol-, diethanol- or triethanolamine, tris(hydroxymethyl)methylamine or 2-hydroxy-tertbutylamine, N,N-di-lower alkyl-N-(hydroxy-lower alkyl)-amines, such as N,N-dimethyl-N-(2-hydroxyethyl)-amine, or N-methyl-D-glucamine, or quaternary ammonium hydroxides, such as tetrabutylammonium hydroxide. The compounds of formula I having a basic group, for example an amino group, can form acid addition salts, for example with suitable inorganic acids, for example hydrohalic acids, such as hydrochloric acid or hydrobromic acid, or sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic, sulfo or phosphonic acids or N-substituted sulfamic acids, for example acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid, as well as with amino acids, such as the .alpha.-amino acids mentioned hereinbefore, and with methanesulfonic acid, ethanesulfonic acid, 2-hydroxyethanesulfonic acid, ethane-1,2-disulfonic acid, benzenesulfonic acid, 4-methylbenzenenesulfonic acid, naphthalene-2-sulfonic acid, 2- or 3-phosphoglycerate, glucose-6-phosphate, or N-cyclohexylsulfamic acid (forming cyclamates) or with other acidic organic compounds, such as ascorbic acid. Compounds of formula I having acid and basic groups can also form internal salts.

For isolation and purification purposes it is also possible to use pharmaceutically unacceptable salts.

The present invention may be better understood with reference to the following examples. These examples are intended to be representative of specific embodiments of the invention, and are not intended as limiting the scope of the invention.

### BIOLOGY EXAMPLES

### Example A

### Enzyme Inhibition Assay

The compounds of the invention are analyzed for inhibitory activity by use of the MBP-C125 assay. This assay determines the relative inhibition of beta-secretase cleavage of a model APP substrate, MBP-C125SW, by the compounds assayed as compared with an untreated control. A detailed description of the assay parameters can be found, for example, in U.S. Patent No. 5,942,400. Briefly, the substrate is a fusion peptide formed of maltose binding protein (MBP) and the carboxy terminal 125 amino acids of APP-SW, the Swedish mutation. The beta-secretase enzyme is derived from human brain tissue as described in Sinha et al, 1999, *Nature* 40:537-540) or recombinantly produced as the full-length enzyme (amino acids 1-501), and can be prepared, for example, from 293 cells expressing the recombinant cDNA, as described in WO00/47618.

Inhibition of the enzyme is analyzed, for example, by immunoassay of the enzyme's cleavage products. One exemplary ELISA uses an anti-MBP capture antibody that is deposited on precoated and blocked 96-well high binding plates, followed by incubation with diluted enzyme reaction supernatant, incubation with a specific reporter antibody, for example, biotinylated anti-SW192 reporter antibody, and further incubation with streptavidin/alkaline phosphatase. In the assay, cleavage of the intact MBP-C125SW fusion protein results in the generation of a truncated amino-terminal fragment, exposing a new SW-192 antibody-positive epitope at the carboxy terminus. Detection is effected by a fluorescent substrate signal on cleavage by the phosphatase. ELISA only detects cleavage following Leu 596 at the substrate's APP-SW 751 mutation site.

### Specific Assay Procedure:

Compounds are diluted in a 1:1 dilution series to a six- point concentration curve (two wells per concentration) in one 96-plate row per compound tested. Each of the test compounds is prepared in DMSO to make up a 10 millimolar stock solution. The stock solution is serially diluted in DMSO to obtain a final compound concentration of 200 micromolar at the high point of a 6-point dilution curve. Ten (10) microliters of each dilution is added to each of two wells on row C of a corresponding V-bottom plate to which 190 microliters of 52 millimolar NaOAc, 7.9% DMSO, pH 4.5 are pre-added. The NaOAc diluted compound plate is spun down to pellet precipitant and 20 microliters/well is transferred to a corresponding flat-bottom plate to which 30 microliters of ice-cold enzyme-substrate mixture (2.5 microliters MBP-C125SW substrate, 0.03 microliters enzyme and 24.5 microliters ice cold 0.09% TX100 per 30 microliters) is added. The final reaction mixture of 200 micromolar compound at the highest curve point is in 5% DMSO, 20 millimolar NaOAc, 0.06% TX100, at pH 4.5.

Warming the plates to 37 degrees C starts the enzyme reaction. After 90 minutes at 37 degrees C, 200 microliters/well cold specimen diluent is added to stop the reaction and 20 microliters/well was transferred to a corresponding anti-MBP antibody coated ELISA plate for capture, containing 80 microliters/well specimen diluent. This reaction is incubated overnight at 4 degrees C and the ELISA is developed the next day after a 2 hour incubation with anti-192SW antibody, followed by Streptavidin-AP conjugate and fluorescent substrate. The signal is read on a fluorescent plate reader.

Relative compound inhibition potency is determined by calculating the concentration of compound that showed a fifty percent reduction in detected signal (IC₅₀) compared to the enzyme reaction signal in the control wells with no added compound.

### Example B

### Cell Free Inhibition Assay Utilizing a Synthetic APP Substrate

A synthetic APP substrate that can be cleaved by beta-secretase and having N-terminal biotin and made fluorescent by the covalent attachment of Oregon green at the Cys residue is used to assay beta-secretase activity in the presence or absence of the inhibitory compounds of the invention. Useful substrates include the following:
Biotin-SEVNLDAEFRC[Oregon green]KK [SEQ ID NO: 1]
Biotin-SEVKMDAEFRC[Oregon green]KK [SEQ ID NO: 2]
Biotin-GLNIKTEEISEISYEVEFRC[Oregon green]KK [SEQ ID NO: 3]
Biotin-ADRGLTTRPGSGLTNIKTEEISEVNLDAEFC[Oregon green]KK [SEQ ID NO: 4]
Biotin-FVNQHLCₒₓGSHLVEALY-LVCₒₓGERGFFYTPKAC[Oregon green] KK [SEQ ID NO: 5]

The enzyme (0.1 nanomolar) and test compounds (0.001 - 100 micromolar) are incubated in pre-blocked, low affinity, black plates (384 well) at 37 degrees for 30 minutes. The reaction is initiated by addition of 150 millimolar substrate to a final volume of 30 microliter per well. The final assay conditions are: 0.001 - 100 micromolar compound inhibitor; 0.1 molar sodium acetate (pH 4.5); 150 nanomolar substrate; 0.1 nanomolar soluble beta-secretase; 0.001% Tween 20, and 2% DMSO. The assay mixture is incubated for 3 hours at 37 degrees C, and the reaction is terminated by the addition of a saturating concentration of immunopure streptavidin. After incubation with streptavidin at room temperature for 15 minutes, fluorescence polarization is measured, for example, using a LJL Acqurest (Ex485 nm/ Em530 nm). The activity of the beta-secretase enzyme is detected by changes in the fluorescence polarization that occur when the substrate is cleaved by the enzyme. Incubation in the presence or absence of compound inhibitor demonstrates specific inhibition of beta-secretase enzymatic cleavage of its synthetic APP substrate.

### Example C

### Beta-Secretase Inhibition: P26-P4'SW Assay

Synthetic substrates containing the beta-secretase cleavage site of APP are used to assay beta-secretase activity, using the methods described, for example, in published PCT application WO00/47618. The P26-P4'SW substrate is a peptide of the sequence:
(biotin)CGGADRGLTTRPGSGLTNIKTEEISEVNLDAEF [SEQ ID NO: 6]
The P26-P1 standard has the sequence:
(biotin)CGGADRGLTTRPGSGLTNIKTEEISEVNL [SEQ ID NO: 7].

Briefly, the biotin-coupled synthetic substrates are incubated at a concentration of from about 0 to about 200 micromolar in this assay. When testing inhibitory compounds, a substrate concentration of about 1.0 micromolar is preferred. Test compounds diluted in DMSO are added to the reaction mixture, with a final DMSO concentration of 5%. Controls also contain a final DMSO concentration of 5%. The concentration of beta secretase enzyme in the reaction is varied, to give product concentrations with the linear range of the ELISA assay, about 125 to 2000 picomolar,.after dilution.

The reaction mixture also includes 20 millimolar sodium acetate, pH 4.5, 0.06% Triton X100, and is incubated at 37 degrees C for about 1 to 3 hours. Samples are then diluted in assay buffer (for example, 145.4 nanomolar sodium chloride, 9.51 millimolar sodium phosphate, 7.7 millimolar sodium azide, 0.05% Triton X405, 6g/liter bovine serum albumin, pH 7.4) to quench the reaction, then diluted further for immunoassay of the cleavage products.

Cleavage products can be assayed by ELISA. Diluted samples and standards are incubated in assay plates coated with capture antibody, for example, SW192, for about 24 hours at 4 degrees C. After washing in TTBS buffer (150 millimolar sodium chloride, 25 millimolar Tris, 0.05% Tween 20, pH 7.5), the samples are incubated with streptavidin-AP according to the manufacturer's instructions. After a one hour incubation at room temperature, the samples are washed in TTBS and incubated with fluorescent substrate solution A (31.2 g/liter 2-amino-2-methyl-1-propanol, 30 mg/liter, pH 9.5). Reaction with streptavidin-alkaline phosphate permits detection by fluorescence. Compounds that are effective inhibitors of beta-secretase activity demonstrate reduced cleavage of the substrate as compared to a control.

### Example D

### Assays using Synthetic Oligopeptide-Substrates

Synthetic oligopeptides are prepared that incorporate the known cleavage site of beta-secretase, and optionally detectable tags, such as fluorescent or chromogenic moieties. Examples of such peptides, as well as their production and detection methods are described in U.S. Patent No: 5,942,400, herein incorporated by reference. Cleavage products can be detected using high performance liquid chromatography, or fluorescent or chromogenic detection methods appropriate to the peptide to be detected, according to methods well known in the art.

By way of example, one such peptide has the sequence (biotin)-SEVNLDAEF [SEQ ID NO: 8], and the cleavage site is between residues 5 and 6. Another preferred substrate has the sequence ADRGLTTRPGSGLTNIKTEEISEVNLDAEF [SEQ ID NO: 9], and the cleavage site is between residues 26 and 27.

These synthetic APP substrates are incubated in the presence of beta-secretase under conditions sufficient to result in beta-secretase mediated cleavage of the substrate. Comparison of the cleavage results in the presence of the compound inhibitor to control results provides a measure of the compound's inhibitory activity.

### Example E

### Inhibition of Beta-Secretase Activity - Cellular Assay

An exemplary assay for the analysis of inhibition of beta-secretase activity utilizes the human embryonic kidney cell line HEKp293 (ATCC Accession No. CRL-1573) transfected with APP751 containing the naturally occurring double mutation Lys651Met52 to Asn651Leu652 (numbered for APP751), commonly called the Swedish mutation and shown to overproduce A beta (Citron et al., 1992, Nature 360:672-674), as described in U.S. Patent No. 5,604,102.

The cells are incubated in the presence/absence of the inhibitory compound (diluted in DMSO) at the desired concentration, generally up to 10 micrograms/ml. At the end of the treatment period, conditioned media is analyzed for beta-secretase activity, for example, by analysis of cleavage fragments. A beta can be analyzed by immunoassay, using specific detection antibodies. The enzymatic activity is measured in the presence and absence of the compound inhibitors to demonstrate specific inhibition of beta-secretase mediated cleavage of APP substrate.

### Example F

### Inhibition of Beta-Secretase in Animal Models of AD

Various animal models can be used to screen for inhibition of beta-secretase activity. Examples of animal models useful in the invention include, but are not limited to, mouse, guinea pig, dog, and the like. The animals used can be wild type, transgenic, or knockout models. In addition, mammalian models can express mutations in APP, such as APP695-SW and the like described herein. Examples of transgenic non-human mammalian models are described in U.S. Patent Nos. 5,604,102, 5,912,410 and 5,811,633.

PDAPP mice, prepared as described in Games et al., 1995, Nature 373:523-527 are useful to analyze in vivo suppression of A beta release in the presence of putative inhibitory compounds. As described in U.S. Patent No. 6,191,166, 4 month old PDAPP mice are administered compound formulated in vehicle, such as corn oil. The mice are dosed with compound (1-30 mg/ml; preferably 1-10 mg/ml). After time, e.g., 3-10 hours, the animals are sacrificed, and brains removed for analysis.

Transgenic animals are administered an amount of the compound inhibitor formulated in a carrier suitable for the chosen mode of administration. Control animals are untreated, treated with vehicle, or treated with an inactive compound. Administration can be acute, i.e., single dose or multiple doses in one day, or can be chronic, i.e., dosing is repeated daily for a period of days. Beginning at time 0, brain tissue or cerebral fluid is obtained from selected animals and analyzed for the presence of APP cleavage peptides, including A beta, for example, by immunoassay using specific antibodies for A beta detection. At the end of the test period, animals are sacrificed and brain tissue or cerebral fluid is analyzed for the presence of A beta and/or beta-amyloid plaques. The tissue is also analyzed for necrosis.

Animals administered the compound inhibitors of the invention are expected to demonstrate reduced A beta in brain tissues or cerebral fluids and reduced beta amyloid plaques in brain tissue, as compared with non-treated controls.

### Example G

### Inhibition of A Beta Production in Human Subjects

Subjects suffering from Alzheimer's Disease (AD) demonstrate an increased amount of A beta in the brain. AD subjects and subjects are administered an amount of the compound inhibitor formulated in a carrier suitable for the chosen mode of administration. Administration is repeated daily for the duration of the test period. Beginning on day 0, cognitive and memory tests are performed, for example, once per month.

Subjects administered the compound inhibitors are expected to demonstrate slowing or stabilization of disease progression as analyzed by changes in one or more of the following disease parameters: A beta present in CSF or plasma; brain or hippocampal volume; A beta deposits in the brain; amyloid plaque in the brain; and scores for cognitive and memory function, as compared with control, non-treated subjects.

### Example H

### Prevention of A Beta Production in Subjects at Risk for AD

Subjects predisposed or at risk for developing AD are identified either by recognition of a familial inheritance pattern, for example, presence of the Swedish Mutation, and/or by monitoring diagnostic parameters. Subjects identified as predisposed or at risk for developing AD are administered an amount of the compound inhibitor formulated in a carrier suitable for the chosen mode of administration. Administration is repeated daily for the duration of the test period. Beginning on day 0, cognitive and memory tests are performed, for example, once per month.

Subjects administered the compound inhibitors are expected to demonstrate slowing or stabilization of disease progression as analyzed by changes in one or more of the following disease parameters: A beta present in CSF or plasma; brain or hippocampal volume; amyloid plaque in the brain; and scores for cognitive and memory function, as compared with control, non-treated subjects.

### Preparation of the Compounds

The compounds of the invention may be prepared according to the procedures set forth in published PCT application WO 01/68593.

Also, methods for preparing the compounds of the invention are set forth in Schemes 1-7. Tables 1 and 2 which follow the schemes illustrate the compounds that can be synthesized by Schemes 1-7, but Schemes 1-7 are not limited by the compounds in the tables nor by any particular substituents employed in the schemes for illustrative purposes. The examples specifically illustrate the application of the following schemes to specific compounds.

The compounds of the present invention have an affinity for aspartyl proteases, in particular, beta-secretase. Therefore, these compounds are useful as inhibitors of such proteases.

As mentioned above heterocycle refers to a stable 5 - 7 membered monocycle or bicyclic heterocycle; it may be optionally benzofused or heterocyclofused. Each heterocycle consists of carbon atoms and from one to four heteroatoms selected from the group consisting of nitrogen, oxygen and sulfur. As used herein, the terms "nitrogen and sulfur heteroatoms" include any oxidized form of nitrogen and sulfur, and the quaternized form of any basic nitrogen. The heterocyclic ring may be attached by any heteroatom or carbon atom of the cycle, which results in the benzimidazolyl, imidazolyl, imidazolinyl, imidazolidinyl, quinolyl, isoquinolyl, indolyl, pyridyl, pyrrolyl, pyrrolinyl, pyrrolidinyl, pyrazolyl, pyrazinyl, quinoxolylj piperidinyl, morpholinyl, P-carbolinyl, tetrazolyl, thiazolidinyl, benzofuranyl, thiamorpholinyl, benzoxazolyl, oxopiperidinyl, oxopyrroldinyl, oxoazepinyl, azepinyl, isoxazolyl, tetrahydropyranyl, tetrahydrofuranyl, thiadiazolyl, thiadiazinyl, benzodioxolyl, thiophenyl, tetrahydrothiophenyl, nicoticoyl, morpholinecarbodithioyl and sulfolanyl, As mentioned above R2 and R4 are each independently (i.e. same or different) selected from the above mentioned class of substituents; the may in particular be 9 fluorenylmethoxycarbonyl (Fmoc), tert-butoxycarbonyl (t-Boc), benzyloxycarbonyl (Cbz), 2-chlorobenzyloxycarbonyl (2-ClCbz), substituted arylSO₂, substituted arylalkylSO₂, heteroarylSO₂, acyl, substituted arylalkylacyl or heteroalkylacyl groups.

The configuration of the asymmetric centre can be D, L and DL, preferably the configuration corresponding to that found in L-lysine and L-ornithine.

In addition, this invention provides pharmaceutical compositions in which these novel compounds of formula I derived from L- amino acids are used to inhibit aspartyl proteases, including beta secretase.

The term "pharmaceutically effective amount" refers to an amount effective in treating Alzheimer's disease in a subject.

The term "prophylactically effective amount" refers to an amount effective in preventing Alzheimer's disease in a subject. As used herein, the term "subject" refers to a mammal, including a human.

The term "pharmaceutically acceptable carrier or adjuvant" and "physiologically acceptable vehicle" refer to a non-toxic carrier or adjuvant that may be administered to a subject, together with a compound of this invention, and which does not destroy the pharmacological activity thereof.

As used herein, the compounds of this invention, including the compounds of formula I are defined to include pharmaceutically acceptable derivatives thereof. A "pharmaceutically acceptable derivative" means any pharmaceutically acceptable salt, ester, or salt of such ester, of a compound of this invention or any other compound which, upon administration to a recipient, is capable of providing (directly or indirectly) a compound of this invention or an antivirally active metabolite or residue thereof.

The compounds of this invention contain one or more asymmetric carbon atoms and thus may occur as racemates and racemic mixtures, single enantiomer, diastereomeric mixtures and individual diastereoisomers. All such isomeric forms of these compounds are expressly included in the present invention. Each stereogenic carbon may be of the R or S configuration.

Combinations of substituents and variables envisioned by this invention are only those that result in the formation of stable compounds. The term "stable", as used herein, refers to compounds that possess stability sufficient to allow manufacture and administration to a mammal by methods known in the art. Typically, such compounds are stable at a temperature 15 to 40°C or less, in the absence of moisture or other chemically reactive conditions, for at least a week.

The compounds of the present invention as mentioned above include salts. Salts of the compounds of this invention include those derived from pharmaceutically acceptable inorganic and organic acids and bases (e.g. salts of acidic compounds of formula I with bases). Salts derived from appropriate inorganic and organic bases include for example, alkali metal (e.g., sodium), alkaline earth metal (e.g., magnesium), ammonium and N - (C₁₋₄ alkyl)₄+ salts.

This invention also envisions ammonium salts (i.e. salts of amino groups) such as for example halide acid salts (e.g. hydrochloride, hydrobromide, hydroiodide salts). Thus the invention envisions the quaternization of any basic nitrogen containing groups (i.e. amino group(s)) of the compounds disclosed herein. The basic nitrogen can be quaternized with any agents known to those of ordinary skill in the art including, for example, lower alkyl halides, such as methyl, ethyl, propyl and butyl chlorides, bromides and iodides; dialkyl sulfates including dimethyl, diethyl, dibutyl and diamyl sulfates; long chain halides such as decyl, lauryl, myristyl and stearyl chlorides, bromides and iodides, and aralkyl halides including benzyl and phenethyl bromides. Water or oil-soluble or dispersible products may be obtained by such quaternization.

Other examples of acid salts include: acetate, adipate, alginate, aspartate, benzoate, benzenesulfonate, bisulfate, butyrate, citrate, camphorate, camphorsulfonate, cyclopentanepropionate, digluconate, dodecylhydrogensulfate, dodecylsulfate, 16 ethanesulfonate, formate, fumarate, glucoheptanoate, glycerophosphate, glycollate, hemisulfate, heptanoate, hexanoate, 2-hydroxyethanesulfonate, lactate, maleate, malonate, methanesulfonate, 2-naphthylsulfonate, nicotinate, nitrate, oxalate, pamoate, pectinate, perchlorate, persulfate, 3-phenylpropionate, phosphate, picrate, pivalate, propionate, salicylate, succinate, sulfate, tartrate, thiocyanate, tosylate, and undecanoate.

The compounds of this invention are readily prepared using conventional techniques from commercially available starting materials.

In the following the preparation of compounds in accordance with the present convention will be described with reference to a number of process schemes wherein the various starting reactants as well as products thereof are designated by reference numbers e.g. in scheme 1 the starting ornithine or lysine is designated with the reference number 1.
Some abbreviations that may appear in this application are as follows:

### ABBREVIATIONS

| Designation | Protecting Group |
|---|---|
| BOC (Boc) | t-butyloxycarbonyl |
| CBZ (Cbz) | benzyloxycarbonyl(carbobenzoxy) |
| TBS (TBDMS) | t-butyl-dimethylsilyl |

| | Activating Group |
|---|---|
| HBT(HOBT or HOBt) | 1-hydroxybenzotriazole hydrate |

| Designation | Coupling Reagent |
|---|---|
| BOP reagent | benzotriazol-1-yloxytris(dimethylamino)phosphonium hexafluorophosphate |
| BOP-C1 | bis(2-oxo-3-oxazolidinyl) phosphinic chloride |
| EDC | 1-ethyl-3-(3-dimethylaminopropyl) carbodiimide hydrochloride |

| | Other |
|---|---|
| (BOC)₂O (BOC₂O) | di-t-butyl dicarbonate |
| n-Bu₄N⁺F⁻ | tetrabutyl ammonium fluoride |
| DABCYL | 4-[[4'-(dimethylamino)phenyl]azo]benzoic acid |
| DEAD | Diethyl azodicarboxylate |
| DIEA | N,N-Diisopropylethylamine |
| DTT | Dithiothreitol |
| EDANS | 5-[(2'-aminoethyl)amino]naphthalene sulfonic acid |
| EDC | 1-Ethyl-3-(3-dimethylaminopropyl)carbodiimide hydrochloride |
| EDTA | Ethylenediaminetetraacetic acid |
| Fmoc | 9-Fluorenylmethoxycarbonyl |
| LC-MS | liquid chromotography-mass spectrometry |
| MP | Melting point |
| Za | Benzyloxycarbonyl |
| | |
| nBuLi (n-Buli) | n-butyllithium |
| DMF | dimethylformamide |
| Et₃N | triethylamine |
| EtOAc | ethyl acetate |
| TFA | trifluoroacetic acid |
| DMAP | dimethylaminopyridine |
| DME | dimethoxyethane |
| LDA | lithium diisopropylamide |
| THF, THIF | tetrahydrofuran |

| | Amino Acid |
|---|---|
| Ile | L-isoleucine |
| Val | L-valine |

In general, amino acid derivatives of formula I are readily obtained from commercially available sources. Following the indications summarized in Scheme 1, the *N*ω-benzyloxycarbonyl blocking group of *N*α-(9-fluorenylmethoxycarbonyl)- *N*ω-benzyloxycarbonyl ornithine or lysine 1 is removed by a treatment with TFA in CH₂Cl₂ according to the indications found in protective groups in Organic Synthesis, 3rd Edition, p. 520-521 (*T.W.* Greene and P. G. M. Wuts (John Wiley & Sons, Inc. 1999). The intermediate is obtained by the evaporation of the solvent and then reacted with a sulfonyl chloride or an acyl chloride derivative in the presence of a base such as 1 M potassium carbonate, affording after normal work-up the desired product 2 in excellent yields. Another possible starting material could be Nα-*tert*-butoxycarbonyl-Nω-benzyloxycarbonyl-L-ornithine or L-lysine la with the removal of the *tert*-butoxycarbonyl group being also achieved by a treatment with TFA in CH₂C₂-. Products 2 with the Fmoc or the t-Boc groups are obtained in excellent yields. 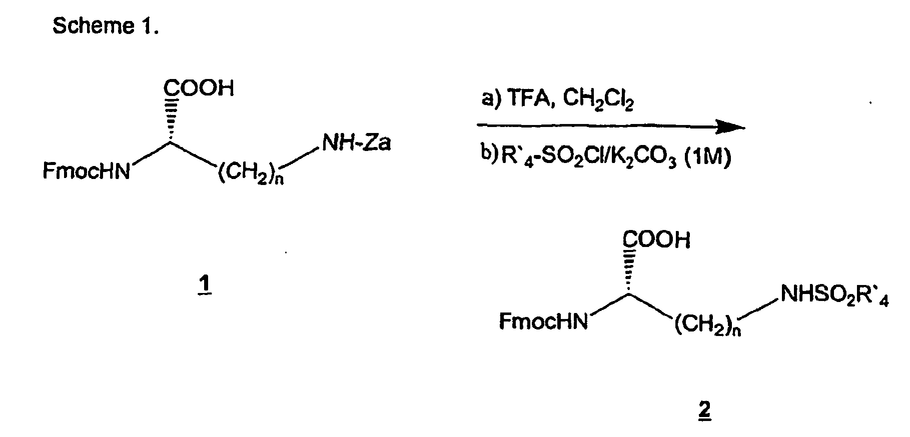 where
Fmoc = 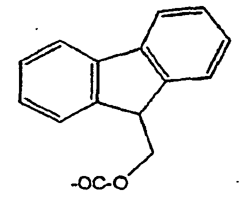 R'₄SO₂ = R₄ wherein R₄ is a sulfonyl group of formula (3) as defined herein
Za = C₆H₅CH₂O-CO-
n = 3 Ornithine
n = 4 Lysine

Scheme 2, below, illustrates the preparation of Nα-isobutyl-Nα- (substituted benzenesulfonyl N∈- (9-fluorenylmethoxycarbonyl) derivatives 9 from readily available material Nc&-tert butoxycarbonyl-Ne- benzyloxycarbonyl-L-lysine 3. The esterification with methyl iodide is achieved by treatment of the potassium salt in DMF with methyl iodide. Removal of the tert butoxycarbonyl group from product 4 is done by treatment with TFA in methylene chloride.

Reductive alkylation of the free amino group with isobutyraldehyde utilizing sodium cyanoborohydride provided the Nα-isobutylamino acid derivative 6. Reaction with a substituted benzenesulfonyl chloride provides the product 7, the HCl scavenger being triethylamine or diisopropylethylamine. Hydrolysis of the methyl ester is accomplished with sodium hydroxide in methanol providing the acid 8 in good yield. It should be noted that extensive epimerisation takes place in this base catalysed hydrolytic reaction. The DL derivative 8 is then submitted to hydrogenolysis to remove the terminal blocking group and the free amino group can then be acylated with 9-fluorenylmethyl chloroformate or N-(9 fluorenylmethoxycarbonyloxy) succinimide to provide the desired product 9 in its racemized form. At that step, use of a substituted sulfonyl chloride provided the corresponding sulfonyl derivative and an acylation of the same amino group with an acyl chloride or an activated acid provided the acylated derivative of general structure 9.

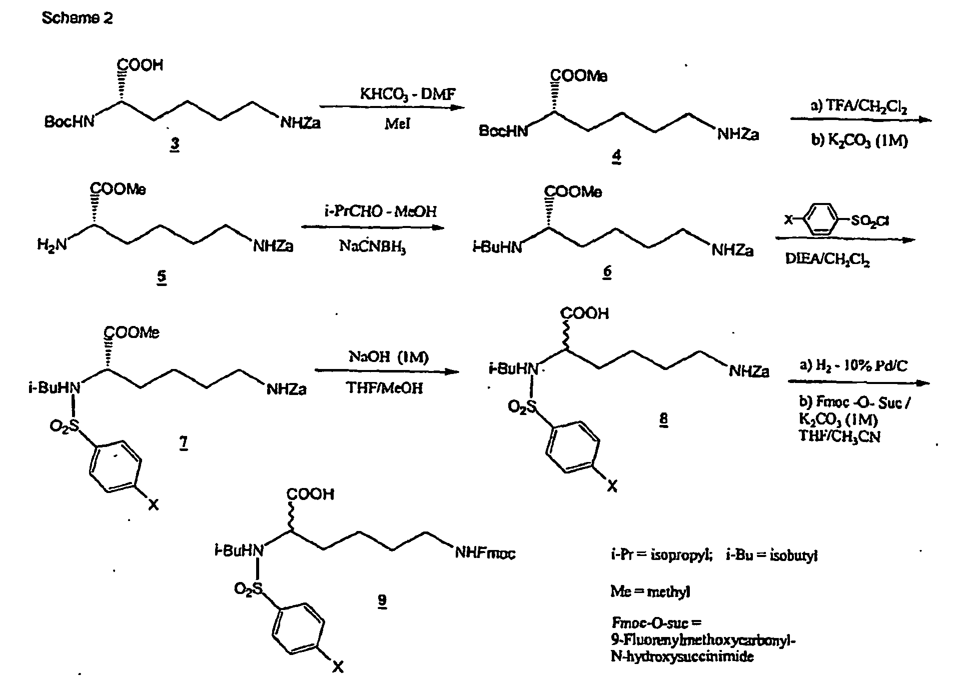

The problem of racemization is resolved by the use of a benzyl ester to block the carboxylic acid instead of a methyl ester. An additional advantage is the simultaneous removal of the two blocking groups (ester and carbamate) by hydrogenolysis, thus shortening the sequence by one step. The scheme 3, outlined below exemplifies this approach clearly.

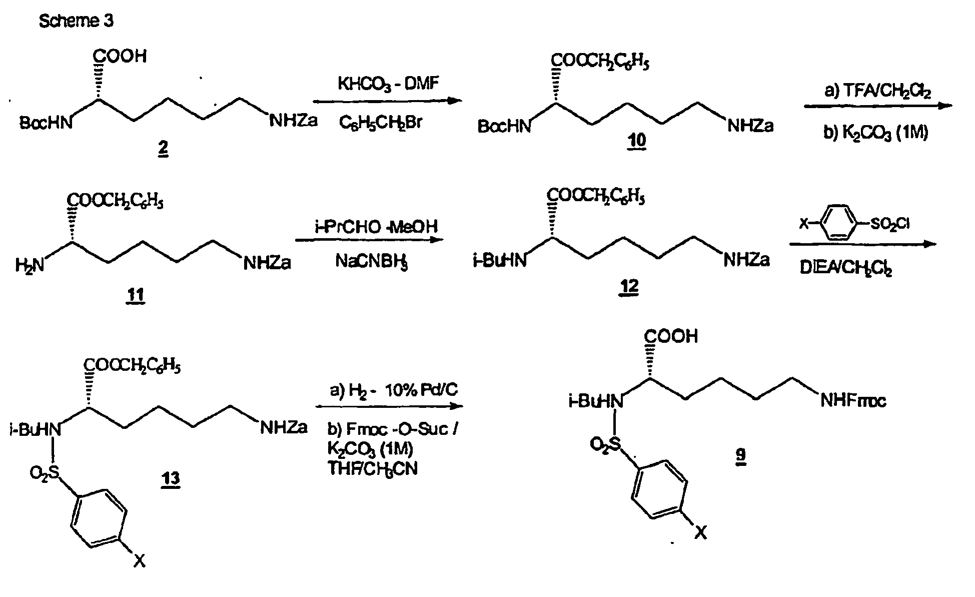

Scheme 4 demonstrates another improved approach to similar derivatives in a much shorter sequence and provide higher yields and avoid the use of protection- deprotection steps. The starting material for this sequence is a readily available commercial product, L-a-amino-E caprolactam 14. Reductive alkylation utilizing the sodium cyanoborohydride conditions provided the alkylated derivative 1.5 in 95% yield as a crystalline solid that can then be subjected to reaction with a substituted sulfonyl chloride in presence of triethylamine in methylene chloride. Product 16 is obtained in 87% yield. Treatment with 12N HCI and acetic acid for 2 hours at reflux provided the lysine, deriv ative 17 quantitatively and the terminal amino group is then acylated with an acyl chloride or an activated carboxylic acid to provide compound 18. Scheme 4a illustrates a particular example of the process of scheme 4.

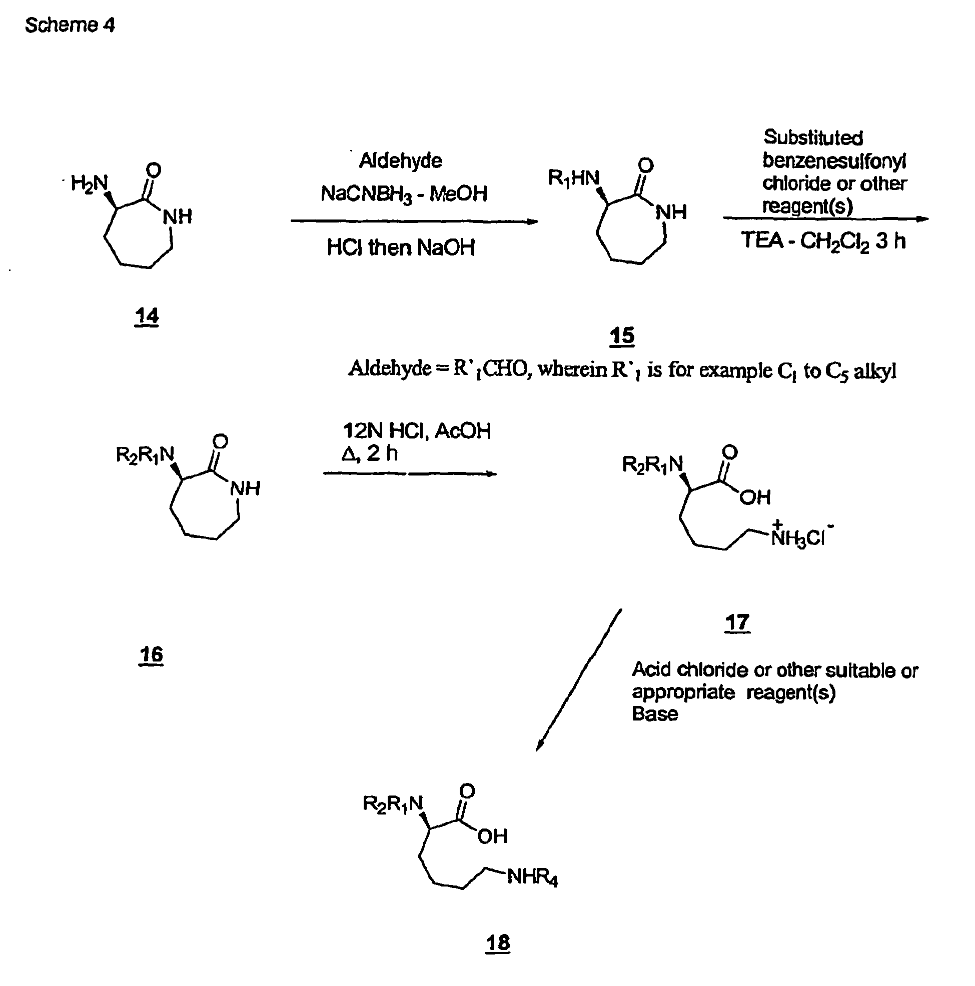

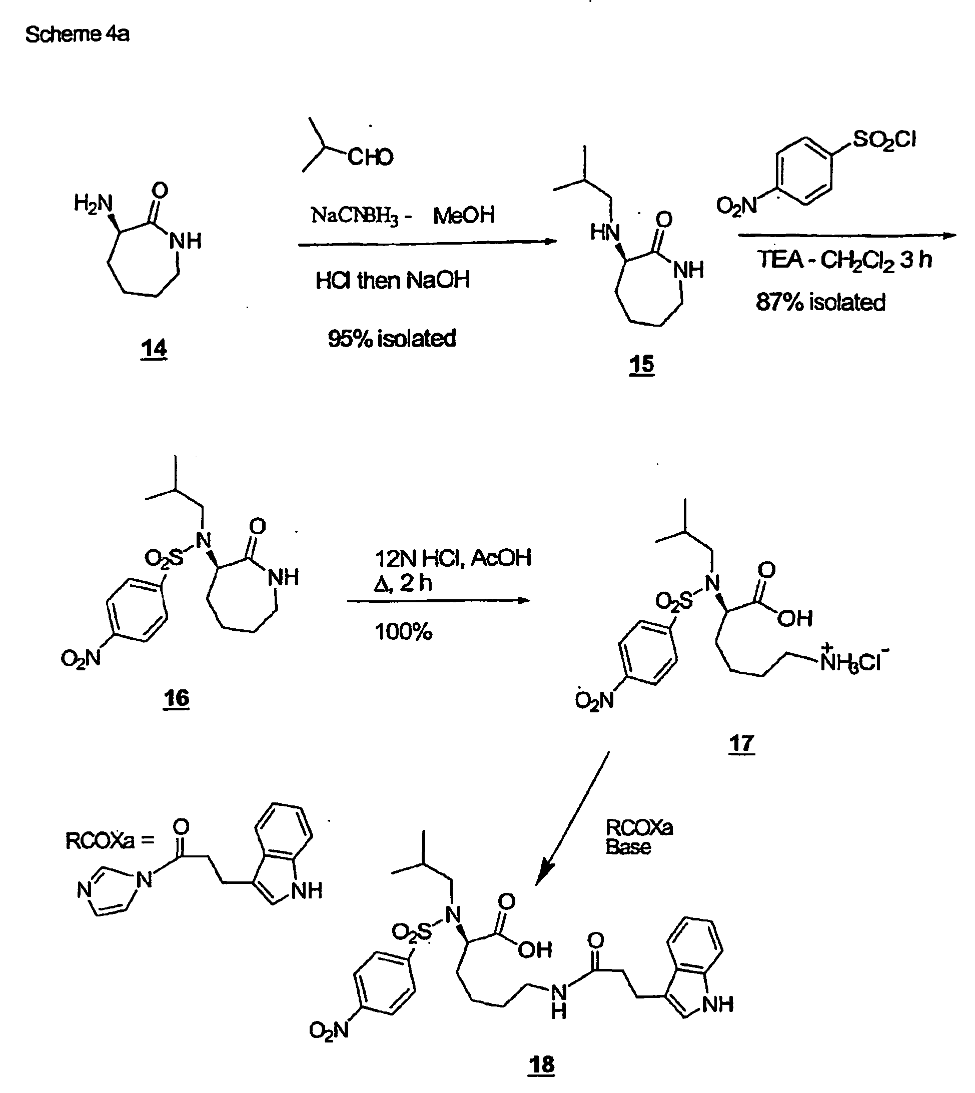

Scheme 5 summarizes the work done to obtain derivatives of structure I where n is 1. The starting material is L-serine 19a. Treatment with DEAD and triphenyl phosphine provided the *β-*lactone 20 that is then treated with ammonia in ethanol. The Nα-tert-butoxycarbonyl-*Nβ*-amino propionic acid derivative is then reacted as usual with-a substituted benzenesulfonyl chloride, providing product 21. The removal of the blocking group and its replacement by another one (v.g. Fmoc) provided compound 22. Scheme 5a illustrates a particular example of the process of scheme 5.

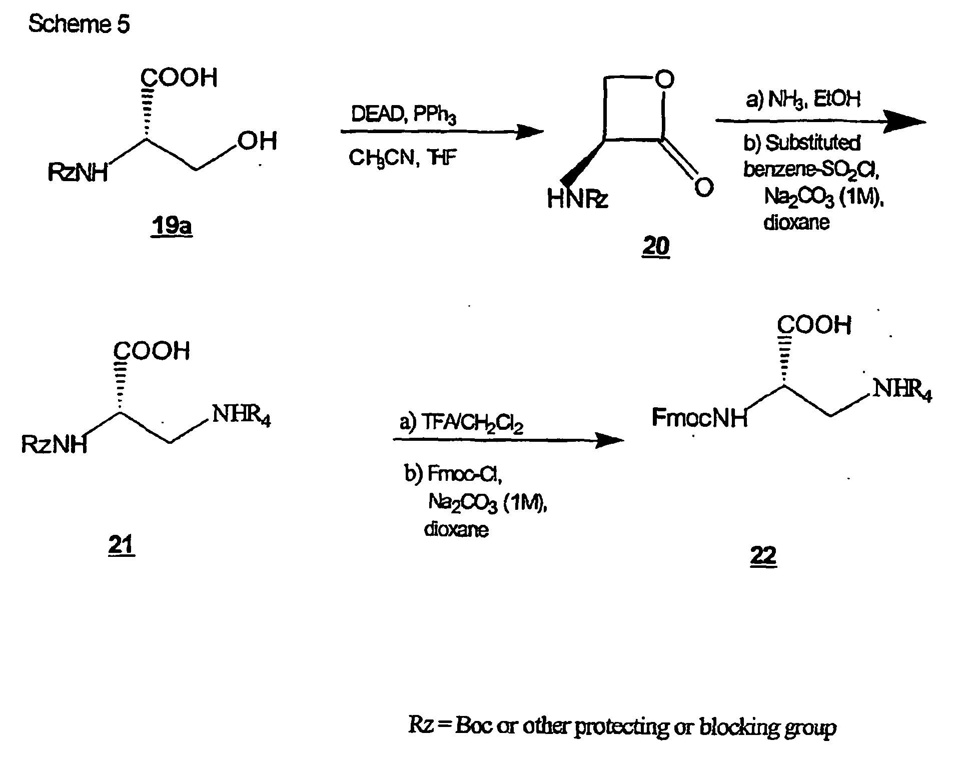

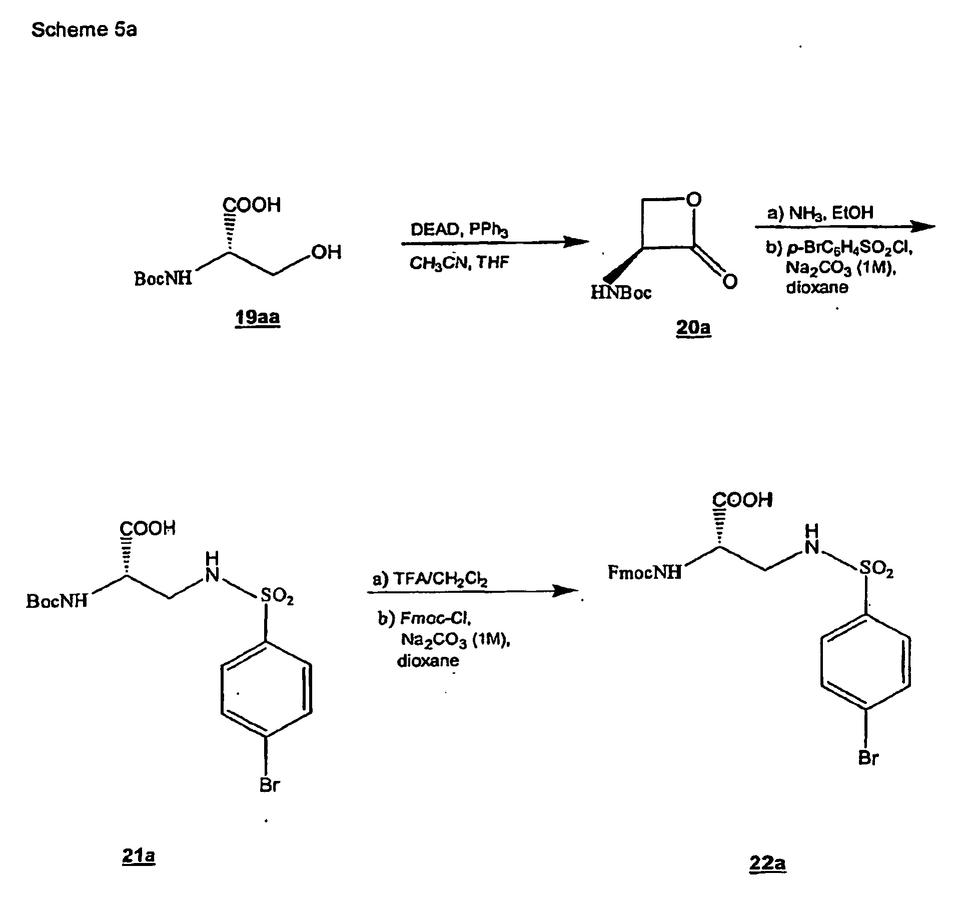

Scheme 6 below relates to an alternative process whereby compounds of formula I as defined herein may be obtained wherein W is -CH₂-XX-CH₂-CH₂-, XX being as defined herein. Thus reductive alkylation of L-serine methyl ester 19b may give rise to compound 23 which may be treated with a substituted benzenesulfonyl chloride to give a compound 24. Further treatment of compound 24 with tosyl chloride in dichloromethane and triethylamine may give rise to a *α,β*-unsaturated ester 25. Michael addition of a substituted ethylenediamine and saponification may give rise to compound 26. The *α,β* -unsaturated ester 25 may be treated with a variety of reagents to provide compounds containing a heteroatom as shown in Table 2 for compound nos. 205, 206 and 207. The chiral derivatives may also be obtained via ring opening of a P-lactone derived form 24 to give pure L isomers 26.

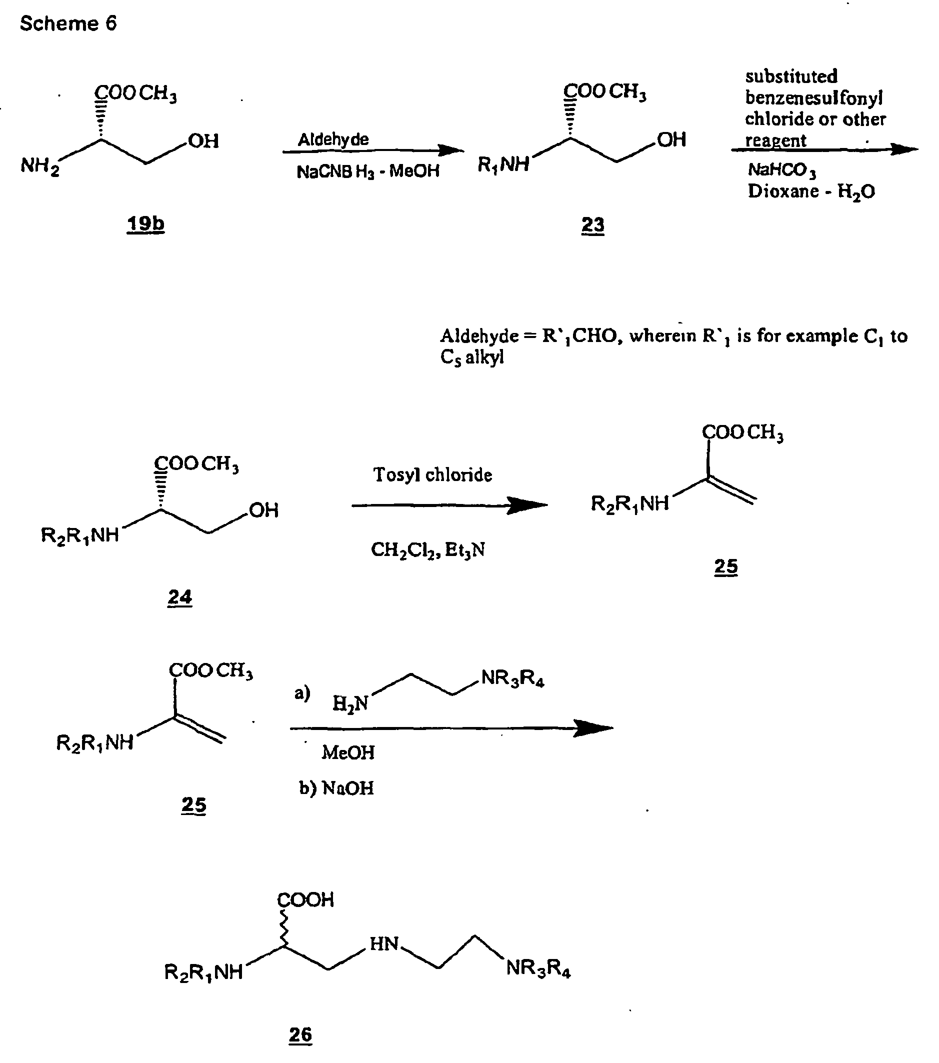

Scheme 7 provides a summary of the approach of products of structure I where n is 2. Again the starting material is a simple product L-homoserine 27. The amino group is protected by the *tert*-butoxycarbonyl group and treatmemt with diazomethane in ether provided derivative 28. The next sequence is the transformation of the hydroxyl group to an amino group, which is easily achieved by treatment of 28 with 4-methylbenzenesulfonyl chloride in pyridine and methylene chloride followed by displacement of the tosyl group by azide in DMF. The product 29 is then reduced by hydrogen gas in presence of 10% Pd/C and the resulting amino group is reacted with a substituted berizenesulfonyl chloride, providing an excellent yield of derivative 30. Its conversion to another group on the alpha amino group is performed as previously described by the removal of the tert-butoxycarbonyl group with TFA in methylene chloride and then reaction with 9-fluorenylmethyl chloroformate or N-(9 fluorenylmethoxycarbonyloxy) succinimide, providing the final compound 31.

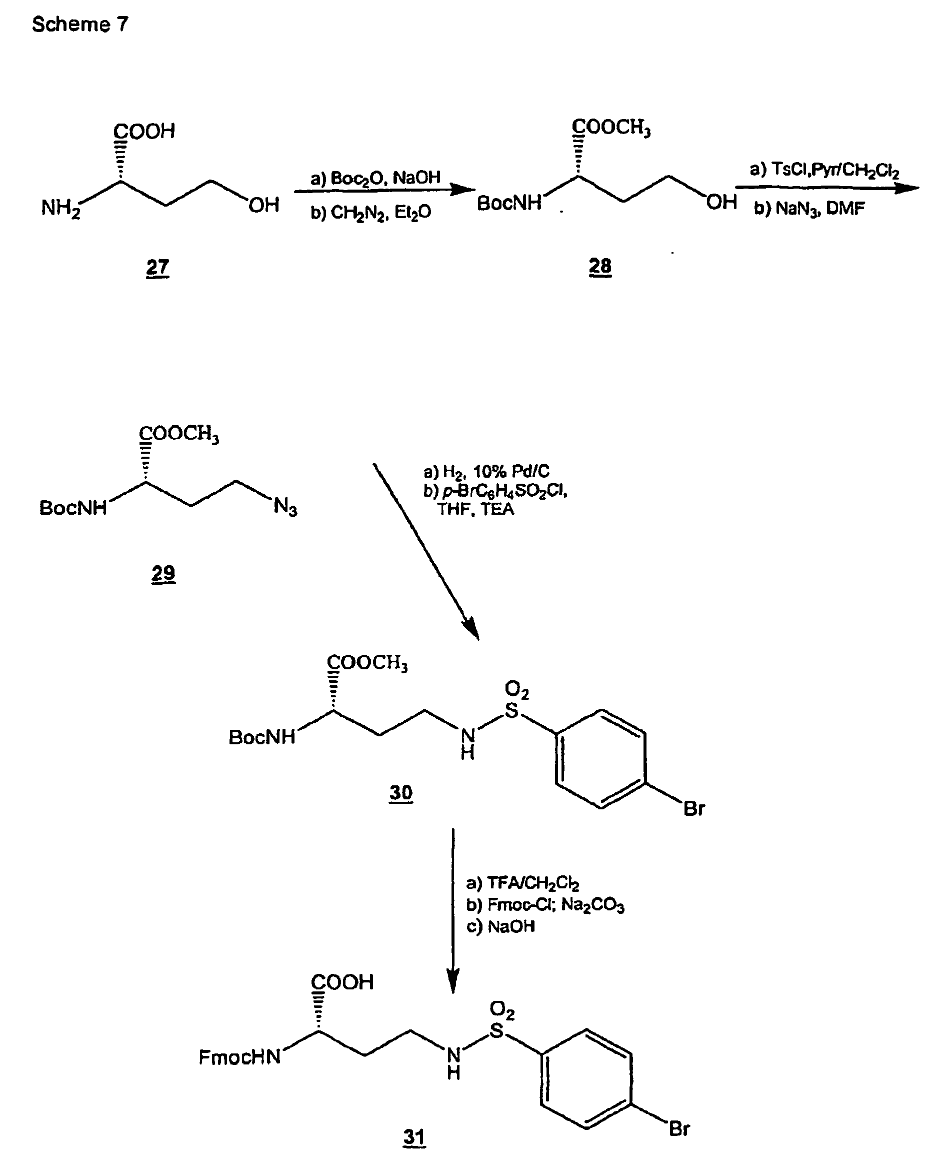

As it can be appreciated by the skilled artisan, the above synthetic schemes are not intended to be a comprehensive list of all means by which the compounds described and claimed in this application may be synthesized. Further methods will be evident to those of ordinary skill in the art.

The compounds of this invention may be modified by appending appropriate functionalities to enhance selective biological properties. Such modifications are known in the art and include those which increase biological penetration into a given biological system (e.g., blood, lymphatic system, central nervous system), increase oral availability, increase solubility to allow administration by injection, alter metabolism and alter rate of excretion.

As discussed above, the novel compounds of the present invention are excellent ligands for aspartyl proteases, particularly beta-secretase.

Pharmaceutical compositions of this invention comprise any of the compounds of the present invention, and pharmaceutically acceptable salts thereof, with any pharmaceutically acceptable carrier, adjuvant or vehicle. Pharmaceutically acceptable carriers, adjuvants and vehicles that may be used in the pharmaceutical compositions of this invention include, but are not limited to ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodiurn hydrogen phosphate, potassium hydrogen phosphate, sodium chloride, zinc salts, colloidal silica, magnesium trisilicate, polyvinyl pyrrolidone, cellulose-based substances, polyethyleneglycol, sodium carboxymethylcellulose, polyacrylates, waxes, polyethylene- polyoxypropylene-block polymers, polyethylene glycol and wool fat.

The pharmaceutical compositions of this invention may be administered orally, parenterally by inhalation spray, topically, rectally, nasally, buccally, vaginally or via an implanted reservoir. We prefer oral administration or administration by injection. The pharmaceutical compositions of this invention may contain any conventional non-toxic pharmaceutically acceptable carriers, adjuvants or vehicles. The term "parenteral" as used herein includes subcutaneous, intracutaneous, intravenous, intramuscular, intra-articular, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3- butanediol. Among the acceptable vehicles and solvents that may be employed are amino acid, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv. or a similar alcohol.

The pharmaceutical compositions of this invention may be orally administered in any orally acceptable dosage form including, but not limited to, capsules, tablets, and aqueous suspension and solutions. In the case of tablets for oral use, carriers that are comm only used include lactose and com starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added.

The pharmaceutical compositions of this invention may also be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax, and polyethylene glycols.

Topical administration of the pharmaceutical compositions of this invention is especially useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene or polyoxypropylene compound, emulsifying wax and water. Alternatively, the pharmaceutical compositions can be formulated with a suitable lotion or cream containing the active compound suspended or dissolved in a carrier. Suitable carriers include, but are not limited to, mineral oil, sorbitan monostearate, polysorbate 60, cetyl esters wax cetearyl alcohol, 2-octyldodecanol, benzyl alcohol and water. The pharmaceutical compositions of this invention may also be topically applied to the lower intestinal tract by rectal suppository 36 formulation or in a suitable neat formulation. Topically-transdermal patches are also included in this invention.

The pharmaceutical compositions of this invention may be administered by nasal aerosol or inhalation. Such compositions are prepared according to techniques well- known in the art of pharmaceutical formulation and may be prepared as solutions in saline employing benzyl alcohol or other suitable preservatives, absorption promoters to enhance bioavailability, fluorocarbons, and/or other solubilizing or dispersing agents known in the art.

Dosage levels of between about 0.01 and about 25 mg/kg body weight per day, preferably between about 0.5 and about 25 mg/kg body weight per day of the active ingredient compound are useful in the prevention and treatment of viral infection, including HIV infection. Typically, the pharmaceutical compositions of this invention will be administered from about I to about 5 times per day or alternatively, as a continuous infusion. Such administration can be used as a chronic or acute therapy. The amount of active ingredient that may be combined with the carrier materials to produce a single dosage form will vary depending upon the subject treated and the particular mode of administration. A typical preparation will contain from about 5% to about 95% active compound (w/w). Preferably, such preparations contain from about 20% to about 80% active compound.

Upon improvement of a subject's condition, a maintenance dose of a compound, composition or combination of this invention may be administered if necessary. Subsequently, the dosage or frequency of admin istration, or both, may be reduced, as a function of the symptoms, to a 37 level at which the improved condition is retained. When the symptoms have been alleviated to the desired level, treatment should cease. Subjects may, however, require intermittent treatment on a long-term basis, upon any recurrence of disease symptoms.

As the skilled artisan will appreciate, lower or higher doses than those recited above may be required. Specific dosage and treatment regimen for any particular subject will depend upon a variety of factors, including the activity of the specific compound employed, the age, body weight, general health status, sex, diet, time of administration, rate of excretion, drug combination, the severity and course of the infection, the subject's disposition to the infection and the judgment of the treating physician.

The compounds of this invention are also useful as commercial reagents which effectively bind to aspartyl proteases, particularly beta sercetase. As commercial reagents, the compounds of this invention, and their derivatives, may be used to block proteolysis of a target peptide, such as an aspartyl protease, or may be derivatized to bind to a stable resin as a tethered substrate for affinity chromatography applications. These and other uses which characterize commercial aspartyl protease inhibitors will be evident to those of ordinary skill in the art.

The compounds listed in Tables 1 and 2 below are prepared by following Schemes 1, 2, 3, 4, 5, 6 or 7 above or using reaction conditions known to those skilled in the art. The activities of the compounds are also listed in the same table demonstrating their potential usefulness. In Table 1 are shown compounds of formula Ia, as defined above, wherein W is -(CH₂)ₙ- and wherein n, Cₓ, R₁, R₂, R₃, and R₄ are set forth for each compound mentioned therein. In Table 2 are shown compounds of formula Ia, as defined above, wherein W is -CH₂-XX-CH₂CH₂- and wherein Cₓ, R₁, R₂, R₃, and R₄ are set forth for each compound mentioned therein.

**TABLE 1**

| compound No. Cx | R₁ | R₂ | R₃ | R₄ | n | Ki (nM) | D, L, DL *R, S, RS* |
|---|---|---|---|---|---|---|---|
| 1 COOH | i-C₄H₉ | 4-ClC₆H₄SO₂ | H | Fmoc | 4 | 20.5 | DL |
| 2 COOH | i-C₄H₉ | 4-CH₃C₆H₄SO₂ | H | Fmoc | 4 | 5.0 | DL |
| 3 COOH | i-C₄H₉ | 4-FC₆H₄SO₂ | H | Fmoc | 4 | 17.4 | DL |
| 4 COOH | i-C₄H₉ | 4-BrC₆H₄SO₂ | H | Fmoc | 4 | 11.4 | L |
| 5 COOH | i-C₄H₉ | 4-CH₃C₆H₄SO₂ | H | Fmoc | 4 | 334 | D |
| 6 COOH | i-C₄H₉ | 4-CH₃C₆H₄SO₂ | H | Fmoc | 3 | 180 | DL |
| 7 COOH | i-C₄H₉ | 4-CH₃OC₆H₄SO₂ | H | Fmoc | 4 | 10.6 | L |
| 8 COOH | i-C₄H₉ | 1-naphthyl-SO₂ | H | Fmoc | 4 | 54.9 | L |
| 9 COOH | i-C₄H₉ | C₆H₅SO₂ | H | Fmoc | 4 | 18.7 | L |
| 10 COOH | i-C₄H₉ | 4-t-BuC₆H₄SO₂ | H | Fmoc | 4 | 257 | L |
| 11 COOH | i-C₄H₉ | 4-BrC₆H₄SO₂ | H | Fmoc | 4 | 15.4 | DL |
| 12 COOH | H | 4-CH₃C₆H₄SO₂ | H | Fmoc | 4 | 8,000 | L |
| 13 COOH | H | Fmoc | H | t-Boc | 3 | 25,000 | L |
| 14 COOH | H | Fmoc | H | 4-BrC₆H₄SO₂ | 2 | 49,000 | L |
| 15 COOCH₃ | H | Fmoc | H | 4-BrC₆H₄SO₂ | 4 | 47,000 | L |
| 16 COOH | H | Fmoc | H | 4-BrC₆H₄SO₂ | 4 | 13,000 | L |
| 17 COOH | H | 4-BrC₆H₄SO₂ | H | 4-BrC₆H₄SO₂ | 4 | 7,700 | L |
| 18 COOH | H | Fmoc | H | 4-BrC₆H₄SO₂ | 1 | 75,000 | S |
| 19 COOH | H | Fmoc | H | 4-BrC₆H₄SO₂ | 4 | 12,900 | D |
| 20 CONH₂ | H | Fmoc | H | 4-BrC₆H₄SO₂ | 4 | 23,000 | RS |
| 21 COOH | H | Fmoc | i-C₄H₉ | 4-BrC₆H₄SO₂ | 4 | 2,000 | L |
| 22 CH2OH | H | Fmoc | H | 4-BrC₆H₄SO₂ | 4 | >6,000 | *S* |
| 23 COOH | H | 3-CO-4-OH-7-CF₃-quinoline | H | 4-BrC₆H₄SO₂ | 4 | >50,000 | L |
| 24 COOH | H | Fmoc | H | 2-BrC₆H₄SO₂ | 4 | 36,000 | L |
| 25 COOH | H | Fmoc | H | 2,4,6-(I-C₃H₇)₃C₆H₂SO₂ | 4 | >3,100 | L |
| 26 CO-NH-Fmoclysyl | H | Fmoc | H | 2,4,6-(CH₃)₃C₆H₂SO₂ | 4 | 63,000 | L |
| 27 COOH | H | Fmoc | H | 2,4,6-(CH₃)₃C₆H₂SO₂ | 4 | >50,000 | L |
| 28 COOH | H | Fmoc | H | 8-quinoline-SO₂ | 4 | 15,000 | L |
| 29 COOH | H | CO-CH₂-3-indole | H | 4-BrC₆H₄SO₂ | 4 | 10,500 | L |
| 30 COOH | H | CO-CH₂-9-fluorene | H | 4-BrC₆H₄SO₂ | 4 | 71,000 | L |
| 31 COOH | H | Fmoc | H | 1-naphthyl-SO₂ | 4 | 11,500 | L |
| 32 COOH | H | Fmoc | H | 2-naphthyl-SO₂ | 4 | 10,000 | L |
| 33 COOH | H | Fmoc | H | C₆H₅CH₂SO₂ | 4 | 20,000 | L |
| 34 COOH | H | Fmoc | H | 3-CF₃C₆H₄SO₂ | 4 | 22,000 | L |
| 35 COOH | H | Fmoc | H | camphor-10-CH₂SO₂ | 4 | 33,000 | L |
| 36 COOH | H | CO-CH(C₆H₅)₂ | H | 4-BrC₆H₄SO₂ | 4 | 24,000 | L |
| 37 COOH | H | CO-COH(C₆H₅)₂ | H | 4-BrC₆H₄SO₂ | 4 | 54,000 | L |
| 38 COOH | H | CO-9-fluorene | H | 4-BrC₆H₄SO₂ | 4 | 4,700 | L |
| 39 COOH | l-C₄H₉ | 4-ClC₆H₄SO₂ | H | COOCH₂C₆H₅ | 4 | >100 | L |
| 40 COOH | C₆H₅CH₂ | 4-CH₃C₆H₂SO₂ | H | Fmoc | 4 | 19.6 | L |
| 41 COOH | H | Fmoc | l-C₄H₉ | 4-BrC₆H₄SO₂ | 3 | >50,000 | L |
| 42 COOH | i-C₄H₉ | 2,4,6-(CH₃)₃C₆H₂SO₂ | H | Fmoc | 4 | >100 | L |
| 43 COOH | CH₂-c-C₃H₅ | 4-CH₃C₆H₄SO₂ | H | Fmoc | 4 | 3.9 | L |
| 44 COOH | i-C₄H₉ | 4-NH₂C₆H₄SO₂ | H | Fmoc | 4 | 2.1 | L |
| 45 COOH | i-C₄H₉ | 4-BrC₆H₄SO₂ | H | COOCH₂C₆H₆ | 4 | >100 | DL |
| 46 COOH | i-C₄H₉ | COC₆H₅ | H | Fmoc | 4 | >100 | L |
| 47 COOH | i-C₄H₉ | 4-CH₃C₆H₄SO₂ | H | COOCH₂C₆H₅ | 4 | >100 | L |
| 48 COOH | i-C₄H₉ | 4-lC₆H₄SO₂ | H | COOCH₂C₆H₅ | 4 | >100 | DL |
| 49 COOH | H | Fmoc | H | C₆H₅SO₂ | 4 | 8,900 | L |
| 50 COOH | H | Fmoc | H | 4-NO₂C₆H₄SO₂ | 4 | 6,000 | L |
| 51 COOH | H | CO-9-fluorene | H | 4-BrC₆H₄SO₂ | 3 | 9,300 | L |
| 52 COOH | H | Fmoc | H | 4-NH₂C₆H₄SO₂ | 4 | 6,800 | L |
| 53 COOH | H | Fmoc | H | 4-ClC₆H₄SO₂ | 4 | 4,800 | L |
| 54 COOH | H | Fmoc | H | 2,5-Cl₂C₆H₃SO₂ | 4 | >6,250 | L |
| 55 COOH | H | Fmoc | H | 4-FC₆H₄SO₂ | 4 | 6,500 | L |
| 56 COOH | t-Boc | 4-BrC₆H₄CH₂ | 4-BrC₆H₄CH₂ | 4-BrC₆H₄SO₂ | 4 | >25,000 | L |
| 57 COOH | H | 4-BrC₆H₄SO₂ | H | 4-BrC₆H₄SO₂ | 3 | 42,400 | L |
| 58 COOH | H | 4-BrC₆H₄SO₂ | 4-FC₆H₄CH₂ | COOCH₂C₆H₅ | 4 | >6,500 | L |
| 59 COOH | H | 4-BrC₆H₄SO₂ | 4-FC₆H₄CH₂ | 4-BrC₆H₄SO₂ | 3 | >34,900 | L |
| 60 COOH | H | 4-BrC₆H₄SO₂ | 4-FC₆H₄CH₂ | 4-BrC₆H₄SO₂ | 4 | >50,000 | L |
| 61 COOH | H | Fmoc | H | 4-CH₃OC₆H₄SO₂ | 4 | 17,400 | L |
| 62 COOH | H | Fmoc | H | 4-NO₂C₆H₄SO₂ | 3 | 26,200 | L |
| 63 COOH | H | Fmoc | H | 4-CH₃C₆H₄SO₂ | 4 | | L |
| 64 COOH | H | Fmoc | H | 4-lC₆H₄SO₂ | 4 | 19,200 | L |
| 65 COOH | H | C₈H₅CO | H | 4-BrC₆H₄SO₂ | 4 | >50,000 | L |
| 66 COOH | H | 4-BrC₆H₄SO₂ | H | Fmoc | 4 | 1,900 | L |
| 67 COOH | l-C₄H₉ | 4-CH₃C₆H₄SO₂ | H | Fmoc | 4 | 4.3 | L |
| 68 COONa | l-C₄H₉ | 4-CH₃C₆H₄SO₂ | H | Fmoc | 4 | 8.1 | L |
| 69 COOH | H | Fmoc | H | 4-NH₂C₆H₄SO₂ | 3 | 23,800 | L |
| 70 COOH | H | Fmoc | H | 2-NO₂C₆H₄SO₂ | 4 | >12,500 | L |
| 71 COOH | H | Fmoc | H | Fmoc | 4 | 44,800 | L |
| 72 COOH | H | 4-ClC₆H₄SO₂ | H | Fmoc | 4 | 2,385 | L |
| 73 COOH | H | Fmoc | H | Fmoc | 3 | 1,300 | L |
| 74 COOH | H | 4-ClC₆H₄SO₂ | H | Fmoc | 3 | 14,200 | L |
| 75 COOCH₂C₆H₅ | i-C₄H₉ | 4-CH₃C₆H₄SO₂ | H | COOCH₂C₆H₅ | 4 | >1,250 | L |
| 76 COOCH₂C₆H₅ | i-C₄H₉ | C₆H₅CH=CHSO₂ | H | COOCH₂C₆H₅ | 4 | >1,250 | L |
| 77 COOCH₂C₆H₅ | i-C₄H₉ | 4-AcNHC₆H₄SO₂ | H | COOCH₂C₆H₅ | 4. | >1,250 | L |
| 78 COOCH₂C₆H₅ | i-C₄H₉ | 4-NO₂C₆H₄SO₂ | H | COOCH₂C₆H₅ | 4 | >1,250 | L |
| 79 COOCH₂C₆H₅ | i-C₄H₉ | 2-NO₂C₈H₄SO₂ | H | COOCH₂C₆H₅ | 4 | >1,250 | L |
| 80 COOCH₂C₆H₅ | Et₂CHCH₂ | 4-MeC₆H₄SO₂ | H | COOCH₂C₆H₅ | 4 | >1,250 | L |
| 81 COOCH₂C₆H₅ | MeEtCHCH₂ | 4-MeC₆H₄SO₂ | H | COOCH₂C₆H₅ | 4 | >1,250 | L |
| 82 COOH | H | Fmoc | H | Fmoc | CH₂SSCH₂ | >1,250 | L |
| 83 COOH | i-C₄H₉ | 4-CH₃C₆H₄SO₂ | H | CO-9-fluorene | 4 | 105 | L |
| 84 COOH | i-C₄H₉ | 4-CH₃C₆H₄SO₂ | H | CO-CH₂-9-fluorene | 4 | 89.7 | L |
| 85 COOH | i-C₄H₉ | 4-CH₃C₆H₄SO₂ | H | CO-9-xanthene | 4 | 14.5 | L |
| 86 COOH | i-C₄H₉ | 4-CH₃C₆H₄SO₂ | H | CO-CH(C₆H₅)₂ | 4 | 171 | L |
| 87 COOH | i-C₄H₉ | 4-CH₃C₆H₄SO₂ | H | CO-3-indole | 4 | >1,250 | L |
| 88 COOH | i-C₄H₉ | 4-CH₃C₆H₄SO₂ | H | CO-2-indole | 4 | >625 | L |
| 89 COOH | i-C₄H₉ | 4-CH₃C₆H₄SO₂ | H | COCH₂CH₂-3-indole | 4 | 6.9 | L |
| 90 COOH | i-C₄H₉ | 4-CH₃C₆H₄SO₂ | H | COCH=CHC₆H₅ | 4 | 747 | L |
| 91 COOH | i-C₄H₉ | 4-CH₃C₆H₄SO₂ | H | COCH₂CH₂C₆H₅ | 4 | 8.0 | L |
| 92 COOH | i-C₄H₉ | 4-CH₃C₆H₄SO₂ | H | COO-Cholesteryl | 4 | >1,250 | L |
| 93 COOH | i-C₄H₉ | 4-CH₃C₆H₄SO₂ | H | CO-2-quinoline | 4 | 152 | L |
| 94 COOH | i-C₄H₉ | 4-NO₂C₆H₄SO₂ | H | COCH₂CH₂C₆H₅ | 4 | 33.9 | L |
| 95 COOH | i-C₄H₉ | 4-NO₂C₆H₄SO₂ | H | COCH₂CH₂-3-indole | 4 | 33.4 | L |
| 96 COOH | i-C₄H₉ | 4-NO₂C₆H₄SO₂ | H | CO-9-xanthene | 4 | 43.9 | L |
| 97 COOH | i-C₄H₉ | 4-C₆H₅CH₂CH₂CONHC₆H₄SO₂ | H | COCH₂CH₂C₆H₅ | 4 | 33.0 | L |
| 98 COOH | i-C₄H₉ | 4-NH₂C₆H₄SO₂ | H | COCH₂CH₂-3-indole | 4 | 10.1 | L |
| 99 COOH | i-C₄H₉ | 4-NH₂C₆H₄SO₂ | H | CO-9-xanthene | 4 | 12.1 | L |
| 100 COOH | i-C₄H₉ | 4-NH₂C₆H₄SO₂ | H | COCH₂CH₂C₆H₅ | 4 | 18.1 | L |
| 101 COOH | i-C₄H₉ | 4-CH₃C₆H₄SO₂ | H | C₆H₅CH=CHSO₂ | 4 | >3,000 | L |
| 102 COOH | H | 1-naphthyl-SO₂ | H | Fmoc | 4 | 2,320 | L |
| 103 COOH | H | 4-NO₂C₆H₄SO₂ | H | Fmoc | 4 | 3,300 | L |
| 104 COOH | H | 4-CH₃OC₆H₄SO₂ | H | Fmoc | 4 | 3,160 | L |
| 105 COOH | H | 2-NH₂C₆H₄SO₂ | H | Fmoc | 4 | 16,000 | L |
| 106 COOH | H | 4-NH₂C₆H₄SO₂ | H | Fmoc | 4 | 4,490 | L |
| 107 COOH | H | 2-NO₂C₆H₄SO₂ | H | Fmoc | 4 | 3,970 | L |
| 108 COOH | i-C₄H₉ | 4-NO₂C₆H₄SO₂ | H | 3-NO₂C₆H₄CH=CHCO | 4 | >300 | L |
| 109 COOH | i-C₄H₉ | 4-NO₂C₆H₄SO₂ | H | 2-NO₂C₆H₄CH=CHCO | 4 | >300 | L |
| 110 COOH | i-C₄H₉ | 4-NO₂C₆H₄SO₂ | H | 2,3-(CH₃O)₂C₆H₃CH=CHCO | 4 | >300 | L |
| 111 COOH | i-C₄H₉ | 4-NO₂C₆H₄SO₂ | H | 4-NO₂C₆H₄CH=CHCO | 4 | 34.4 | L |
| 112 COOH | i-C₄H₉ | 4-NO₂C₆H₄SO₂ | H | C₆H₅CH₂CH=CHCO | 4 | 21.7 | L |
| 113 COOH | i-C₄H₉ | 4-NO₂C₆H₄SO₂ | H | 4-CH₃OC₆H₄CH=CHCO | 4 | >300 | L |
| 114 COOH | i-C₄H₉ | 4-NO₂C₆H₄SO₂ | H | 4-CH₃C₆H₄CH=CHCO | 4 | >300 | L |
| 115 COOH | i-C₄H₉ | 4-NO₂C₆H₄SO₂ | H | C₆H₅CH₂SO₂ | 4 | >300 | L |
| 116 COOH | i-C₄H₉ | 4-NO₂C₆H₄SO₂ | H | 4-NO₂C₆H₄SO₂ | 4 | >300 | L |
| 117 COOH | i-C₄H₉ | 4-NH₂C₆H₄SO₂ | H | 4-NH₂C₆H₄SO₂ | 4 | | L |
| 118 COOH | i-C₄H₉ | 4-NH₂C₆H₄SO₂ | H | 3-NH₂C₆H₄CH₂CH₂CO | 4 | 20.5 | L |
| 119 COOH | i-C₄H₉ | 4-NH₂C₆H₄SO₂ | H | 2,3-(CH₃O)₂C₆H₃CH₂CH₂CO | 4 | 6.2 | L |
| 120 COOH | i-C₄H₉ | 4-NH₂C₆H₄SO₂ | H | 4-CH₃OC₆H₄CH₂CH₂CO | 4 | 12.4 | L |
| 121 COOH | i-C₄H₉ | 4-NH₂C₆H₄SO₂ | H | C₆H₅CH₂CH₂CH₂CO | 4 | >300 | L |
| 122 COOH | i-C₄H₉ | 4-NH₂C₆H₄SO₂ | H | 2-NH₂C₆H₄CH₂CH₂CO | 4 | >300 | L |
| 123 COOCH₃ | i-C₄H₉ | 4-CH₃C₆H₄SO₂ | H | Fmoc | 4 | >300 | L |
| 124 CH₂OH | i-C₄H₉ | 4-CH₃C₆H₄SO₂ | H | Fmoc | 4 | 208 | R |
| 125 CONH₂ | i-C₄H₉ | 4-CH₃C₆H₄SO₂ | H | Fmoc | 4 | 107 | RS |
| 126 COOH | i-C₄H₉ | 4-CH₃C₆H₄SO₂ | H | 4-HOC₆H₄CH₂CH₂CO | 4 | >300 | L |
| 127 COOH | H | t-Boc | H | COOCH₂C₆H₄-2-Cl | 4 | >200 | L |
| 128 COOH | H | t-Boc | H | COOCH₂C₆H₄-2-Cl | 4 | >200 | D |
| 129 COOH | H | Fmoc | H | t-Boc | 4 | 28,000 | L |
| 130 COOH | H | Fmoc | H | COOCH₂C₆H₅ | 4 | 20,000 | L |
| 131 COOH | H | Fmoc | H | Ac | 4 | 56,000 | L |
| 132 COOH | H | Fmoc | H | t-Boc | 4 | 8,400 | D |
| 133 COOH | H | Fmoc | H | COOCH₂C₆H₅ | 4 | 16,000 | D |
| 134 COOH | H | COOCH₂C₆H₅ | H | t-Boc | 4 | >200,000 | L |
| 135 COOH | H | COOCH₂C₆H₅ | H | COOCH₂C₆H₅ | 3 | >200,000 | L |
| 136 COOH | H | COOCH₂C₆H₅ | H | t-Boc | 4 | >200,000 | D |
| 137 COOH | H | t-Boc | H | COOCH₂C₆H₅ | 3 | >200,000 | L |
| 138 COOH | H | t-Boc | H | Fmoc | 4 | >200 | L |
| 139 COOH | H | Fmoc | H | 3-NO₂C₆H₄SO₂ | 4 | | L |
| 140 COOH | H | Fmoc | H | 4-t-BuC₆H₄SO₂ | 4 | | L |
| 141 CONHOH | i-C₄H₉ | 4-CH₃C₆H₄SO₂ | H | Fmoc | 4 | 245 | RS |
| 142 COOH | H | Fmoc | H | 4-CH₃C₆H₄SO₂ | 4 | 9,400 | D |
| 143 COOH | H | t-Boc | H | 4-BrC₆H₄SO₂ | 4 | 660,000 | L |
| 144 COOH | H | Fmoc | H | H | 3 | | L |
| 145 COOH | H | Fmoc | H | H | 4 | | L |
| 146 COOH | H. | Fmoc | H | 3-NO₂C₆H₄SO₂ | 3 | | L |
| 147 COOH | H | Fmoc | H | 4-BrC₆H₄SO₂ | 3 | | L |
| 148 COOH | H | Fmoc | H | 4-CH₃OC₆H₄SO₂ | 3 | | L |
| 149 COOH | H | Fmoc | H | 4-CH₃C₆H₄SO₂ | 3 | 10,100 | L |
| 150 COOH | H | Fmoc | H | 4-FC₆H₄SO₂ | 3 | | L |
| 151 COOH | H | 4-CH₃C₆H₄SO₂ | H | 4-CH₃C₆H₄SO₂ | 4 | | L |
| 152 COOH | i-C₄H₉ | 4-NO₂C₆H₄SO₂ | H | Fmoc | 4 | | L |
| 153 COOH | i-C₄H₉ | 4-CH₃C₆H₄SO₂ | H | C₆H₅CH₂CH₂CS | 4 | 151 | DL |
| 154 COOH | i-C₄H₉ | 4-NO₂C₆H₄SO₂ | H | C₆H₅SCH₂CO | 4 | 10.2 | L |
| 155 COOH | i-C₄H₉ | 4-NH₂C₆H₄SO₂ | H | 3,4-(OCH₂O)C₆H₃CH₂CH₂CO | 4 | 15.7 | L |
| 156 COOH | i-C₄H₉ | 4-NH₂C₆H₄SO₂ | H | 3-CH₃OC₆H₄CH₂CH₂CO | 4 | 16.7 | L |
| 157 COOH | i-C₄H₉ | 4-NH₂C₆H₄SO₂ | H | 2-CH₃OC₆H₄CH₂CH₂CO | 4 | 18.9 | L |
| 158 COOH | i-C₄H₉ | 4-NH₂C₆H₄SO₂ | H | C₆H₅CH₂CH₂CO | 4 | >300 | L |
| 159 COOH | i-C₄H₉ | 4-CH₃C₆H₄SO₂ | i-C₄H₉ | C₆H₅CH₂CH₂CO | 4 | >300 | DL |
| 160 COOH | i-C₄H₉ | 4-NO₂C₆H₄SO₂ | H | C₆H₅OCH₂CO | 4 | 24.5 | L |
| 161 COOH | i-C₄H₉ | 4-NO₂C₆H₄SO₂ | H | 2-CH₃OC₆H₄CH=CHCO (trans) | 4 | >300 | L |
| 162 COOH | i-C₄H₉ | 4-NO₂C₆H₄SO₂ | H | 3-CH₃OC₆H₄CH=CHCO | 4 | >300 | L |
| 163 COOH | i-C₄H₉ | 4-NO₂C₆H₄SO₂ | H | 3,4-(OCH₂O)C₆H₃CH=CHCO | 4 | >300 | L |
| 164 COOH | i-C₄H₉ | 4-NO₂C₆H₄SO₂ | H | 3-C₅H₄NCH=CHCO (trans) | 4 | >300 | L |
| 165 CONHOH | i-C₄H₉ | 4-CH₃C₆H₄SO₂ | H | Fmoc | 4 | | L |
| 166 COOH | H | 4-FC₆H₄SO₂ | H | 4-FC₆H₄SO₂ | 3 | | L |
| 167 COOH | H | Fmoc | H | 2-FC₆H₄SO₂ | 4 | | L |
| 168 COOH | H | Fmoc | H | 1-naphthyl-SO₂ | 3 | | L |
| 169 COOCH₂C₆H₅ | i-C₄H₉ | C₆H₅SO₂ | H | COOCH₂C₆H₅ | 4 | | L |
| 170 COOCH₂C₆H₅ | i-C₄H₉ | 4-CH₃OC₆H₄SO₂ | H | COOCH₂C₆H₅ | 4 | | L |
| 171 COOCH₂C₆H₅ | i-C₄H₉ | 4-BrC₆H₄SO₂ | H | COOCH₂C₆H₅ | 4 | | L |
| 172 COOCH₂C₆H₅ | i-C₄H₉ | 2-NO₂C₆H₄SO₂ | H | COOCH₂C₆H₅ | 4 | | L |
| 173 COOCH₂C₆H₅ | Et₂CHCH₂ | C₆H₅SO₂ | H | COOCH₂C₆H₅ | 4 | | L |
| 174 COOCH₂C₆H₅ | Et₂CHCH₂ | 4-CH₃OC₆H₄SO₂ | H | COOCH₂C₆H₅ | 4 | | L |
| 175 COOCH₂C₆H₅ | Et₂CHCH₂ | C₆H₅CH=CHSO₂ | H | COOCH₂C₆H₅ | 4 | | L |
| 176 COOCH₂C₆H₅ | Et₂CHCH₂ | 4-AcNHC₆H₄SO₂ | H | COOCH₂C₆H₅ | 4 | | L |
| 177 COOCH₂C₆H₅ | Et₂CHCH₂ | 4-BrC₆H₄SO₂ | H | COOCH₂C₆H₅ | 4 | | L |
| 178 COOCH₂C₆H₅ | Et₂CHCH₂ | 4-NO₂C₆H₄SO₂ | H | COOCH₂C₆H₅ | 4 | | L |
| 179 COOCH₂C₆H₅ | MeEtCHCH₂ | C₆H₅SO₂ | H | COOCH₂C₆H₅ | 4 | | L |
| 180 COOCH₂C₆H₅ | MeEtCHCHz | 4-CH₃OC₆H₄SO₂ | H | COOCH₂C₆H₅ | 4 | | L |
| 181 COOCH₂C₆H₅ | MeEtCHCH₂ | C₆H₅CH=CHSO₂ | H | COOCH₂C₆H₅ | 4 | | L |
| 182 COOCH₂C₆H₅ | MeEtCHCH₂ | 4-AcNHC₆H₄SO₂ | H | COOCH₂C₆H₅ | 4 | | L |
| 183 COOCH₂C₆H₅ | MeEtCHCH₂ | 4-BrC₆H₄SO₂ | H | COOCH₂C₆H₅ | 4 | | L |
| 184 COOCH₂C₆H₅ | MeEtCHCH₂ | 4-NO₂C₆H₄SO₂ | H | COOCH₂C₆H₅ | 4 | | L |
| 185 COOCH₂C₆H₅ | MeEtCHCH₂ | 2-NO₂C₆H₄SO₂ | H | COOCH₂C₆H₅ | 4 | | L |
| 186 COOH | i-C₄H₉ | 4-NO₂C₆H₄SO₂ | H | 2-CH₃OC₆H₄CH=CHCO (cis) | 4 | 39.8 | L |
| 187 COOH | i-C₄F₉ | 4-NO₂C₆H₄SO₂ | H | C₆H₅CH₂CH=CHCO (trans) | 4 | | L |
| 188 COOH | i-C₄H₉ | 4-CH₃C₆H₄SO₂ | H | 4-HOC₆H₄CH=CHCO (trans) | 4 | | L |
| 189 COOH | i-C₄H₉ | 4-NO₂C₆H₄SO₂ | H | 3,5-(CH₃O)₂C₆H₃CH=CHCO | 4 | 108 | L |
| 190 COOH | i-C₄H₉ | 4-NO₂C₆H₄SO₂ | H | 2,5-(CH₃O)₂C₆H₃CH=CHCO | 4 | >75 | L |
| 191 COOH | i-C₄H₉ | 4-NO₂C₆H₄SO₂ | H | 2,4-(CH₃O)₂C₆H₃CH=CHCO | 4 | >75 | L |
| 192 COOH | i-C₄H₉ | 4-NH₂C₆H₄SO₂ | H | C₆H₅OCH₂CO | 4 | 8.2 | L |
| 193 COOH | i-C₄H₉ | 4-NO₂C₆H₄SO₂ | H | 3,4-(CH₃O)₂C₆H₃CH=CHCO | 4 | >75 | L |
| 194 COOH | i-C₄H₉ | 4-NH₂C₆H₄SO₂ | H | 4-NH₂C₆H₄CH₂CH₂CO | 4 | >300 | L |
| 195 COOH | i-C₄H₉ | 4-NH₂C₆H₄SO₂ | H | 3-C₅H₃NCH₂CH₂CO | 4 | >300 | L |
| 196 COOH | i-C₄H₉ | 4-NH₂C₆H₄SO₂ | H | 2,4-(CH₃O)₂C₆H₃CH₂CH₂CO | 4 | 17.1 | L |
| 197 COOH | i-C₄H₉ | 4-NH₂C₆H₄SO₂ | H | 2,5-(CH₃O)₂C₆H₃CH₂CH₂CO | 4 | 20.7 | L |
| 198 COOH | i-C₄H₉ | 4-NH₂C₆H₄SO₂ | H | 3,5-(CH₃O)₂C₆H₃CH₂CH₂CO | 4 | 32.2 | L |
| 199 COOH | i-C₄H₉ | 4-NO₂C₆H₄SO₂ | H | 4-CH₃C₆H₄SO₂ | 4 | | L |
| 200 COOH | i-C₄H₉ | 4-NH₂C₆H₄SO₂ | H | 3,4-(CH₃O)₂C₆H₃CH₂CH₂CO | 4 | 132 | L |
| 201 COOH | i-C₄H₉ | 4-CH₃C₆H₄SO₂ | H | 2,3-(CH₃O)₂C₆H₃CH₂CH₂CO | 4 | 15.0 | L |
| 202 COOH | i-C₄H₉ | 4-CH₃C₆H₄SO₂ | H | C₆H₅SCH₂CO | 4 | 18.0 | L |
| 203 COOH | i-C₄H₉ | 4-CH₃C₆H₄SO₂ | H | C₆H₅OCH₂CO | 4 | 17.5 | L |
| 204 COOH | i-C₄H₉ | 4-CH₃C₆H₄SO₂ | H | C₆H₆CH₂CH₂C=N-CN | 4 | 34.0 | DL |

**TABLE 2**

| Compound No. Cx | R₁ | R₂ | R₃ | R₄ | XX | Ki (nM) | D, L, DL *R, S, RS* |
|---|---|---|---|---|---|---|---|
| 205 COOH | i-C₄H₉ | 4-CH₃C₆H₄SO₂ | H | COCH₂CH₂C₅H₅ | O | | DL |
| 206 COOH | i-C₄H₉ | 4-CH₃C₆H₄SO₂ | H | COCH₂CH₂C₆H₅ | NH | >300 | DL |
| 207 COOH | i-C₄H₉ | 4-CH₃C₆H₄SO₂ | H | COCH₂CH₂C₅H₅ | S | | DL |

In order that this invention be more fully understood, the following examples are set forth relating to the preparation of example compounds in accordance with the present invention. These examples are for the purpose of illustration only and are not to be construed as limiting the scope of the invention in any way. When an example relates to the preparation of a compound identified in Table 1 or 2 above, the compound number used in Table 1 or 2 will appear after the name of the compound prepared in accordance to the example; additionally with respect to the compound numbers used in the tables of examples 80, and 81 these numbers identify the compounds as the compounds corresponding to that respective number which appears in Table 1.

### Materials and Methods

Analytical thin layer chromatography (TLC) is carried out with 0.25 mm silica gel E. Merck 60 F₂₅₄ plates and eluted with the indicated solvent systems. Preparative chromatography is performed either by flash chromatography, using Silica Gel 60 (EM Science) with the indicated solvent systems and a positive nitrogen pressure to allow proper elution, or by preparative thin layer chromatography, again employing E. Merck 60 F₂₅₄ plates of 0.5, 1.0, or 2.0 mm thickness.

Detection of the compounds is carried out by exposing eluted plates, analytical or preparative, to UV light and treating analytical plates either with a 2% p-anisaldehyde solution in ethanol containing 1% acetic acid and 3% sulfuric acid or with a 0.3% ninhydrin solution in ethanol containing 3% acetic acid, followed by heating.

Nuclear magnetic resonance (NMR) spectra are recorded on a Bruker AMX-2 500 MHz equipped with a reversed or QNP probe. Samples are dissolved in deuterochloroform (CDCl₃), deuteroacetone (acetone-d₃) or deuterated dimethylsulfoxide (DMSO-d₃) for data acquisition using tetramethylsilane (TMS) as internal standard. Chemical shifts are expressed in parts per million (ppm), the coupling constants J are expressed in hertz (Hz) and multiplicities (denoted as s for singlet, d for doublet, dd for doublet of doublets, t for triplet, q for quartet, m for multiplet, and br s for broad singlet).

The following compounds are prepared either from a derivative of a L-amino acid or, when indicated, from a derivative of a D-amino acid using the procedures summarized in Schemes 1, 2, 3, 4, 4a, 5, 5a, 6 or 7.

### Example 1. Preparation of Nα-(9-fluorenylmethoxycarbonyl)-L-lysine (compound no. 145)

*N*α-(9-fluorenylmethoxycarbonyl)-*N*∈-benzyloxycarbonyl-L-lysine (502 mg, 1.00 mmol) is dissolved in TFA/CH₂Cl₂ (3 mL/3 mL) and stirred at room temperature for 1 h. The volatiles are removed *in vacuo* to afford the title compound quantitatively as a white solid.

¹H NMR (DMSO-d₆) : 1.30 - 1.43 (m, 2H), 1.50 - 1.78 (m, 6H), 2.78 (d, J = 5.5, 2H), 3.94 (m, 1H), 4.22 (m, 1H), 4.25 - 4.33 (m, 2H), 7.31 (dd, J = 7.4, 7.4, 2H), 7.40 (dd, J = 7.5, 7.4, 2H), 7.61 (d, J = 7.7, 1H), 7.71 (m, 2H), 7.82 (br s, 3H), 7.88 (d, J = 7.5, 2H).

The D-isomer is obtained by using *N*α-(9-fluorenyl methoxycarbonyl)-*N*∈-benzyloxycarbonyl-D-lysine.

### Example 2. Preparation of N∈-(4-bromobenzenesulfonyl)-Nα-(9-fluorenylmethoxycarbonyl)-L-lysine (compound no. 16)

The product of example 1 (368 mg, 1.00 mmol) is dissolved in a 1M aqueous K₂CO₃ solution (5 mL) and THF (3 mL). The reaction mixture is cooled to 0 °C, before a solution of 4 bromobenzenesulfonyl chloride (280 mg; 1.10 mmol) in dioxane (6 mL) is added. The mixture is stirred at 0 °C for 1 h and then at room temperature for 2 h. The pH of the reaction mixture is acidified (pH ~ 3) with 1N HCl. The mixture is then extracted with EtOAc. The organic layer is washed with brine and dried over MgSO₄. After filtration, the filtrate is evaporated to dryness *in vacuo*, and the crude material is purified by flash chromatography eluting with 70% EtOAc in hexane containing 0.4% AcOH, to yield 417 mg (71%) of the title compound.

¹H NMR (DMSO-d₆): 1.20 - 1.80 (m, 6H), 2.70 (dd, J = 12.8, 6.5, 2H), 3.88 - 3.92 (m, 1H), 4.20 (t, J = 7.0, 1H), 4.30 (d, J = 7.0,2H), 7.20 - 7.40 (m, 5H), 7.55 - 7.60 (m, 1H), 7.67 - 7.92 (m, 8H), 12.50 (br s, 1H).

Utilising the D-isomer and following the indications of example 2, the D isomer is obtained.

### Example 3. Preparation of N∈-(4-nitrobenzenesulfonyl)-Nα-(9-fluorenylmethoxycarbonyl)-L-lysine (compound no. 50)

*N*α-(9-fluorenylmethoxycarbonyl)-L-lysine is reacted with 4-nitrobenzenesulfonyl chloride under the conditions used in example 2 giving 89% of the title compound.

¹H NMR (DMSO-d₆): 1.22 - 1.65 (m, 6H), 2.79 (dd, J = 12.8,6.2,2H), 3.85 (m, 1H), 4.20 (t, J = 7.0, 1H), 4.28 (d, J = 7.0, 2H), 7.28 - 7.42 (m, 4H), 7.56 (d, J = 8.1, 2H), 7.70 (d, J = 6.3, 2H), 7.88 (d, J = 7.4, 2H), 7.98 (t, J = 5.4, 1H), 8.03 (d, J = 8.5, 2H), 8.40 (d, J = 8.4, 2H), 12.40 (br s, 1H).

### Example 4. Preparation of N∈-(4-aminobenzenesulfonyl)-Nα- (9-fluorenylmethoxycarbonyl)-L-lysine (compound no. 52)

The product obtained from example 3 (553 mg, 1.00 mmol) is dissolved in EtOAc (10 mL) and then hydrogenated using 10% Pd on charcoal as catalyst at atmospheric pressure for 2 h. The catalyst is filtered off and the filtrate is evaporated in vacuo to yield the title compound in 95% yield.

¹H NMR (DMSO-d₆) : 1.20 - 1.72 (m, 6H), 2.60 (dd, J = 12.8, 6.2, 2H), 3.80 (m, 1H), 4.20 (m, 2H), 4.31 (m, 1H), 5.90 (br s, 2H), 6.61 (d, J = 8.2, 2H), 7.00 - 7.10 (m, 2H), 7.28 - 7.48 (m, 6H), 7.68 - 7.90 (m, 4H).

### Example 5. Preparation of N∈-(4-iodobenzenesulfonyl)-Nα-(9-fluorenylmethoxycarbonyl)-L-lysine (compound no. 64)

*N*α-(9-fluorenylmethoxycarbonyl)-L-lysine is reacted with 4-iodobenzenesulfonyl chloride under the conditions used in example 2 giving 68% of the title compound.

¹H NMR (DMSO-d₆): 1.23 - 1.45 (m, 4H), 1.50 - 1.68 (m, 2H), 2.70 (dd, J = 13.0, 6.9, 2H), 3.38 (m, 1H), 4.20 (t, J = 7.0, 1H), 4.30 (d, J = 7.0, 2H), 7.28 - 7.42 (m, 4H), 7.52 - 7.60 (m, 1H), 7.67 (t, J = 5.5, 1H), 7.70 (d, J = 7.4,2H), 7.88 (d, J = 7.4,2H), 7.97 (d, J = 8.6,2H), 11.30 (br s, 1H).

### Example 6.Preparation of N∈-(4-fluorobenzenesulfonyl)-Nα-(9-fluorenylmethoxycarbonyl)-L-lysine (compound no. 55)

*N*α-(9-fluorenylmethoxycarbonyl)-L-lysine is reacted with 4-fluorobenzenesulfonyl chloride under the conditions used in example 2 giving 51% of the title compound.

¹H NMR (DMSO-d₆): 1.22 - 1.70 (m, 6H), 2.75 (dd, J = 12.8, 6.2, 2H), 3.85 - 3.92 (m, 1H), 4.20 (t, J = 7.0, 1H), 4.30 (d, J = 7.0, 2H), 7.25 - 7.45 (m, 6H), 7.57 (d, J = 8.3, 1H) , 7.62 (t, J = 5.2, 1H), 7.72 (d, J = 6.5, 2H), 7.82 - 7.90 (m, 4H) , 12.40 (br s, 1H).

### Example 7. Preparation of N∈-(2,5-dichlorobenzenesulfonyl)-Nα-(9-fluorenylmethoxycarbonyl)-L-lysine (compound no. 54)

*N*α-(9-fluorenylmethoxycarbonyl)-L-lysine is reacted with 2,5- dichlorobenzenesulfonyl chloride under the conditions used in example 2 giving 28% of the title compound.

¹H NMR (DMSO-d₆): 1.20 - 1.45 (m, 6H), 1.48 - 1.68 (m, 2H), 2.70 (dd, J = 12.8, 6.7, 2H), 3.83 - 3.89 (m, 1H), 4.20 (t, J = 7.0, 1H), 4.28 (d, J = 6.8, 2H), 7.30 (t, J = 7.3, 2H), 7.40 (t, J = 7.3, 2H), 7.55 (d, J = 8.1, 1H), 7.62 - 7.65 (m, 4H), 7.78 (d, J = 7.8, 2H), 7.92 (d, J = 7.9, 2H).

### Example 8. Preparation of N∈-(4-methylbenzenesulfonyl)-Nα-(9- fluorenylmethoxycarbonyl)-L-lysine (compound no. 63)

*N*α-(9-fluorenylmethoxycarbonyl)-L-lysine is reacted with 4-methylbenzenesulfonyl chloride under the conditions used in example 2 giving 71% of the title compound.

¹H NMR (DMSO-d₆): 1 .20 - 1.75 (m, 6H), 2.35 (s, 3H), 2.70 (dd, J = 12.9, 7.0, 2H), 3.82 - 3.90 (m, 1H) , 4.20 (t, J = 7.0, 1H), 4.30 (d, J = 7.0,2H), 7.20 - 7.50 (m, 7H), 7.52 - 7.90 (m, 7H), 12.30 (br s, 1H).
The D-isomer is prepared by following essentially the same conditions.

### Example 9. Preparation of N∈-(3-nitrobenzenesulfonyl)-Nα-(9-fluorenylmethoxycarbonyl)-L-lysine (compound no. 139)

*Nα*-(9-fluorenylmethoxycarbonyl)-L-lysine is reacted with 3-nitrobenzenesulfonyl chloride under the conditions used in example 2 giving 42% of the title compound.

¹H NMR: 1.3 - 1.7 (m, 6H) , 2.76 (m, 2H), 3.76 (m, 1H), 4.0 - 4.5 (m, 1H), 4.22 (m, 2H), 4.32 (m, 1H), 6.3-7.0 (m, 1H), 7.9 - 8.2 (m, 1H), 7.2 - 8.6 (m, 12H).

### Example 10. Preparation of N∈-(4-methoxybenzenesulfonyl)-Nα-(9-fluorenylmethoxycarbonyl)-L-lysine (compound no. 61)

*N*α-(9-fluorenylmethoxycarbonyl)-L-lysine is reacted with 4-methoxybenzenesulfonyl chloride under the conditions used in example 2 giving 61% of the title compound.

¹H NMR (DMSO-d₆): 1.10 - 1.68 (m, 6H), 2.70 (m, 2H), 3.80 (s, 3H), 3.88 (m, 1H), 4.20 (t, J = 7.0, 1H), 4.28 (t, J = 7.0, 2H), 7.08 (d, J = 8.3, 2H), 7.30 - 7.45 (m, 4H), 7.60 (d, J = 7.7, 1H), 7.70 (m, 2H), 7.90 (d, J = 7.4, 2H), 12.50 (br s, 1H).

### Example 11. Preparation of N∈-(2,4,6-triisopropylbenzencsulfonyl)-Nα-(9-fluorenylmethoxycarbonyl)-L-lysine (compound no. 25)

Nα-(9-fluorenylmethoxycarbonyl)-L-lysine is reacted with 2,4,6- triisopropylbenzenesulfonyl chloride under the conditions used in example 2 giving 34% of the title compound.

¹H NMR (DMSO-d₆): 1.17 (d, J = 6.0, 6H), 1.20 (d, J = 6.8, 12H), 1.22 - 1. 65 (m, 6H), 2.78 (dd, J = 13.0, 6.9, 2H), 2.90 (h, J = 6.5, 1H), 3.85 (m, 1H), 4.13 (h, J = 7.0, 1H), 4.27 (d, J = 7.0, 2H), 7.21 (s, 2H), 7.29 - 7.40 (m, 4H), 7.44 (t, J = 5.3, 1H), 7.53 (d, J = 7.7, 1H), 7.70 (m, 2H), 7.88 (d, J = 7.4, 2H), 12.20 (br s, 1H).

### Example 12. Preparation of Ne-(2,4,6-trimethylbcnzenesulfonyl)-Nα-(9-fluorenylmethoxycarbonyl)-L-lysine (compound no. 27)

*N*α-(9-fluorenylmethoxycarbonyl)-L-lysine is reacted with 2,4,6- trimethylbenzenesulfonyl chloride under the conditions used in example 2 giving 37% of the title compound.

¹H NMR (DMSO-d₆): 1.22 - 1.45 (m, 4H), 1.50 - 1.70 (m, 2H), 2.24 (s, 3H), 2.56 (s, 6H), 2.74 (dd, J = 13.0, 6.9, 2H), 3.90 (m, 1H), 4.23 (t, J = 7.0, 1H), 4.30 (d, J = 7.0, 2H), 7.00 (s, 2H), 7.29 - 7.45 (m, 6H), 7.71 (m, 2H), 7.88 (d, J = 7.5, 2H), 12.30 (br s, 1H).

Also isolated in small yield (25%) from the reaction mixture is *N*α-(9-fluorenylmethoxycarbonyl)-L-lysyl-*N*α-(9-fluorenylmethoxy-carbonyl)-*N*∈-(2,4,6trimethylbenzenesulfonyl)-L-lysine (compound no. 26).

¹H NMR (DMSO-d₆): 1.10 - 1.75 (m, 12H), 2.22 (s, 3H), 2.52 (s, 6H), 2.68 (m, 2H), 3.02 (m, 2H), 3.82 (m, 1H), 3.90 (m, 1H), 4.20 (m, 2H), 4.28 (m, 4H), 6.98 (s, 2H), 7.28 - 7.42 (m, 1H), 7.57 (d, J = 7.5, 2H), 7.70 (m, 4H), 7.80 (t, J = 5,0 1H), 7.89 (d, J = 7.3, 4H), 12.20 (br s, 1H) .

### Example 13. Preparation of N∈-(4-tert-butylbenzenesulfonyl)-Nα-(9- fluorenylmethoxycarbonyl)-L-lysine (compound no. 140)

*N*α-(9-fluorenylmethoxycarbonyl)-L-lysine is reacted with 4-*tert*-butylbenzenesulfonyl chloride under the conditions used in example 2 giving 72% of the title compound.

¹H NMR (DMSO-d₆) : 1.20 - 1.45 (m, 4H), 1.29 (s, 9H), 1.50 - 1.65 (m, 2H), 2.70 (dd, J = 13.0, 6.9, 2H), 3.85 (m, 1H), 4.22 (t, J = 7.0, 1H) , 4.28 (d, J = 7.5, 2H), 4.47 (t, J = 5.5, 1H) , 7.28 - 7.43 (m, 6H), 7.55 (d, J 8.2, 2H), 7.60 (d, J = 8.5, 2H), 7.70 (d, J = 7.0, 2H), 7.88 (d, J = 7.3, 2H), 57 12,30 (br s, 1H).

### Example 14. Preparation of N∈-benzenesulfonyl-Nα-(9-fluorchylmethoxycarbonyl)-L-lysine (compound no. 49)

*N*α-(9-fluorenylmethoxycarbonyl)-L-lysine is reacted with benzenesulfonyl chloride under the conditions used in example 2 giving 68% of the title compound.

¹H NMR (DMSO-d₆) : 1.15 - 1.45 (m, 4H) , 1.50 - 1.65 (m, 2H), 2.70 (m, 1H) , 3.77 (m, 1H) , 4.20 (t, J = 7.0, 1H) , 4.28 (t, J = 7.0, 2H), 7.30 - 7.80 (m, 15H), 12.70 (br s, 1H).

### Example 15. Preparation of N∈-(3-trifluoromethylbenzenesulfonyl)-Nα-(9-fluorenylmethoxycarbonyl)-L-lysine (compound no. 34)

*N*α-(9-fluorenylmethoxycarbonyl)-L-lysine is reacted with 3-trifluoromethylbenzenesulfonyl chloride under the conditions used in example 2 giving 61% of the title compound.

¹H NMR (DMSO-d₆): 1 .20 - 1.68 (m, 6H), 2.75 (dd, J = 12.8, 6.8, 2H), 3.87 (m, 1H), 4.21 (t, J = 7.0, 1H), 4.28 (d, J = 7.0, 2H), 7.30 - 7.42 (m, 4H), 7.52 (d, J = 7.8, 1H), 7.70 (d, J = 6.4, 2H), 7.8 0 - 7.90 (m, 4H), 8.02 - 8.10 (m, 3H), 12.50 (br s, 1H).

### Example 16. Preparation of N∈-(1-naphthalenesulfonyl)-Nα-(9-fluorenylmethoxycarbonyl)-L-lysine (compound no. 31)

*Nα*-(9-fluorenylmethoxycarbonyl)-L-lysine is reacted with 1-naphthalenesulfonyl chloride under the conditions used in example 2 giving 66% of the title compound.

¹H NMR (DMSO-d₆): 1.18 - 1.60 (m, 6H), 2.75 (dd, J = 13.0, 7.0, 2H), 3.80 (m, 1H), 4.21 (t, J = 7.0, 1H), 4.27 (d, J = 7.0, 2H), 7.28 - 7.40 (m, 4H), 7.51 (d, J = 7.7, 1H), 7.61 - 7.71 (m, 5H), 7.86 (d, J = 7.1, 2H), 7.91 (t, J = 5.2, 1H), 8.06 (d, J 8.2, 1H) , 8.11 (d, J = 7.3, 1H), 8.20 (d, J = 8.3, 1H), 8.66 (d, J = 8.5, 1 H), 12.30 (br s, 1H).

### Example 17. Preparation of N∈-(2-naphthalenesulfonyl)-Nα-(9-fluorenylmethoxycarbonyl)-L-lysine (compound no. 32)

*N*α-(9-fluorenylmethoxycarbonyl)-L-lysine is reacted with 2-naphthalenesulfonyl chloride under the conditions used in example 2 giving 71% of the title compound.

¹H NMR (DMSO-d₆): 1.25 - 1.60 (m, 6H), 2.74 (dd, J = 12.6, 6.5, 2H), 3.85 (m, 1H), 4.19 (t, J 6.9, 1H), 4.28 (d, J = 7.0, 2H), 7.25 - 7.40 (m, 4H) , 7.53 (d, J = 8.2, 1H), 7.64 - 7.87 (m, 7H), 8.00 - 8.20 (m, 3H), 8.42 (s, 1H), 12.50 (br s, 1H).

### Example 18. Preparation of N∈-(8-quinolinesulfonyl)-Nα-(9-fluorenylmethoxycarbonyl)-L-lysine (compound no. 28)

*N*α-(9-fluorenylmethoxycarbonyl)-L-lysine is reacted with 8-quinolinesulfonyl chloride under the conditions used in example 2 giving 81% of the title compound.

¹H NMR (DMSO-d₆): 1.20 - 1.52 (m, 6H), 2.70 (dd, J = 12.9, 6.9, 2H), 3.3 8 (m, 1H), 4.20 (t, J = 6.9, 1H), 4.30 (d, J = 7.0, 2H), 7.15 (t, J = 5.6, 1H), 7.28 - 7.40 (m, 4H), 7.50 (d, J = 7.6, 1H), 7.68 - 7.76 (m, 6H), 8.28 (dd, J = 13.0, 8.0, 2H), 8.53 (d, J = 8.3, 1H), 9.05 (d, J = 3.0, 1H), 12.30 (br s, 1H).

### Example 19. Preparation of N∈-phenylmethylsulfonyl-Nα-(9-fluorenylmethoxycarbonyl)-L-lysine (compound no. 33)

*N*α-(9-fluorenylmethoxycarbonyl)-L-lysine is reacted with phenylmethylsulfonyl chloride under the conditions used in example 2 giving 15% of the title compound.

¹H NMR (DMSO-d₆): 1.20 - 1.80 (m, 6H), 2.86 (dd, J = 12.5, 6.5, 2H), 3.90 (m, 1H) , 4.20 (t, J = 7.0, 1H), 4.26 (d, J = 7.0, 2H), 4.29 (s, 2H), 7.28 - 7.45 (m, 9H), 7.60 (d, J = 8.3, 1H), 7.72 (d, J = 7.4, 2H), 7.89 (d, J = 7.4, 2H), 12.50 (br s, 1H).

### Example 20. Preparation of N∈-(1S)-(10-camphorsulfonyl)-Nα-(9-fluorenylmethoxycarbonyl)-L-lysine (compound no. 35)

*N*α-(9-fluorenylmethoxycarbonyl)-L-lysine is reacted with (1*S*)-(+)-10-camphorsulfonyl chloride under the conditions used in example 2 giving 72% of the title compound.

¹H NMR (DMSO-d₆) : 0.80 (s, 3H), 1.00 (s, 3H), 1.30 - 1.78 (m, 7H), 1.88 - 1.92 (m, 2H), 2.05 (m, 1H), 2 .30 - 2.42 (m, 2H), 2.87 (d, J = 14.9, 2H), 2.90 - 3.03 (m, 2H), 3.31 (s, 2H), 3.90 (m, 1H), 4.20 (t, J = 7.0, 1H), 4.30 (d, J = 7.0, 2H), 7.00 (t, J = 5.3, 1H), 7.28 - 7.45 (m, 4H), 7.60 (d, J = 7.9, 1H), 7.70 (d, J = 7.3, 2H), 7.89 (d, J = 7.4, 2H), 12.50 (br s, 1H).

### Example 21. Preparation of Nα-(2-nitrobenzenesulfonyl)- N∈-(9-fluorenylmethoxycarbonyl)-L-lysine (compound no. 70)

*N*α-(9-fluorenylmethoxycarbonyl)-L-lysine is reacted with 2-nitrobenzenesulfonyl chloride under the conditions used in example 2 giving 44% of the title compound.

¹H NMR (DMSO-d₆): 1.18 - 1.40 (m, 4H), 1. 52 - 1.73 (m, 2H), 2.90 (m, 2H), 3.82 (m, 1H), 4.20 (t, J = 6.3, 1H), 4.28 (d, J = 7.0, 1H), 7.22 (t, J = 5.2, 1H), 7.31 - 7.45 (m, 4H), 7.67 (d, J = 7.3, 1H), 7.80 - 8.08 (m, 6H), 8.45 (d, J = 8.4, 1H).

### Example 22. Preparation of N∈-(4-chlorobenzenesulfonyl)-Nα- (9-fluorenylmethoxycarbonyl)-L-lysine (compound no. 53)

*Nα*-(9-fluorenylmethoxycarbonyl)-L-lysine is reacted with 4-chlorobenzenesulfonyl chloride under the conditions used in example 2 giving 37% of the title compound.

¹H NMR (DMSO-d₆): 1.20 - 1.70 (m, 6H), 2.72 (dd, J = 13.5, 6.8, 2H), 3.85 (m, 1H), 4.21 (t, J = 7.0, 1H), 4.27 (d, J = 7.1, 2H), 7.25 - 7.42 (m, 4H), 7.56 (d, J = 8.1, 1H), 7.63 - 7.67 (m, 5H), 7.78 (d, J = 7.8, 2H), 7.88 (d, J = 7.5, 2H), 12.50 (br s, 1H).

### Example 23. Preparation of N∈-(2-bromobenzenesulfonyl)-Nα- (9-fluorenylmcthoxycarbonyl)-L-lysine (compound no. 24)

*N*α-(9-fluorenylmethoxycarbonyl)-*L*-lysine is reacted with 2-bromobenzenesulfonyl chloride under the conditions used in example 2 giving 61% of the title compound.

¹H NMR (DMSO-d₆): 1.20 - 1.70 (m, 6H), 2.80 (dd, J = 12.8, 6.9, 2H), 3.80 (m, 1H), 4.20 (t, J = 7.0, 1H), 4.28 (d, J = 6.9, 2H), 7.30 - 7.57 (m, 7H), 7.66 - 7.88 (m, 6H), 7.98 (d, J = 7.5, 1H).

### Example 24. Preparation of Nα-(9-fluorenylmethoxycarbonyl)-L-ornithine trifluoroacetate salt (compound no. 144)

*N*α-(9-fluorenylmethoxycarbonyl)-N-δ-*tert*-butoxycarbonyl-L-ornithine (454 mg, 1.00 mmol) is reacted under the conditions used in example 1 to afford the title compound quantitatively as a white solid.

¹H NMR (DMSO-d₆): 1.60 - 1.86 (m, 4H), 2.80 (m, 2H), 4.00 (m, 1H), 4.20 - 4.38 (m, 3H), 7.30 (t, J = 7.4, 2H), 7.40 (t, J = 7.3, 2H), 7.68 (d, J = 8.1, 1H), 7.72 (d, J = 7.4, 2H), 7.80 (br s, 2H), 7.90 (d, J = 7.4, 2H).

### Example 25. Preparation of Nδ-(3-nitrobenzenesulfonyl)-Nα-(9-fluorenylmethoxycarbonyl)-L-ornithine (compound no. 146)

The product of example 24 is reacted with 3-nitrobenzenesulfonyl chloride under the conditions of example 2 giving 64% of the title compound.

¹H NMR (DMSO-d₆): 1.3 - 1.8 (m, 4H), 2.76 (t, 2H, J = 7 Hz), 3.71 (d, 1H), 4.19 (m, 2H), 4.28 (m, 1H), 6.2 - 7.8 (m, 1H) , 7.5 -8.2 (m, 1H), 7.3 - 8.6 (m, 12H).

### Example 26. Preparation of Nδ-(4-bromosulfonyl)-Nα-(9-fluorenylmethoxycarbonyl)-L-ornithine (compound no. 147)

The product of example 24 is reacted with 4-bromobenzenesulfonyl chloride under the conditions of example 2 giving 67% of the title compound.

¹H NMR (DMSO-d₆): 1.38 - 1.62 (m, 3H), 1.65 - 1.80 (m, 1H), 2.75 (dd, J = 13.0, 6.9, 2H), 3.78 (m, 1H), 4.21 (t, J = 6.9, 1H), 4.27 (d, J = 6.9, 2H), 7.30 - 7.43 (m, 4H), 7.58 (d, J = 7.7, 1H), 7.71 (m, 4H), 7.79 (d, J = 8.1, 2H), 7.89 (d, J = 7.3, 2H), 12.30 (br s, 1H).

### Example 27. Preparation of Nδ-(4-methoxybenzenesulfonyl)-Nα-(9-fluorenylmethoxycarbonyl)-L-ornithine (compound no. 148)

The product of example 24 is reacted with 4-methoxybenzenesulfonyl chloride under the conditions of example 2 giving 61% of the title compound.

¹H NMR (DMSO-d₆): 1.40 - 1.62 (m, 3H), 1. 68 - 1.78 (m, 1H), 2.70 (dd, J = 13.0, 6.8, 2H), 3.81 (s, 3H), 3.86 (m, 1H), 4.21 (t, J = 7.0, 1H), 4.27 (d, J = 6.9, 2H), 7.08 (d, J = 8.3, 2H), 7.28 - 7.42 (m, 4H), 7.58 (d, J = 7.7, 1H), 7.70 (m, 2H), 7.89 (d, J = 7.4, 2H), 12.35 (br s, 1H).

### Example 28. Preparation of Nδ-(4-nitrobenzenesulfonyl)-Nα-(9-fluorenylmethoxycarbonyl)-L-ornithine (compound no. 62)

The product of example 24 is reacted with 4-nitrobenzenesulfonyl chloride under the conditions of example 2 giving 71% of the title compound.

¹H NMR (DMSO-d₆): 1.42 - 1.65 (m, 3H), 1.68 - 1.70 (m, 1H), 2.80 (dd, J = 12.6, 6.8, 2H), 3.85 (m, 1H), 4.21 (t, J = 6.9, 1H), 4.27 (d, J = 7.0, 2H), 7.30 - 7.45 (m, 2H), 7.60 (d, J = 8.4, 1H), 7.71 (d, J = 7.3, 2H), 7.88 (d, J = 7.4, 2H), 8.00 (d, J = 5.3, 1H), 8.03 (d, J = 8.2, 2H), 8.40 (d, J = 7.8, 2H), 12.40 (br s, 1H).

### Example 29. Preparation of Nδ-(4-methylbenzenesulfonyl)-Nα-(9-fluorenylmethoxycarbonyl)-L-ornithine (compound no. 149)

The product of example 24 is reacted with 4-methylbenzenesulfonyl chloride under the conditions of example 2 giving 71 % of the title compound.

¹H NMR (DMSO-d₆): 1.30 - 1.80 (m, 4H) , 2.33 (s, 3H), 2.71 (m, 2H), 2.90 - 3.2 (m, 1H), 3.82 (m, 1H), 4.21 (m, 2H), 4.31 (m, 1H), 6.40-6.90 (m, 1H), 7.50 - 7.70 (m, 1H), 7.20 - 7.90 (m, 12H).

### Example 30. Preparation of Nδ-(4-fluorobenzenesulfonyl)-Nα-(9-fluorenylmethoxycarbonyl)- L-ornithine (compound no. 150)

The product of example 24 is reacted with 4-fluorobenzenesulfonyl chloride under the conditions of example 2 giving 46% of the title compound.

¹H NMR (DMSO-d₆): 1.3 - 1.8 (m 4H), 2.71 (m, 2H), 3.77 (m, 1H), 4.22 (m, 2H) 4.27 (m, 1H), 6.4 - 7.1 (m, 1H), 7.5 - 8.2 (m, 1H), 7.3 - 7.9 (m, 12H)

### Example 31. Preparation of Nδ-(4-aminobenzenesulfonyl)-Nα-(9-fluorenylmethoxycarbonyl)-L-ornithine (compound no. 69)

The product obtained from example 28 (54.0 mg, 0.10 mmol) is dissolved in MeOH (5 mL) and then hydrogenated using 10% Pd/C as catalyst at atmospheric pressure for 1 h. The catalyst is filtered off and the filtrate is evaporated *in vacuo* to yield 96% of the title compound.

¹H NMR (DMSO-d₆): 1.3 - 1.8 (m, 4H), 2.79 (m, 2H), 3.14 (m, 1H), 5.76 (5.76 (s, 1H), 6.28 (s, 2H), 7.3 - 7.8 (m, 14H).

### Example 32. Preparation of Nα, N∈-di-(4-methylbenzenesulfonyl)-L-lysine (compound no. 151)

To a stirred solution of L-lysine dihydrochloride (1 mmol) in a mixture of THF and 1M K₂CO₃ (3 mL/3 mL) is added 4-methylbenzenesulfonyl chloride (381 mg, 2.00 mmol). The reaction mixture is stirred for 2 h and then quenched with 1N HCl and extracted twice with EtOAc. The combined organic extracts are dried over MgSO₄ and concentrated. The crude is purified by flash chromatography using hexane/EtOAc/AcOH (30:69.4/0.6) to give 75% of the desired product.

¹H NMR (DMSO-d₆): 1.05 - 1.30 (m, 4H), 1.32 - 1.52 (m, 2H), 2.34 (s, 3H), 2.37 (s, 3H), 2.60 (dd, J = 12.9, 6.9, 2H), 3.56 (m, 1H), 7.32(d, J = 7.9, 2H), 7.38 (d, J = 8.0, 2H), 7.42 (t, J = 5.9, 1H), 7.62 (d, J = 8.3, 2H), 7.66 (d, J = 8.5, 2H), 7.97 (d, J = 7.7, 1H), 12.4 (br s, 1H).

### Example 33. Preparation of Nα,N∈-di-(4-bromobenzenesulfonyl)-L-lysine (compound no. 17)

Following the indications of example 32 substituting 4-methylbenzenesulfonyl chloride with 4-bromobenzenesulfonyl chloride, the product is obtained in 78% yield.

¹H NMR (DMSO-d₆): 1.12 - 1.35 (m, 4H), 1.40 - 1.58 (m, 2H), 2.60 - 2.68 (m, 2H), 3.42 - 3.50 (m, 1H), 7.60 - 7.80 (m, 10H), 12.80 (br s, 1H).

### Example 34. Preparation of Nα,Nδ-di-(4-bromobenzenesulfonyl)-L-ornithine (compound no. 57)

Following the indications of example 32 substituting L-lysine with L-ornithine and using 4-bromobenzenesulfonyl chloride instead of 4-methylbenzenesulfonyl chloride, the title product is obtained in 66% yield.

¹H NMR (DMSO-d₆): 1.30 - 1.52 (m, 3H), 1.58 - 1.67 (m, 1H), 2.62 - 2.70 (m, 2H), 3.61 - 3.70 (m, 1H), 7.62 - 7.82 (m, 9H), 12.70 (br s, 1H).

### Example 35. Preparation of Nα-isobutyl-Nα-(4-methylbenzenesulfonyl)- N∈-(9-fluoreaylmethoxycarbonyl)-DL-lysine (compound no. 2)

### Step A. Preparation of N∈- benzyloxycarbonyl-L-lysine methyl ester

To a stirred *N*α-*tert*-butoxycarbonyl-*N*∈-benzyloxycarbonyl-L-lysine (7.6 g, 20 mmol) in DMF (120 mL) is added KHCO₃ (2.2 g, 22 mmol). After stirring the suspension for 1 h, methyl iodide (3.6 g, 25 mmol) is added dropwise. The reaction mixture is stirred overnight. It is quenched with 1N HCl until acidic (app. pH = 3) and extracted with EtOAc. The organic layer is washed twice with brine, dried over MgSO₄ and concentrated in vacuo to afford the methyl ester that is used without further purification. It is dissolved in CH₂Cl₂ (60 mL), and to this solution is added TFA (20 mL). The reaction mixture is stirred at room temperature for 2 h, evaporated *in vacuo*, and taken up in 1M K₂CO₃ and EtOAc. The aqueous layer is extracted with EtOAc. The combined organic layers are dried over MgSO₄ and concentrated to give 5.42 g (92%) of the title compound as a colorless oil.

¹H NMR (CDCl₃): 1.35 - 1.48 (m, 2H), 1.50 - 1.65 (m, 3H), 1.70 - 1.79 (m, 1H), 1.82 (br s, 2H), 3.17 (m, 2H), 3.43 (t, J = 6.5, 1H), 3.71 (s, 3H), 4.90 (br s, 1H), 5.09 (s, 2H), 7.27 - 7.35 (m, 5H).

### Step B. Preparation of N∈-benzyloxycarbonyl-Nα-isobutyl-L-lysine methyl ester

To a stirred solution of amine from step A of this example (5.0 g, 17 mmol), AcOH (2.0 mL, 42 mmol) and NaCNBH₃ (1.39 g, 22.1 mmol) in MeOH (200 mL) at 0°C is added a solution of isobutyraldehyde (2.02 mL, 22.1 mmol) in MeOH (10 mL). The solution is warmed to room temperature and stirred for 2h. The mixture is quenched with a saturated solution of K₂CO₃ (106 mL). The solution is filtered and the filtrate is evaporated *in vacuo*. The residue is taken up in EtOAc (200 mL) and water (150 mL). The organic layer is separated, washed successively with 1M K₂CO₃ and brine, dried over Na₂SO₄ and concentrated *in vacuo*. The residue is filtered on silica gel, giving 4.04 g (68%) of the title compound.

¹H NMR (CDCl₃): 0.88 (d, J = 7.4, 6H), 1.32 - 1.70 (m, 7H), 2.22 and 2.35 (ABX, J = 11.0, 7.1, 2H), 3.16 (m, 2H), 3.69 (s, 3H), 4.95 (br s, 1H) , 5.07 (s, 2H), 7.28 - 7.34 (m, 5H).

### Step C. Preparation of Nα-isobutyl-Nα-(4-methylbenzenesulfonyl)-N∈-benzyloxycarbonyl-L-lysine methyl ester

To a stirred solution of the amine obtained in step B of this example (1.00 g, 2.34 mmol) in CH₂Cl₂ (3 mL) is added 4-methylbenzenesulfonyl chloride (670 mg, 3.51 mmol) and diisopropylethylamine (0.5 mL, 2.8 mmol). The reaction mixture is stirred overnight at room temperature. The mixture is treated with 1N HCl and extracted with CH₂Cl₂. The organic layer is dried over MgSO₄ and concentrated *in vacuo*. The crude material is purified by flash chromatography eluting with 30% EtOAc in hexane to yield 1.3 g (89%) of the title compound as a colorless oil.

¹H NMR (DMSO-d₆): 0.84 (d, J = 7.2, 3H), 0.86 (d, J = 6.3, 3H), 1.30 - 1.68 (m, 5H), 1.88 - 2.00 (m, 2H), 2.42 (s, 3H), 2.92 and 3.00 (ABX, J = 14.7, 8.2, 2H), 3.18 (m, 2H), 3.50 (s, 3H), 4.40 (t, J = 7.4, 1H), 4.78 (br s, 1H), 5.11 (s, 2H), 7.27 - 7.71 (m, 9H).

### Step D. Preparation of Nα-isobutyl-Nα-(4-methylbenzenesulfonyl)-N∈- benzyloxycarbonyl-DL-lysine

To a stirred solution of the ester obtained in step C of this example (505 mg, 1.00 mmol) in a mixture of 50% MeOH in THF (4 mL) is added a 1N NaOH solution (3 mL, 3 mmol). The reaction is stirred at room temperature overnight, then diluted with 1N HCl until acidic and extracted twice with EtOAc. The combined organic layers are dried with MgSO₄ and concentrated *in vacuo* to give the title compound (490 mg, 100%) as an amorphous solid.

¹H NMR (DMSO-d₆): 0.78 (d, J = 6.9, 3H), 0.81 (d, J = 6.5, 3H), 1.15 - 1.50 (m, 5H), 1.75 - 1.80 (m, 2H), 2.36 (s, 3H), 2.75 - 3.00 (m, 4H), 4.20 (t, J = 7.0, 1H), 5.00 (s, 2H), 7.20 (t, J = 5.0, 1H), 7.30 - 7.67 (m, 9H), 12.70 (br s, 1H).

### Step E. Preparation of Nα-isobutyl-Nα-(4-methylbenzenesulfonyl)-N∈-(9- fluorenylmethoxycarbonyl)-DL-lysine

10% Pd/C (120 mg) is added to a stirred solution of the product from step D of this example (490 mg, 1.00 mmol). The suspension is flushed with hydrogen gas and maintained under H₂ pressure for 2h. It is then filtered and concentrated *in vacuo.* The resulting white solid is partially dissolved in 1M K₂CO₃ (4 mL, 4 mmol), THF (6 mL) and acetonitrile (4 mL). To this suspension is added N- (9-fluorenylmethoxycarbonyloxy) succinimide (371 mg, 1.10 mmol). The reaction became clear and is stirred for 1h at room temperature. The mixture is quenched by the addition of 2N HCl until acidic. The mixture is extracted twice with EtOAc, the combined organic layer is washed with brine, dried over MgSO₄ and concentrated *in vacuo*. The residue is purified by flash chromatography eluting with 60% EtOAc in hexane containing 0.4% AcOH to yield 480 mg (83%) of the title compound as a white solid.

¹H NMR (DMSO-d₆): 0.79 (d, J = 7.1, 3H), 0.81 (d, J = 7.1, 3H), 1.12 - 1.25 (m, 2H), 1.30 - 1.40 (m, 2H), 1.42 - 1.50 (m, 2H), 1.75 - 1.90 (m, 2H), 2.36 (s, 3H), 2.85 (m, 2H), 2.90 and 3.00 (ABX, J = 14.3, 7.3, 2H), 4.16 - 4.21 (m, 2H), 4.28 (d, J = 7.0, 2H), 7.21 (t, J = 5.2, 1H), 7.30 - 7.42 (m, 6H), 7.60 (m, 4H), 7.88 (d, J = 7.5, 2H), 12.69 (br s, 1H).

### Example 36. Nα-isobutyl-Nα-(4-chlorobenzenesulfonyl)-N∈-(9-fluorenylmethoxycarbonyl)-DL-lysine (compound no. 1)

Following the indications found in example 35 step C and substituting 4-methylbenzenesulfonyl chloride with 4-chlorobenzenesulfonyl chloride, the title compound is obtained (67% yield).

¹H NMR (DMSO-d₆): 0.78 (d, J = 6.1, 3H), 0.81 (d, J = 6.1, 3H), 1.15 - 1.52 (m, 5H) , 1.75 - 1.91 (m, 2H), 2.80 - 2.95 (m, 3H), 3.00 (dd, J = 14.2, 7.2, 1H), 4.20 (m, 2H), 4.31 (d, J = 6.5, 2H), 7.20 (t, J = 5.6, 1H), 7.23 - 7.42 (m, 4H), 7.53 - 7.68 (m, 4H), 7.79 (d, J = 7.4, 2H), 7.88 (d, J = 7.4, 2H), 12.70 (br s, 1H).

### Example 37. Preparation of Nα-isobutyl-Nα-(4-fluorobenzenesulfonyl)- N∈-(9- fluorenylmethoxycarbonyl)-DL-lysine (compound no. 3)

Following the indications found in example 35 and substituting 4-bromobenzenesulfonyl chloride with 4-fluorobenzenesulfonyl chloride, the title compound is obtained (62% yield).

¹H NMR (DMSO-d₆) : 0.78 (d, J = 6.8, 3H), 0.81 (d, J = 6.9, 3H), 1.18 - 1.28 (m, 2H), 1.30 - 1.42 (m, 2H), 1.45 - 1.53 (m, 1H), 1.79 - 1.95 (m, 2H), 2.90 (m, 3H), 3.00 (dd, J = 14.6, 7.4, 1H), 4.20 (m, 2H), 4.31 (d, J = 6.4, 2H), 7.22 (t, J = 5.0, 1H), 7.30 - 7.45 (m, 6H), 7.67 (d, J = 7.5, 1H), 7.82 - 7.91 (m, 4H).

### Example 38. General preparation of Nα-isobutyl-Nα-(4-substituted benzenesulfonyl)-N∈-(9-fluorenylmethoxycarbonyl)-L-lysine

### Step A. Preparation of N∈-benzyloxycarbonyl-L-lysine benzyl ester

To a stirred solution of *N*α-*tert*-butoxycarbonyl-*N*∈-benzyloxycarbonyl-L-lysine (7.6 g, 20 mmol) in DMF (120 mL) is added potassium bicarbonate. After stirring the suspension for 1h, benzyl bromide (1.31 mL, 11.0 mmol) is added dropwise. The reaction mixture is stirred overnight, then diluted with 1N HCl until acidic (pH approximately 3) and extracted with EtOAc. The organic layer is washed twice with brine, dried over MgSO₄ and concentrated in vacuo to yield the benzyl ester that is dissolved in CH₂Cl₂/TFA (60 mL/20 mL). The mixture is stirred until the disappearance of the starting material (1.2 h). The volatiles are removed *in vacuo* and dissolved in EtOAc and a solution of 1M K₂CO₃. The two phases are separated and the aqueous layer is washed twice with EtOAc. The combined layers are dried over MgSO₄ and concentrated *in vacuo* to give the title compound as a colorless oil (8.9 g, 95%).

¹H NMR (DMSO-d₆): 1. 22 - 1.50 (m, 5H), 1.53 - 1.62 (m, 1H), 2.00 (br s, 2H), 2.95 (m, 2H), 3.30 (m, 1H), 5.00 (s, 2H), 5.10 (s, 2H), 7.20 (t, J = 5.0, 1H), 7.25 - 7.40 (m, 5H).

### Step B. Preparation of Nα-alkyl-N∈-benzyloxycarbonyl-L-lysine benzyl ester

To a stirred solution of the product obtained in step A (4.32 g, 9.17 mmol), acetic acid (1.3 mL, 23 mmol) and sodium cyanoborohydride (691 mg, 11.0 mmol) in MeOH (120 mL) at 0°C is added a solution of aldehyde (11.0 mmol) in MeOH (40 mL). The reaction mixture is warmed to room temperature and stirred for a period of 1h. A saturated solution of K₂CO₃ (55 mL) is added and the mixture is partitioned between EtOAc (150 mL) and water (100 mL). The organic layer is washed with 1M K₂CO₃ and with brine, then dried over MgSO₄. The organic solvent is removed *in vacuo* and the residue is purified by flash chromatography eluting with hexane/EtOAc (60:40) to yield 65 - 95% of the title compound.

### Step C. Preparation of Nα-(4-substitutedbenzenesulfonyl)-Nα-alkyl-N∈-benzyloxycarbonyl-L-lysine benzyl ester

To a stirred solution of the product of step B of this example (1.0 mmol) in CH₂Cl₂ (1 mL) is added io a substituted benzenesulfonyl chloride (1.5 mmol) followed by the addition of diisopropylethyl amine (174 µL). The reaction mixture is stirred for three days at room temperature. It is then diluted with 1N HCl The organic phase is dried over MgSO₄ and concentrated *in vacuo*. The crude residue is flash chromatographed eluting with 40% EtOAc in hexane to yield the title compound at about 85%.

### Step D. Preparation of Nα-(4-substituted benzenesulfonyl)-Nα-alkyl-N∈-(9-fluorenylmethoxycarbonyl)-L-lysine

To the product obtained in step C of this example (1 mmol) in AcOH (5 mL) is added 10% Pd/C (120 mg). The suspension is flushed with hydrogen gas and maintained under H₂ atmosphere for 2 h. After filtering and evaporating *in vacuo*, the resulting white solid is partially dissolved in K₂CO₃ (1M)/THF/CH₃CN (4 mL/4 mL/4 mL). To this suspension is added N-(9-fluorenylmethoxycarbonyloxy) succinimide (371 mg, 1.10 mmol). The reaction turned slowly to colorless and is left stirring for 1h. HCl (1M) is added until acidic pH and the reaction mixture is extracted twice with EtOAc. The combined organic layers are washed with brine, dried over MgSO₄ and concentrated. The residue is purified by flash chromatography eluting with a mixture of hexane/EtOAc containing 0.4% AcOH to yield 69 - 88% of the title compound.

### Example 39. Preparation of Nα-isobutyl-Nα-(4-bromobenzenesulfonyl)-N∈-(9-fluorenylmethoxycarbonyl)-L-lysine (compound no. 4)

### Step A. Preparation of Nα-isobutyl-N∈-benzyloxycarbonyl-L-lysine benzyl ester

The title compound is prepared by reacting *N∈-*benzyloxycarbonyl-L-lysine benzyl ester with isobutyraldehyde according to the indications of step B of example 38.

¹H NMR (CDCl₃): 0.88 (d, J = 5.0, 6H), 1.30 - 1.41 (m, 2H), 1,42 - 1.53 (m, 2H), 1.58 - 1.62 (m, 3H), 2.28 and 2.35 (ABX, J = 15.2, 7.4, 2H), 3 .10 - 3.18 (m, 2H), 3.25 (t, J = 7.0, 1H), 4.85 (br s, 1H), 5.10(s, 2H), 5.12 and 5.20 (AB, J = 12.5, 2H), 7.30 - 7.38 (m, 10H).

### Step B. Preparation of Nα-isobutyl-Nα-(4-bromobenzenesulfonyl)-N∈-benzyloxycarbonyl-L-lysine benzyl ester

The product obtained in step A of this example is treated as described in step C of example 38 with 4-bromobenzenesulfonyl chloride to yield the title compound.

¹H NMR (CDCl₃): 0.78 (d, J = 6.7, 3H), 0.83 (d, J = 6.1, 3H), 1.35 - 1.60 (m, 4H), 1.65 - 1.74 (m, 1H), 1.86 - 2.06 (m, 2H), 2.85 and 3.00 (ABX, J = 14.5, 7.4, 2H), 3.17 - 3.24 (m, 2H), 4.45 (t, J = 7.2, 1H), 4.84 (br s, 1H), 4.93 (s, 2H), 5.11 (s, 2H), 7.21 - 7.62 (m, 14H) .

### Step C. Preparation of Nα-isobutyl-Nα-(4-bromobenzenesulfonyl)-N∈-(9-fluorenylmethoxycarbonyl)-L-lysine

The title compound is prepared by following the indications of step D of example 38 using the product obtained in step B of this example and reacting it with N-(9-fluorenylmethoxycarbonyloxy) succinimide.

¹H NMR (DMSO-d₆): 0.79 (d, J = 7.0, 3H), 0.81 (d, J = 7.1, 3H), 1.15 - 1.25 (m, 2H), 1.30 - 1.40 (m, 2H), 1.42 - 1.50 (m, 1H), 1.78 - 1.92 (m, 2H), 2.89 (m, 2H), 2.95 and 3.00 (ABX, J = 14.8, 7.3, 2H), 4.20 (m, 2H), 4.30 (d, J = 6.4, 2H), 7.21 (t, J = 5.0, 1H), 7.30 - 7.52 (m, 6H), 7.62 (d, J = 7.4, 1H), 7.67 - 7.90 (m, 6H), 12.70 (br s, 1H).

The D-lysine derivative is prepared in a similar manner.

### Example 40. Preparation of Nα-(4-aminobenzenesulfonyl)-Nα-isobutyl-N∈- (9-fluorenylmethoxycarbonyl)-L-lysine (compound no. 44)

### Step A. Preparation of Nα-isobutyl-Nα-(4-nitrobenzenesulfonyl)-N∈-benzyloxycarbonyl-L-lysine benzyl ester (compound no. 78).

The product obtained in step A of example 39 is treated as described in step C of example 38 with 4-nitrobenzenesulfonyl chloride to yield the title compound.

¹H NMR (CDCl₃): 0.79 (d, J = 6.0, 3H), 0.85 (d, J = 6.1, 3H), 1.42 - 1.65 (m, 4H), 1.67 - 1.73 (m, 1H), 1.93 (h, J= 6.0, 1H), 2.00 - 2.10 (m, 1H), 2.90 and 3.05 (ABX, J = 14.5, 7.4, 2H), 3.20 (m, 2H), 4.51 (t, J = 7.2, 1 H), 4.80 (br s, 1H), 4.91 (s, 2H), 5.10 (s, 2H), 7.15 (d, J = 7.0, 2H), 7.30 - 7.42 (m, 9H).

### Step B. Preparation of Nα-(4-aminobenzenesulfonyl)-Nα-isobutyl-N∈-(9-fluorenylmethoxycarbonyl)-L-lysine

The title compound is prepared by following the indications of step D of example 38 using the product obtained in step A of this example and reacting it with N-(9-fluorenylmethoxycarbonyloxy) succinimide. In this case, the hydrogenolysis of the benzyl groups and the reduction of the nitro group took place simultaneously.

¹H NMR (DMSO-d₆): 0.78 (d, J = 6.9, 3H), 0.80 (d, J = 6.0, 3H), 1.18 - 1.48 (m, 5H), 1.73 - 1.82. (m, 2H), 2.82 - 3.00 (m, 4H), 4.10 (t, J = 7.1, 1H), 4.20 (t, J = 7.0, 1H), 4.28 (d, J = 7.6, 2H), 5.95 (br s, 2H), 6.57 (d, J = 7.6, 2H), 7.22 (t, J = 5.2, 1H), 7.30 - 7.45 (m, 6H), 7.67 (d, J = 7.1, 2H), 7.88 (d, J = 7.3, 2H), 12.60 (br s, 1H).

### Example 41. Preparation of Nα-isobutyl-Nα-benzenesulfonyl-N∈-(9-fluorenylmethoxycarbonyl)-L-lysine (compound no. 9)

### Step A. Preparation of Nα-isobutyl-Nα-benzenesulfonyl-N∈-benzyloxycarbonyl-L-lysine benzyl ester

The product obtained in step A of example 39 is treated as described in step C of example 38 with benzenesulfonyl chloride to yield the title compound.

¹H NMR (CDCl₃) : 0.78 (d, J = 6.0, 3H), 0.83 (d, J = 6.8, 3H), 1.30 - 1.73 (m, 5H), 1.85 - 2.00 (m, 2H), 2.88 and 3.15 (ABX, J = 14.0, 7.2, 2H), 3.16 (m, 2H), 4.45 (t, J = 7.2, 1H) , 2.82 (br s, 1H), 4.91 (s, 2H), 5.10 (s, 2H), 7.21 - 7.55 (m, 13H), 7.79 (d, J = 7.7, 2H).

### Step B. Preparation of Na-isobutyl-Na-benzenesulfonyl-Ne-(9-fluorenylmethoxycarbonyl)-L-lysine

The title compound is prepared by following the indications of step D of example 38 using the product obtained in step A of this example and reacting it with N-(9-fluorenylmethoxycarbonyloxy) succinimide.

¹H NMR (DMSO-d₆): 0.79 (d, J = 6.1, 3H), 0.81 (d, J = 6.7, 3H), 1.15 - 1.50 (m, 5H), 1.82 - 1.93 (m, 2H), 2.89 (m, 2H), 2.93 and 3.00 (ABX, J = 14.7, 7.1, 2H), 4.20 (m, 2H), 4.30 (d, J = 6.5, 2H), 7.22 (t, J = 5.2, 1H), 7.31 - 7.42 (m, 4H), 7.52 - 7.70 (m, 5H), 7.80 (d, J = 7.7, 2H), 7.87 (d, J = 7.3, 2H), 12.70 (br s, 1H).

### Example 42. Preparation of Nα-isobutyl-Nα-(1-naphthalenesulfonyl)-N∈-(9-fluorenylmethoxycarbonyl)-L-lysine (compound no. 8)

### Step A. Preparation of Nα-isobutyl-Nα-(1-naphthalenesulfonyl)-N∈-benzyloxycarbonyl-L-lysine benzyl ester

The product obtained in step A of example 39 is treated as described in step C of example 38 with 1-naphthalenesulfonyl chloride to yield the title compound.

¹H-NMR (CDCl₃): 0.71 (d, J = 7.3, 3H), 0.78 (d, J = 7.0, 3H), 1.20 - 1.48 (m, 4H), 1.55 - 1.65 (m, 1H), 1.82 - 2.00 (m, 2H), 3.00 and 3.20 (ABX, J = 14.2, 7.4, 2H), 3.12 (m, 2H), 4.50 (t, J = 7.2, 1H), 4.71 - 4.82 (m, 3H), 5.10 (s, 2H), 7.10 - 7.60 (m, 4H), 7.90 (d, J = 6.4, 1H), 8.00 (d, J = 8.0, 1H), 8.29 (d, J = 7.3, 1H), 8.76 (d, J = 7.8, 1H).

### Step B. Preparation of Nα-isobutyl-Nα-(1-naphthalenesulfonyl)-N∈-(9-fluorenylmethoxycarbonyl)-L-lysine

The title compound is prepared by following the indications of step D of example 38 using the product obtained in step A of this example and reacting it with N-(9-fluorenylmethoxycarbonyloxy) succinimide.

¹H NMR (DMSO-d₆): 0.70 (d, J = 6.2, 3H), 0.73 (d, J = 6.3, 3H), 1.10 - 1.18 (m, 2H), 1.20 - 1.28 (m, 2H), 1.34 - 1.45 (m, 1H), 1.75 - 1.92 (m, 2H), 2.80 (m, 2H), 3.00 and 3.11 (ABX, J = 14.6, 6.2, 2H), 4.20 (m, 1H), 4.30 (m, 2H), 5.00 (s, 1H), 7.21 (m, 1H), 7.28 - 7.45 (m, 4H), 7.60 - 8.30 (m, 9H), 8.65 (d, J = 9.0, 1H).

### Example 43. Preparation of Nα-isobutyl-Nα-(4-tert-butylbenzenesulfonyl)-N∈-(9-fluorenylmethoxycarbonyl)-L-lysine (compound no. 10)

### Step A. Preparation of Nα-isobutyl-Nα-(4-tert-butylbenzenesulfonyl)-N∈-benzyloxycarbonyl-L-lysine benzyl ester

The product obtained in step A of example 39 is treated as described in step C of example 38 with 4-*tert-*butylbenzenesulfonyl chloride to yield the title compound.

¹H NMR (CDCl₃): 0.77 (d, J = 6.0, 3H), 0.82 (d, J = 7.0, 3H), 1.32 (s, 9H), 1.28 - 1.70 (m, 5H), 1.88 - 2.00 (m, 2H), 2.87 and 3.00 (ABX, J = 14.0, 7.0, 2H), 3.15 (m, 2H), 4.47 (t, J = 7.2, 1H), 4.83 (br s, 1H), 4.90 (s, 2H), 5.10 (s, 2H), 7.20 - 7.43 (m, 12H), 7.72 (d, J = 7.8, 2H).

### Step B. Preparation of Nα-isobutyl-Nα-(4-tert-butylbenzenesulfonyl)-N∈-(9-fluorenylmethoxycarbonyl)-L-lysine

The title compound is prepared by following the indications of step D of example 38 using the product obtained in step A of this example and reacting it with N-(9-fluorenylmethoxycarbonyloxy) succinimide.

¹H NMR (CDCl₃): 0.80 (d, J = 6.9, 3H), 0.82 (d, J = 6.2, 3H), 1.10 - 1.20 (m, 2H), 1.27 (s, 9H), 1.28 - 1.42 (m, 3H), 1.75 - 1.92 (m, 2H), 2.82 (m, 2H), 2.95 (m, 2H), 4.15 (t, J = 6.5, 1H) , 4.20 (t, J = 7.1, 1H), 4.28 (d, J = 6.6, 2H), 7.20 (t, J = 5.2, 1H), 7.28 - 7.45 (m, 4H), 7.56 (d, J = 7.0, 2H), 7.67 (m, 4H), 7.88 (d, J = 7.1, 2H), 12.70 (br s, 1H).

### Example 44. Preparation of Nα-isobutyl-Nα-(4-methoxybenzenesulfonyl)-N∈-(9-fluorenylmethoxycarbonyl)-L-lysine (compound no. 7)

### Step A. Preparation of Nα-isobutyl-Nα-(4-methoxybenzenesulfonyl)-N∈-benzyloxycarbonyl-L-lysine benzyl ester

The product obtained in step A of example 39 is treated as described in step C of example 38 with 4-methoxybenzenesulfonyl chloride to yield the title compound.

¹H NMR (CDCl₃): 0.78 (d, J 6.6, 3H), 0.83 (d, J = 6.1, 3H), 1.33 - 1.80 (m, 5H), 1.86 - 2.00 (m, 2H), 2.90 and 3.00 (ABX, J = 14.3, 7.6, 2H), 3.15 - 3.20 (m, 2H), 3.82 (s, 3H), 4.43 (t, J = 7.3, 1H), 4.82 (br s, 1H), 4.94 and 4.96 (AB, J = 12.6, 2H), 5.10 (s, 2H), 6.83 (d, J = 8.4, 2H), 7.20 - 7.40 (m, 10H), 7.70 (d, J = 8.1, 2H).

### Step B. Preparation of Nα-isobutyl-Nα-(4-methoxybenzenesulfonyl)-N∈-(9-fluorenylmethoxycarbonyl)-L-lysine

The title compound is prepared by following the indications of step D of example 38 using the product obtained in step A of this example and reacting it with N-(9-fluorenylmethoxycarbonyloxy) succinimide.

¹H NMR (CDCl₃): 0.78 (d, J = 6.9, 3H), 0.81 (d, J = 6.9, 3H), 1.15 - 1.51 (m, 5H), 1.75 - 1.90 (m, 2H), 2.88 - 2.92 (m, 3H), 2.97 (dd, J = 14.5, 7.6, 2H), 3.81 (s, 3H) , 4.15 (t, J = 6.8, 1H), 4.18 (t, J = 6.7, 1H), 4.20 (d, J = 6.6, 2H), 7.06 (d, J = 8.7, 2H) , 7.22 (t, J = 4.9, 1H), 7.70 (m, 4H), 7.89 (d, J = 7.4, 2H), 12.60 (br s, 1H).

### Example 45. Preparation of Nα-isobutyl-Nα-(4-methylbenzenesulfonyl)-N∈-(9-fluorenylmethoxycarbonyl)-L-lysine (compound no. 67)

### Step A. Preparation of Nα-isobutyl-Nα-(4-methylbenzenesulfonyl)-N∈-benzyloxycarbonyl-L-lysine benzyl ester (compound no. 75)

The product obtained in step A of example 39 istreated as described in step C of example 38 with 4-methylbenzenesulfonyl chloride to yield the title compound.

¹H NMR (CDCl₃): 0.79 (d, J = 7.0, 3H), 0.83 (d, J = 7.0, 3H), 1.30 - 1.45 (m, 2H), 1.48 - 1.57 (m, 2H), 1.60 - 1.72 (m, 1H), 1.91 - 2.00 (m, 2H), 2.40 (s, 3H), 2.88 and 3.14 (ABX, J = 14.5, 7.4, 2H), 3.16 (m, 2H), 4.44 (t, J = 7.3, 1H), 4.85 (br s, 1H), 4.93 (s, 2H), 5.10 (s, 2H), 7.16 (d, J = 7.7, 2H), 7.20 - 7.42 (m, 10H), 7.65 (d, J = 8.3, 2H).

### Step B. Preparation of Nα-isobutyl-Nα-(4-methylbenzenesulfonyl)-N∈-(9-fluorenylmethoxycarbonyl)-L-lysine

The title compound is prepared by following the indications of step D of example 38 using the product obtained in step A of this example and reacting it with N-(9-fluorenylmethoxycarbonyloxy) succinimide.

¹H NMR (CDCl₃) : 0.79 (d, J = 7.1, 3H), 0.81 (d, J = 7.1, 3H), 1.12 - 1.25 (m, 2H), 1.30 - 1.40 (m, 2H), 1.42 - 1.50 (m, 2H), 1.78 - 1.90 (m, 2H), 2.36 (s, 3H), 2.85 (m, 2H), 2.88 and 3.04 (ABX, J = 14.3, 7.3, 2H), 4.16 - 4.21 (m, 2H), 4.28 (d, J = 7.0, 2H), 7.30 - 7.42 (m, 6H), 7.60 (m, 4H), 7.88 (d, J = 7.5, 2H), 12.69 (br s, 1H).

### Example 46. Preparation of Nα-isobutyl-Nα-(2,4,6-trimethylbenzenesulfonyl)-N∈-(9-fluorenyltaethoxycarbonyl)-L-lysine (compound no. 42)

### Step A. Preparation of Nα-isobutyl-Nα-(2,4,6-trimethylbenzenesulfonyl)-N∈-benzyloxycarbonyl-L-lysine benzyl ester

The product obtained in step A of example 39 is treated as described in step C of example 38 with 2,4,6-trimethylbenzenesulfonyl chloride to yield the title compound.

¹H NMR (CDCl₃) : 0.70 (d, J = 6.7, 3H), 0.78 (d, J = 6.5, 3H), 1.22 - 1.55 (m, 4H), 1.65 - 1.80 (m, 2H), 1.95 - 2.05 (m, 1H), 2.27 (s, 3H), 2.56 (s, 6H), 3.10 - 3.20 (m, 4H), 4.26 (t, J = 6.5, 1H), 4.83 (br s, 1H), 5.06 and 5.11 (AB, J = 12.6, 2H), 5.10 (s, 2H), 6.87 (s, 2H), 7.27 - 7.36 (m, 10H).

### Step B. Preparation of Nα-isobutyl-Nα-(2,4,6-trimethylbenzenesulfonyl)-N∈-(9-fluorenylmethoxycarbonyl)-L-lysine

The title compound is prepared by following the indications of step D of example 39 using the product obtained in step A of this example and reacting it with N-(9-fluorenylmethoxycarbonyloxy) succinimide.

¹H NMR (CDCl₃): 0.69 (d, J = 6.8, 3H), 0.73 (d, J = 6.0, 3H), 1.15 - 1.40 (m, 4H), 1.52 - 1.62 (m, 1H), 1.72 (h, J = 6.5, 1H), 1.82 - 1.93 (m, 1H), 2.24 (s, 3H), 2.53 (s, 6H), 2.90 (m, 2H), 3.10 (t, J = 7.2, 2H), 3.98 (t, J = 7.0, 1H), 4.20 (t, J = 6.7, 1H), 4.28 (d, J = 6.8, 2H), 7.03 (s, 2H), 7.20 (t, J = 5.2, 1H), 7.30 - 7.45 (m, 4H), 7.67 (d, J = 7.3, 2H) , 7.87 (d, J = 7.5, 2H), 12.80 (br s, 1H).

### Example 47. Preparation of Nα-isobutyl-Nα-(4-iodobenzenesulfonyl)-N∈-benzyloxycarbonyl-DL-lysine (compound no. 48)

### Step A. Preparation of Nα-isobutyl-Nα-(4-iodobenzenesulfonyl)-N∈-benzyloxycarbonyl-L-lysine benzyl ester

The product obtained in step A of example 39 istreated as described in step C of example 38 with 4-iodobenzenesulfonyl chloride to yield the title compound.

¹H NMR (CDCl₃): 0.78 (d, J = 6.1, 3H), 0.83 (d, J = 6.3, 3H), 1.38 - 1.60 (m, 4H), 1.65 - 1.75 (m, 1H), 1.90 (h, J = 6.2, 1H), 1.91 - 2.02 (m, IH), 2.85 and 3.00 (ABX, J = 14.5, 7.4, 2H), 3.20 (m, 2H), 4.45 (t, J = 7.2, 1H), 4.83 (br s, 1H), 4.93 (s, 2H), 5.11 (s, 2H), 7.20 - 7.70 (m, 14H)

### Step B. Preparation of Nα-isobutyl-Nα-(4-iodobenzenesulfonyl)-N∈-benzyloxycarbonyl-DL-lysine

The product from step A of this example issaponified according to the indication of step D of example 35 to provide the title compound.

¹H NMR (DMSO-d₆): 0.79 (d, J = 6.1, 3H), 0.82 (d, J = 6.4, 3H), 1.18 - 1.55 (m, 5H), 1.74 - 1.93 (m, 2H), 2.86 - 2.99 (m, 3H), 3.00 (dd, J = 15.5, 7.7, 1H), 4.20 (t, J = 7.2, 1H), 5.00 (s, 2H), 7.20 (br s, 1H), 7.28 - 7.36 (m, 5H), 7.55 (d, J = 7.0, 2H), 7.93 (d, J = 8.0, 2H), 12.73 (br s, 1H).

### Example 48. Preparation of Nα-isobutyl-Nα-(4-methylbenzenesulfonyl)- Nδ-(9-fluorenylmethoxycarbonyl)-DL-ornithine (compound no. 6)

### Step A. Preparation of Nα-isobutyl-Nδ-benzyloxycarbonyl-DL-omithine methyl ester

The title compound is prepared by reacting *Nα-tert-*butoxycarbonyl-*N*δ-benzyloxycarbonyl-DL- ornithine with methyl iodide according to the indications of step A of example 35. The product treated with TFA in CH₂Cl₂ and the residue isdirectly subjected to the reductive alkylation as described in step B of example 35.

¹H NMR (CDCl₃) : 0.87 (d, J = 6.5, 6H), 1.50 (br s, 1H), 1.55-1.72 (m, 5H), 2.25 and 2.38 (ABX, J = 11.1, 6.0, 2H), 3.16 - 3.25 (m, 3H), 3.70 (s, 3H), 5.08 (s, 2H), 5.25 (br s, 1H), 7.29 - 7.40 (m, 5H).

### Step B. Preparation of Nα-isobutyl-Nα-(4-methylbenzenesulfonyl)-Nδ-benzyloxycarbonyl-DL-ornithine methyl ester

The title compound is prepared (89% yield) by following the indications of step C of example 35 using the product obtained in step B of this example and reacting it with 4-methylbenzenesulfonyl chloride.

¹H NMR (CDCl₃): 0.84 (d, J = 7.4, 3H), 0.87 (d, J = 7.8, 3H), 1.52 - 1.70 (m, 3H), 1.88 - 2.00 (m, 2H), 2.90 and 3.05 (ABX, J = 14.5, 7.5, 2H), 3.15 - 3.22 (m, 2H), 3.48 (s, 3H), 4.41 (t, J = 6.3, 1H), 4.90 (br s, 1H), 5.10 (s, 2H), 7.27 (d, J = 8.1, 2H), 7.31 - 7.36 (m, 5H), 7.70 (d, J = 7.5, 2H).

### Step C. Preparation of Nα-isobutyl-Nα-(4-methylbenzenesulfonyl)-Nδ-(9-fluorenylmethoxycarbonyl)-DL-ornithine

The title compound is prepared by following the indications of step D of example 35 using the product obtained in step B of this example and reacting it with N-(9-fluorenylmethoxycarbonyloxy) succinimide.

¹H NMR (DMSO-d₆): 0.77 (d, J = 7.2, 3H), 0.80 (d, J = 7.1, 3H), 1.30 - 1.52 (m, 3H), 1.68 - 1.90 (m, 2H), 2.35 (s, 3H), 2.85 and 2.95 (ABX, J = 14.0, 7.3, 2H), 2.90 (m, 2H), 4.20 (m, 2H), 4.30 (d, J = 6.4, 2H), 7.20 - 7.42 (m, 7H), 7.60 (m, 4H), 7.88 (d, J = 7.5, 2H), 12.65 (br s, 1H).

### Example 49. Preparation of Nα-isobutyl-Nα-benzoyl-N∈-(9-fluorenylmethoxycarbonyl)-Llysine (compound no. 46)

To a stirred solution of *N*α-isobutyl-*N*∈-benzyloxycarbonyl-L-lysine (213 mg, 0.50 mmol) in CH₂Cl₂ (5 mL) is added benzoyl chloride (140 mg, 1.00 mmol) and DIEA (130 mg, 1.00 mmol). The reaction mixture is stirred at room temperature for 1 h and then diluted with 1N HCl The mixture isextracted with EtOAc, dried over MgSO₄ and evaporated to dryness. The residue is purified by flash chromatography. Elution with 70% EtOAc in hexane provided *N*α- isobutyl-*N*α-benzoyl-*N*∈-benzyloxycarbonyl-L-lysine that is further hydrogenolyzed using 10% Pd/C and then treated with 9-fluorenylmethyl chloroformate instead of N-(9-fluorenylmethoxycarbonyloxy) succinimide as outlined in step D of example 38 to provide the title compound (90% yield).

¹H NMR (DMSO-d₆): 0.70 (d, J = 6.3, 3H), 0.89 (d, J 6.5, 3H), 1.22 - 1.55 (m, 4H), 1.62 - 1.90 (m, 3H), 2.90 - 3.22 (m, 4H), 3.92 - 4.12 (m, 1H), 4.20 (t, J = 6.8, 1H), 4.28 (t, J = 6.2, 2H), 7.20 - 7.45 (m, 10H), 7.70 (d, J = 7.2, 2H), 7.90 (d, J = 7.5, 2H), 12.50 (s, 1H).

### Example 50. Preparation of Nα-benzyl-Nα-(4-methylbenzenesulfonyl)-N∈-(9-fluorenylmethoxycarbonyl)-L-lysine (compound no. 40)

### Step A. Preparation of Nα-benzyl-N∈-benzyloxycarbonyl-L-lysine benzyl ester

The title compound is prepared by reacting *N*∈-benzyloxycarbonyl-L-lysine benzyl ester according to the indications of step B of example 38 using benzaldehyde instead of isobutyraldehyde.

¹H NMR (CDCl₃): 1.30 - 1.52 (m, 4H), 1. 60 - 1.74 (m, 2H), 3.15 (m, 2H), 3.30 (t, J = 6.5, 1H), 3.60 and 3.80 (AB, J = 16.7, 2H), 4.76 (br s, 1H), 5.11 (s, 2H), 5.18 (q, J = 11.7, 2H), 7.22 - 7.40 (m, 15H).

### Step B. Preparation of Nα-benzyl-Nα-(4-methylbenzenesulfonyl)-N∈-benzyloxycarbonyl-L-lysine benzyl ester

The product obtained in step A of this example is treated as described in step C of example 38 with 4-methylbenzenesulfonyl chloride to yield the title compound.

¹H NMR (CDCl₃): 1.05 - 1.32 (m, 4H), 1.45 - 1.58 (m, 1H), 1.72 - 1.80 (m, 1H), 2.40 (s, 3H), 3.00 (m, 2H), 4.31 and 4.70 (AB, J = 16.1, 2H), 4.57 (dd, J = 9.0, 5.7, 1H), 4.70 (d, J = 16.1, 1H), 4.80 (br s, 1H), 4.85 (s, 2H), 5.11 (s, 2H), 7.16 - 7.37 (m, 17H), 7.68 (d, J = 7.5, 2H).

### Step C. Preparation of Nα-benzyl-Nα-(4-methylbenzenesulfonyl)-N∈-(9-fluorenylmethoxycarbonyl)-L-lysine

The title compound is prepared by following the indications of step D of example 38 using the product obtained in step B of this example and reacting it with N-(9-fluorenylmethoxycarbonyloxy) succinimide.

¹H NMR (DMSO-d₆): 1.00 - 1.20 (m, 4H), 1.27 - 1.40 (m, 1H), 1.55 - 1.62 (m, 1H), 2.37 (s, 3H), 2.75 (m, 2H), 4.20 (t, J = 6.5, 1H), 4.25 - 4.30 (m, 3H), 4.33 and 4.65 (AB, J = 16.4, 2H), 7.15(t, J = 5.2, 1H), 7.20 - 7.42 (m, 11H), 7.67 (d, J = 7.3, 4H), 7.88 (d, J = 7.5, 2H), 12.70 (br s, 1H).

### Example 51. Preparation of Nα-cyclopropylmethyl-Nα-(4-methylbenzenesulfonyl)-N∈-(9-fluorenylmethoxycarbonyl)-L-lysine (compound no. 43)

### Step A. Preparation of Nα-cyclopropylmethyl-N∈-benzyloxycarbonyl-L-lysine benzyl ester.

The title compound is prepared by reacting *N*∈-benzyloxycarbonyl-L-lysine benzyl ester according to the indications of step B of example 38 using cyclopropylcarboxaldehyde instead of isobutyraldehyde.

¹H NMR (CDCl₃): 0.00 - 0.07 (m, 2H), 0.41 - 0.47 (m, 2H), 0.86 - 0.93 (m, 1H), 1.22 - 1.70 (m, 6H), 1.78 (br s, 1H), 2.20 and 2.50 (ABX, J = 12.0, 8.1, 2H), 3.10 (m, 2H), 3.30 (m, 1H), 5.00 (br s, 1H) , 5.12 (s, 2H), 5.15 and 5.18 (AB, J = 12.7, 2H), 7.30 - 7.36 (m, 10H).

### Step B. Preparation of Nα-cyclopropylmethyl-Nα-(4-methylbenzenesulfonyl)-N∈-benzyloxycarbonyl-L-lysine benzyl ester.

The product obtained in step A of this example is treated as described in step C of example 38 with 4-methylbenzenesulfonyl chloride to yield the title compound.

¹H NMR (CDCl₃) : 0.04 (m, 1H) , 0.15 (m, 1H) , 0.41 (d, J = 7.7, 2H), 0.90 (m, 1H), 1.22 - 1.60 (m, 4H), 1.65 - 1.80 (m, 1H), 1.90 - 2.03 (m, 1H) , 2.35 (s, 3H), 2.90 and 3.20 (ABX, J = 15.3, 7.2, 2H), 3.15 (m, 2H), 4.58 (dd, J = 9.1, 5.4, 1H), 4.90 (s, 2H), 5.00(br s, 1H), 5.10(s, 2H), 7.10 - 7.40(m, 12H), 7.67 (d, J = 8.5, 2H).

### Step C. Preparation of Nα-benzyl-Nα-(4-methylbenzenesulfonyl)-N∈-(9-fluorenylmethoxycarbonyl)L-lysine.

The title compound is prepared by following the indications of step D of example 38 using the product obtained in step B of this example and reacting it with N-(9-fluorenylmethoxycarbonyloxy) succinimide.

¹H NMR (DMSO-d₆): 0.12 (m, 1H), 0.21 (m, 1H), 0.40 (d, J = 7.8, 2H), 0.95 - 1.03 (m, 1H), 1.17 - 1.45 (m, 4H), 1.55 - 1.68 (m, 1H), 1.80 - 1.90 (m, 1H), 2.90 and 3.20 (ABX, J = 15.4, 5.8, 2H), 2.95 (m, 2H), 4.20 (t, J = 6.5, 1H), 4.29 (d, J = 6.6, 2H), 7.25 (t, J = 5.4, 1H), 7.30 - 7.45 (m, 6H), 7.69 (d, J = 7.5, 4H), 7.88 (d, J = 7.4, 2H), 12.70 (br s, 1H).

### Example 52. Preparation of Nα,N∈-di-(9-fluorenylmethoxycarbonyl)-L-lysine (compound no. 71)

The reaction of 9-fluorenylmethyl chloroformate with L-lysine according to the conditions described in example 2 provided the title product in 71% yield.

¹H NMR (DMSO-d₆): 1.20 - 1.50 (m, 4H), 1.55 - 1.78 (m, 1H), 3.00 (m, 2H), 3.92 (m, 1H), 4.20 (t, J = 6.3, 2H) , 4.29 (d, J = 7.0, 4H), 7.27 (t, J = 5.3, 1H), 7.29 - 7.42 (m, 8H), 7.60 (d, J = 7.9, 1H), 7.67 - 7.73 (m, 4H), 7.88 (m, 4H), 12.50 (br s, 1H).

### Example 53. Preparation of NαNδ-di-(9-fluorenylmethoxycarbonyl)-L-ornithine (compound no. 73)

The reaction of 9-fluorenylmethyl chloroformate with L-ornithine according to the conditions described in example 2 provided the title product in 79% yield.

¹H NMR (DMSO-d₆): 1.42 - 1.80 (m, 4H), 3.00 (m, 2H), 3.94 (m, 1H), 4.20 (t, J = 6.3, 2H), 4.29 (d, J = 7.0, 4H), 7.28 (t, J = 5.2, 1H), 7.30 - 7.48 (m, 8H), 7.63 (d, J = 7.6, 1H) , 7.67 - 7.73 (m, 4H), 7.88 (m, 4H), 12.50 (br s, 1H).

### Example 54. Preparation of Nα-(4-nitrobenzenesulfonyl)-N∈-(9- fluorenylmethoxycarbonyl)-L-lysine (compound no. 103)

*N*α-*tert*-butoxycarbonyl-*N*∈-(9-fluorenylmethoxycarbonyl)-L-lysine is deprotected at the a position by treatment with TFA/CH₂Cl₂ as described in the procedure outlined in example 24 and the resulting trifluoroacetate salt is alkylated with 4-nitrobenzenesulfonyl chloride as described in example 2 affording the title compound in 51% yield.

¹H NMR (DMSO-d₆): 1.13 - 1.33 (m, 4H), 1.45 - 1.70 (m, 2H), 2.90 (m, 2H), 3.75 (dd, J = 13.0, 7.3, 1H), 4.20 (t, J = 6.3, 1H), 4.28 (d, J = 7.0, 2H), 7.20 (t, J = 5.2, 1H), 7.30 - 7.48 (m, 4H), 7.67 (d, J = 7.3, 1H), 7.88 (d, J = 7.3, 2H), 8.01 (d, J = 8.8, 2H), 8.38 (d, J = 8.1, 2H), 8.50 (d, J = 8.1, 1H).

### Example 55. Preparation of Nα-(4-chlorobenzenesulfonyl)-N∈-(9-fluorenylmethoxycarbonyl)L-lysine (compound no. 72)

*N*α-*tert*-butoxycarbonyl-*N*∈-(9-fluorenylmethoxycarbonyl)-L-lysine is deprotected at the a position by treatment with TFA/CH₂Cl₂ as described in the procedure outlined in example 24 and the resulting trifluoroacetate salt is alkylated with 4-chlorobenzenesulfonyl chloride as described in example 2, affording the title compound in 38% yield.

¹H NMR (DMSO-d₆): 1.12 - 1.38 (m, 4H), 1.42 - 1.65 (m, 2H), 2.90 (m, 2H), 3.67 (dd, J = 13.0, 7.7, 1H), 4.20 (t, J = 6.5, 1H), 4.29 (d, J = 6.9, 2H), 7.20 (t, J = 5.2, 1H), 7.30 - 7.42 (m, 4H), 7.62 (d, J = 7.3, 1H), 7.67 (d, J = 7.9, 2H), 7.75 (d, J = 7.9, 2H), 7.88 (d, J = 8.2, 2H), 8.23 (d, J = 8.9, 1H).

### Example 56. Preparation of Nα-(4-chlorobenzenesulfonyl)-Nδ-(9-fluorenylmethoxycarbonyl)-L-ornithine (compound no. 74)

*N*α-*tert*-butoxycarbonyl-*N*δ-(9-fluorenylmethoxycarbonyl)-L-ornithine is deprotected at the a position by treatment with TFA/CH₂Cl₂ as described in the procedure outlined in example 24 and the resulting trifluoroacetate salt is alkylated with 4-chlorobenzenesulfonyl chloride as described in example 2, affording the title compound in 33% yield.

¹H NMR (DMSO-d₆): 1.32 - 1.52 (m, 3H), 1.56 - 1.68 (m, 1H), 2.90 (m, 2H), 3.70 (dd, J = 13.1, 7.2, 1H), 4.20 (t, J = 6.3, 1H), 4.28 (d, J = 6.7, 2H), 7.26 (t, J = 5.1, 1H), 7.31 - 7.45 (m, 4H), 7.60 (d, J = 8.3, 2H), 7.67 (d, J = 7.3, 2H), 7.75 (d, J = 8.3, 2H), 7.87 (d, J = 7.2, 2H), 8.25 (d, J = 8.9, 1H).

### Example 57. Preparation of Nα-(2-nitrobenzenesulfonyl)-N∈-(9-fluorenylmethoxycarbonyl)-L-lysine (compound no. 107)

*N*α-*tert*-butoxycarbonyl-*N*∈-(9-fluorenylmethoxycarbonyl)-L-lysine is deprotected at the a position by treatment with TFA/CH₂Cl₂ as described in the procedure outlined in example 24 and the resulting trifluoroacetate salt is alkylated with 2-nitrobenzenesulfonyl chloride as described in example 2, affording the title compound in 48% yield.

¹H NMR (DMSO-d₆): 1.32 - 1.52 (m, 3H), 1.56 - 1.68 (m, 1H), 2.90 (m, 2H), 3.70 (dd, J = 13.1, 7.2, 1H), 4.20 (t, J = 6.3, 1H), 4.28 (d, J = 6.7, 2H), 7.26 (t, J = 5.1, 1H), 7.31 - 7.45 (m, 4H), 7.60 (d, J = 8.3, 2H), 7.67 (d, J = 7.3, 2H), 7.75 (d, J = 8.3, 2H), 7.87 (d, J = 7.2, 2H), 8.25 (d, J = 8.9, 1H) .

### Example 58. Preparation of Nα-(4-bromobenzenesulfonyl)-N∈-(9-fluorenylmcthoxycarbonyl)-L-lysine (compound no. 66)

*N*α-*tert*-butoxycarbonyl-*N*∈-(9-fluorenylmethoxycarbonyl)-L-lysine is deprotected at the a position by treatment with TFA/CH₂Cl₂ as described in the procedure outlined in example 24 and the resulting trifluoroacetate salt is alkylated with 4-bromobenzenesulfonyl chloride as described in example 2, affording the title compound in 65% yield.

¹H NMR (DMSO-d₆) : 1.15 - 1.38 (m, 4H), 1.42 - 1.55 (m, 2H), 2.90 (m, 2H), 3.67 (dd, J = 12.0, 5.6, 1H), 4.20 (t, J = 7.0, 1H), 4.27 (d, J = 7.0, 2H), 7.20 (t, J = 5.0, 1H), 7.30 - 7.90 (m, 12H), 8.24 (d, J = 8.8, 1H), 12.50 (br s, 1H).

### Example 59. Preparation of Nα-(1-naphthalenesulfonyl)-N∈-(9-fluorenylmethoxycarbonyl)-L-lysine (compound no. 102)

*N*α-*tert*-butoxycarbonyl-*N*∈-(9-fluorenylmethoxycarbonyl)-L-lysine is deprotected at the a position by treatment with TFA/CH₂Cl₂ as described in the procedure outlined in example 24 and the resulting trifluoroacetate salt is alkylated with 1-naphthalenebenzenesulfonyl chloride as described in example 2, affording the title compound in 71% yield.

¹H NMR (DMSO-d₆): 1.15 - 1.38 (m, 4H), 1.42 - 1.63 (m, 2H), 2.80 (m, 2H), 3.61 (m, 1H), 4.20 (t, J = 7.0, 1H), 4.27 (d, J = 7.0, 2H), 7.17 (t, J = 5.0, 1H), 7.25 - 8.13 (m, 15H), 8.40 (s, 1H), 12.40 (br s, 1H).

### Example 60. Preparation of Nα-(4-methoxylbenzenesulfonyl)-N∈-(9-fluorenylmethoxycarbonyl)-L-lysine (compound no. 104)

*N*α-*tert*-butoxycarbonyl-*N*∈- (9-fluorenylmethoxycarbonyl)-L-lysine is deprotected at the α position by treatment with TFA/CH₂Cl₂ as described in the procedure outlined in example 24 and the resulting trifluoroacetate salt is alkylated with 4-methoxybenzenesulfonyl chloride as described in example 2, affording the title compound in 65% yield.

¹H NMR (DMSO-d₆): 1.10 - 1.40 (m, 4H), 1.42 - 1.60 (m, 2H), 2.86 (m, 2H), 3.60 (m, 1H), 3.80 (s, 3H), 4.20 (t, J = 7.0, 1H), 4.27 (d, J = 7.0, 2H), 7.05 (d, J = 8.5, 2H), 7.20 (t, J = 5.0, 1H), 7.25 - 7.90 (m, 11H), 12.50 (br s, 1H).

### Example 61. Preparation of Nα-(4-aminobenzenesulfonyl)-N∈-(9-fluorenylmethoxycarbonyl)-L-lysine (compound no. 106)

The product of example 54 is hydrogenolized following the conditions found in example 4 affording the title compound in 90% yield.

¹H NMR (DMSO-d₆): 1.12 - 1.38 (m, 4H), 1.42 - 1.60 (m, 2H), 2.90 (m, 2H), 3.42 (m, 1H), 4.20 (t, J = 7.0, 1H), 4.27 (d, J = 7.0, 2H), 5.86 (s, 2H), 6.55 (d, J = 8.6, 2H), 7.20 (t, J = 5.0, 1H), 7.25 - 7.90 (m, 11H), 12.35 (br s, 1H).

### Example 62. Preparation of Nα-(2-aminobenzenesulfonyl)-N∈-(9-fluorenylmethoxycarbonyl)-L-lysine (compound no. 105)

The product of example 57 is hydrogenolized following the conditions found in example 4 affording the title compound in 88% yield.

¹H NMR (DMSO-d₆): 1.12 - 1.38 (m, 4H), 1.48 - 1.60 (m, 2H), 2.80 (m, 2H), 3.55 (m, 1H), 4.20 (t, J = 7.2, 1H), 4.27 (d, J = 7.0, 2H), 5.88 (s, 2H), 6.55 (t, J = 7.4, 1H), 6.76 (d, J = 7.8, 1H), 7.16 (t, J = 5.0, 1H), 7.22 (t, J = 7.4, 1H), 7.30 - 7.92 (m, 10H), 12.60 (br s, 1H).

### Example 63. Preparation of Nα-(9-fluorenylmethoxycarbonyl)-N∈-isobutyl-N∈-(4-bromobenzenesulfonyl)-L-lysine (compound no. 21)

### Step A. Preparation of Nα-tert-butoxycarbonyl-N∈-isobutyl-N∈- (4-bromobenzenesulfonyl)-L-lysine methyl ester

To a stirred solution of *Nα-tert*-butoxycarbonyl-*N∈-*benzyloxycarbonyl-L-lysine methyl ester (380 mg, 1.00 mmol) in MeOH (5 mL) is added 10% Pd/C (70 mg), followed by isobutyraldehyde (91 µL, 2.0 mmol). This suspension is maintained under hydrogen atmosphere for 1 h. The solids are filtered off and to the filtrate is added triethylamine (210 µL, 1.50 mmol) and 4-bromobenzenesulfonyl chloride (765 mg, 3.00 mmol) in 3 portions (1.00 mmol per hour). The reaction mixture is concentrated, diluted with 1N HCl and extracted with EtOAc. The organic layer is dried (MgSO₄) and concentrated *in vacuo*. The residue is purified by flash chromatography eluting with 25% EtOAc in hexane to yield 444 mg (83%) of the title compound.

¹H NMR (DMSO-d₆) : 0.83 (d, J = 6.0, 6H), 1.18 - 1.30 (m, 2H), 1.32 - 1.48 (m, 2H), 1.37 (s, 9H), 1.50 - 1.65 (m, 2H), 1.84 (m, 1H), 2.83 (d, J = 7.4, 2H) , 3. 00 (m, 2H), 3.60 (s, 3H) , 3.91 (m, 1H), 7.18 (d, J = 7.6, 1H), 7.71 (d, J = 7.9, 2H), 7.80 (d, J = 8.1, 2H).

### Step B. Preparation of Nα-(9-fluorenylmethoxycarbonyl)-N∈-isobutyl-N∈-(4-bromobenzenesulfonyl)-L-lysine

The product from step A of this example is reacted utilizing the conditions found in step D of example 38 to yield 67% of the title compound.

¹H NMR (DMSO-d₆): 0.80 (d, J =, 6H), 1.20 - 1.50 (m, 4H), 1.52 - 1.70 (m, 2H), 1.75 - 1. 84 (m, 1H) , 2.82 (d, J = 7.3, 2H), 3.00 (m, 2H), 3.90 (m, 2H), 4.23 (t, J = 6.8, 1H), 4.27 (d, J = 6.7, 2H), 7.33 (t, J = 7.4, 2H), 7.40 (t, J = 7.4, 2H), 7.59 (d, J = 8.1, 1H), 7.72 (m, 4H), 7.79 (d, J = 8.1, 2H), 7.89 (d, J = 7.8, 2H), 12.55 (br s, 1H).

### Example 64. Preparation of Nα-(9-fluorenylmethoxycarbonyl)-Nδ-isobutyl-Nδ-(4-bromobenzenesulfonyl)-L-ornithine (compound no. 41)

### Step A. Preparation of Nα-tert-butoxycarbonyl-Nδ-isobutyl-Nδ-(4-bromobenzenesulfonyl)-L-ornithine methyl ester

Following the indications of example 63 substituting *N*α-*tert-* butoxycarbonyl-*N*∈-benzyloxycarbonyl-L-lysine methyl ester with *N*α-*tert*-butoxycarbonyl-*N*δ-benzyloxycarbonyl-L-ornithine methyl ester, the title compound is obtained in 72% yield.

¹H NMR (DMSO-d₆) : 0.88 (d, J = 6.0, 3H), 0.89 (d, J = 6.0, 3H), 1.44 (s, 9H), 1.55 - 1.88 (m, 4H), 1.90 (h, J = 6.1, 1H), 2.86 (d, J = 7.5, 2H), 3.10 (d, J = 6. 3, 2H), 3.73 (s, 3H), 4.25 (br s, 1H) , 5.05 (d, J = 7.5, 1H), 7.65 (s, 4H).

### Step B. Preparation of Nα-(9-fluorenylmethoxycarbonyl)-Nδ-isobutyl-Nδ-(4-bromobenzenesulfonyl)-L-ornithine.

The product from step A of this example is reacted utilizing the conditions found in step D of example 38 to yield 63% of the title compound.

¹H NMR (DMSO-d₆) : 0.79 (d, J =, 3H) , 0.81 (d, J = 6. 0, 3H) , 1.47 - 1.61 (m, 3H), 1.63 - 1.76 (m, 1H), 1.85 (h, J = 6.1, 1H), 2.81 (d, J = 7.3, 2H), 3.05 (m, 2H), 3.92 (m, 1H), 4.22 (t, J = 7.2, 1H), 4.28 (d, J = 7.2, 2H), 7.28 - 7.45 (m, 4H), 7.65 (d, J = 8.0, 1H), 7.72 (d, J = 7.4, 4H), 7.78 (d, J = 8.7, 2H), 7.88 (d, J = 7.5, 2H), 12.55 (br s, 1H).

### Example 65. Preparation of Nα-(9-fluorenylmethoxycarbonyl)-N∈-(2-fluorobenzenesulfonyl)-L-lysine (compound no. 167)

*N*α-(9-fluorenylmethoxycarbonyl)-*N*∈-*tert*-butoxycarbonyl-L-lysine (234 mg, 0.50 mmol) is treated with TFA/CH₂Cl₂ to remove the *tert*-butoxycarbonyl and the product obtained from evaporating off the volatiles is reacted with 2-fluorobenzenesulfonyl chloride under the conditions indicated in example 2 to afford a 67% yield of the title compound.

¹H NMR (DMSO-d₆): 1.20 - 1.70 (m, 6H), 2.80 (dd, J = 12.8, 6.9, 2H), 3.84 (m, 1H), 4.20 (t, J = 7.0, 1H), 4.28 (d, J = 6.9, 2H), 7.30 - 7.57 (m, 7H), 7.66 - 7.88 (m, 6H), 7.98 (d, J = 7.5, 1H).

### Example 66. Preparation of Nα-(9-fluorenylmethoxycarbonyl)-Nδ-(1-naphthalenesulfonyl)-L-ornithine (compound no. 168)

*N*α-(9-fluorenylmethoxycarbonyl)-*N*δ-*tert*-butoxycarbonyl-L-ornithine (234 mg, 0.50 mmol) is treated with TFA/CH₂Cl₂ to remove the *tert*-butoxycarbonyl and the product obtained from evaporating off the volatiles is reacted with 1-naphthalenesulfonyl chloride under the conditions indicated in example 2 to afford a 50% yield of the title compound.

¹H NMR (DMSO-d₆): 1.38 - 1.62 (m, 3H), 1.65 - 1.80 (m, 1H), 2.75 (dd, J = 13.0, 6.9, 2H), 3.78 (m, 1H), 4.21 (t, J = 6.9, 1H), 4.27 (d, J = 6.9, 2H), 7.30 - 7.43 (m, 4H), 7.58 (d, J = 7.7, 1H), 7.71 (m, 4H), 7.79 (d, J = 8.1, 2H), 7.89 (d, J = 7.3, 12), 12.30 (br s, 1H).

### Example 67. Preparation of (S)-2-(9-fluorenylmethoxycarbonylamino)-4-(4-bromobenzenesulfonylamino)-butanoic acid (compound no. 14)

### Step A. Preparation of N-tert-butoxycarbonyl-L-homoserine methyl ester

To a stirred solution of L-homoserine (1.0 g, 8.4 mmol) in dioxane/water (35 mL/70 mL) is added sodium hydroxide (738 mg; 18.5 mmol). After stirring for 5 min, di-tert-butyl dicarbonate (2.20 g, 10.0 mmol) is added in one portion and the mixture is stirred for 2 h and then diluted with 1N HCl (pH ~ 3) and extracted twice with EtOAc. The combined organic layers are dried over magnesium sulfate and concentrated. The crude is diluted in MeOH and CH₂N₂ in ether is added until the yellow colour persisted. Excess diazomethane is destroyed by the addition of AcOH. The mixture is concentrated *in vacuo* to afford a colorless oil that is flash chromatographed eluting with 60% EtOAc in hexane to yield 1.4 g (71%) of the title compound.

¹H NMR (CDCl₃): 1.43 (s, 9H), 1.60 - 1.72 (m, 1H), 2.10 - 2.22 (m, 1H), 2.60 - 2.76 (m, 2H), 2.74 (s, 3H), 4.50 (br s, 1H), 5.42 (br s, 1H).

Step B. Preparation of (*S*)-2-*tert*-butoxycarbonylamino-4-azido-butanoic acid methyl ester.

4-Methylbenzenesulfonyl chloride (572 mg, 3.00 mmol) is added to a stirred solution of the product of step A of this example in a mixture of pyridine/CH₂Cl₂ (7.5 mL/7.5 mL). The mixture is stirred at room temperature until complete disappearance of the starting material and is then diluted with 10% HCl, and extracted with CH₂Cl₂. The organic layer is dried over MgSO₄ and concentrated *in vacuo*. The residue is diluted in DMF to which is added sodium azide (260 mg, 4.00 mmol). The suspension is heated at 70°C for 3h, cooled to room temperature, diluted with 1N HCl and extracted with EtOAc. The organic layer is washed with brine, dried over MgSO₄ and concentrated *in vacuo*. The residue is purified by flash chromatography eluting with 30% EtOAc in hexane to afford 470 mg (91%) of the title compound.

¹H NMR (CDCl₃): 1.42 (s, 9H), 1.82 - 1.92 (m, 1H), 2.07 - 2.15 (m, 1H), 3.38 (t, J = 6.0, 2H), 3.74 (s, 3H), 4.38 (br s, 1H), 5.24 (br s, 1H).

### Step C. Preparation of methyl (S)-2-tert-butoxycarbonylamino-4-(4-bromobenzenesulfonylamino)butanoate

To a stirred solution of the product of step B of this example (520 mg, 2.00 mmol) in EtOAc (6 mL) is added 10% Pd/C (60 mg). The suspension is stirred under hydrogen for 1 h, filtered and concentrated *in vacuo.* The residue is diluted with THF (6 mL) and 4-bromobenzenesulfonyl chloride (613 mg, 2.40 mmol) is added followed by triethylamine (557 µL, 4.00 mmol). The mixture is stirred for 3 h and then acidified with 1N HCl and extracted with EtOAc. The organic layer is dried over MgSO₄ and concentrated *in vacuo*. The crude material is purified by flash chromatography eluting with 30% EtOAc in hexane to afford 770 mg (85%) of the title compound.

¹H NMR (DMSO-d₆): 1.35 (s, 9H), 1.65 - 1.72 (m, 1H), 1.75 - 1.85 (m, 1H), 2.35 - 2.42 (m, 2H), 3.90 (m, 1H), 7.10 (d, J = 6.3, 1H), 7.70 (d, J = 7.0, 2H), 7.80 (d, J = 7.0, 2H), 12.40 (br s, 1H).

### Step D. Preparation of (S)-2-(9-fluorenymethoxycarbonylamino)-4-(4-bromobenzenesulfonylamino)butanoic acid

A solution of the product of step C of this example (225 mg, 0.50 mmol) in TFA/CH₂Cl₂ (2 mL/2 mL) is stirred for 2 h, then concentrated under reduced pressure. The residue is dissolved in THF/H₂O (1 mL/ 1 mL) to which is added sodium carbonate (159 mg, 1.50 mmol) and 9-fluorenylmethyl chloroformate (155 mg, 0.60 mmol). The mixture is stirred for 1 h, then 1M sodium hydroxide (0.5 mL) is added. After stirring for 30 min, the reaction mixture is acidified with 1N HCl and extracted twice with EtOAc. The combined organic layers are dried over MgSO₄ and concentrated *in vacuo*. The residue is purified by flash chromatography eluting with 5% MeOH in CH₂Cl₂ to afford 187 mg (67%) of the title compound.

¹H NMR (DMSO-d₆): 1.70 - 1.80 (m, 1H), 1.82 - 1.90 (m, 1H), 2.78 - 2.87 (m, 2H), 3.95 (m, 1H), 4.18 - 4.27 (m, 3H), 7.28 - 7.45 (m, 4H), 7.50 (d, J = 7.0, 1H), 7.72 - 7.92 (m, 9H).

### Example 68. Preparation of (S)-2-(9-fluorenylmethoxycarbonylamino)-3-(4-bromobenzenesulfonylamino)-propanoic acid (compound no. 18)

### Step A. Preparation of (S) - tert-butoxycarbonylamino-β-propiolactone

DEAD (1.76 g, 10.0 mmol) is added to a cold (-78°C) solution of triphenylphosphine (2.62 g, 10.0 mmol) in THF (30 mL). The mixture is stirred for 15 min and a solution of tert-butoxycarbonyl L-serine (2.05, 10.0 mmol) in acetonitrile (10 mL) is added. The mixture is stirred for 30 min then allowed to warm up to room temperature. The solvent is then removed under reduced pressure and the crude is purified by flash chromatography eluting with 30% EtOAc in hexane to afford 1.37 g (73%) of the title compound.

¹H NMR (CDCl₃) : 1.43 (s, 9H), 4.40 - 4.50 (m, 2H), 5.10 (br s, 1H), 5.45 (br s, 1H) .

### Step B. Preparation of (S)-2-tert-butoxycarbonylamino-3-(4-bromobenzenesulfonylamino)-propionic acid

To a stirred solution of the product prepared in step A of this example (561 mg, 4.00 mmol) in CH₃CN (20 mL) is added NH₃ (2M solution in EtOH, 10 mL). The mixture is stirred at 0°C for 2 h and then at room temperature. After 3 h, it is concentrated and rediluted with dioxane (10 mL). To this solution is added 4-bromobenzenesulfonyl chloride (2.04 g, 8.00 mmol) followed by 1M Na₂CO₃ (8 mL). The reaction mixture is vigorously stirred for 2 h and then acidified with 1N HCl and extracted with EtOAc. The organic layer is dried over MgSO₄ and concentrated *in vacuo*.

The crude material is purified by flash chromatography eluting with 5% MeOH in CH₂Cl₂ containing 0.5% AcOH, affording 500 mg (30%) of the title compound.

H NMR (DMSO-d₆) : 1.34 (s, 9H), 2.96 - 3.12 (m, 2H), 3.90 (m, 1H), 6.65 (br s, 1H) , 7.70 (d, J = 7.2, 2H) , 7.80 (d, J = 7.0, 2H).

### Step C. Preparation of (S)-2-(9-fluorenylmethoxycarbonylamino)-3-(4-bromobenzenesulfonylamino)-propionic acid

A solution of the acid prepared in step B of this example (50 mg, 0.12 mmol) in TFA/CH₂Cl₂ (1 mL/1 mL) is stirred for 1 h and concentrated *in vacuo*. The residue is taken up in a mixture of 1M Na₂CO₃ and dioxane (1 mL/1 mL), to which is added 9-fluorenylmethyl chloroformate (37 mg, 0.10 mmol). The reaction mixture is stirred for Ih and then diluted with 1N HCl and extracted with EtOAc. The organic layers are dried over MgSO₄ and concentrated *in vacuo*. The residue is purified by flash chromatography eluting with 10% MeOH in CH₂Cl₂ containing 1% AcOH affording 42 mg (62%) of the title compound.

¹H NMR (DMSO-d₆): 2.95 - 3.03 (m, 1H), 3.08 - 3.15 (m, 1H), 3.78 - 3.85 (m, 1H), 4.20 - 4.31 (m, 3H), 7.00 (br s, 1H), 7.25 - 7.50 (m, 4H), 7.68 - 7.92 (m, 9H), 12.30 (br s, 1H).

### Example 69. Preparation of Nα-isobutyl-Nα-(4-nitrobenzenesulfonyl)-N∈- (3-indolepropionyl)-L-lysine (compound no. 95)

### Step A. Preparation of L-Nα-isobutyl-∈-caprolactam

L-α-amino-∈-caprolactam (6.0 g, 47 mmol) is dissolved in MeOH (300 mL) containing AcOH (3.5 mL). Isobutyraldehyde (3.0 g, 50 mmol) is added to the solution followed by sodium cyanoborohydride (3.3 g, 50 mmol). The mixture is stirred at room temperature for 2 h after which MeOH is removed *in vacuo*. 1M K₂CO₃ (30 mL) is added to the residue which is then extracted with two 100 mL portions of EtOAc. The organic layer is dried with MgSO₄, filtered and concentrated *in vacuo*. The crude residue, which contains traces of dialkylated product is taken up in hot EtOH (8 mL) and diluted with ~300 mL ice cold ether until two phases began to appear. 10 mL of trimethylsilyl chloride is then added slowly which gave a precipitate of pure product which is filtered and dried under vacuum affording 9.57g (95%) of the title compound as the HCl salt. The salt is suspended in 200 mL EtOAc and 20% NaOH slowly until the solid disappears. The organic layer is dried with MgSO₄ and concentrated *in vacuo* to give 7.55 g (91%) of a thick oil which crystallized on standing. MP 52-54°C.

¹H NMR (CDCl₃): 0.93 (d, J = 6.8, 3H), 0.97 (d, J = 6.5, 3H), 1.39.(t, J = 9.8, 1H), 1.47 (m, 1H), 1.61 (m, 1H), 1.65 - 1.78 (m, 2H), 1.93 - 2.01 (m, 2H), 2.20 - 2.32 (m, 2H), 2.38 (t, J = 9.7, 1H), 3.16 (m, 3H), 6.62 (s, 1H).

### Step B. Preparation of L-Nα-isobutyl-Nα-(4-nitrobenzenesulfonyl)-∈-caprolactam

To the product from step A of this example (4.14 g, 22.5 mmol) dissolved in CH₂Cl₂ (50 mL) is added diisopropylethyl amine (6.00 mL, 30.0 mmol) and 4-nitrobenzenesulfonyl chloride (5.09 g, 23.0 mmol). The mixture is stirred overnight. Afterwards, the solution is acidified with 1N HCl and extracted with EtOAc. The organic layer is dried and concentrated *in vacuo*. The residue is recrystallized from MeOH. The thin needles are filtered off and air dried giving 6.9g (83%) of the pure title product. MP 152-154°C.

¹H NMR (CDCl₃) : 0.93 (d, J = 6.0, 3H) , 0.96 (d, J = 6.0, 3H), 1.39 (t, J = 12.0, 1H), 1.65 - 1.85 (m, 3H), 2.08 - 2.18 (m, 3H), 3.06 (dd, J = 14.3, 8.5, 1H), 3.35 (dd, J = 14.2, 8.5, 1H), 4.65 (d, J = 8.7, 1H), 5.7 (s, 1H), 7.92 (d, J = 8.8, 2H), 8.3 (d, J = 8.8, 2H).

### Step C. Preparation of Nα-isobutyl-Nα-(4-nitrobenzenesulfonyl)-L-lysine hydrochloride

The product of step B of this example (1.0 g, 2.7 mmol) is dissolved in AcOH (4 mL). This solution is added to 12N HCl and the mixture isrefluxed for 2 h until all solids had disappeared. The solution is evaporated *in vacuo* to give 1.12 g (quantitative yield) of the desired product as its hydrochloride salt.

¹H NMR (DMSO-d₆ + 10% D₂O): 0.79 (d, J = 6.8, 3H), 0.86 (d, J = 6.8, 3H), 1.25 (t, J = 11.9, 2H), 1.28 - 1.32 (m, 2H), 1.45 - 1.51 (m, 2H), 1.75 - 1.85 (m, 2H), 1.70 (m, 1H), 2.83 - 2.87 (m, 1H), 3.03 - 3.07 (m, 1H), 4.21 (t, J = 10.1, 1H), 8.10 (d, J = 7.9, 2H), 8.37 (d, J = 7.9, 2H).

### Step D. Preparation of Nα-isobutyl-Nα-(4-nitrobenzenesulfonyl)-N∈-(3-indolepropionyl)-L-lysine

The product of step C of this example (100 mg, 0.24 mmol) is weighed in the Bohdahn robotic reaction vessels. 3.3M Cs₂CO₃ (1 mL) and THF (2 mL) are then added. The tube is then stirred vigorously and indole-3-proprionic acid (80 mg, 0.4 mmol), activated by carbonyl diimidazole (65 mg, 0.4 mmol) in THF (1 mL), is added. Gas evolution is observed. The stirring continued for 2 h. EtOAc (3 mL) is then added and the organic phase is removed. This phase is washed with 1N HCl and the organic phase isconcentrated *in vacuo* giving a very crude product which is purified by flash chromatography to yield 140 mg of the title product 90%).

1H NMR (DMSO-d₆) : 0.79 (d, J = 6.8, 3H) , 0.86 (d, J = 6.8, 3H), 0.91 (m, 1H), 1.25 (t, J = 10.6, 2H), 1.28 - 1.34 (m, 2H), 1.45 - 1.52 (m, 2H), 1.75 - 1.85 (m, 2H), 2.35 (t, J = 7.1, 2H), 2.60 (t, J = 7.1, 2H), 2.85 - 3.05 (m, 2H), 4.18 (t, J = 5.2, 1H), 6.85 - 6.91 (m, 1H), 6.96 - 7.11 (m, 3H), 7.22 - 7.31 (m, 2H), 7.45 (d, J = 7.9, 2H), 7.67 (d, J = 7.9, 2H).

### Example 70. Preparation of Nα-(9-fluorenylmethoxycarbonyl)-N∈-(4-bromobenzenesulfonyl)-L-lysine methyl ester (compound no. 15)

A solution of diazomethane in ether is added to a solution of N-α-(9-fluorenylmethoxycarbonyl)-*N*∈-(4-bromobenzenesulfonyl)-L-lysine (35 mg, 0.06 mmol) in MeOH (0.5 mL) until the yellow color persisted. The solvents are removed *in vacuo* and the residue is purified by flash chromatography eluting with 5% MeOH in CH₂Cl₂, affording 20 mg (55%) of the title compound.

¹H NMR (DMSO-d₆): 1.20 - 1.40 (m, 4H) , 1.51 - 1.65 (m, 2H), 2.70 (dd, J = 12.3, 6.0, 2H), 3.61 (s, 3H), 3.95 (dd, J = 13.0, 6. 1, 2H), 4.20 (t, J = 7.0, 1H), 4.30 (d, J = 7.0, 2H) , 7.30 - 7.42 (m, 4H), 7.68 - 7.72 (m, 4H), 7.80 (d, J = 8.1, 2H), 7.88 (d, J = 8.1, 2H).

### Example 71. Preparation of (2S)-(9-fluorenylmethoxycarbonylamino)-6-(4-bromobenzenesulfonylamino)-1-hexanol (compound no. 22)

### Step A. Preparation of (2S)-tert-butoxycarbonylamino-6-(4-bromobenzenesulfonylamino)-1-hexanol

To a cold (0°C) solution of the ester (240 mg, 0.50 mmol) in ether (4 mL) is added in one portion LiAlH₄ (76 mg, 2.0 mmol). The reaction mixture is stirred at 0°C for 1 h and at room temperature for an additional 30 min. The mixture is quenched with water and 1N HCl and extracted with EtOAc. The organic extract is dried over MgSO₄ and concentrated *in vacuo.* The residue is purified by flash chromatography eluting with 30% EtOAc in hexane to provide 207 mg (92%) of the title compound.

¹H NMR (DMSO-d₆) : 1.30 - 1.58 (m, 6H), 1.43 (s, 9H), 2.92 (dd, J = 12.4, 6.5, 2H), 2.70 (br s, 1H), 3.50 - 3.68 (m, 2H), 4.80 (d, J = 7.1, 1H) , 5.30 (t, J = 8.2, 1H) , 7.63 (d, J = 8.2, 2H), 7.72 (d, J = 8.0, 2H).

### Step B. Preparation of (2S)-(9-fluorenylmethoxycarbonylamino)-6-(4-bromobenzenesulfonylamino)-1-hexanol.

A solution of the alcohol from step A of this example in TFA/CH₂Cl₂ (1 mL/1 mL) is stirred for 1 h and then concentrated *in vacuo*. The residue is taken up in a mixture of THF and 1M K₂CO₃ (1 mL/1 mL). To this solution is added 9-fluorenylmethyl chloroformate (103 mg, 0.40 mmol) and the mixture is stirred at room temperature for 1 h. The reaction is quenched by adding 1N HCl and is extracted with EtOAc. The organic extracts are washed with brine, dried over MgSO₄ and concentrated *in vacuo*. The residue is purified by flash chromatography eluting with 50% EtOAc in hexane, providing 142 mg (75%) of the title compound.

¹H NMR (DMSO-d₆): 1.12 - 1.50 (m, 6H), 2.70 (dd, J = 12.8, 6.8, 2H), 3.18 - 3.22 (m, 1H), 3.27 - 3.36 (m, 1H), 4.19 - 4.30 (m, 3H), 4.58 (t, J = 5.4, 1H), 6.92 (d, J = 8.5, 1H), 7.25 - 7.42 (m, 4H), 7.65 - 7.73 (m, 5H) , 7.80 (d, J = 8.6, 2H), 7.86 (d, J = 8.0, 2H).

### Example 72. Preparation of (2R,2S)-(9-fluorenylmethoxycarbonylamino)-6-(4-bromobenzenesulfonylamino)-1-hexanamide (compound no. 20)

### Step A. Preparation of (2R,2S)-tert-butoxycarbonyl-6-(4-bromobenzenesulfonylamino)-1-hexanamide

To a stirred solution of methyl (*2R, 2S) -tert-*butoxycarbonyl-6-(4-bromobenzenesulfonylamino)-1-hexanoate (415 mg, 1.00 mmol) in THF (5 mL) is added ammonium hydroxide (3 mL) and sodium hydroxide (3 mL). The mixture is stirred for 2 h, diluted with 1N HCl until acidic and extracted twice with EtOAc. The extracts are dried over MgSO₄ and concentrated *in vacuo.* Purification by flash chromatography eluting with 5% MeOH in CH₂Cl₂ afforded 350 mg (82%) of the title compound.

¹H NMR (DMSO-d₆): 1.44 (s, 9H), 1.47 - 1.90 (m, 6H), 3.20 (m, 2H), 4.12 (br s, 1H), 5.03 (m, 1H), 5.20 (br s, 1H), 5.88 (br s, 1H), 6.30 (br s, 1H), 7.20 - 7.45 (m, 4H).

### Step B. Preparation of (2R, 2S)-(9-fluorenylmethoxycarbonylamino)-6-(4- bromobenzenesulfonylamino)-1-hexanamide

The *tert*-butoxycarbonyl is removed as indicated in example 24 and the resulting salt is treated with *N*-(9-fluorenylmethoxycarbonyloxy) succinimide as in step D of example 38 to afford the title product in 67% yield.

¹H NMR (DMSO-d₆) : 1.15 - 1.62 (m, 6H), 2.70 (dd, J ₌ 12.6, 6.5, 2H), 3.85 (m, 1H), 4.20 - 4.35 (m, 3H), 6.94 (s, 1H), 7.24 (s, 1H), 7.28 - 7.42 (m, 4H), 7.68 - 7.90 (m, 8H).

### Example 73. Preparation of Nα-benzoyl-N∈-(4-bromobenzenesulfonyl)-L-lysine (compound no. 65)

### Step A. Preparation of Nα-tert-butoxycarbonyl-N∈-(4-bromobenzenesulfonylamino)-L-lysine methyl ester

The title compound is prepared by reacting *N*α-*tert-*butoxycarbonyl-*N*∈-(4-benzyloxycarbonyl)-L-lysine with diazomethane using conditions similar to those found in example 70. The product is then hydrogenolyzed (H₂, 10% Pd/C, MeOH) following indications of example 4. The product is treated under the conditions of example 2 to provide after purification by flash chromatography the title compound (72% yield).

¹H NMR (DMSO-d₆): 1.32 - 1.42 (m, 2H), 1.45 (s, 9H), 1.47 - 1.62 (m, 3H), 1.68 - 1.72 (m, 2H), 2.95 (dd, J = 13.0, 6.4, 2H), 3.74 (s, 3H), 4.28 (br s, 1H), 4.80 (t, J = 5.3, 1H), 5.07 (br s, 1H), 7.66 (d, J = 8.3, 2H), 7.73 (d, J = 8.5, 2H).

### Step B. Preparation of Nα-benzoyl-N∈-(4-bromobenzenesulfonyl)-L-lysine

The product from step A of this example (0.30 mmol) is taken up in a mixture of TFA/CH₂Cl₂ (1 mL/1 mL) for 1 h and the solution is concentrated to dryness. The crude product is dissolved in DMF (2 mL) to which is added benzoic acid, the BOP reagent (159 mg, 0.36 mmol) and DIEA (156 µL, 0.90 mmol). The reaction mixture is stirred overnight and then quenched with 1N HCl and extracted with EtOAc. The organic extract is washed with brine and concentrated *in vacuo*. The residue is dissolved in THF to which is added 1N NaOH (0.3 mL). The mixture is stirred for 2 h and 1N HCl is added. The mixture is extracted with EtOAc, washed with brine, dried over MgSO₄ and concentrated *in vacuo*. The crude material is purified by flash chromatography to yield the title compound in 83% yield.

¹H NMR (DMSO-d₆) : 1.30 - 1.47 (m, 4H), 1.57 - 1.70 (m, 2H), 2.73 (dd, J = 11.5, 6.1, 2H) , 4.31 (dd, J = 13.1, 7.7, 1H), 7.42 - 7.55 (m, 3H), 7.65 - 7.90 (m, 7H), 8.52 (d, J = 7.6, 1H), 12.40 (br s, 1H).

### Example 74. Preparation of Nα-(4-hydroxy-7-trifluoromethylquinoline-3-carbonyl)-N∈-(4-bromobenzenesulfonyl)-L-lysine (compound no. 23)

Following the indications of example 73 and substituting benzoic acid by 4-hydroxy-7-trifluoromethylquinoline-3-carboxylic acid, the title compound is obtained in 25% yield.

¹H NMR (DMSO-d₆): 1.22 - 1.57 (m, 4H), 1.65 - 1.82 (m, 2H), 2.70 (m, 2H), 4.46 (m, 1H), 7.67 - 7.82 (m, 7H), 8.08 (s, 1H), 8.45 (d, J = 8.5, 1H), 10.25 (d, J = 7.5, 1H), 12.80 (br s, 1H).

### Example 75. Preparation of Nα-(9-fluorenemethylcarbonyl)-N∈- (4-bromobenzenesulfonyl)-L-lysine (compound no. 30)

Following the indications of example 73 and substituting benzoic acid by 9-fluoreneacetic acid, the title compound is obtained in 71% yield.

¹H NMR (DMSO-d₆): 1.22 - 1.45 (m, 4H), 1.47 - 1.55 (m, 1H), 1.62 - 1.70 (m, 1H), 2.58 (dd, J = 14.5, 6.5, 2H), 2.70 - 2.74 (m, 2H), 4.25 (m, 1H), 4.34 (t, J = 7.5, 1H), 7.20 - 7.38 (m, 4H), 7.48 (d, J = 7.5, 1H), 7.60 (d, J = 7.5, 1H), 7.70 - 7.90 (m, 6H), 8.12 (d, J = 6.6, 1H), 12.50 (br s, 1H).

### Example 76. Preparation of Nα-(9-fluorenecarbonyl)-N∈-(4-bromobenzenesulfonyl)-L-lysine (compound no. 38)

Following the indications of example 73 and substituting benzoic acid by 9-fluoreneacetic acid, the title compound is obtained in 71% yield. The NMR indicates a 1:1 equilibrium between the amide form and its enol form.

¹H NMR (DMSO-d₆): 1.26 - 1.45 (m, 4H), 1.72 - 1.80 (m, 2H), 2.72 (m, 2H), 4.18 (dd, J = 12.5, 6.5, 0.5H), 4.25 (dd, J = 12.5, 0.5H), 6.76 (s, 0.5H, fluorene methine), 7.22 - 7.30 (m, 2H), 7.32 - 7.43 (m, 3H), 7.53 (d, J = 7.6, 0.5H), 7.56 (d, J = 7.5, 0.5H), 7.68 - 7.80 (m, 7H) , 8.15 (d, J = 8.4, 0.5H) , 8.26 (d, J = 8.0, 0.5H), 12.21 (br s, 0.5H, OH, enol), 12.71 (br s, 1H).

### Example 77. Preparation of Nα-(diphenylhydroxyacetyl)-N∈-(4-bromobenzenesulfonyl)-L--lysine (compound no. 37)

Following the indications of example 73 and substituting benzoic acid by benzilic acid, the title compound is obtained in 55% yield.

¹H NMR (DMSO-d₆): 1.13 - 1.20 (m, 2H), 1.28 - 1.37 (m, 2H), 1.60 - 1.75 (m, 2H), 2.65 (dd, 12.5, 6.1, 2H), 4.22 (dd, J = 12.7, 7.8, 2H), 6.83 (s, 1H) , 7.20 - 7.42 (m, 11H) , 7.65 (t, J = 5.5, 1H), 7.70 (d, J = 8.1, 2H), 7.80 (d, J = 8.1, 2H) , 8.04 (d, J = 8.3, 1H), 12.70 (br s, 1H).

### Example 78. Preparation of Nα-(diphenylacetyl)-N∈-(4-bromobenzenesulfonyl)-L-lysine (compound no. 36)

Following the indications of example 73 and substituting benzoic acid by diphenylacetic acid, the title compound is obtained in 67% yield.

¹H NMR (DMSO-d₆): 1.18 - 1.25 (m, 4H), 1.48 - 1.68 (m, 2H), 2.67 (dd, J = 12.3, 6.3, 2H), 4.17 (dd, J = 12.1, 7.3, 1H), 5.05 (s, 1H), 7.17 - 7.30 (m, 10H), 7.65 (t, J = 5.3, 1H), 7.70 (d, J - 8.4, 2H), 7.80 (d, J = 8.3, 2H), 8.51 (d, J = 8.3, 2H), 12.50 (br s, 1H).

### Example 79. Preparation of Nα-(3-indoleacetyl)-N∈-(4-bromobenzenesulfonyl)-L-lysine (compound no. 29)

Following the indications of example 73 and substituting benzoic acid by 3-indoleacetic acid, the title compound is obtained in 32% yield.

¹H NMR (DMSO-d₆) : 1.20 - 1.40 (m, 4H), 1.45 - 1.70 (m, 2H), 2.70 (dd, J = 12.5, 7.5, 2H), 3.55 (d, J = 11.2, 2H), 4.10 (dd, J = 12.5, 7.4, 1H), 6.90 - 7.05 (m, 2H) , 7.18 (s, 1H), 7.30 (d, J = 7.8, 1H), 7.52 (d, J = 7.7, 1H), 7.68 - 7.75 (m, 3H), 7.80 (d, J = 8.1, 2H), 8.05 (d, J = 7.1, 1H), 10.82 (br s, 1H).

### Example 80. General preparation of Nα-alkyl-Nα-(substituted benzenesulfonyl)-N∈-benzyloxycarbonyl-L-lysine benzyl ester

The products of reductive alkylation with isobutyraldehyde (BSP-4), 2-ethylbutyraldehyde (BSP-5) and 2-methylpentanaldehyde (BSP-6) are dissolved in CH₂Cl₂ at a concentration of 100 mg/mL and a volume of 8 mL. The three solutions are added (1 mL aliquots) to 24 reactor block tubes in the Bohdahn AWS and purged with argon. A solution of 400 mg DIPEA in 10 mL _{CH2Cl2} is made and aliquots of 1 ml, are placed in all the tubes. The solution is stirred for 20 min. The solutions of substituted sulfonyl chlorides are added in 2 ml, aliquots. The concentrations are as follows:

| Substituted benzenesulfonyl chloride | Concentration in CH₂Cl₂ |
|---|---|
| tosyl chloride | 25 mg/mL |
| benzenesulfonyl chloride | 25 mg/mL |
| *trans*-β-styrenesulfonyl chloride | 25 mg/mL |
| acetamidobenzenesulfonyl chloride | 25 mg/mL |
| methoxybenzenesulfonyl chloride | 25 mg/mL |
| bromobenzenesulfonyl chloride | 30 mg/mL |
| 4-nitrobenzenesulfonyl chloride | 30 mg/mL |
| 2-nitrobenzenesulfonyl chloride | 30 mg/mL |

The solutions are then subjected to a gentle reflux and the CH₂Cl₂ is reduced to about 0.5 mL. The solutions are stirred under argon for 72 h. The CH₂Cl₂ is then removed *in vacuo* and replaced with 1 mL of acetone. 2 mL of 1M K₂CO₃ is then added and the tubes shaken manually.

CH₂Cl₂ (4 mL) is added and the organic phase is separated and evaporated off. A small aliquot is then provided for LC-MS.

| **Compound no.** | **MASS** | **YIELD mg** | **LC-MS purity (%)** |
|---|---|---|---|
| 75 | 580.73 | 145 | > 90 |
| 169 | 566.71 | 122 | > 90 |
| 170 | 596.73 | 136 | > 90 |
| 76 | 592.75 | 85 | > 90 |
| 77 | 623.76 | 137 | > 90 |
| 171 | 645.6 | 210 | > 90 |
| 79 | 611.71 | 116 | > 90 |
| 78 | 611.71 | 106 | > 90 |
| 80 | 608.79 | 140 | > 90 |
| 172 | 594.76 | 112 | > 90 |
| 173 | 624.79 | 139 | > 90 |
| 175 | 620.80 | 125 | > 90 |
| 174 | 651.81 | 129 | > 90 |
| 176 | 673.66 | 131 | > 90 |
| 177 | 639.76 | 110 | > 90 |
| 178 | 639.76 | 129 | Impure |
| 88 | 608.79 | 124 | > 90 |
| 179 | 594.76 | 128 | > 90 |
| 180 | 624.79 | 125 | > 90 |
| 181 | 620.8 | 112 | > 80 |
| 182 | 651.81 | 134 | > 90 |
| 183 | 673.66 | 117 | > 90 |
| 184 | 639.76 | 101 | = 60 |
| 185 | 639.76 | 84 | Impure |

In some cases some DIPEA remained. Excess tosylate is hydrolyzed and extracted during work up.

### Example 81. Preparation of Nα-isobutyl-Nα-(4-methylbenzenesulfonyl)-N∈-acyl-L-lysine

*N*α-isobutyl-*N*α-4-methylbenzenesulfonyl)-L-lysine acetate salt, is weighed in Bohdahn robotic reaction vessels. The mass varied from 80 to 100 mg. These are then suspended in a 3.3M Cs₂CO₃ solution and THF (2 mL) is added. This formed a white suspension. The tubes are then stirred vigorously and the various acid chlorides dissolved in THF (1 mL) are added. In most cases gas evolution is observed. The stirring continued for 2 h.

Initial weights:

| Product No. | Starting material (mg) | mmol | Carboxylic acid chloride (mg) (Carboxylic acid precursors) |
|---|---|---|---|
| 83 | 105 | 0.25 | 60 (9-Fluorenecarboxylic acid) |
| 84 | 94 | 0.22 | 73 (9-Fuoreneacetic acid) |
| 85 | 106 | 0.25 | 73 (Xanthene-9-carboxylic acid) |
| 86 | 87 | 0.21 | 70 (Diphenylacetic acid) |
| 87 | 81 | 0.2 | 60 (Indolyl-3-carboxylic acid) |
| 88 | 83 | 0.2 | 60 (Indolyl-2-carboxylic acid) |
| 89 | 93 | 0.22 | 60 (3-Indolepropionic acid) |
| 90 | 88 | 0.21 | 60 (trans-Cinnamic acid) |
| 91 | 86 | 0.21 | 60 (3-Phenylpropionic acid) |
| 92 | 87 | 0.21 | 112 (Cholesteyl chloroformate) |
| 93 | 86 | 0.21 | 60 (2-Quinolinecarboxylic acid) |

After 2 h, EtOAc (3 mL) is added to each flask and the two phases are separated. In the case of the reaction producing derivatives no. 90, 92, 95 and 96, an insoluble precipitate is formed. These are acidified with 1N HCl that gave two clear phases. The organic layers are separated and evacuated to leave the crude products as either acids or as the cesium salt. These are placed under high vacuum for 16 h. The flasks are weighed and tabulated above. The products are then analysed by MS to determine if the reaction had taken place and to get an estimate of the purity of the final adducts.

Results:

| Product No. | Yield | MW | % Purity |
|---|---|---|---|
| 83 | 98 | 548.69 | >50 |
| 84 | 89 | 562.72 | >85 |
| | | 694.72 (Cs) | |
| 85 | 95 | 564.69 | >85 |
| 86 | 90 | 550.71 | >85 |
| | | 682.71 (Cs) | |
| 87 | 123 | 499.62 | >50 |
| 88 | 91 | 499.62 | >50 |
| | | 631.62 (Cs) | |
| 89 | 101 | 527.68 | >85 |
| 90 | 105 | 486.62 | >85 |
| | | 618.62 (Cs) | |
| 91 | 97 | 488.64 | >80 |
| | | 620.64 (Cs) | |
| 92 | 115 | 511.63 | >85 |
| | | 643.63 (Cs) | |
| 93 | 112 | 769.13 | >85 |
| | | 900.14 (Cs) | |

### Example 82. Preparation of Nα-isobutyl-Nα-(4-methylbenzenesulfonyl)-N∈-(9-fluorenylmethoxycarbonyl)-L-lysine methyl ester (compound no. 123)

The product from example 45 is treated with excess diazomethane, yielding the title compound in 68% yield.

¹H NMR (DMSO-d₆): 0.84 (d, J = 7.1, 3H), 0.87 (d, J = 7.0, 3H), 1.35 - 1.65 (m, 5H), 1.90 - 2.00 (m, 2H), 2.40 (s, 3H), 2.95 and 3.04 (ABX, J = 14.3, 7.7, 2H), 3.18 (m, 2H), 3.49 (s, 3H), 4.20 (t, J = 7.0, 1H), 4.40 (m, 2H), 4.85 (t, J = 5.5, 1H), 7.23 - 7.40 (m, 6H), 7.55 - 7.80 (m, 6H).

### Example 83. Preparation of (2R) -N-isobutyl-N- (4-methylbenzenesulfonylamino)-6-(9-fluorenylmethoxycarboxylamino)-1-hexanol (compound no. 124)

### Step A. Preparation of (2R)-N-isobutyl-N-(4-methylbenzene-sulfonylamino)-6-(9-fluorenylmethoxycarbonylamino)-1-hexanol

The product from example 35 step C is treated under conditions described in example 71 step 1 to yield the title compound in 92% yield.

¹H NMR (DMSO-d₆): 0.90 (d, J = 6.5, 3H), 0.92 (d, J = 6.7, 3H), 1.25 - 1.50 (m, 5H), 1.88 - 2.00 (m, 2H), 2.39 (s, 3H), 2.90 (dd, J = 14.5, 7.5, 1H), 2.95 - 3.10 (m, 3H), 3.50 - 3.65 (m, 3H), 4.80 (br s, 1H), 5.10 (s, 2H), 7.26 (d, J = 7.3, 2H), 7.30 - 7.40 (m, 5H), 7.68 (d, J = 7.8, 2H).

### Step B. Preparation of (2R) -N-isobutyl-N- (4-methylbenzene-sulfonylamino)-6-(9-fluorenylmethoxycarboxylamino)-1-hexanol

The alcohol of step A of this example (150 mg, 0.31 mmol) is dissolved in MeOH (3 mL) and hydrogenated in the presence of 10% Pd/C (50 mg). After 1h, N- (9-fluorenylmethoxycarbonyloxy) succinimide (177 mg, 0.34 mmol) and triethylamine (62 mg, 0.62 mmol) are added. The reaction mixture is stirred at room temperature for 1 h, then filtered and concentrated *in vacuo*. The residue is purified by flash chlormatography eluting with 70% EtOAc in hexane to provide 90% yield of the title compound.

¹H NMR (DMSO-d₆): 0.82 (d, J = 7.0, 3H), 0.84 (d, J = 7.0, 3H), 0.90 - 1.30 (m, 5H), 1.45 - 1.55 (m, 1H), 1.82 - 1.90 (m, 1H), 2.36 (s, 3H), 2.53 (s, 1H), 2.78 and 2.95 (ABX, J = 15.0, 7.5, 2H), 2.82 (m, 2H), 3.26 and 3.55 (ABX, J = 14.0, 7.0, 2H), 3.50 (m, 1H), 4.20 (t, J = 7.0, 1H) , 4.30 (t, J = 7.0, 2H) , 7.18 (t, J = 5.0, 1H), 7.30 - 7.42 (m, 6H), 7.65 (m, 4H), 7.90 (d, J = 7.4, 2H).

### Example 84. Preparation of (2R,2S)-N-isobutyl-N-(4-methylbenzenesulfonylamino)-6-(9-fluorenylmethoxycarboxylamino)-1- hexanamide (compound no. 125)

### Step A. Preparation of (2R,2S)-N-isobutyl-N- (4-methylbenzenesulfonylamino)-6-benzyloxycarbonylamino-1-hexanamide

To a stirred solution of the product of example 35 step D (245 mg, 0.50 mmol) in DMF (4 mL) is added successively ammonium chloride (106 mg, 2.00 mmol), triethylamine (202 mg, 2.00 mmol) and EDC·HCl. The reaction mixture is stirred for 36 h, then quenched with water and extracted with EtOAc. The organic layer is dried over MgSO₄, concentrated and purified by flash chromatography, eluting with 10% MeOH in CH₂Cl₂, affording 190 mg (77%) of the title compound.

¹H NMR (DMSO-d₆) : 0.80 (d, J = 7.0, 3H), 0.81 (d, J - 7.0, 3H), 1.00 - 1.32 (m, 5H), 1.60 - 2.00 (m, 2H), 2.37 (s, 3H), 2.85 (m, 2H), 2.90 and 3.17 (ABX, J = 13.5, 7.5, 2H), 4.10 (t, J = 7.2, 1H), 5.00 (s, 2H), 7.07 (s, 1H), 7.14 (s, 1H), 7.16 (m, 1H), 7.30 - 7.40 (m, 7H) , 7.71 (d, J = 7.8, 2H).

### Step B. Preparation of (2R,2S)-N-isobutyl-N-(4-methylbenzene-sulfonylamino)-6-(9-fluorenylmethoxycarbonylamino)-1-hexanamide

The title product is obtained in 61% yield by following the indications of step B of example 83, substituting the hexanol derivative by the product obtained in step A of this example.

¹H NMR (DMSO-d₆): 0.79 (d, J = 6.5, 3H), 0.82 (d, J 6.5, 3H), 1.00 - 1.35 (m, 5H), 1.60 - 1.99 (m, 2H), 2.37 (s, 3H), 2.85 (m, 2H), 2.90 and 3.20 (ABX, J = 13.5, 7.5, 2H), 4.10 (t, J = 7.1, 1H), 4.20 (t, J = 7.0, 1H), 4.27 (d, J = 7.0, 2H), 7.07 (s, 1H), 7.14 (s, 1H), 7.20 (m, 1H), 7.30 - 7.45 (m, 5H), 7.60 - 7.72 (m, 6H), 7.89 (d, J = 7.5, 2H).

### Example 85. Preparation of (2R,2S)-N-isobutyl-N-(4-methylbenzenesulfonylamino)-6-(9-fluorenylmethoxycarboxylamino)-1-hydroxylhexamide (compound no. 141)

### Step A. (2R,2S)-N-isobutyl-N-(4-methylbenzenesulfonylamino)-6- benzyloxycarbonylamino-1-benzyloxylaminohexane

The product of example 35 step D is reacted under the conditions outlined in step A of example 84 substituting ammonium chloride with benzyloxyamine, the crude material (38%) isused without purification in step B.

### Step B. Preparation of (2R,2S)-N-isobutyl-N-(4-methylbenzenesulfonylamino)-6-(9-fluorenylmethoxycarboxylamino)-1- hydroxylaminohexane

The title product is obtained in 82% yield by following the indications of step B of example 83, substituting the hexanol derivative by the product obtained in step A of this example.

¹H NMR (DMSO-d₆): 0.76 (d, J = 6.6, 3H), 0.79 (d, J = 6.6, 3H), 1.00 - 1.32 (m, 5H), 1.63 - 1.69 (m, 1H), 2.00 - 2.10 (m, 1H), 2.36 (s, 3H) , 2.85 (m, 2H), 2.90 and 3.16 (ABX, J = 13.5, 7.5, 2H), 4. 05 (t, J = 7.2, 1H), 4.20 (t, J = 7.0, 1H), 4.28 (d, J = 7.0, 2H), 7.20 (t, J = 5.5, 2H), 7.30 - 7.45 (m, 6H), 7.70 (m, 4H), 7.90 (d, J = 7.4, 2H), 8.86 (s, 1H), 10.63 (br s, 1H).

### Example 86. Preparation of Nα-isobutyl-Nα-(4-nitrobenzenesulfonyl)-N∈-(trans-2-methoxycinnamoyl)-L-lysine (compound no. 161)

A mixture of *trans*-2-methoxycinnamic acid (106 mg, 0.55 mmol) and carbonyldiimidazole (89 mg, 0.55 mmol) in THF (3 mL) is stirred at room temperature for 1 h, and then at 40°C -until gas evolution ceased. The mixture is cooled to room temperature and *N*α-isobutyl-*N*α-(4-nitrobenzenesulfonyl)-L-lysine (212 mg, 0.50 mmol) in solution in 1M K₂CO₃ is added. The reaction mixture is stirred at room temperature for 3 h, then diluted with 1N HCl and extracted with EtOAc. The organic layer is dried over MgSO₄, concentrated *in vacuo* and purified by flash chromatography eluting with 70% EtOAc in hexane containing 0.4% AcOH to give the title compound (71% yield).

¹H NMR (DMSO-d₆): 0.82 (d, J = 6.0, 3H), 0.87 (d, J = 6.4, 3H), 1.25 - 1.63 (m, 5H), 1.85 - 2.00 (m, 2H), 2.95 (dd, J = 13.5, 7.5, 1H), 3.05 - 3.15 (m, 3H), 3.85 (s, 3H), 4.28 (t, J = 7.8, 1H) , 6.60 (d, J = 16.3, 1H), 6.90 - 7.50 (m, 4H) , 7.63 (d, J = 16.3, 1H), 8.02 (t, J = 8.7, 2H), 8.37 (d, J = 8.6, 2H), 12.70 (br s, 1H).

### Example 87. Preparation of Nα-isobutyl-Nα-(4-nitrobenzenesulfoayl)-N∈-(cis-2-methoxycinnamoyl)-L-lysine (compound no. 186)

*N*α-isobutyl-*N*α-(4-nitrobenzenesulfonyl)-L-lysine is reacted with *cis*-2-methoxycinnamic acid under the conditions described in example 86 to yield 32% of the desired product.

¹H NMR (DMSO-d₆): 0.82 (d, J = 6.0, 3H), 0.87 (d, J = 6.4, 3H), 1.20 - 1.64 (m, 7H), 2.95 (dd, J = 13.5, 7.5, 1H), 3.00 (m, 2H), 3.10 (m, 1H), 3.78 (s, 3H), 4.25 (t, J = 7.8, 1H), 5.95 (d, J = 12.4, 1H) , 6.80 (d, J = 12.4, 1H), 6.85 (t, J = 7.2, 1H), 7.00 (m, 1H), 7.26 (t, J = 7.0, 1H), 7.55 (d, J = 7.2, 1H), 7.95 (t, J = 5.5, 1H), 8.06 (d, J = 8.8, 2H), 8.37 (d, J = 8.8, 2H), 12.75 (br s, 1H).

### Example 88. Preparation of Nα-isobutyl-Nα-(4-nitrobenzenesulfonyl)-N∈-dihydrocinnamoyl-L-lysine (compound no. 94)

*N*α-isobutyl-*N*α-(4-nitrobenzenesulfonyl)-L-lysine is reacted with dihydrocinnamic acid under the conditions described in example 86 to yield 81% of the desired product.

¹H NMR (DMSO-d₆): 0.82 (d, J = 7.0, 3H), 0.86 (d, J = 7. 0, 3H), 1.18 - 1.60 (m, 5H), 1.80 - 1.95 (m, 2H), 2.33 (t, J = 7.2, 2H), 2.80 (t, J = 7.2, 2H), 2.91 - 3.00 (m, 3H), 3.10 (dd, J = 13.2, 7.0, 1H) , 4.27 (t, J = 7.2, 1H) , 7.15 7.3 0 (m, 5H) , 7.74 (t, J = 5.2, 1H), 8.06 (d, J = 8.0, 2H), 8.38 (d, J = 8.0, 2H), 12.70 (br s, 1H).

### Example 89. Preparation of Nα-isobutyl-Nα-(4-nitrobenzenesulfonyl)-N∈-(9xanthenecarbonyl)-L-lysine (compound no. 96)

*N*α-isobutyl-*N*α-(4-nitrobenzenesulfonyl)-L-lysine is reacted with xanthene-9-carboxylic acid under the conditions described in example 86 to yield the desired product.

¹H NMR (DMSO-d₆): 0.75 (d, J = 6.5, 3H), 0.78 (d, J = 6.8, 3H), 1.2 (br s, 2H), 1.32 - 1.42 (m, 3H), 1.74 - 1.86 (m, 2H), 2.82 - 2.90 (m, 4H), 4.12 - 4.15 (t, J = 14, 1H), 4.85 (s, 1H), 7.04 - 7.16 (q, J = 6.2, 4H), 7.22 - 7.32 (q, J = 6.2, 4H), 8.05 (d, J = 14, 2H), 8.45 (d, J = 5.14, 2H).

### Example 90. Preparation of Nα-isobutyl-Nα-(4-aminobenzenesulfonyl)-N∈-(3-indolepropionyl)-L-lysine (compound no. 98)

The product of example 69 is reduced by catalytic hydrogenation under the conditions described in example 4 to yield the desired product (95% yield).

LC-MS: 529.3 (M⁺ +H).

### Example 91. Preparation of Nα-isobutyl-Nα-(4-nitrobenzenesulfonyl)-N∈-(3-nitrocinnamoyl)-L-lysine (compound no. 108)

*Nα*-isobutyl-*Nα*-(4-nitrobenzenesulfonyl)-L-lysine is reacted with 3-nitrocinnamic acid under the conditions described in example 86 to yield 52% of the desired product.

¹H NMR (DMSO-d₆): 0.82 (d, J = 6.3, 3H), 0.86 (d, J = 6.1, 3H), 1.28 - 1.62 (m, 5H), 1.88 - 1.96 (m, 2H), 2.95 and 3.10 (ABX, J = 14.3, 7.5, 2H), 3.15 (m, 2H), 4.30 (t, J = 7.0, 1H), 6.60 (d, J = 15.5, 1H), 7.62 (m, 1H), 7.68 (d, J = 15.5, 1H), 7.80 (m, 2H), 8.00 - 8.10 (m, 3H), 8.20 (t, J = 5.5 1H), 8.40 (d, J = 8.8, 2H), 12.80 (br s, 1H).

### Example 92. Preparation of Nα-isobutyl-Nα-(4-nitrobeiizenesulfonyl)-N∈-(2-nitrocinnamoyl)-L-lysine (compound no. 109)

*N*α-isobutyl-*N*α-(4-nitrobenzenesulfonyl)-L-lysine is reacted with 2-nitrocinnamic acid under the conditions described in example 86 to yield 42% of the desired product.

¹H NMR (DMSO-d₆): 0.82 (d, J = 6.7, 3H), 0.86 (d, J = 7.0, 3H), 1.27 - 1.63 (m, 5H), 1.85 - 1.95 (m, 2H), 2.92 and 3.10 (ABX, J = 14.3, 7.5, 2H), 3.13 (m, 2H), 4.30 (t, J = 7.0, 1H), 6.80 (d, J = 15.1, 1H), 7.50 (d, J = 15.1, 1H), 7.70 (t, J = 7.8, 1H), 8.00 - 8.40 (m, 8H), 12.80 (br s, 1H).

### Example 93. Preparation of Nα-isobutyl-Nα-(4-nitrobenzenesulfonyl)-N∈-(2,3-dimethoxycinnamoyl)-L-lysine (compound no. 110)

*N*α-isobutyl-*N*α-(4-nitrobenzenesulfonyl)-L-lysine is reacted with 2,3-dimethoxycinnamic acid under the conditions described in example 86 to yield 70% of the desired product.

¹H NMR (DMSO-d₆): 0.81 (d, J = 6.5, 3H), 0.86 (d, J = 6.5, 3H), 1.15 - 1.60 (m, 5H), 1.82 - 1.95 (m, 2H), 2.53 (s, 3H), 2.90 (dd, J = 14.3, 7.3, 1H), 3.10 - 3.18 (m, 3H), 3.74 (s, 3H), 3.82 (s, 3H), 4.30 (t, J = 7.2, 1H), 6.60 (d, J = 15.5, 1H), 7.05 - 7.15 (m, 3H), 7.60 (d, J = 15.5, 1H), 8.10 (m, 3H), 8.36 (d, J = 8.0, 1H), 12.80 (br s, 1H).

### Example 94. Preparation of Nα-isobutyl-Nα-(4-nitrobenzenesulfonyl)-N∈-(3,5-dimethoxycinnamoyl)-L-lysine (compound no. 189)

Na-isobutyl-Na-(4-nitrobenzenesulfonyl)-L-lysine is reacted with 3,5-dimethoxycinnamic acid under the conditions described in example 86 to yield 66% of the desired product.

¹H NMR (DMSO-d₆) : 0.79 (d, J = 7.0, 3H), 0.82 (d, J = 6.1, 3H), 1.25 - 1.60 (m, 5H), 1.85 - 2.00 (m, 2H), 2.90 (dd, J = 13.5, 7.5, 1H) , 3.05 - 3.15 (m, 3H), 3.76 (s, 6H), 4.27 (t, J = 7.0, 1H), 6.51 (s, 1H), 6.60 (d, J = 16.5, 1H), 6.71 (s, 2H), 7.30 (d, J = 16.5, 1H) , 8.02 (t, J = 5.5, 1H) , 8.10 (d, J = 8.2, 2H), 8.38 (d, J = 8.2, 2H) , 12.70 (br s, 1H).

### Example 95. Preparation of Nα-isobutyl-Nα-(4-nitrobenzenesulfonyl)-N∈-(2,5-dimethoxycinnamoyl)-L-lysine (compound no. 190)

*N*α-isobutyl-*N*α-(4-nitrobenzenesulfonyl)-L-lysine is reacted with 2,5-dimethoxycinnamic acid under the conditions described in example 86 to yield 69% of the desired product.

¹H NMR (DMSO-d₆) : 0.82 (d, J = 6.8, 3H), 0.87 (d, J = 6.8, 3H), 1.25 - 1.62 (m, 5H), 1.82 - 1.98 (m, 2H), 2.95 (dd, J = 13.5, 7.3, 1H), 3.10 - 3.18 (m, 3H), 3.73 (s, 3H), 3.78 (s, 3H), 4.28 (t, J = 7.2, 1H), 6.60 (d, J = 16.5, 1H), 6.90 - 7.05 (m, 3H), 7.60 (d, J = 16.5, 1H), 8.00 (t, J = 5.5, 1H), 8.10 (d, J = 8.3, 2H), 8.40 (d, J = 8.2, 2H), 12.70 (br s, 1H).

### Example 96. Preparation of Nα-isobutyl-Nα-(4-nitrobenzenesulfonyl)-N∈-(2,4-dimethoxycinnamoyl)-L-lysine (compound no. 191)

*N*α-isobutyl-*N*α-(4-nitrobenzenesulfonyl)-L-lysine is reacted with 2,4-dimethoxycinnamic acid under the conditions described in example 86 to yield 72% of the desired product.

¹H NMR (DMSO-d₆): 0.81 (d, J = 6.0, 3H), 0.86 (d, J = 6.2, 3H), 1.25 - 1.62 (m, 5H), 1.85 - 1.98 (m, 2H), 2.95 (dd, J = 13.5, 7.5, 1H), 3.05 - 3.12 (m, 3H), 3.80 (s, 3H), 3.84 (s, 3H), 4.30 (t, J = 7.2, 1H), 6.48 (d, J = 16.5, 1H), 6.60 (m, 2H), 7.42 (d, J = 8.6, 1H), 7.55 (d, J = 16.2, 1H), 7.89 (t, J = 5.5, 1H), 8.10 (d, J = 8.8, 2H), 8.38 (d, J = 8.8, 2H), 12.70 (br s, 1H).

### Example 97. Preparation of Nα-isobutyl-Nα-(4-nitrobenzenesulfonyl)-N∈-(4-nitrocinnamoyl)-L-lysine (compound no. 111)

*N*α-isobutyl-*N*α-(4-nitrobenzenesulfonyl)-L-lysine is reacted with 4-nitrocinnamic acid under the conditions described in example 86 to yield 49% of the desired product.

¹H NMR (DMSO-d₆) : 0.81 (d, J = 6.0, 3H), 0.86 (d, J = 6.0, 3H), 1.25 - 1.60 (m, 5H), 1.85 - 1.95 (m, 2H), 2.72 (m, 2H), 2.90 and 3.10 (ABX, J = 14.3, 7.5, 2H), 4.15 (m, 2H), 6.80 (d, J = 15.5, 1H), 7.50 (d, J = 15.5, 1H), 7.82 (d, 8.7, 2H), 8.10 (d, J = 8.5, 2H), 8.22 (t, J = 5.0, 1H) , 8.25 (d, J = 8.8, 2H), 8.38 (d, J = 8.8, 2H), 12.80 (br s, 1H).

### Example 98. Preparation of Nα-isobutyl-Nα-(4-nitrobenzenesulfonyl)-N∈-(trans-4-phenylbuten-2-oyl)-L-lysine (compound no. 187)

*N*α-isobutyl-*N*α-(4-nitrobenzenesulfonyl)-L-lysine is reacted with *trans*-4-phenylbuten-2-oic acid under the conditions described in example 86 to yield 45% of the desired product.

¹H NMR (DMSO-d₆): 0.81 (d, J = 6.1, 3H), 0.86 (d, J = 6.7, 3H), 1.22 - 1.62 (m, 5H), 1.84 - 1.95 (m, 2H), 2.92 and 3.10 (ABX, J = 13.5, 7.5, 2H), 3.00 (m, 2H), 4.28 (t, J = 7.1, 1H), 6.30 (dt, 16.3, 7.6, 1H), 6.45 (d, J = 16.0, 1H), 7.20 - 7.40 (m, 5H), 7.85 (t, J = 5.3, 1H) , 8.06 (d, J = 8.0, 2H), 8.38 (d, J = 8.0, 2H), 12.70 (br s, 1H).

### Example 99. Preparation of Nα-isobutyl-Nα-(4-nitrobenzenesulfonyl)-N∈-(4-methoxycinnamoyl)-L-lysine (compound no. 113)

*N*α-isobutyl-*N*α-(4-nitrobenzenesulfonyl)-L-lysine is reacted with 4-methoxycinnamic acid under the conditions described in example 86 to yield 65% of the desired product.

¹H NMR (DMSO-d₆): 0.81 (d, J = 6.0, 3H), 0.86 (d, J = 6.9, 3H), 1.25 - 1.62 (m, 5H), 1.85 - 1.97 (m, 2H), 2.90 (dd, J = 14.5, 7.5, 1H), 3.05 - 3.14 (m, 3H), 3.78 (s, 3H), 4.30 (t, J = 7.0, 1H), 6.42 (d, J = 15.3, 1H), 7.00 (d, J = 8.0, 2H), 7.34 (d, J = 15.3, 1H), 7.50 (d, J = 8.0, 2H), 7.95 (t, J = 5.5, 1H), 8.02 - 8.40 (m, 4H), 12.70 (br s, 1H).

### Example 100. Preparation of Nα-isobutyl-Nα-(4-nitrobenzenesulfonyl)-N∈-benzylsulfonyl-L-lysine (compound no. 115)

*N*α-isobutyl-*N*α-(4-nitrobenzenesulfonyl)-L-lysine is reacted with benzylsulfonyl chloride under the conditions described in example 2 to yield 24% of the desired product.

¹H NMR (DMSO-d₆): 0.82 (d, J = 6.5, 3H), 0.88 (d, J = 6.5, 3H), 1.22 - 1.60 (m, 5H), 1.80 - 1.98 (m, 2H), 2.80 (m, 2H), 2.92 and 3.10 (ABX, J = 14.5, 7.3, 2H), 4.25 (m, 1H), 4.28 (s, 2H), 7.00 (t, J = 5.5, 1H), 7.30 - 7.40 (m, 5H), 8.08 (d, J = 8.7, 2H), 8.40 (d, J = 8.5, 2H), 12.70 (br s, 1H).

### Example 101. Preparation of Nα-isobutyl-Nα,N∈-di-(4-nitrobenzenesulfonyl)-L-lysine (compound no. 116)

*N*α-isobutyl-*N*α-(4-nitrobenzenesulfonyl)-L-lysine is reacted with 4-nitrobenzenesulfonyl chloride under the conditions described in example 2 to yield 32% of the desired product.

¹H NMR (DMSO-d₆): 0.80 (d, J = 6.2, 3H), 0.84 (d, J = 7.1, 3H), 1.18 - 1.55 (m, 5H), 1.75 - 1.90 (m, 2H), 2.72 (m, 2H), 2.90 and 3.07 (ABX, J = 14.5, 7.5, 2H), 4.20 (dd, J = 8.5, 6.0, 1H), 7.90 (t, J = 5.5, 1H), 8.02 (d, J = 8.0, 2H), 8.06 (d, J = 8.0, 2H), 8.35 (d, J = 8.2, 2H), 8.42 (d, J = 8.0, 2H), 12.80 (br s, 1H)

### Example 102. Preparation of Nα-isobutyl-Nα-(4-nitrobenzenesulfonyl)-N∈-(4-methylbenzenesulfonyl)-L-lysine (compound no. 199)

*N*α-isobutyl-*N*α-(4-nitrobenzenesulfonyl)-L-lysine is reacted with 4-methylbenzylsulfonyl chloride under the conditions described in example 2 to yield 28% of the desired product.

¹H NMR (DMSO-d₆): 0.80 (d, J = 7.0, 3H), 0.84 (d, J = 6.2, 3H), 1.18 - 1.55 (m, 5H), 1.72 - 1.95 (m, 2H), 2.38 (s, 3H), 2.62 (m, 2H), 2.90 and 3.10 (ABX, J = 14.5, 7.5, 2H), 4.22 (t, J = 6.1, 1H), 7.37 (d, J = 8.2, 2H), 7.40 (t, J = 5.5, 2H), 7.65 (d, J = 8.2, 2H), 8.10 (d, J = 8.0, 2H), 8.40 (d, J = 8.2, 2H), 12.70 (br s, 1H).

### Example 103. Preparation of Nα-isobutyl-Nα-(4-Tnitrobenzenesulfonyl)-N∈-phenylthioacetyl-L-lysine (compound no. 154)

*N*α-isobutyl-*N*α-(4-nitrobenzenesulfonyl)-L-lysine is reacted with (phenylthio)acetyl chloride under the conditions described in example 2 to yield 74% of the desired product.

¹H NMR (DMSO-d₆): 0.81 (d, J = 5.8, 3H), 0.85 (d, J = 6.9, 3H), 1.16 - 1.55 (m, 5H), 1.80 - 1.95 (m, 2H), 2.90 and 3.10 (ABX, J = 14.5, 7.5, 2H), 3.00 (m, 2H), 3.60 (s, 3H), 4.23 (t, J = 7.0, 1H), 7.18 (t, J = 5.5, 1H), 7.27 - 7.35 (m, 4H), 8.05 (m, 3H), 8.40 (d, J = 8.0, 2H), 12.75 (br s, 1H).

### Example 104. Preparation of Nα-isobutyl-Nα-(4-nitrobenzenesulfonyl)-N∈-phenoxyacetyl-L-lysine (compound no. 160)

*N*α-isobutyl-*N*α-(4-nitrobenzenesulfonyl)-L-lysine is reacted with phenoxyacetyl chloride under the conditions described in example 2 to yield 88% of the desired product.

¹H NMR (DMSO-d₆): 0.82 (d, J = 6.0, 3H), 0.85 (d, J = 6.0, 3H), 1.20 - 1.60 (m, 5H), 1.80 - 1.96 (m, 2H), 2.92 (dd, J = 14.2, 7.5, 1H) , 3.05 - 3.12 (m, 3H) , 4.28 (t, J = 7.0, 1H) , 4.45 (s, 2H), 6.90 - 7.00 (m, 3H), 7.30 (m, 2H), 8.00 (t, J = 4.5, 1H), 8.08 (d, J = 8.8, 2H), 8.37 (d, J = 8.5, 2H), 12.50 (br s, 1H).

### Example 105. Preparation of Nα-isobutyl-Nα-(4-nitrobenzenesulfonyl)-N∈-(3-methoxycinnamoyl)-L-lysine (compound no. 162)

*N*α-isobutyl-*N*α-(4-nitrobenzenesulfonyl)-L-lysine is reacted with 3-methoxycinnamic acid under the conditions described in example 86 to yield 50% of the desired product.

¹H NMR (DMSO-d₆): 0.82 (d, J = 7.0, 3H), 0.87 (d, J = 7.0, 3H), 1.27 - 1.62 (m, 5H), 1.85 - 1.95 (m, 2H), 2.95 and 3.10 (ABX, J = 14.3, 7.3, 2H), 3.12 (m, 2H), 3.78 (s, 3H), 4.30 (t, J = 6.5, 1H), 6.60 (d, J = 16.4, 1H), 6.95 (m, 1H), 7.10 (m, 2H), 7.30 - 7.40 (m, 2H), 8.03 (t, J = 5.0, 1H), 8.08 (d, J = 9.0, 2H), 8.38 (d, J = 8.8, 2H), 12.70 (br s, 1H).

### Example 106. Preparation of Nα-isobutyl-Nα-(4-nitrobenzenesulfonyl)-N∈-(3,4-methylenedioxycinnamoyl)-L-lysine (compound no. 163)

Na-isobutyl-Na-(4-nitrobenzenesulfonyl)-L-lysine is reacted with 3,4-methylenedioxycinnamic acid under the conditions described in example 86 to yield 76% of the desired product.

¹H NMR (DMSO-d₆): 0.81 (d, J = 6.0, 3H), 0.86 (d, J = 6.8, 3H), 1.25 - 1.60 (m, 5H), 1.84 - 2.00 (m, 2H), 2.93 and 3.10 (ABX, J = 14.8, 7.4, 2H), 3.13 (m, 2H), 4.30 (t, J = 6.2, 1H), 6.05 (s, 2H), 6.42 (d, J = 15.2, 1H), 6.93 (d, J = 7.5, 1H), 7.05 (d, J = 7.5, 1H), 7.12 (s, 1H), 7.30 (d, J = 15.3, 1H), 7.90 (t, J = 5.2, 1H), 8.10 (d, J = 8.0, 2H), 8.37 (d, J = 8.3, 2H), 12.70 (br s, 1H).

### Example 107. Preparation of Nα-isobutyl-Nα-(4-nitrobenzenesulfonyl)-N∈-(3,4-dimethoxycinnamoyl)-L-lysine (compound no. 193)

*N*α-isobutyl-*N*α-(4-nitrobenzenesulfonyl)-L-lysine is reacted with 3,4-dimethoxycinnamic acid under the conditions described in example 86 to yield 73% of the desired product.

¹H NMR (DMSO-d₆): 0.82 (d, J = 7.0, 3H), 0.87 (d, J = 7.0, 3H), 1.20 - 1.60 (m, 5H), 1.82 - 1.98 (m, 2H), 2.95 (dd, J = 13.5, 7.5, 2H), 3.10 - 3.17 (m, 2H), 3.78 (s, 3H), 3.79 (s, 3H), 4.30 (m, 1H), 6.56 (d, J = 16.5, 1H), 7.00 (d, J = 8.0, 1H), 7.10 (d, J = 8.2, 1H), 7.13 (s, 1H), 7.32 (d, J = 16.5, 1H), 7.93 (t, J = 5.5, 1H), 8.10 (d, J = 8.3, 2H), 8.38 (d, J = 8.0, 2H), 12.70 (br s, 1H).

### Example 108. Preparation of Nα-isobutyl-Nα-(4-nitrobenzenesulfonyl)-N∈-(trans-3-(3-pyridyl)acryloyl)-L-lysine (compound no. 164)

*N*α-isobutyl-*N*α-(4-nitrobenzenesulfonyl)-L-lysine is reacted with trans-3-(3-pyridyl)acrylic acid under the conditions described in example 86 to yield 60% of the desired product.

¹H NMR (DMSO-d₆): 0.82 (d, J = 7.0, 3H), 0.87 (d, J = 6.1, 3H), 1.25 - 1.62 (m, 5H), 1.88 - 1.92 (m, 2H), 2.93 and 3.08 (ABX, J = 13.5, 7.3, 2H), 3.15 (m, 2H), 4.30 (t, J = 6.3, 1H), 6.70 (d, J = 15.2, 1H), 7.45 (m, 2H), 7.95 (m, 1H), 8.08 (d, J = 8.8, 2H), 8.12 (t, J = 5.4, 1H), 8.40 (d, J = 8.5, 2H), 12.70 (br s, 1H).

### Example 109. Preparation of Nα-isobutyl-Nα-(4-methylbenzenesulfonyl)-N∈-(trans-4-hydroxycinnamoyl)-L-lysine (compound no. 188)

*N*α-isobutyl-*N*α-(4-methylbenzenesulfonyl)-L-lysine is reacted with *trans*-4-hydroxycinnamic acid under the conditions described in example 86 to yield 45% of the desired product.

¹H NMR (DMSO-d₆) : 0.80 (d, J = 6.1, 3H), 0.82 (d, J = 6.2, 3H), 1.20 - 1.55 (m, 5H), 1.78 - 1.95 (m, 2H), 2.37 (s, 3H), 2.90 and 3.00 (ABX, J = 14.3, 7.0, 2H), 3.10 (m, 2H), 4.17 (t, J = 6.5, 1H), 6.40 (d, J = 16.0, 1H), 6.80 (d, J = 7.5, 2H), 7.30 (d, J = 16.0, 1H), 7.38 (m, 4H), 7.78 (d, J = 7.0, 2H) , 7.90 (t, J = 5.0, 1H), 9.80 (s, 1H), 12.70 (br s, 1H).

### Example 110. Preparation of Nα-isobutyl-Nα-(4-aminobenzenesulfonyl)-N∈-(3-aminodihydrocinnamoyl)-L-lysine (compound no. 118)

The product of example 91 is reduced by catalytic hydrogenation under the conditions described in example 4 to yield the desired product. The yields of the catalytic hydrogenation are usually ranging form 85% to 100%.

¹H NMR (DMSO-d₆): 0.78 (d, J = 7.2, 3H), 0.80 (d, J = 7.0, 3H), 1.15 - 1.46 (m, 5H), 1.72 - 1.90 (m, 2H), 2.30 (t, J = 7.0, 2H), 2.62 (t, J = 7.0, 2H), 2.90 (m, 2H), 3.00 (m, 2H), 4.10 (t, J = 7.0, 1H), 5.90 (br s, 2H), 6.42 - 6.60 (m, 4H), 6.88 (m, 2H), 7.40 (d, J = 7.2, 2H), 7.80 (t, J = 5.0, 1H), 12.70 (br s, 1H).

### Example 111. Preparation of Nα-isobutyl-Nα-(4-aminobenzenesulfonyl)-N∈-(2,3-dimethoxydihydrocinnamoyl)-L-lysine (compound no. 119)

The product of example 93 is reduced by catalytic hydrogenation under the conditions described in example 4 to yield the desired product.

¹H NMR (DMSO-d₆): 0.78 (d, J = 6.5, 3H), 0.80 (d, J = 6.5, 3H), 1.15 - 1.42 (m, 5H), 1.72 - 1.90 (m, 2H), 2.30 (t, J = 7.2, 2H), 2.76 (t, J = 7.2, 2H), 2.82 - 3.00 (m, 4H), 3.71 (s, 3H), 3.77 (s, 3H), 4.10 (t, J = 7.2, 1H), 5.95 (s, 2H), 6.60 (d, J = 7.6, 2H), 6.73 (d, J = 7.5, 1H), 6.86 (d, J = 7.4, 1H), 6.95 (t, J = 8.5, 1H), 7.40 (d, J = 7.7, 2H), 7.75 (br s, 1H), 12.55 (br s, 1H).

### Example 112. Preparation of Nα-isobutyl-Nα-(4-aminobenzenesulfonyl)-N∈-(4-methoxydihydrocinnamoyl)-L-lysine (compound no. 120)

The product of example 99 is reduced by catalytic hydrogenation under the conditions described in example 4 to yield the desired product.

¹H NMR (DMSO-d₆): 0.78 (d, J = 7.0, 3H), 0.80 (d, J = 6.5, 3H), 1.15 - 1.48 (m, 5H), 1.70 - 1.90 (m, 2H), 2.30 (t, J = 7.0, 2H), 2.75 (t, 7.0, 2H), 2.70 - 3.00 (m, 4H), 3.73 (s, 3H), 4.10 (t, J = 7.0, 1H), 5.95 (s, 2H), 6.46 (d, J = 7.5, 2H), 6.57 (d, J = 7.5, 2H) 6.82 (d, J = 7.8, 2H), 7.40 (d, J = 7.5, 2H), 7.69 (t, J = 5.2, 1H), 12.60 (br s, 1H).

### Example 113. Preparation of Nα-isobutyl-Nα-(4-aminobenzenesulfonyl)-N∈-(2-aminodihydrocinnamoyl)-L-lysine (compound no. 122)

The product of example 92 is reduced by catalytic hydrogenation under the conditions described in example 4 to yield the desired product.

¹H NMR (DMSO-d₆): 0.78 (d, J = 6.0, 3H), 0.80 (d, J = 6.0, 3H), 1.18 - 1.50 (m, 5H), 1.72 - 1.90 (m, 2H), 2.27 (t, J = 7.0, 2H), 2.60 (t, J = 7.0, 2H), 2.85 - 3.00 (m, 4H), 4.00 (t, J = 7.0, 1H), 5.94 (s, 2H), 6.31 - 6.37 (m, 3H), 6.58 (d, J = 8.2, 2H), 6.89 (t, J = 8.2, 1H), 7.39 (d, J = 8.2, 2H), 7.73 (t, J = 4.9, 1H).

### Example 114. Preparation of Nα-isobutyl-Nα-(4-aminobenzenesulfonyl)-N∈-(3,4-methylenedioxydihydrocinnamoyl)-L-lysine (compound no. 155)

The product of example 106 is reduced by catalytic hydrogenation under the conditions described in example 4 to yield the desired product.

¹H NMR (DMSO-d₆): 0.78 (d, J = 7.0, 3H), 0.80 (d, J = 6.2, 3H), 1.18 - 1.50 (m, 5H), 1.70 - 1.90 (m, 2H), 2.30 (t, J = 7.2, 2H), 2.70 (t, J = 7.2, 2H), 2.80 - 3.00 (m, 4H), 4.12 (t, J = 7.0, 1H), 5.93 (s, 2H), 5.95 (s, 2H), 6.80 (m, 2H), 7.38 (d, J = 8.4, 2H), 7.78 (t, J = 4.5, 1H), 12.55 (br s, 1H).

### Example 115. Preparation of Nα-isobutyl-Nα-(4-aminobenzenesulfonyl)-N∈-(3,4-dimethoxydihydrocinnamoyl)-L-lysine (compound no. 200)

The product of example 107 is reduced by catalytic hydrogenation under the conditions described in example 4 to yield the desired product.

¹H NMR (DMSO-d₆): 0.78 (d, J = 6.5, 3H), 0.80 (d, J = 6.5, 3H), 1.17 - 1.50 (m, 5H), 1.72 - 1.90 (m, 2H), 2.30 (t, J = 7.2, 2H), 2.72 (t, J = 7.2, 2H), 2.80 - 3.00 (m, 4H), 3.69 (s, 3H), 3.72 (s, 3H), 4.10 (t, J = 6.7, 1H) , 5.94 (br s, 2H), 6.55 - 6.82 (m, 5H), 7.40 (d, J = 8.2, 2H), 7.74 (t, J = 4.5, 1H), 12.45 (br s, 1H).

### Example 116. Preparation of Nα-isobutyl-Nα-(4-aminobenzenesulfonyl)-N∈-(3-methoxydihydrocinnamoyl)-L-lysine (compound no. 156)

The product of example 105 is reduced by catalytic hydrogenation under the conditions described in example 4 to yield the desired product.

¹H NMR (DMSO-d₆): 0.78 (d, J = 7.0, 3H), 0.80 (d, J = 7.0, 3H), 1.12 - 1.48 (m, 5H), 1.71 - 1.82 (m, 2H), 2.33 (t, J = 7.2, 2H), 2.78 (t, J = 7.2, 2H), 2.80 - 3.00 (m, 4H), 3.70 (s, 3H), 4.10 (t, J = 7.0, 1H) , 5.95 (s, 2H), 6.60 (d, J = 8.0, 2H), 6.75 (m, 3H), 7.17 (m, 1H), 7.40 (d, J = 8.0, 2H), 7.75 (t, J = 5.5, 1H), 12.60 (br s, 1H).

### Example 117. Preparation of Nα-isobutyl-Nα-(4-aminobenzenesulfonyl)-N∈-(2-methoxydihydrocinnamoyl)-L-lysine (compound no. 157)

The product of example 86 is reduced by catalytic hydrogenation under the conditions described in example 4 to yield the desired product.

¹H NMR (DMSO-d₆): 0.78 (d, J = 6.1, 3H), 0.80 (d, J = 6.5, 3H) , 1.15 - 1.48 (m, 5H), 1.70 - 1.92 (m, 2H) , 2.30 (t, J = 7.6, 2H), 2.75 (t, J = 7.6, 2H), 2.80 - 3.00 (m, 4H), 3.80 (s, 3H), 4.10 (t, J = 6.0, 1H) , 5.95 (s, 2H), 6.57 (d, J = 7.8, 2H) , 6.82 (t, J = 7.2, 1H) , 6.92 (d, J = 8.0, 1H), 7.11 (d, J = 8.1, 1H), 8.17 (t, J = 7.2, 1H), 7.70 (t, J = 5.0, 1H), 12.50 (br s, 1H).

### Example 118. Preparation of Nα-isobutyl-Nα-(4-aminobenzenesulfonyl)-N∈-(4-phenylbutanoyl)-L-lysine (compound no. 121)

The product of example 98 is reduced by catalytic hydrogenation under the conditions described in example 4 to yield the desired product.

¹H -NMR (DMSO-d₆) : 0.76 (d, J = 7.0, 3H) , 0.79 (d, J = 7.0, 3H), 1.18 - 1.50 (m, 5H) , 1.72 - 1.80 (m, 4H), 2.06 (t, J = 7.0, 2H) 2.54 (t, J = 7.2, 2H), 2.82 - 2.92 (m, 2H), 2.97 (m, 2H), 4.10 (t, J = 7.0, 1H), 5.95 (s, 2H), 6.60 (d, J = 8.2, 2H), 7.18 (d, J = 8.0, 3H), 7.26 (m, 2H), 7.47 (d, J = 7.5, 2H), 7.70 (d, J = 4.2, 1H) , 12.70 (br s, 1H) .

### Example 119. Preparation of Nα-isobutyl-Nα-(4-aminobenzenesulfonyl)-N∈-(4-aminodihydrocinnamoyl)-L-lysine (compound no. 194)

The product of example 97 is reduced by catalytic hydrogenation under the conditions described in example 4 to yield the desired product.

¹H NMR (DMSO-d₆): 0.78 (d, J = 7.0, 3H), 0.80 (d, J = 6.5, 3H), 1.15 - 1.48 (m, 5H), 1.70 - 1.90 (m, 2H), 2.21 (t, J = 7.6, 2H), 2.62 (t, J = 7.6, 2H), 2.70 - 3.00 (m, 4H), 4.12 (t, J = 7.0, 1H), 5.94 (s, 2H), 6.46 (d, J = 7.5, 2H), 6.57 (d, J = 7.5, 2H), 6.82 (d, J = 7.5, 2H), 7.40 (d, J = 7.2, 2H), 7.69 (t, J = 5.2, 1H), 12.60 (br s, 1H).

### Example 120. Preparation of Nα-isobutyl-Nα-(4-aminobenzenesulfonyl)-N∈-[3-(3-pyridyl)propionyl]-L-lysine (compound no. 195)

The product of example 108 is reduced by catalytic hydrogenation under the conditions described in example 4 to yield the desired product.

¹H NMR (DMSO-d₆): 0.77 (d, J = 6.2, 3H) , 0.80 (d, J = 6.5, 3H), 1.10 - 1.48 (m, 5H), 1.70 - 1.90 (m, 2H), 2.38 (t, J = 7.5, 2H), 2.80 (t, J = 7.5, 2H), 2.84 - 3.00 (m, 4H), 4.10 (t, J = 7.0, 1H), 5.95 (s, 2H), 6.58 (d, J = 7.0, 2H), 7.28 (m, 1H), 7.40 (d, J = 7.1, 2H), 7.60 (d, J = 8.0, 1H), 7.78 (d, J = 5.5, 2H), 8.38 (d, J = 4.3, 1H), 8.41 (s, 1H), 12.70 (br s, 1H).

### Example 121. Preparation of Nα-isobutyl-Nα-(4-aminobenzenesulfonyl)-N∈-(2,4-dimethoxydihydrocinnamoyl)-L-lysine (compound no. 196)

The product of example 96 is reduced by catalytic hydrogenation under the conditions described in example 4 to yield the desired product.

¹H NMR (DMSO-d₆): 0.78 (d, J = 7.0, 3H), 0.80 (d, J = 6.5, 3H), 1.17 - 1.50 (m, 5H), 1.70 - 1.95 (m, 2H), 2.22 (t, J = 7.0, 2H), 2.68 (t, J = 7.0, 2H), 2.82 - 3.00 (m, 4H), 3.71 (s, 3H), 3.75 (s, 3H), 4.10 (t, J = 7.0, 1H), 5.95 (s, 2H), 6.40 (m, 1H), 6.50 (s, 1H), 6.58 (d, J = 8.7, 2H), 6.99 (d, J = 8.6, 1H), 7.40 (d, J = 8.7, 2H), 7.70 (t, J = 5.0, 1H), 12.70 (br s, 1H).

### Example 122. Preparation of Nα-isobutyl-Nα-(4-aminobenzenesulfonyl)-N∈-(2,5-dimethoxydihydrocinnamoyl)-L-lysine (compound no. 197)

The product of example 95 is reduced by catalytic hydrogenation under the conditions described in example 4 to yield the desired product.

¹H NMR (DMSO-d₆): 0.78 (d, J = 7.0, 3H) , 0.80 (d, J = 7.0, 3H), 1.15 - 1.50 (m, 5H), 1.72 - 1.90 (m, 2H), 2.26 (t, J = 7.6, 2H), 2.70 (t, J = 7.6, 2H), 2.82 - 3.00 (m, 4H), 3.66 (s, 3H), 3.72 (s, 3H), 4.10 (t, J = 7.0, 1H), 5.95 (s, 2H), 6.58 (d, J = 7.9, 2H), 6.70 (s, 2H), 6.84 (m, 1H), 7.70 (t, J = 5.0, 2H), 12.70 (br s, 1H).

### Example 123. Preparation of Nα-isobutyl-Nα-(4-aminobenzenesulfonyl)-N∈-3,5-dimethoxydihydrocinnamoyl-L-lysine (compound no. 198)

The product of example 94 is reduced by catalytic hydrogenation under the conditions described in example 4 to yield the desired product.

¹H NMR (DMSO-d₆): 0.78 (d, J = 6.7, 3H), 0.80 (d, J = 7.0, 3H), 1.15 - 1.50 (m, 5H), 1.70 - 1.90 (m, 2H), 2.30 (t, J = 7.2, 2H), 2.75 (t, J = 7.2, 2H), 2.82 - 2.99 (m, 4H), 3.70 (s, 6H), 5.95 (s, 2H), 6.30 (s, 1H), 6.35 (s, 2H), 6.57 (d, J 8.0, 2H), 7.40 (d, J = 8.0, 2H), 7.75 (t, J = 5.5, 1H), 12.50 (br s, 1H).

### Example 124. Preparation of Nα-isobutyl-Nα-(4-aminobenzenesulfonyl)-N∈-dihydrocinnamoyl-L-lysine (compound no. 158)

The product of example 88 is reduced by catalytic hydrogenation under the conditions described in example 4 to yield the desired product.

¹H NMR (DMSO-d₆): 0.78 (d, J = 6.2, 3H), 0.81 (d, J = 6.2, 3H), 1.12 - 1.46 (m, 5H), 1.70 - 1.80 (m, 1H), 1.81 - 1.92 (m, 1H) , 2.32 (t, J = 7.0, 2H), 2.78 (t, J = 7.0, 2H), 2.80 - 3.00 (m, 4H), 4.12 (t, J = 7.0, 1H), 5.95 (br s, 2H), 6.60 (d, J = 8.7, 2H), 7.13 - 7.25 (m, 5H), 7.40 (d, J = 8.5, 2H), 7.70 (t, J = 4.0, 1H), 12.70 (br s, 1H).

### Example 125. Preparation of Nα-isobutyl-Nα-(4-methylbenzenesulfonyl)-N∈-(4-hydroxydihydrocinnamoyl)-L-lysine (compound no. 126)

The product of example 109 is reduced by catalytic hydrogenation under the conditions described in example 4 to yield the desired product.

¹H NMR (DMSO-d₆): 0.80 (d, J = 6.1, 3H) , 0.82 (d, J = 6.0, 3H), 1.15 - 1.50 (m, 5H), 1.70 - 1.92 (m, 2H), 2.26 (t, J = 7.5, 2H), 2.37 (s, 3H), 2.67 (t, J = 7.5, 2H), 2.88 - 3.02 (m, 4H) , 4.17 (t, J = 7.0, 1H), 6.63 (d, J = 8.5, 2H), 6.95 (d, J = 7.5, 2H), 7.36 (d, J = 8.2, 2H), 7.66 (d, J = 7.5, 2H), 7.70 (t, J = 5.0, 1H), 9.10 (s, 1H), 12.70 (br s, 1H).

### Example 126. Preparation of Na-isobutyl-Na-(4-methylbenzenesulfonyl)-N∈-dihydrothiocinnamoyi-DL-lysine (compound no. 153)

### Step A. Preparation of Nα-isobutyl-Nα-(4-methylbenzenesulfonyl)-N∈-dihydrocinnamoyl-L-lysine methyl ester

*N*α-isobutyl-*N*α-(4-methylbenzenesulfonyl)-*N*∈-dihydrocinnamoyl-L-lysine is esterified with diazomethane following indications found in example 82 to provide a quantitative yield of the title methyl ester.

¹H NMR (DMSO-d₆): 0.83 (d, J = 6.8, 3H), 0.87 (d, J = 7.0, 3H), 1.32 - 1.75 (m, 5H), 1.88 - 2.00 (m, 2H), 2.42 (s, 3H), 2.50 (t, J = 7.2, 2H), 2.90 and 3.05 (dd, J = 14.5, 7.4, 2H), 3.00 (t, J = 7.0, 2H), 3.50 (s, 3H), 4.40 (t, J = 7.0, 1H), 5.60 (br s, 1H), 7.18 - 7.32 (m, 7H), 7.69 (d, J = 7.8, 2H).

### Step B. Preparation of Nα-isobutyl-Nα-(4-methylbenzenesulfonyl)-N∈-dihydrothiocinnamoyl-L-lysine methyl ester

To a stirred solution of the product from step A of this example (1.0 g, 2.0 mmol) in THF (20 mL) is added Lawesson's reagent (808 mg, 2.00 mmol). The reaction mixture is stirred at room temperature for 3 h, concentrated *in vacuo* and purified by flash chromatography eluting with 60% EtOAc in hexane, providing 980 mg (95%) of the desired thioamide.

¹H NMR (DMSO-d₆): 0.82 (d, J = 7.2, 3H), 0.86 (d, J = 6.2, 3H), 1.35 - 1.45 (m, 2H), 1.55 - 1.98 (m, 5H), 2.45 (s, 3H), 2.88 and 3.05 (dd, J = 14.8, 7.5, 2H), 2.95 (t, J = 7.7, 2H), 3.12 (t, J = 7.5, 2H), 3.50 (s, 3H), 3.60 (m, 2H), 4.42 (t, J = 7.2, 1H), 7.20 - 7.32 (m, 7H), 7.50 (br s, 1H), 7.72 (d, J = 7.6, 2H).

### Step C. Preparation of Nα-isobutyl-Nα-(4-methylbenzenesulfonyl)-N∈-dihydrothiocinnamoyl-DL-lysine

The product from step B of this example is saponified according to the indications of example 35 step D to afford the title compound quantitatively.

¹H NMR (DMSO-d₆): 0.79 (d, J = 6.7, 3H), 0.82 (d, J = 6.5, 3H), 1.40 - 1.50 (m, 4H), 1.78 - 1.92 (m, 3H), 2.37 (s, 3H), 2.80 (t, J = 7.2, 2H), 2.90 (dd, J = 14.3, 7.5, 2H), 2.94 - 3.05 (m, 3H), 4.20 (t, J = 7.0, 1H), 7.17 - 7.30 (m, 5H), 7.37 (d, J = 7.7, 2H), 7.67 (d, J = 7.5, 2H), 9.90 (br s, 1H), 12.70 (br s, 1H).

### Example 127. Preparation of Nα,Nδ-di-(4-bromobenzenesulfonyl)-Nδ-(4-fluorobenzyl)-L-ornithine (compound no. 59)

To a stirred solution of *Nα,Nδ*-di-(4-bromobenzenesulfonyl)-L-ornithine (145 mg, 0.25 mmol) in DMF (2.5 mL) is added NaH. The reaction is stirred at room temperature until the hydrogen evolution stoped. 4-fluorobenzyl bromide (57 mg, 0.3 mmol) in solution in DMF (0.5 mL) is added and the mixture is allowed to stirr at room temperature for 1 h. HCl (1N) is added until acidic pH (-3) and the reaction is extracted with EtOAc. The organic layer is dried (MgSO₄) and concentrated. The crude material is purified by flash chromatography eluting with hexanes:EtOAc:AcOH, 50:50:00; 25:75:00 and then 25:70:0.5 to afford 142 mg (84%) of the title compound.

¹H NMR (DMSO-d₆): 1.20 - 1.50 (m, 4H), 2.98 - 3.10 (m, 2H), 3.55 (m, 1H), 4.20 (s, 2H), 7.10 - 7.33 (m, 4H), 7.60 - 7.80 (m, 8H), 8.18 (d, J = 9.0, 1H), 12.60 (br s, 1H).

### Example 128. Preparation of Nα,N∈-di-(4-bromobenzenesulfonyl)-N∈-(4-fluorobenzyl)-L-lysine (compound no. 60)

*N*α,*N*∈-di-(4-bromobenzenesulfonyl)-L-lysine is reacted with 4- fluorobenzyl bromide under the conditions described in example 127 to yield 85% of the desired product.

¹H NMR (DMSO-d₆) : 1.05 - 1.45 (m, 6H), 2.92 - 3.05 (m, 2H), 3.55 (m, 1H), 4.26 (s, 2H), 7.12 - 7.37 (m, 4H), 7.60 - 7.85 (m, 8H), 8.17 (d, J = 8.1, 1H) , 12.30 (br s, 1H) .

### Example 129. Preparation of Nα,N∈-diisobutyl-Nα-(4-methylbenzenesulfonyl)-N∈-(3-phenylpropanoyl)-DL-lysine (compound no. 159)

### Step A. Preparation of Nα,N∈-diisobutyl-Nα-(4-methylbenzenesulfonyl)-N∈-(phenylpropanoyl)-L-lysine methyl ester

The product of example 35 (step C) (*N*α-isobutyl-*N*α-(4-methylbenzenesulfonyl)-*N*∈-benzyloxycarbonyl-L-lysine methyl ester) is reduced by catalytic hydrogenation under the conditions described in example 4 to yield the free amine which is subjected to reductive alkylation under the conditions described in example 35 (step B) followed by acylation with 3-phenylpropionyl chloride under the conditions described in example 35 (step C) to give the title compound (75% yield).

¹H NMR (CDCl₃): 0.83 - 0.89 (m, 12H, 4 CH₃), 1.15 - 1.65 (m, 5H), 1.82 - 2.00 (m, 3H), 2.42 (s, 3H), 2.60 (m, 2H), 2.70 (m, 2H), 2.93 - 3.05 (m, 5H), 3.17 (m, 1H), 3.22 - 3.40 (m, 1H), 5.5 (s, 3H), 4.40 (m, 1H) , 7.22 - 7.33 (m, 3H).

### Step B. Preparation of Nα,N∈- diisobutyl-Nα-(4-methylbenzenesulfonyl)-N∈-(3-phenylpropanoyl)DL-lysine

The product from step A of this example is saponified according to the indications of example 35 step D to afford the title compound quantitatively.

¹H NMR (DMSO-d₆): 0.76 - 0.83 (m, 12H, 4 CH₃), 1.09 - 1.50 (m, 5H), 1.78 - 1.92 (m, 3H), 2.38 (s, 3H), 2.52 (m, 2H), 2.80 (m, 2H), 2.85 - 3.15 (m, 6H), 4.20 (t, J = 7.0, 1H), 7.38 (m, 2H), 7.67 (t, J = 8.8, 2H), 12.65 (br s, 1H).

### Example 130. Preparation of Nα-isobutyl-Nα-(4-aminobenzenesulfonyl)-N∈-phenoxyacetyl-L-lysine (compound no. 192)

The product of example 104 is reduced by catalytic hydrogenation under the conditions described in example 4 to yield 100% of the title compound.

¹H NMR (DMSO-d₆): 0.78 (d, J = 6.0, 3H), 0.80 (d, J = 6.0, 3H), 1.17 - 1.50 (m, 5H), 1.72 - 1.90 (m, 2H), 2.82 and 2.90 (ABX, J = 14.0, 7.5, 2H), 3.08 (m, 2H), 4.10 (t, J = 7.2, 1H), 4.43 (s, 2H), 5.95 (br s, 2H), 6.60 (d, J = 7.6, 2H), 6.90 - 7.00 (m, 3H), 7.30 (m, 2H), 7.39 (d, J = 7.5, 2H), 8.02 (t, J = 5.0, 1H), 12.70 (br s, 1H).

### Example 131. Preparation of Nα-isobutyl-Nα-(4-methylbenzenesulfonyl)-N∈-(2,3-dimethoxydihydrocinnamoyl)-L-lysine (compound n. 201)

### Step A. Preparation of Nα-isobutyl-Nα-(4-methylbenzenesulfonyl)-N∈-(2,3-dimethoxycinnamoyl)-L-lysine

*N*α-isobutyl-*N*α-(4-methylbenzenesulfonyl)-L-lysine is reacted with 2,3-dimethoxycinnamic acid under the conditions described in example 86. The crude material is purified by flash chromatography (CH₂Cl₂:MeOH, 49:1 to 9:1) to yield 18% of the desired product.

¹H NMR (DMSO-d₆) : 0.81 (m, 6H), 1.24 (m, 2H), 1.40 (m, 3H), 1.87 (m, 2H), 2.37 (s, 3H), 2.95 (m, 2H), 3.09 (s, 2H), 3.74 (s, 3H), 3.82 (s, 3H), 4.19 (s, 1H), 6.60 (d, J = 16.0, 1H), 7.10 (m, 3H), 7.36 (d, J = 7.0, 2H), 7.58 (d, J = 15.0, 1H), 7.68 (d, J = 7.0, 2H), 8.07 (s, 1H), 12.65 (br s, 1H).

### Step B. Preparation of Nα-isobutyl-Nα-(4-methylbenzenesulfonyl)-N∈-(2,3-dimethoxydihydrocinnarnoyl)-L-lysine

The product of step A is reduced by catalytic hydrogenation under the conditions described in example 4 to yield 95% of the title compound.

¹H NMR (CDCl₃): 0.87 (s, 6H), 1.16 (m, 2H), 1.37 (m, 2H), 1.56 (m, 1H), 1.90 (m, 2H), 2.34 (t, J = 8.0, 2H), 2.40 (s, 3H), 2.82 (t, J = 8.0, 2H), 2.99 (m, 2H), 3.75 (s, 3H), 3.81 (s, 3H), 4.23 (t, J = 7.0, 1H), 6.79 (d, J = 7.0, 1H) , 6.90 (d, J = 8.0, 1H), 6.99 (t, J = 8.0, 1H), 7.40 (d, J = 8.0, 2H), 7.72 (d, J = 8.0, 2H), 7.78 (s, 1H).

### Example 132. Preparation of Nα-isobutyl-Nα-(4-methylbenzenesulfonyl)-N∈-phenylthioacetyl-L-lysine (compound no. 202)

*N*α-isobutyl-*N*α-(4-methylbenzenesulfonyl)-L-lysine is reacted with (phenylthio)acetyl choride under conditions described in example 2. The crude material is purified by flash chromatography (CH₂Cl₂:MeOH, 19:1 to 9:1) to yield 38% of the desired product.

¹H NMR (CDCl₃): 0.85 (s, 6H), 1.01 (s, 2H), 1.32 (m, 2H), 1.47 (m, 1H), 1.86 (m, 2H), 2.41 (s 3H), 3.02 (d, J = 10.0, 2H), 3.07 (m, 1H), 3.13 (m, 1H), 3.62 (s, 2H), 4.13 (m, 1H), 6.80 (s, 1H), 7.20 - 7.30 (m, 7H), 7.69 (d, J = 10.0, 2H).

### Example 133. Preparation of Nα-isobutyl-Nα-(4-methylbenzenesulfonyl)-N∈-phenoxyacetyl-L-lysine (compound no. 203)

*N*α-isobutyl-*N*α-(4-methylbenzenesulfonyl)-L-lysine is reacted with phenoxyacetyl chloride under the conditions described in example 2. The crude material is purified by flash chromatography (CH₂Cl₂:MeOH, 19:1 to 9:1) to yield 77% of the desired product.

¹H NMR (CDCl₃): 0.87 (s, 6H), 1.33 (s, 2H), 1.54 (m, 2H), 1.64 (m, 1H), 1.95 (m, 2H), 2.40 (s, 3H), 2.98 (m, 1H), 3.04 (m, 1H), 3.30 (s, 2H), 4.34 (m, 1H), 4.50 (s, 2H), 6.78 (s, 1H), 6.94 (d, J = 7.0, 2H), 7.04 (t, J = 7.0, 1H), 7.29 (m, 2H), 7.33 (m, 2H), 7.72 (d, J = 7.0, 1H), 8.47 (br s, 1H).

### Example 134. Preparation of Nα-isobutyl-Nα-(4-methylbenzenesulfonyl)-N∈-(dihydrothiocinnamoyl-N-cyanoamidine) - DL-lysine (compound no. 204)

To a stirred solution of *Nα*-isobutyl-*Nα*- (4-methylbenzenesulfonyl)-*N∈*-dihydrothiocinnamoyl-L-lysine methyl ester (example 126 step B) (170 mg, 0.33 mmol) in MeOH (3 mL) is added cyanamide (28 mg, 0.66 mmol). The mixture is stirred for 5 min, then mercuric acetate (209 mg, 0.66 mmol) is added. The reaction mixture is stirred for 3 h, then diluted with a saturated solution of NH₄Cl and extracted with EtOAc. The organic layer is concentrated then diluted with THF/MeOH (1 mL/1 mL) and treated with 1N NaOH (1.2 mL). After stirring for 4 h, the reaction is acidified with 1N HCl (pH ~ 1 -2) and extracted with EtOAc. The organic layer is dried, concentrated and purified by flash chromatography (hexane: EtOAc: AcOH, 30:70:0.4) to give 110 mg (65%) of the title compound.

¹H NMR (DMSO-d₆): 0.79 (d, J = 6.0, 3H), 0.83 (d, J = 6.0, 3H), 1.10 - 1.20 (m, 2H), 1.22 - 1.35 (m, 2H), 1.40 - 1.50 (m, 1H), 1.78 (m, 1H), 1.90 (m, 1H), 2.33 (t, J = 7.6, 2H), 2.80 (t, J = 7.5, 2H), 2.88 - 3.00 (m, 4H), 4.20 (t, J = 7.2, 1H), 7.15 - 7.30 (m, 5H), 7.37 (d, J = 7.6, 2H), 7.66 (d, J = 7.5, 2H), 7.73 (t, J = 5.3, 1H), 12.70 (br s, 1H).

### Example 135. Preparation of (2R,2S)-2-[N-isobutyl-N-(4-methylbenzenesulfonyl)]-3-[2'-(N' -dihydrocinnamoyl)ethylamino] - propionic acid (compound no. 206)

### Step A. Preparation of Nα-isobutyl-L-serine methyl ester

L-serine methyl ester is subjected to reductive alkylation under the conditions described in example step B to give 66% of the desired product.

¹H NMR (CDCl₃): 0.89 (d, J = 6.3, 3H) , 0.91 (d, J = 6.3, 3H), 1.70 (h, J = 7.0, 3H), 2.28 and 2.50 (ABX, J = 11.1, 7.3, 2H), 3.33 (m, 1H), 3.20 - 3.40 (br s, 1H), 3.58 (m, 1H), 3.73 (m, 3H), 3.76 (m, 1H).

### Step B. Preparation of Nα-isobutyl-Nα-4-methylbenzenesulfonyl-L-serine methyl ester

To a stirred solution of *N*α-isobutyl-L-serine methyl ester (1.2 g, 6.93 mmol) in dioxane/water (20 mL/10 mL) is added NaHCO₃ (614 mg, 7.63 mmol). The mixture is stirred at 40°C overnight, then acidified with 1N HCl (pH ~ 1) and extracted with EtOAc. The organic layer is dried (MgSO₄) and concentrated. The crude is purified by flash chromatography (hexane:EtOAc, 70:30) to yield 1.3 g (81%) of the iosylate.

¹H NMR (CDCl₃): 0.82 (d, J = 6.3, 3H), 0.85 (d, J = 6.2, 3H), 1.85 - 1.92 (m, 1H), 2.41 (s, 3H), 2.52 (br s, 1H), 2.90 and 3.10 (ABX, J = 15.2, 7.4, 2H), 3.58 (s, 3H), 3.80 (m, 1H), 4.11 (t, J = 8.0, 1H), 4.39 (t, J = 7.2, 1H), 7.28 (d, J = 8.2, 2H) , 7.70 (d, J = 8.0, 2H).

### Step C. Preparation of 2-[N-isobutyl-N-(4-methylbenzenesulfonyl)]methyl acrylate

To a stirred solution of the tosylate (330 mg, 1 mmol) in CH₂Cl₂ (10 mL) is added triethylamine (153 µL, 1.1 mmol) and tosyl chloride (209 mg, 1,1, mmol). The reaction is stirred for 4 h, then triethylamine (306 µL, 2.2 mmol) is added. The reaction mixture is allowed to stir overnight. It is diluted with 1N HCl and EtOAc, the organic layer is collected and concentrated. The crude is purified by flash chromatography (hexane:EtOAc, 4:1) to yield 220 mg (71%) of the acrylate.

¹H NMR (CDCl₃) : 0.89 (d, J = 6.7, 6H), 1.70 (h, J = 7.0, 1H), 2.41 (s, 3H), 3.15 (d, J = 7.5, 2H), 3.65 (s, 3H), 5.71 (s, 1H), 6.36 (s, 1H), 7.28 (d, J = 8.0, 2H), 6.67 (d, J = 8.0, 2H).

### Step D. Preparation of (2R,2S)-2-[N-isobutyl-N-(4-methylbenzenesulfonyl)]-3-[2'-(N'-dihydrocinnamoyl)ethylamino]-propionic acid methyl ester

Triethylamine (55 µL, 0.4 mmol) is added to a stirred solution of the acrylate (104 mg, 0.33 mmol) and N-dihydrocinnamoyl ethylenediamine trifluoroacetic acid salt (111 mg, 0.36 mmol) in MeOH. The mixture is stirred for 2 days then concentrated and purified by flash chromatography (CH₂Cl₂:MeOH, 95:05) to yield the amine ester (80 mg, 50%).

¹H NMR (DMSO-d₆): 0.79 (d, J = 7.2, 3H), 0.80 (d, J = 7.0, 3H), 1.65 (br s, 1H), 1.88 (h, J = 7.2, 1H) , 2.30 - 2.36 (m, 2H), 2.38 (s, 3H), 2.42 (t, J = 6.1, 2H), 2.65 (dd, J = 12.5, 7.0, 1H) , 2.80 (m, 3H), 2.90 - 3.10 (m, 5H), 3.46 (s, 3H), 4.35 (t, J = 7.2, 1H) , 7.14 - 7.30 (m, 5H), 7.39 (d, J = 8.4, 2H) , 7.70 (d, J = 8.2, 2H), 7.73 (t, J = 5.0, 1H).

### Step E. Preparation of (2R,2S)-2- [N-isobutyl-N- (4-methylbenzenesulfonyl)]-3-[2'-(N'-dihydrocinnamoyl)ethylamino]-propionic acid

NaOH (100 µL, 1N) is added to a stirred solution of aminoester (45 mg, 0.089 mmol) in THF/MeOH (1 mL/1 mL). The reaction is stirred for 3 h then acidified with TFA and concentrated. The crude is purified by flash chromatography (CH₂Cl₂:MeOH, 4:1) to yield the desired product (35 mg, 80%).

¹H NMR (DMSO-d₆): 0.75 (d, J = 6.4, 3H), 0.78 (d, J = 7.1, 3H), 1.80 (h, J = 7.0, 1H), 2.36 (s, 3H), 2.39 (m, 2H), 2.80 - 2.88 (m, 4H), 2.90 and 3.00 (ABX, J = 14.5, 7.4, 2H), 3.12 (t, J = 8.0, 1H), 3.20 - 3.28 (m, 1H), 4.20 (dd, J = 11.0, 5.0, 1H), 7.16 - 7.28 (m, 5H), 7.33 (d, J = 8.0, 2H), 7.73 (d, J = 8.0 2H), 7.99 (t, J = 5.0, 1H), 9.25 - 9.75 (br s, 1H).

## Claims

1. Use of a compound of formula (Ia), or a pharmaceutically acceptable salt thereof: 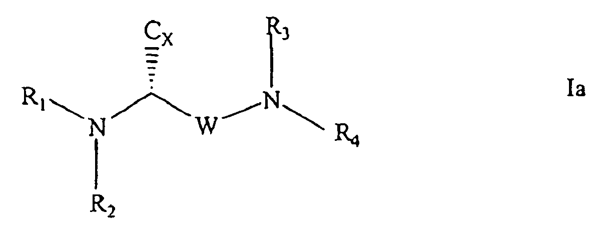 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 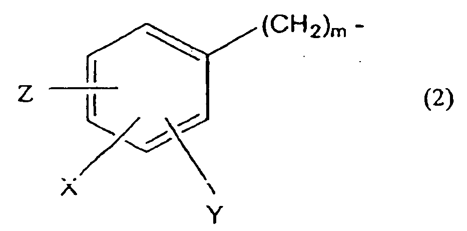 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-,3-indole-CH₂CO-,3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 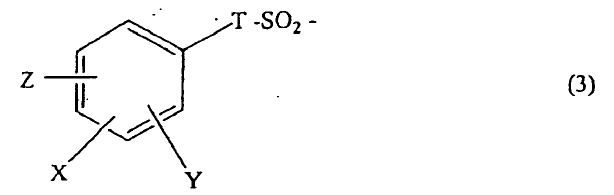 a heterocycle-alkylsulfonyl group of formula heterocycle-(CH₂)ₘ-SO₂- and a carbonyl group of formula (4) 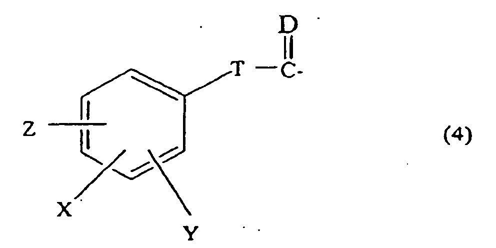 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, - CF₃, - NO₂, -NH₂, -NHR₅, -NR₅R₆, -NHCOR₅, -NHCOheterocycle, heterocycle being as defined above, -OR₅, -SR₅, -SOR₅, -SO₂R₅, - COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂ - NHR₅, -NR₅R₆, -NHCOR_{5,} -OR₅, -SR₅, -SOR₅, -SO₂R₅, -COOR₅, -CH₂OH, - COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings for the preparation of a medicament for treating or preventing Alzheimer's disease in a subject in need of such treatment.

2. The use according to claim 1 for treating Alzheimer's disease in a subject in need of such treatment.

3. The use according to claim 1 or 2 for treating Alzheimer's disease by modulating the activity of beta amyloid converting enzyme.

4. The use according to claim 1, further comprising the use of a P-gp inhibitor, or a pharmaceutically acceptable salt thereof.

5. Use of a compound of formula (Ia), or a pharmaceutically acceptable salt thereof: 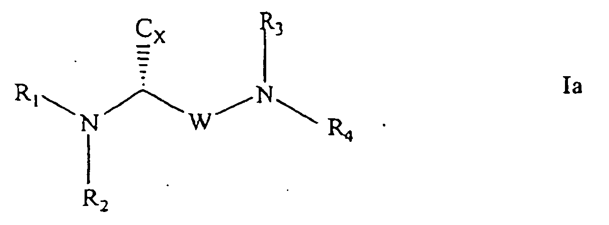 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 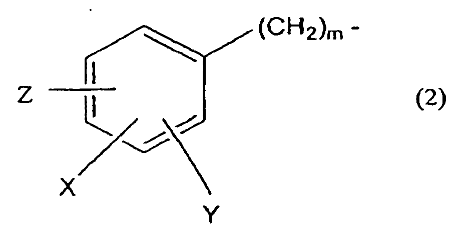 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 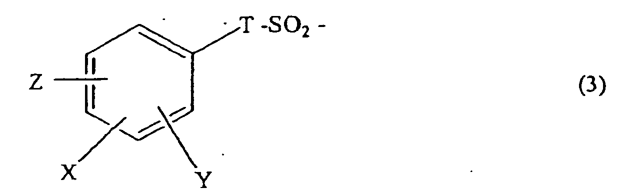 a heterocycle-alkylsulfonyl group of formula heterocycle-(CH₂)ₘ-SO₂- and a carbonyl group of formula (4) 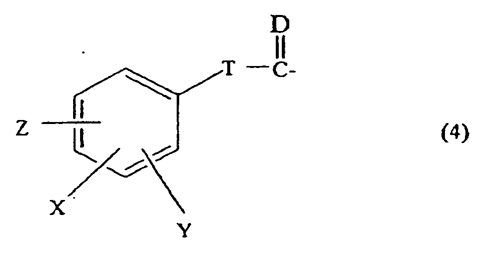 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, - CF₃, - NO₂, -NH₂, -NHR₅, -NR₅R₆, -NHCOR₅, -NHCOheterocycle, heterocycle being as defined above, -OR₅, -SR₅, -SOR₅, -SO₂R₅, - COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂ - NHR₅, -NR₅R₆, -NHCOR_{5,} -OR₅, -SR₅, -SOR₅, -SO₂R₅, -COOR₅, -CH₂OH, - COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings, for the preparation of a medicament for treating a subject who has, or in preventing a subject from getting, a disease or condition selected from the group consisting of Alzheimer's disease, for helping prevent or delay the onset of Alzheimer's disease, for treating subjects with mild cognitive impairment (MCI) and preventing or delaying the onset of Alzheimer's disease in those who would progress from MCI to AD, for treating Down's syndrome, for treating humans who have Hereditary Cerebral Hemorrhage with Amyloidosis of the Dutch-Type, for treating cerebral amyloid angiopathy and preventing its potential consequences, i.e. single and recurrent lobar hemorrhages, for treating other degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease.

6. The use according to any of claim 1-5 wherein the compound of formula Ia is selected from the group consisting of: 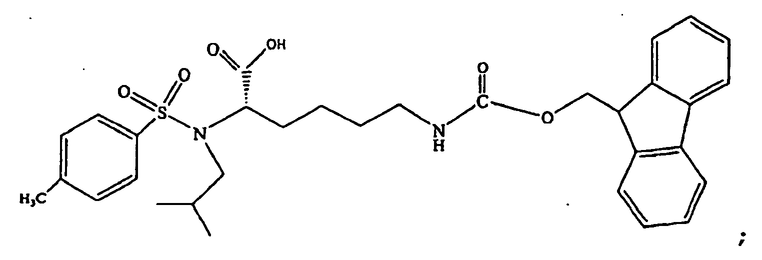 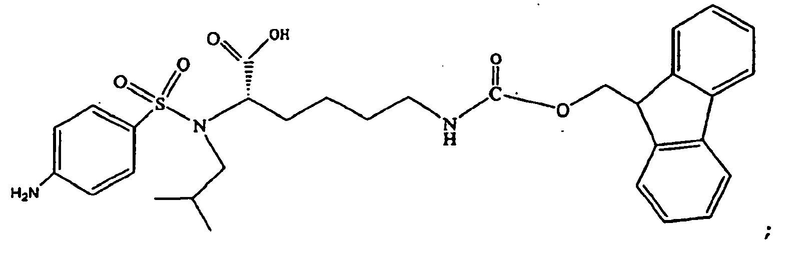 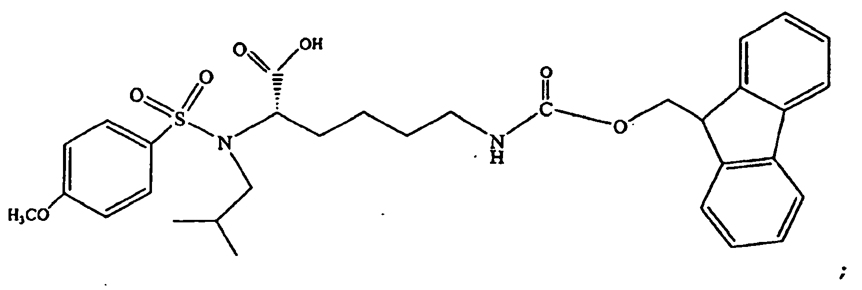 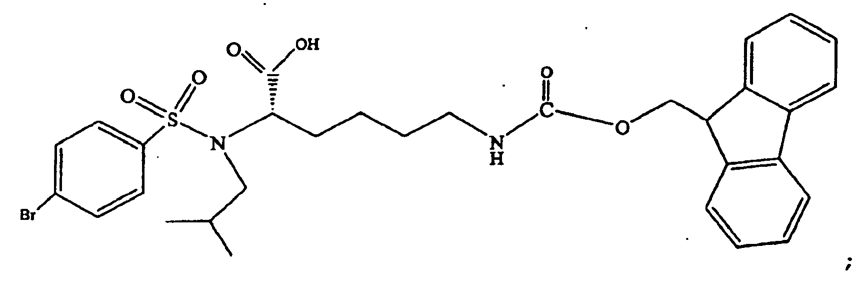 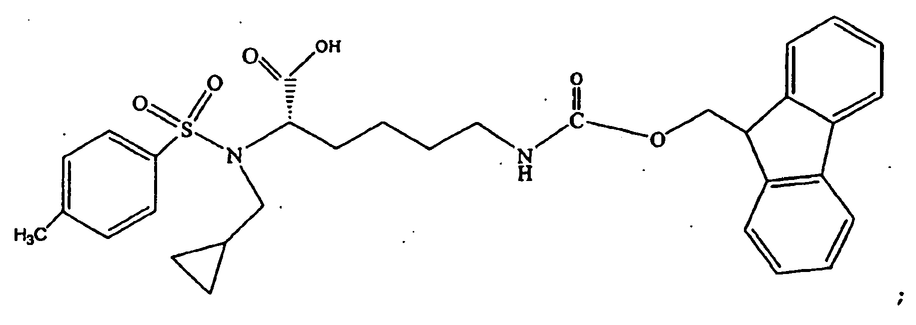 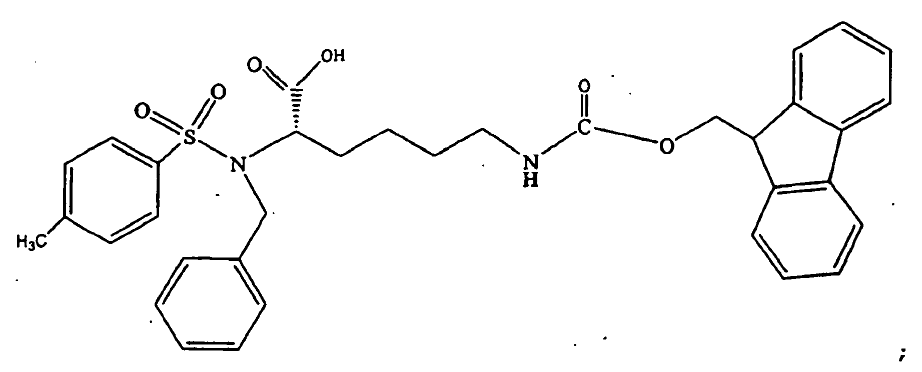 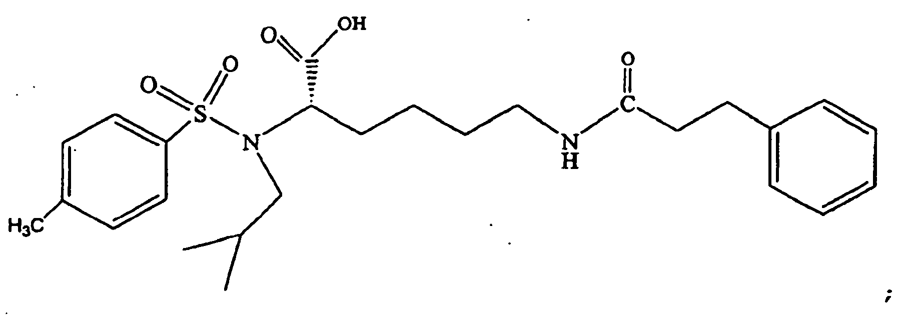 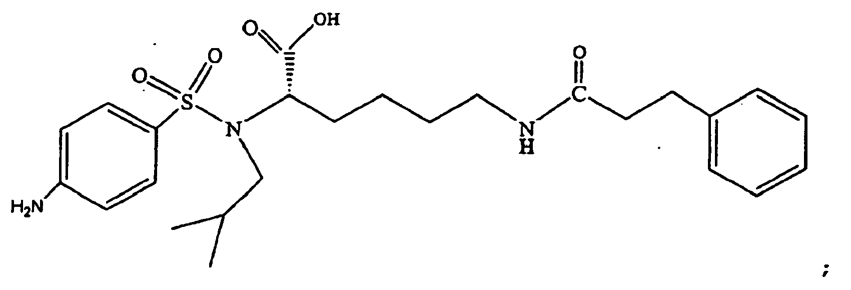 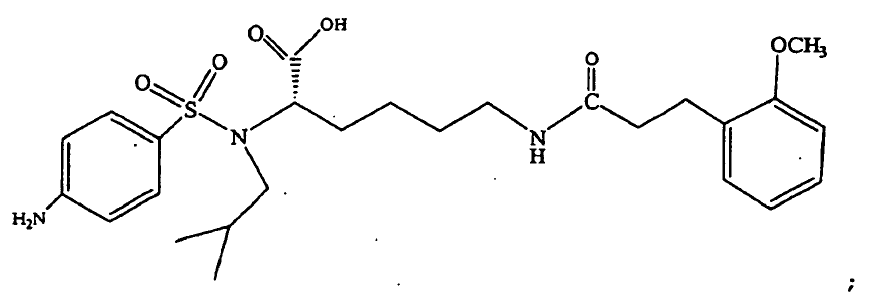 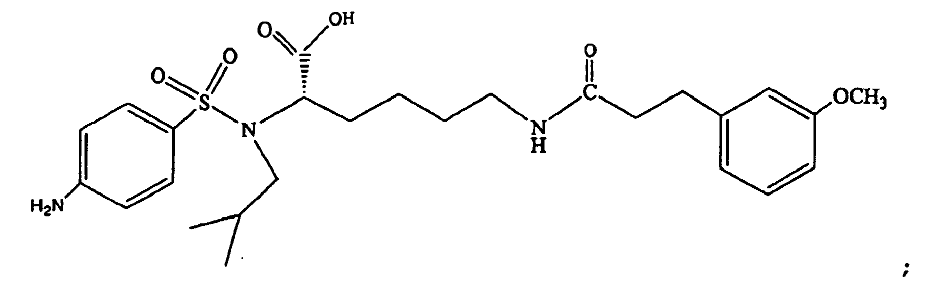 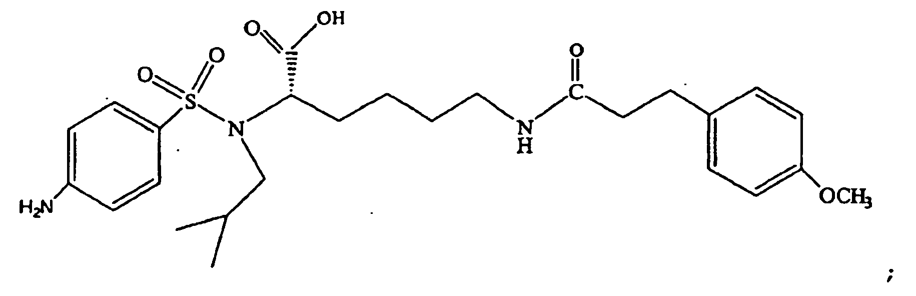 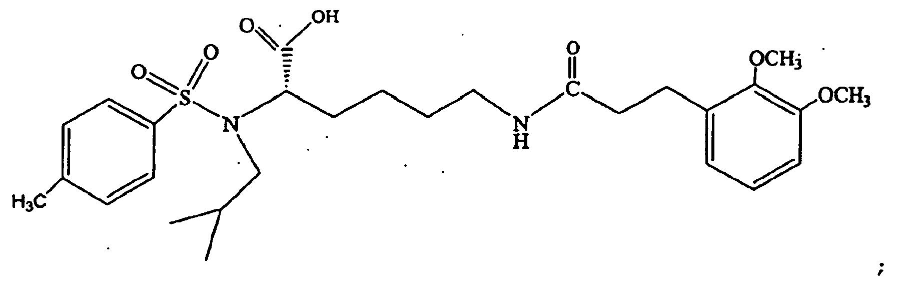 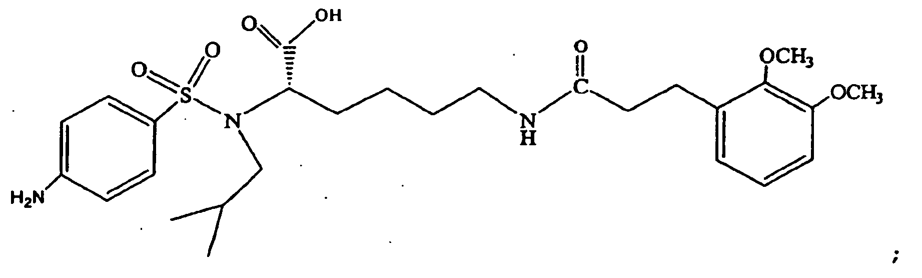 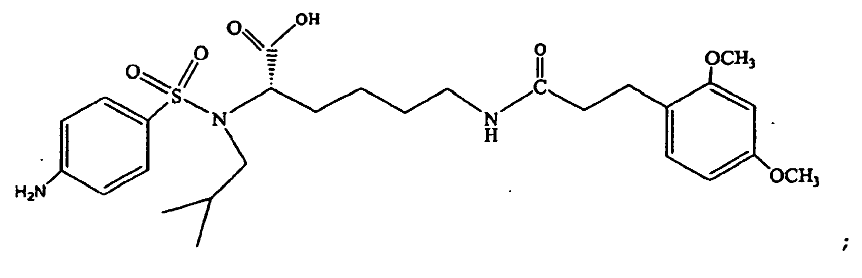 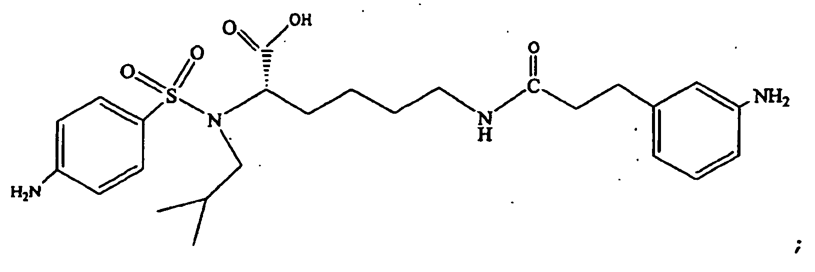 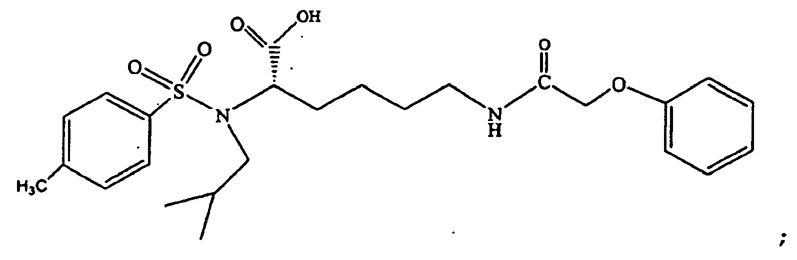 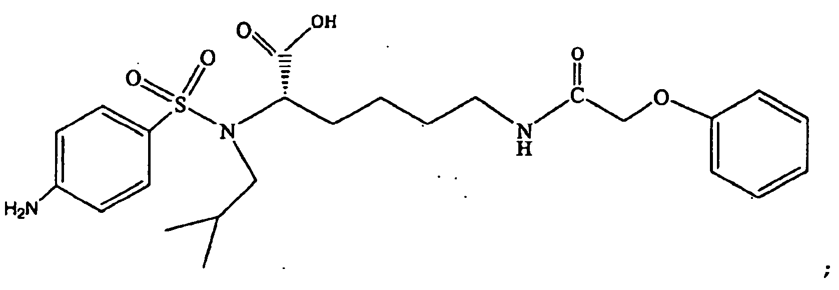 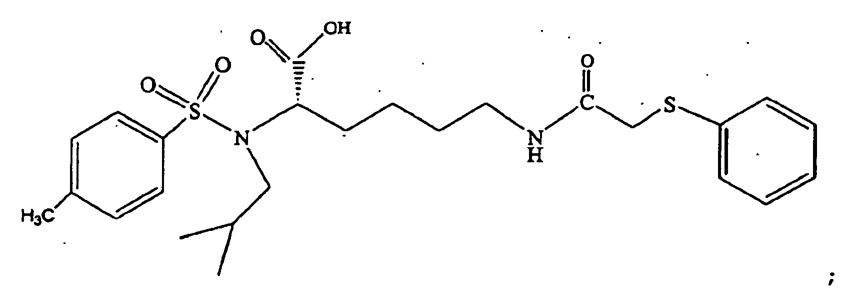 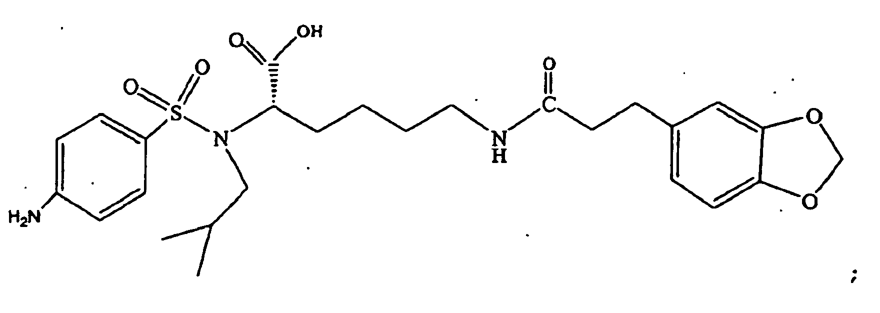 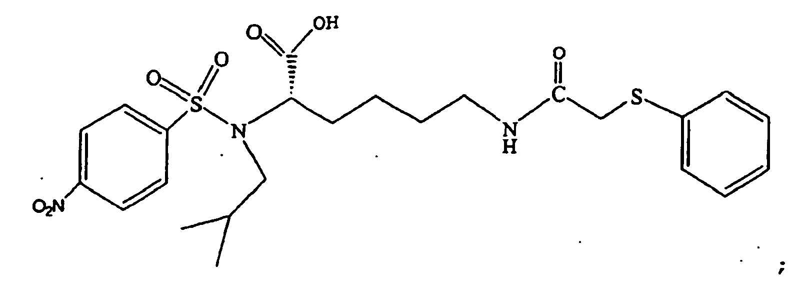 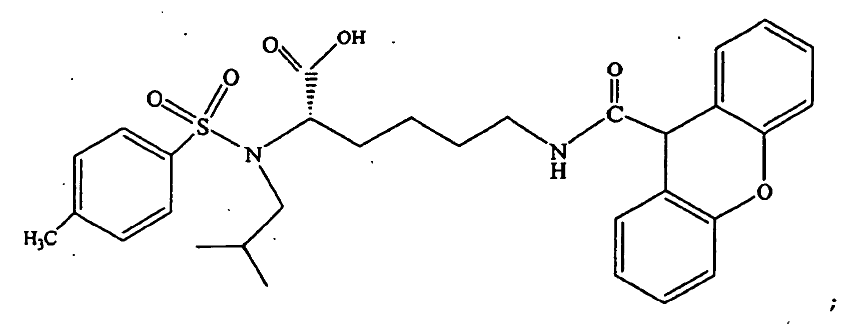 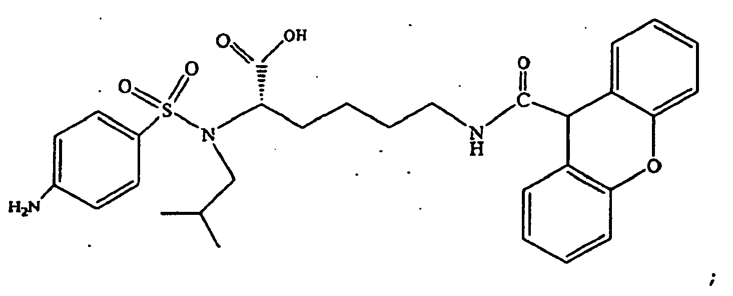 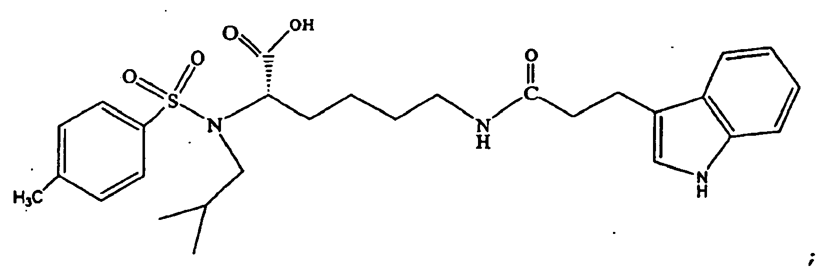 and 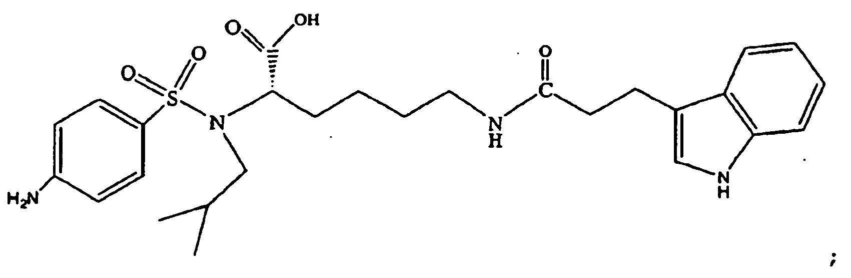 or pharmaceutically acceptable ammonium, K, Na, and Cs salts thereof.

7. Use of a composition comprising one or more pharmaceutically acceptable carriers and a compound of Formula Ia or a pharmaceutically acceptable salt thereof: 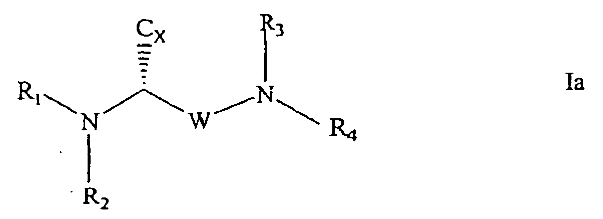 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 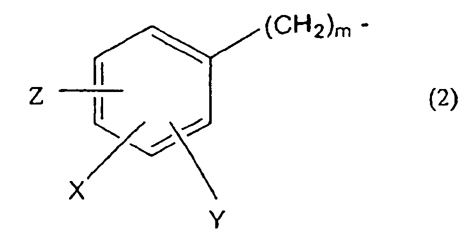 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 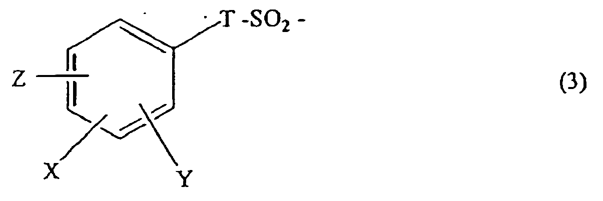 a heterocycle-alkylsulfonyl group of formula heterocycle-(CH₂)ₘ-SO₂- and a carbonyl group of formula (4) 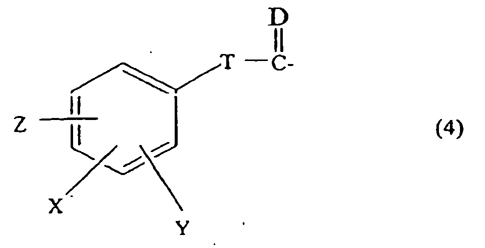 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, -CF₃, - NO₂, -NH₂ -NHR₅, -NR₅R₆, -NHCOR₅, -NHCOheterocycle, heterocycle being as defined above, -OR₅, -SR₅, -SOR₅, -SO₂R₅, -COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂, -NHR₅, -NR₅R₆, - NHCOR₅-OR₅, -SR₅, -SOR₅, -SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings, for the preparation of a medicament for treating or preventing Alzheimer's disease in a subject in need of such treatment.

8. Use of a compound of formula Ia in the manufacture of a medicament for the treatment or prevention of conditions selected from the group consisting of Alzheimer's disease, mild cognitive impairment (MCI), Down's syndrome, Hereditary Cerebral Hemorrhage with Amyloidosis of the Dutch-Type, cerebral amyloid angiopathy, degenerative dementias, including dementias of mixed vascular and degenerative origin, dementia associated with Parkinson's disease, frontotemporal dementias with parkinsonism (FTDP), dementia associated with progressive supranuclear palsy, dementia associated with cortical basal degeneration, or diffuse Lewy body type of Alzheimer's disease: 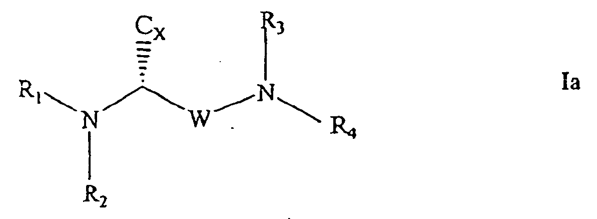 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 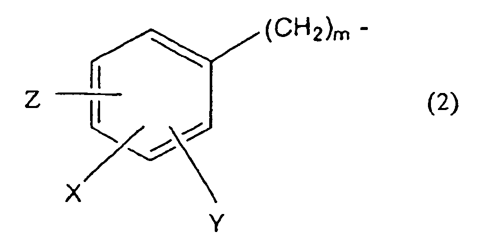 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 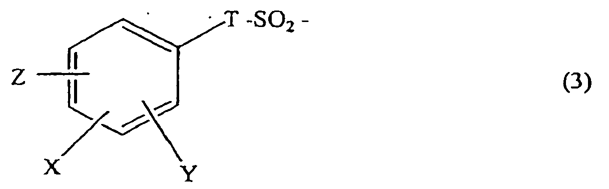 a heterocycle-alkylsulfonyl group of formula heterocycle-(CH₂)ₘ-SO₂- and a carbonyl group of formula (4) 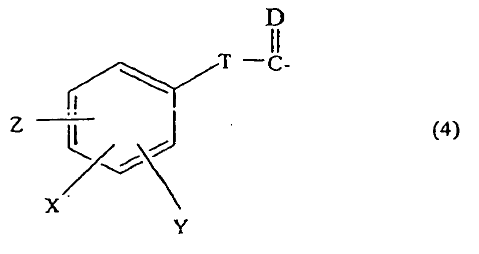 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, - CF₃, - NO₂, -NH₂ -NHR₅, -NR₅R₆, -NHCOR₅, -NHCOheterocycle, heterocycle being as defined above, -OR₅, -SR₅, -SOR₅, -SO₂R₅, - COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂,-NHR₅, -NR₅R₆, -NHCOR₅,-OR₅, -SR₅, -SOR₅, -SO₂R₅, -COOR₅, -CH₂OH,-COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

9. An in vitro method for inhibiting beta-secretase activity, comprising contacting an effective amount for inhibition of a compound of formula Ia: 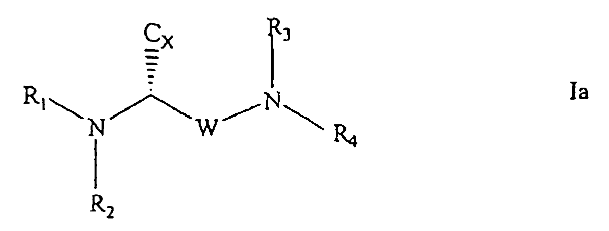 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 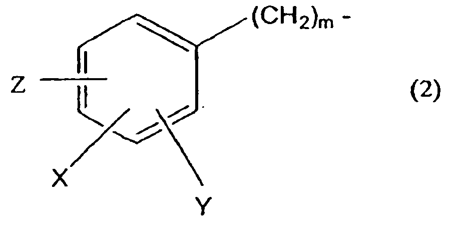 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄⁻-SO₂-, an aryalkyl group of formula (2) as defined above, a sulfonyl group of formula (3) 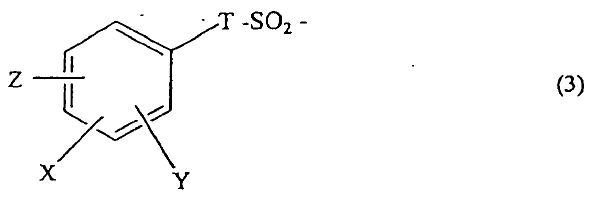 a heterocycle-alkylsulfonyl group of formula heterocycle-(CH₂)ₘ-SO₂- and a carbonyl group of formula (4) 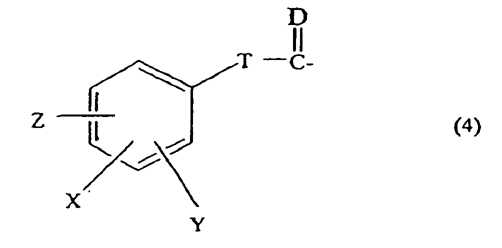 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, -CF₃, - NO₂, -NH₂, -NHR₅, -NR₅R₆, -NHCOR₅, -NHCOheterocycle, heterocycle being as defined above, -OR₅, -SR₅, -SOR₅, -SO₂R₅, -COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂, -NHR₅, -NR₅R₆, - NHCOR₅, -OR₅, -SR₅, -SOR₅, -SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

10. An in vitro method for inhibiting cleavage of an amyloid precursor protein (APP) isotype at a site in the APP isotype that is susceptible to cleavage, comprising contacting said APP isotype with an effective cleavage inhibitory amount of a compound of formula Ia: 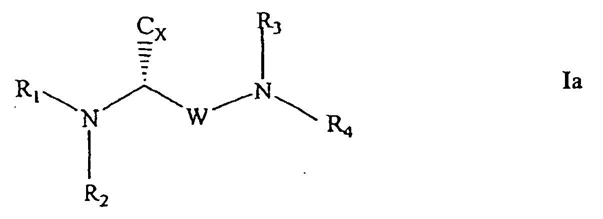 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 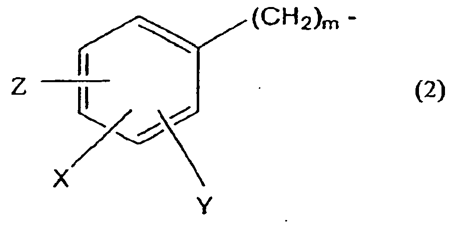 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-,C₆H₅OCH₂CO-, (C₆H₅)₂COHCO-,C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 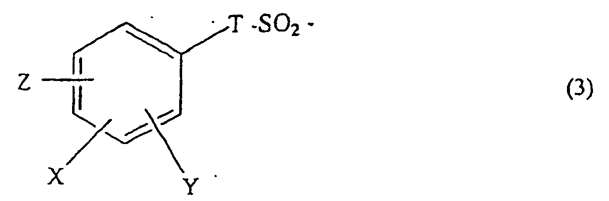 a heterocycle-alkylsulfonyl group of formula heterocycle-(CH₂)ₘ-SO₂- and a carbonyl group of formula (4) 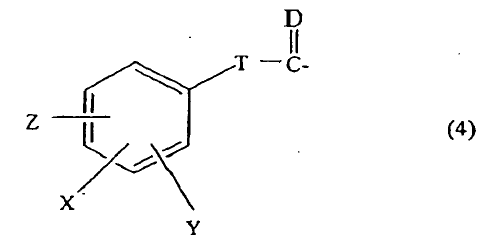 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, -CF₃, - NO₂, -NH₂, -NHR₅, -NR₅R₆, -NHCOR₅, -NHCOheterocycle, heterocycle being as defined above, -OR₅, -SR₅, -SOR₅, -SO₂R₅, -COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂, -NHR₅, -NR₅R₆, - NHCOR₅,-OR₅, -SR₅, -SOR₅, -SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

11. An in vitro method for inhibiting production of amyloid beta peptide (A beta) in a cell, comprising administering to said cell an effective inhibitory amount of a compound of formula Ia: 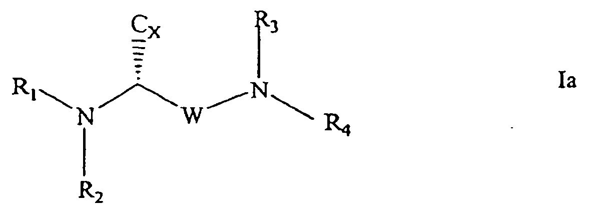 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 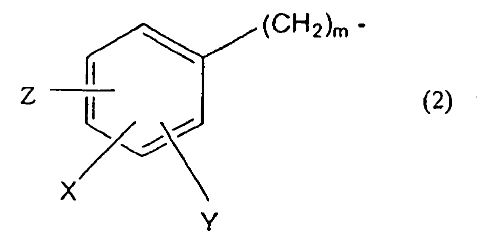 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 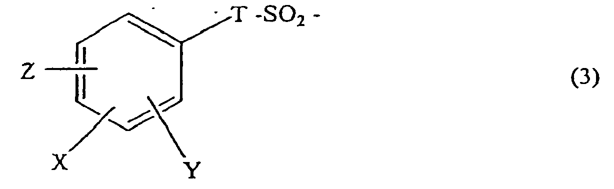 a heterocycle-alkylsulfonyl group of formula heterocycle-(CH₂)ₘ-SO₂- and a carbonyl group of formula (4) 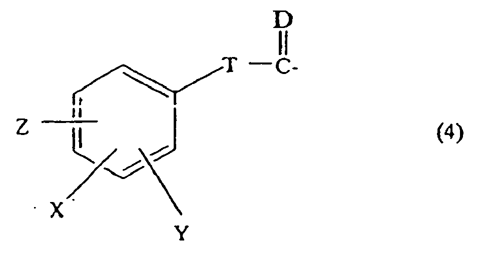 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, -CF₃, - NO₂, -NH₂, -NHR₅, -NR₅R₆, -NHCOR₅, -NHCOheterocycle, heterocycle being as defined above, -OR₅, -SR₅, -SOR₅, -SO₂R₅, -COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂, -NHR₅, -NR₅R₆, - NHCOR₅-OR₅, -SR₅, -SOR₅, -SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

12. The method of claim 11, wherein the cell is an animal cell.

13. The method of claim 12, wherein the animal cell is a mammalian cell.

14. The method of claim 13, wherein the mammalian cell is human.

15. A composition comprising beta-secretase complexed with a compound of formula Ia: 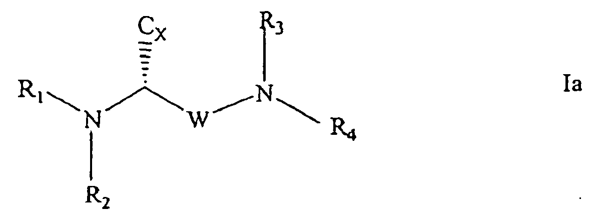 and wherein the compound of formula Ia comprises an amino group or pharmaceutically acceptable ammonium salts thereof,
wherein W is selected from the group consisting of -(CH₂)ₙ, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyllysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 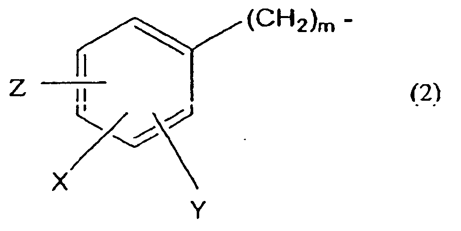 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, *tert-*butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 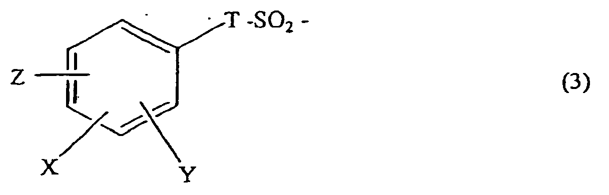 a heterocycle-alkylsulfonyl group of formula heterocycle-(CH₂)ₘ-SO₂- and a carbonyl group of formula (4) 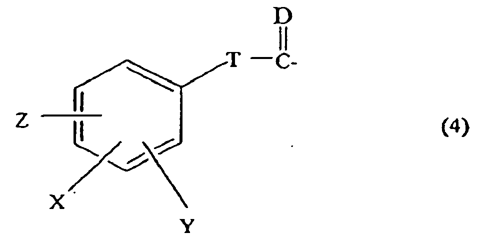 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected (i.e. independently) from the group consisting of H, a straight or branched alkyl group of 1 to 6 carbon atoms, F, Cl, Br, I, -CF₃, - NO₂, -NH₂, -NHR₅, -NR₅R₆, -NHCOR₅, -NHCOheterocycle, heterocycle being as defined above, -OR₅, -SR₅, -SOR₅,-SO₂R₅, -COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂, -NHR₅, -NR₅R₆, - NHCOR₅,-OR₅, -SR₅, -SOR₅, -SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

16. A method for producing a beta-secretase complex comprising the composition of claim 15.

17. Use of a compound of formula Ia: 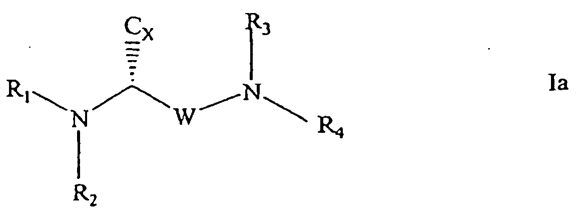 and wherein the compound of formula Ia comprises an amino group or pharmaceutically acceptable ammonium salts thereof,
wherein W is selected from the group consisting of -(CH₂)ₙ₋, and -CH₂-XX-CH₂-CH₂-
wherein n is 1, 2, 3, 4 or 5
wherein XX is selected from the group consisting of 0, NR₅, S, SO and SO₂
wherein Cx is selected from the group consisting of -COOM, -COOR₅, -CH₂OH, -CONR₅R₆, -CONHOH, 9-fluorenylmethoxycarbonyl-lysyl-NH-CO-, benzyloxycarbonyl, and tetrazolyl,
wherein M is an alkali metal or an alkaline earth metal,
wherein R₁ and R₃, the same or different, are selected from the group consisting of H, *tert*-butoxycarbonyl, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof, an arylalkyl group of formula (2) 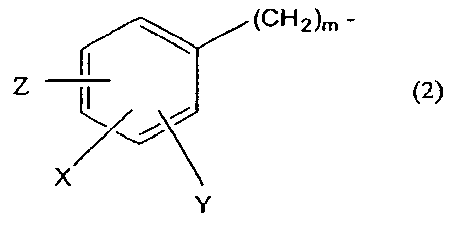 and a heterocycle-alkyl group of formula heterocycle-(CH₂)ₘ- wherein R₂ and R₄ the same or different are independently selected from the group consisting of H, CHO-, CF₃₋,CH₃CO-, benzoyl, 9-fluorenylmethoxycarbonyl, tert-butoxycarbonyl, benzyloxycarbonyl, 2-chlorobenzyloxycarbonyl, 4-OH-7-CF₃- quinoline-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholesteryl-OCO-, 2-quinoline-CO-, xanthene-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorene-CH₂CO-, camphor-10-CH₂-SO₂-, (C₆H₅)₂CH-CO-, fluorene-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄-SO₂-, C₆H₅CHCH-SO₂-, 4-NO₂C₆H₄-SO₂-, an aryalkyl group of formula (2) as defined above, a sulfonyl group of formula (3) 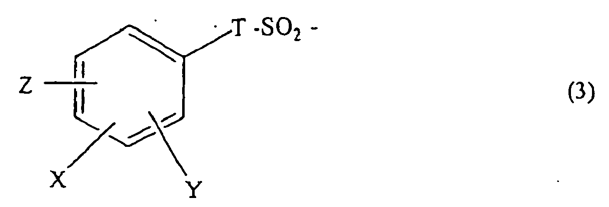 a heterocycle-alkylsulfonyl group of formula heterocycle-(CH₂)ₘ-SO₂- and a carbonyl group of formula (4) 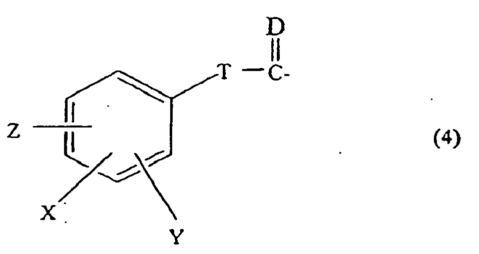 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, -CF₃, - NO₂, -NH₂, -NHR₅, -NR₅R₆, -NHCOR₅, -NHCOheterocycle, heterocycle being as defined above, -OR₅, -SR₅, -SOR₅, -SO₂R₅, -COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂, -NHR₅, -NR₅R₆, - NHCOR₅, -OR₅, -SR₅, -SOR₅, -SO₂R₅, -COOR₅, -CH₂OH, -COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings, for the preparation of a medicament for treating or preventing a disease **characterized by** beta-amyloid deposits on or in the brain.

18. The use according to any of claims 1-5, further comprising use of one or more therapeutic agents selected from the group consisting of an antioxidant, an anti-inflammatory, a gamma secretase inhibitor, a neurotrophic agent, an acetyl cholinesterase inhibitor, a statin, P-gp inhibitors, an A beta peptide, and an anti-A beta peptide.

19. The use of claim 1 wherein the compound of formula Ia comprises an amino group or pharmaceutically acceptable ammonium salts thereof, wherein W is -(CH₂)ₙ-, n is 3 or 4 and D is O.

20. The use of claim 1 wherein the compound of formula Ia comprises an amino group or pharmaceutically acceptable ammonium salts thereof, wherein heterocycle is selected from the group consisting of benzimidazolyl, imidazolyl, imidazolinyl, imidazolidinyl, quinolyl, isoquinolyl, indolyl, pyridyl, pyrrolyl, pyrrolinyl, pyrrolidinyl, pyrazolyl, pyrazinyl, quinoxolyl, piperidinyl, morpholinyl, β-carbolinyl, tetrazolyl, thiazolidinyl, benzofuranyl, thiamorpholinyl, benzoxazolyl, oxopiperidinyl, oxopyrroldinyl, oxoazepinyl, azepinyl, isoxazolyl, tetrahydropyranyl, tetrahydrofuranyl, thiadiazolyl, thiadiazinyl, benzodioxolyl, thiophenyl, tetrahydrothiophenyl, nicoticoyl, morpholinecarbodithioyl and sulfolanyl.

21. The use of claim 1 wherein the compound of formula Ia comprises an amino group or pharmaceutically acceptable ammonium salts thereof, wherein n is 4.

22. The use of claim 1 wherein the compound of formula Ia comprises an amino group or pharmaceutically acceptable ammonium salts thereof, wherein Cₓ is selected from the group consisting of -COOM, -COOR₅, -CH₂OH, -CONHOH, and benzyloxycarbonyl, wherein M is an alkali metal and R, is as defined in claim 1, wherein R₁ and R₃, the same or different, are selected from the group consisting of H, a straight or branched alkyl group of 1 to 6 carbon atoms, a cycloalkylalkyl group having 3 to 7 carbon atoms in the cycloalkyl part thereof and 1 to 3 carbon atoms in the alkyl part thereof and an arylalkyl group of formula (2) as defined in claim 1 wherein Z and Y are each H, m is 1 and X is H, Br or F wherein R₂ and R₄ the same or different are selected from the group consisting of H, 9-fluorenylmethoxycarbonyl, benzyloxycarbonyl, 2- chlorobenzyloxycarbonyl, 4-OH-7-CF₃₋quinoline-3-CO-, 3-indole-CH₂CH₂CO-, 3- indole-CH₂CO-, 3-indole-CO-, 2-indole-CO-, C₆H₅CHCHCO-, C₆H₅CH₂CH₂CO-, C₆H₅CH₂CH₂CH₂CO-, C₆H₅CH₂CHCHCO-, C₆H₅OCH₂CO-, (C₆H₅)₂COHCO-, C₆H₅SCH₂CO-, C₆H₅CH₂CH₂CS-, 4-HOC₆H₄CH₂CH₂CO-, cholesteryl-OCO-, 2-quinoline-CO-, fluorene-CO-, xanthene-9-CO-, 4-C₆H₅CH₂CH₂CONHC₆H₄SO₂-, 4-NO₂C₆H₄CHCHCO-, 3-NO₂C₆H₄CHCHCO-, 2- NO₂C₆H₄CHCHCO-, 2,3-(CH₃O)₂C₆H₃CHCHCO-, 3,4- (CH₃O)₂C₆H₃CHCHCO-, 2,5-(CH₃O)₂C₆H₃CHCHCO-, 2,5-(CH₃O)₂C₆H₃CH₂CH₂CO-, 3, 5- (CH₃O)₂C₆H₃CH₂CH₂CO-,3,4-(CH₃O)₂C₆H₃CH₂CH₂CO-, 2,4-(CH₃O)₂C₆H₃CHCHCO-, 2, 4-(CH₃O)₂C₆H₃CH₂CH₂CO-,3,4(CH₃O)₂C₆H₃CHCHCO-, 2,3-(CH₃O)₂C₆H₃CH₂CH₂CO-, 4-CH₃OC₆H₄CHCHCO-, 4-CH₃OC₆H₄CH₂CH₂CO-, 2- CH₃OC₆H₄CHCHCO-, 3-CH₃OC₆H₄CHCHCO-, 3-CH₃OC₆H₄CH₂CH₂CO-, 2- CH₃OC₆H₄CH₂CH₂CO- , 4-CH₃OC₆H₄CHCHCO-, 4-HOC₆H₄CHCHCO-, 3-NH₂C₆H₄CH₂CH₂CO-, 3-C₅H₄NCHCHCO-3-C₅H₄NCH₂CH₂CO-, fluorene-CH₂CO-, camphor-10-CH₂-SO₂-, (C₆H₅)₂CH-CO-, 1-naphthyl-SO₂-, 2-naphthyl-SO₂-, fluorenyl-SO₂-, phenanthryl-SO₂-, anthracenyl-SO₂-, quinoline-SO₂-, 4-CH₃COONHC₆H₄SO₂-, C₆H₅CHCH-SO₂-, 4-NO₂C₆H₄-SO₂-, and a sulfonyl group of formula (3) 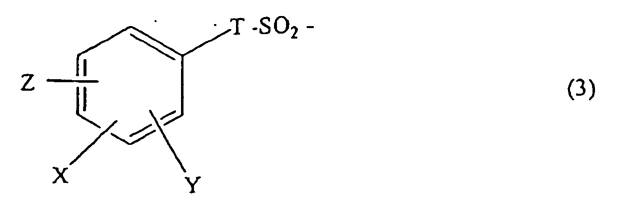 wherein T is -(CH₂)ₘₘ- wherein mm is 0 and wherein X, Y and Z, are independently selected from the group consisting of H, a straight or branched alkyl group of 1 to 6 carbon atoms, F, Cl, Br, I, -CF₃, -NO₂, - NH₂, and -COR₅, wherein R₅ selected from the group consisting of H, and a straight or branched alkyl group of 1 to 6 carbon atoms.

23. The use of claim 1 wherein the compound of formula Ia comprises an amino group or pharmaceutically acceptable ammonium salts thereof, wherein R₂ is a sulfonyl group of formula (3) 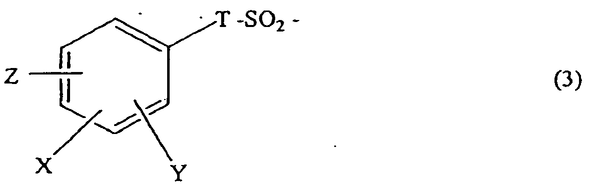 wherein T is selected from the group consisting of -(CH₂)ₘₘ-, -CH=CH-, and -CH₂-CH=CH-;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, - CF₃, - NO₂, -NH₂ -NHR₅, -NR₅R₆, -NHCOR₅, -NHCOheterocycle, heterocycle being as defined above, -OR₅, -SR₅, -SOR₅,-SO₂R₅, - COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂₋NHR₅, -NR₅R₆, -NHCOR₅-OR₅, -SR₅, -SOR₅, -SO₂R₅, -COOR₅, -CH₂OH, - COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings.

24. The use of claim 23 wherein the compound of formula Ia comprises an amino group or pharmaceutically acceptable ammonium salts thereof, wherein W is -(CH₂)ₙ-, and wherein n is 4.

25. The use of claim 1 wherein the compound of formula Ia comprises an amino group or pharmaceutically acceptable ammonium salts thereof, wherein R₂ is a sulfonyl group of formula (3) 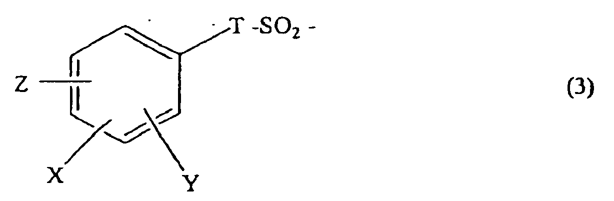 wherein T is selected from the group consisting of -(CH₂)ₘₘ-, -CH=CH-, and -CH₂-CH=CH-;
wherein D is selected from the group consisting of O, NR₇ and S,
wherein m is 1, 2, 3 or 4;
wherein mm is 0, 1, 2, 3 or 4;
wherein X, Y and Z, the same or different, are independently selected from the group consisting of H, a straight or branched alkyl group of 1 to 6 carbon atoms, F, Cl, Br, I, - CF₃, - NO₂, -NH₂ -NHR₅, -NR₅R₆, -NHCOR₅, -NHCOheterocycle, heterocycle being as defined above, -OR₅, -SR₅, -SOR₅, -SO₂R₅, - COOR₅, -CH₂OH, -COR₅, and -NHCOAryl, Aryl being an unsubstituted phenyl group or a phenyl group substituted by one or more members of the group consisting of a straight or branched alkyl group of 1 to 6 carbon atoms, F, Cl, Br, I, -CF₃, - NO₂, -NH₂,-NHR₅, -NR₅R₆, -NHCOR₅,-OR₅, -SR₅, -SOR₅, -SO₂R₅, -COOR₅, -CH₂OH,-COR₅, wherein R₅ and R₆, are independently selected from the group consisting of H, and a straight or branched alkyl group of 1 to 6 carbon atoms wherein R₇ is selected from the group consisting of HO-, CH₃O-, NC-, benzyloxy, and H₂N- and wherein heterocycle is selected from the group consisting of heterocyclic groups comprising 5 to 7 ring atoms, said ring atoms comprising carbon atoms and from one to four heteroatoms selected from the group consisting of nitrogen, oxygen and sulfur, said heterocyclic groups being monocycylic, bicycylic or monocycylic fused with one or two benzene rings;
and wherein R₃ is H.

26. The use of claim 25 wherein the compound of formula Ia comprises an amino group or pharmaceutically acceptable ammonium salts thereof, wherein W is -(CH₂)ₙ-, and wherein n is 4.

27. The use of claim 2 wherein the compound of formula Ia comprises an amino group or pharmaceutically acceptable ammonium salts thereof, wherein R₁ is selected from the group consisting of isobutyl, cyclopropylmethyl and benzyl, wherein R₂ is a sulfonyl group of formula (3) as defined in claim 1, wherein R₃ is H and wherein Cₓ is selected from the group consisting of - COOM, and -COOR₅, M being an alkali metal and R₅ being as defined in claim 1.

28. The use of claim 2 wherein the compound of formula Ia comprises an amino group or pharmaceutically acceptable ammonium salts thereof, wherein n is 4, wherein R₁ is selected from the group consisting of isobutyl, cyclopropylmethyl and benzyl, wherein R₂ is a sulfonyl group of formula (3) as defined in claim 1, wherein T is -(CH₂)ₘₘ-, wherein mm is 0, wherein X, Y and Z, the same or different, are selected from the group consisting of H, a straight or branched alkyl group of 1 to 6 carbon atoms, Br, NO₂,NH₂, and OR₅, wherein R₃ is H, wherein Cx is selected from the group consisting of -COOM, and - COOR₅, wherein M is an alkali metal, wherein R₅ is as defined in claim 1 and wherein R₄ is selected from the group consisting of 9-fluorenylmethoxycarbonyl, 2, 3(CH₃O)₂C₆H₃CH₂CH₂CO-, 2,4-(CH₃O)₂ C6H3CH2CH2CO- , 3-indole-CH₂CH₂CO-, C₆H₅CH₂CH₂CO-, C₆H₅SCH₂CO-, C₆H₅OCH₂CO-, xanthene-9-CO-, 4-CH₃OC₆H₄CH₂CH₂CO-, 3-CH₃OC₆H₄CH₂CH₂CO-, 2-CH₃OC₆H₄CH₂CH₂CO-, 3-NH₂C₆H₄CH₂CH₂CO- and

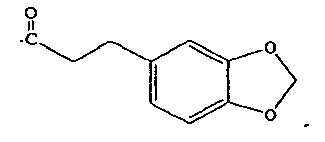

## Patentansprüche

1. Verwendung einer Verbindung der Formel Ia oder eines pharmazeutisch verträglichen Salzes davon: 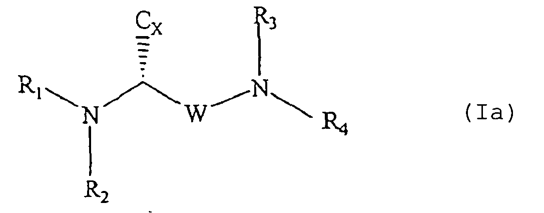 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin Cₓ ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 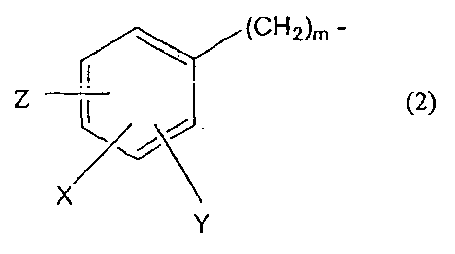 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 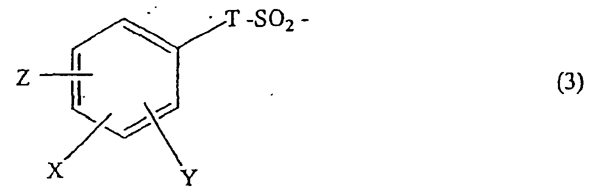 einer Heterozyklus-Alkylsulfonylgruppe der Formel Heterozyklus-(CH₂)ₘ-SO₂- und einer Carbonylgruppe der Formel (4) 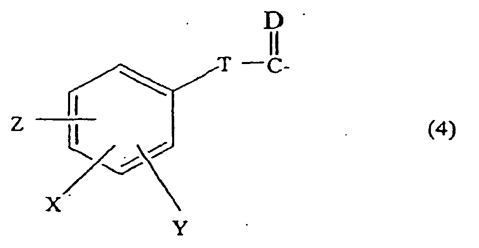 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, - CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind,
zur Herstellung eines Medikaments zur Behandlung oder Prävention von Alzheimer-Krankheit bei einem Patienten, der eine solche Behandlung benötigt.

2. Verwendung nach Anspruch 1 zur Behandlung von Alzheimer-Krankheit bei einem Patienten, der eine solche Behandlung benötigt

3. Verwendung nach Anspruch 1 oder 2 zur Behandlung von Alzheimer-Krankheit durch Modulation der Wirkung von Beta-Amyloid konvertierendem Enzym.

4. Verwendung nach Anspruch 1, des Weiteren umfassend die Verwendung eines P-gp-Inhibitors oder eines pharmazeutisch verträglichen Salzes davon.

5. Verwendung einer Verbindung der Formel (Ia), oder eines pharmazeutisch verträglichen Salzes davon: 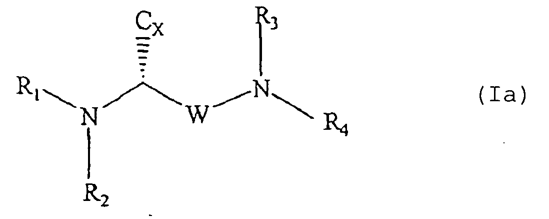 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin C_{X} ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 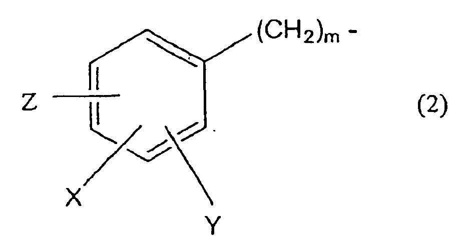 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 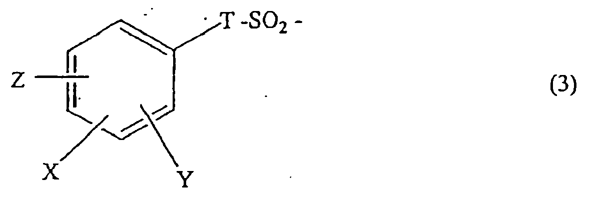 einer Heterozyklus-Alkylsulfonylgruppe der Formel Heterozyklus-(CH₂)ₘ-SO₂- und einer Carbonylgruppe der Formel (4) 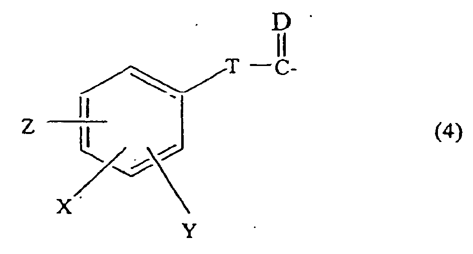 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind,
zur Herstellung eines Medikaments zur Behandlung eines Patienten, der leidet an, oder zur Prävention, das ein Patient bekommt, eine Krankheit oder einen Zustand, ausgewählt aus der Gruppe bestehend aus Alzheimer-Krankheit, zur Unterstützung bei der Verhinderung oder dem Verzögern des Auftretens der Alzheimer-Krankheit, zur Behandlung von Patienten mit geringer kognitiver Beeinträchtigung (MCI) und zur Verhinderung oder zur Verzögerung des Auftretens der Alzheimer-Krankheit in jenen Patienten, die im Krankheitsverlauf von MCI zu AD fortschreiten würden, zur Behandlung des Down-Syndroms, zur Behandlung von Menschen mit hereditärer zerebraler Hämorrhagie mit Amyloidose des Dutch-Typs, zur Behandlung zerebraler amyloider Angiopathie und zur Verhinderung ihrer potenziellen Folgen, nämlich von einmaligen und wiederkehrenden Lobärhämorrhagien, zur Behandlung anderer degenerativer Demenz, einschließlich Demenz gemischten vaskulären und degenerativen Ursprungs, Demenz im Zusammenhang mit Parkinson, frontotemporale Demenzen mit Parkinsonismus (FTDP), Demenz im Zusammenhang mit progressiver supranukleärer Lähmung, Demenz im Zusammenhang mit kortikaler Basaldegeneration oder Alzheimer-Krankheit des diffusen Lewy-Body-Typs.

6. Verwendung nach einem der Ansprüche 1 bis 5, worin die Verbindung der Formel (Ia) ausgewählt ist aus der Gruppe bestehend aus: 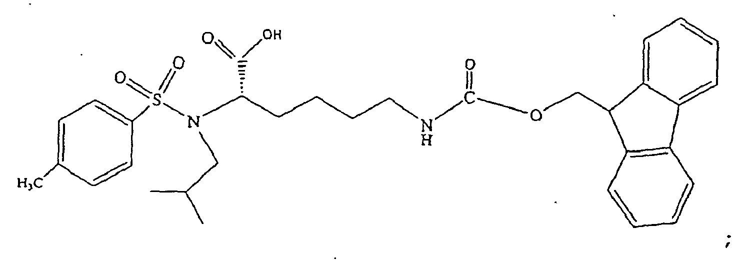 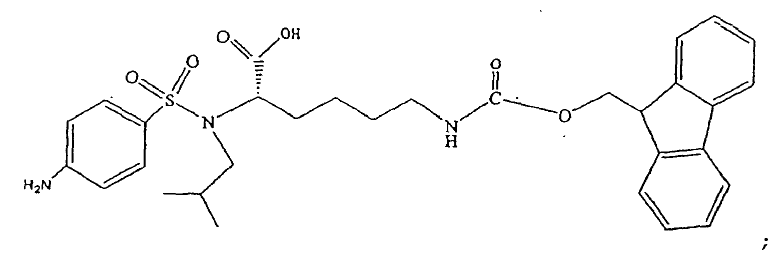 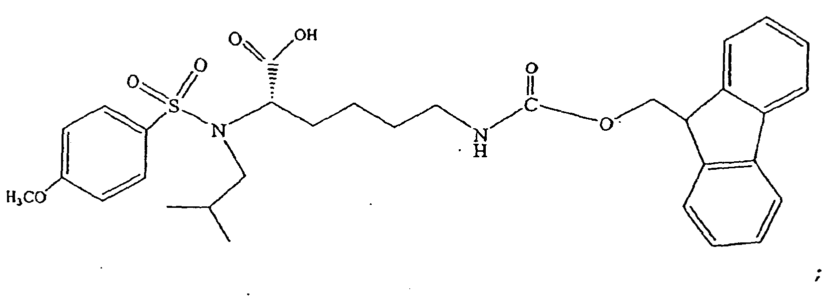 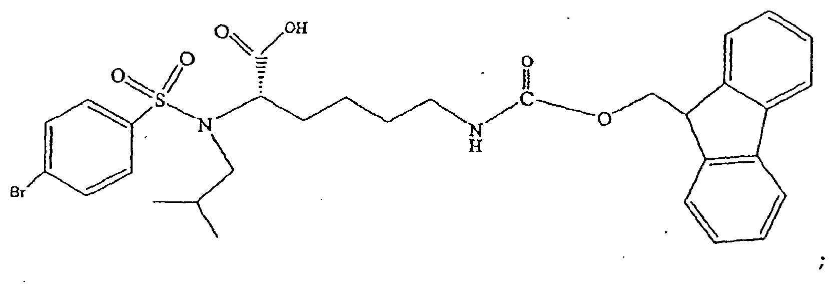 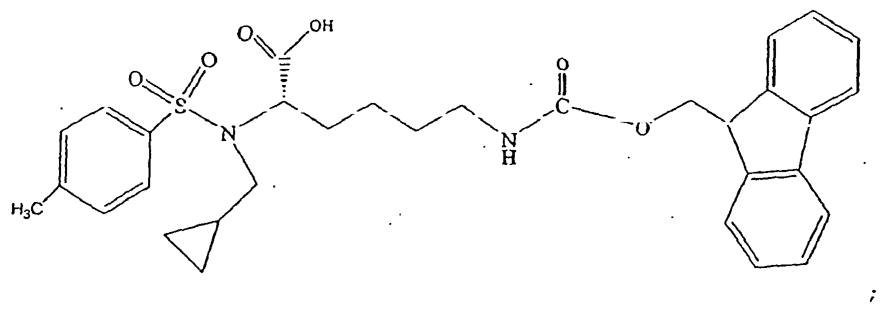 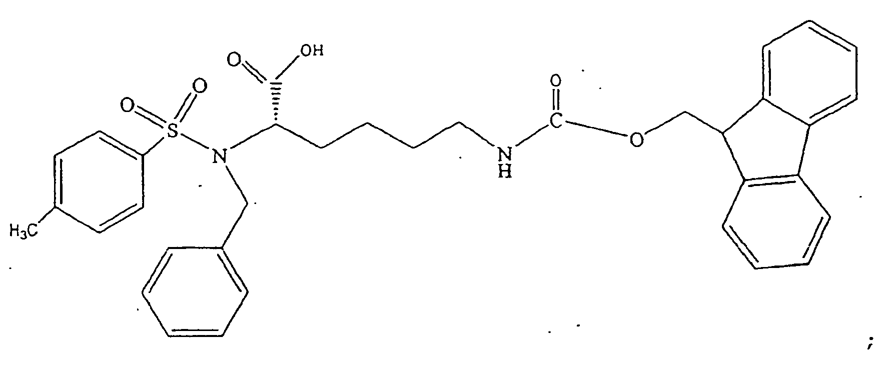 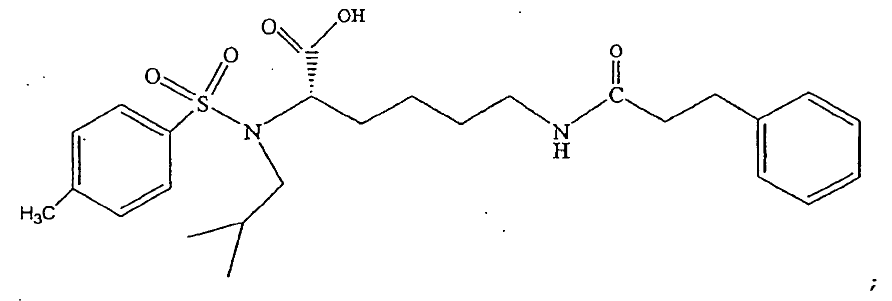 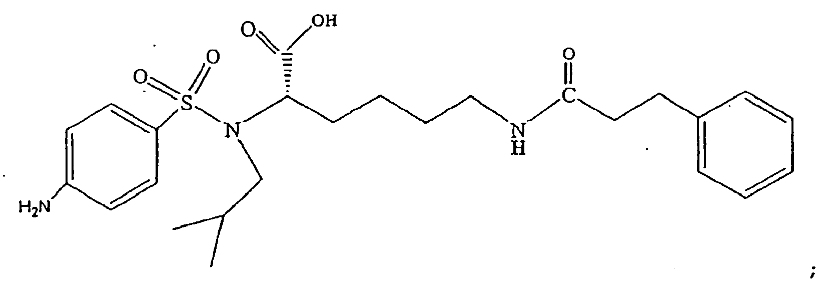 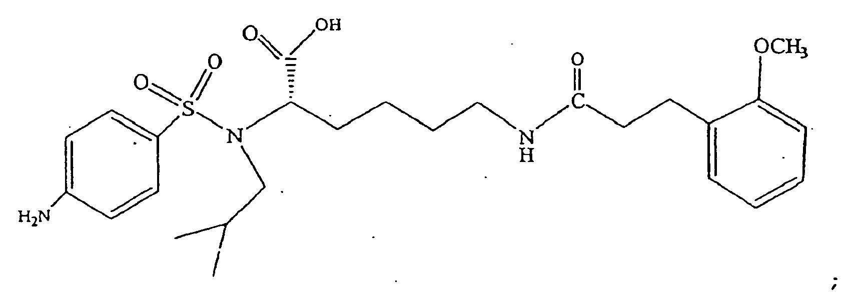 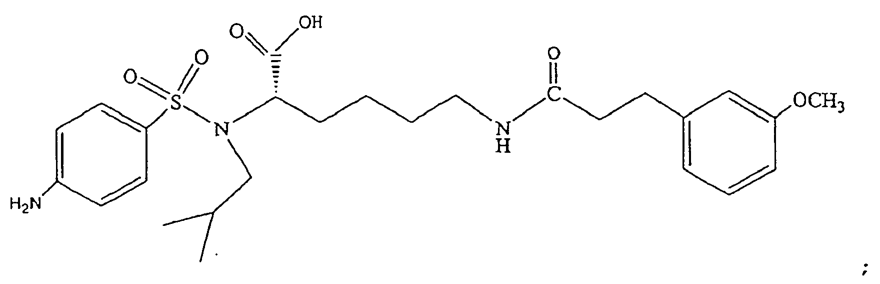 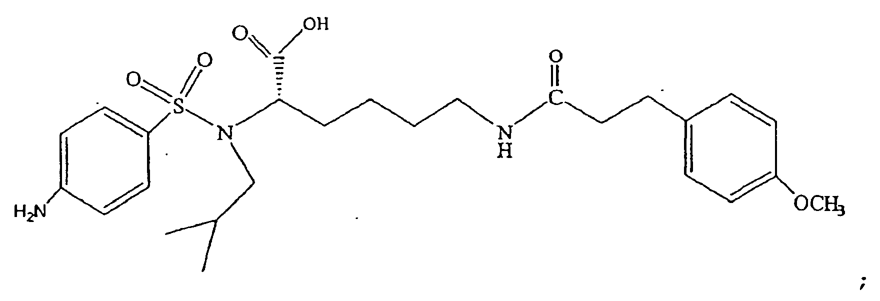 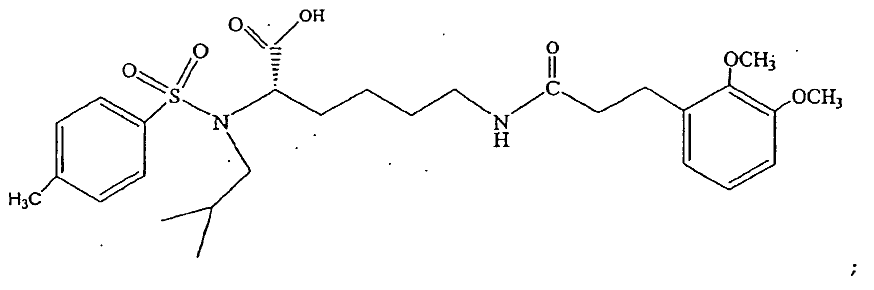 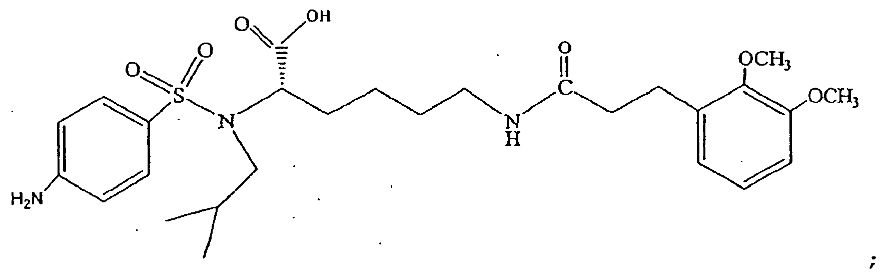 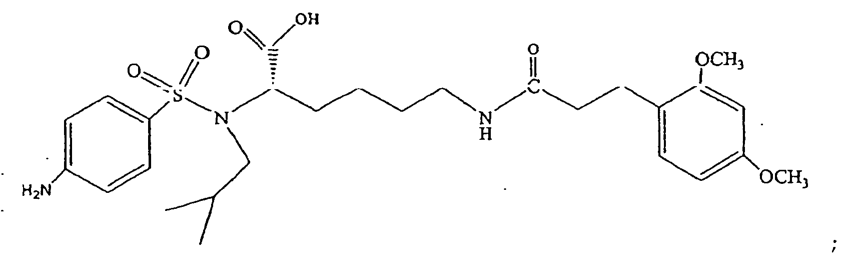 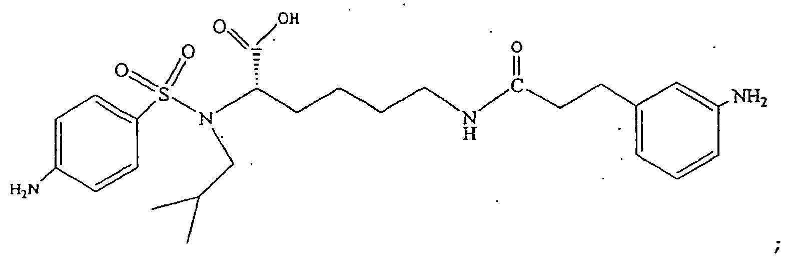 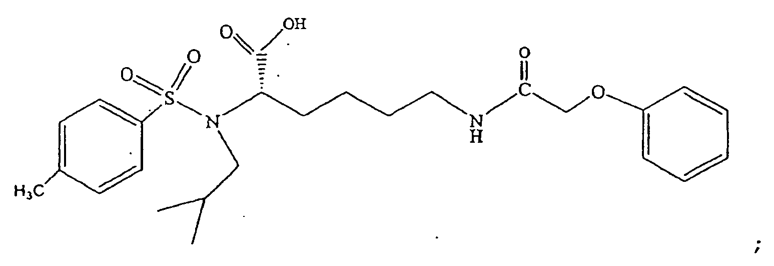 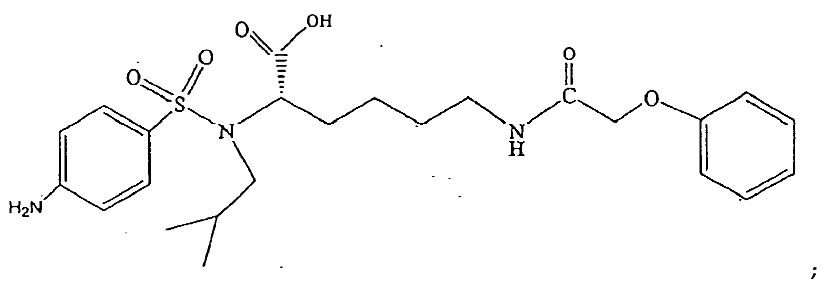 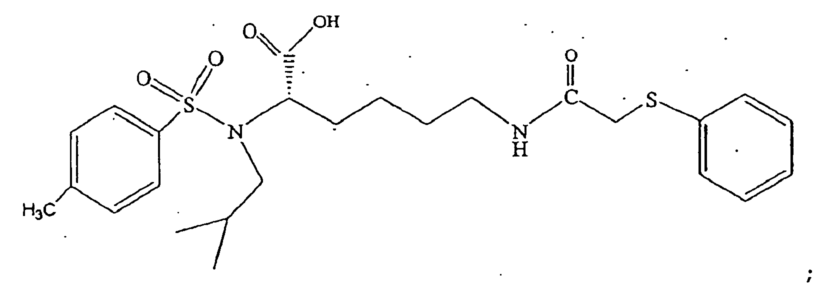 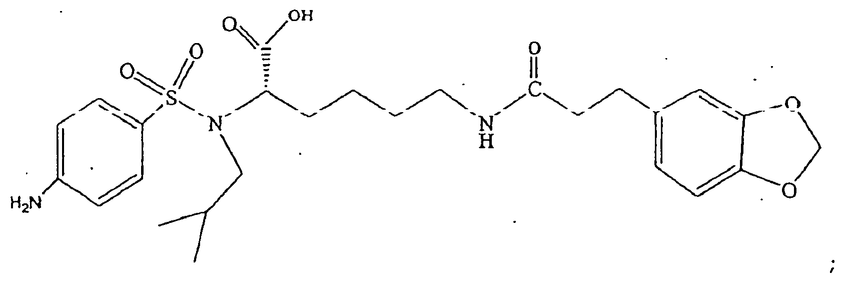 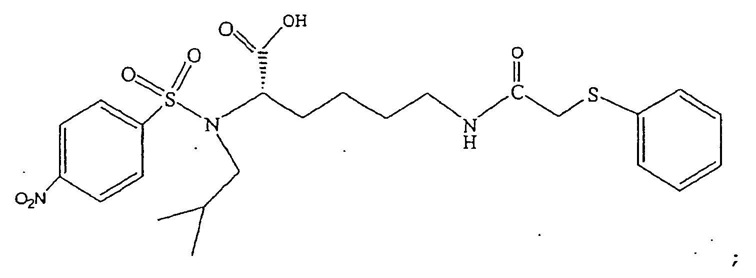 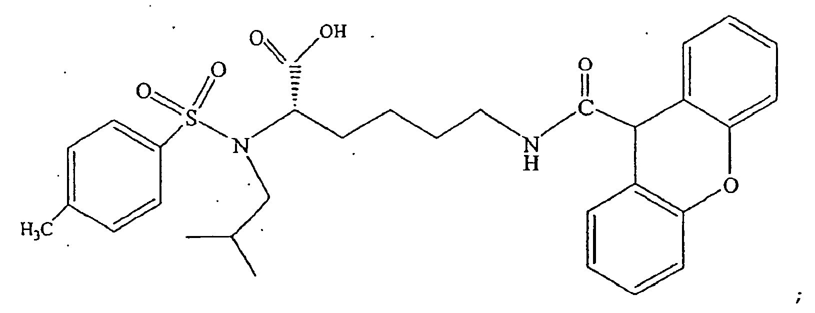 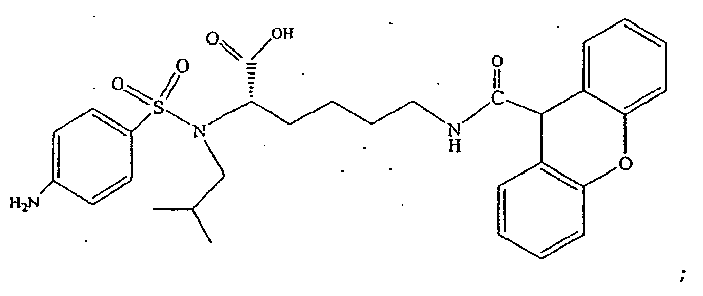 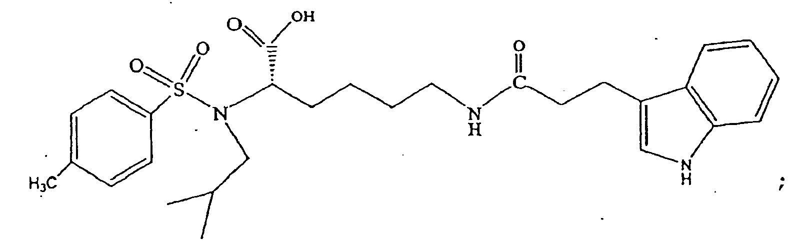 und 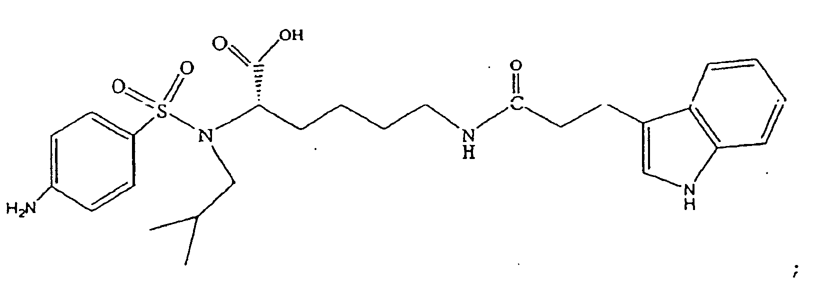 oder pharmazeutisch verträgliche Ammonium-, Kalium (K)-, Natrium (Na)- und Caesium (Cs)-Salze davon.

7. Verwendung einer Zusammensetzung, umfassend einen oder mehrere pharmazeutisch verträgliche Trägerstoffe und eine Verbindung der Formel Ia oder ein pharmazeutisch verträgliches Salz davon: 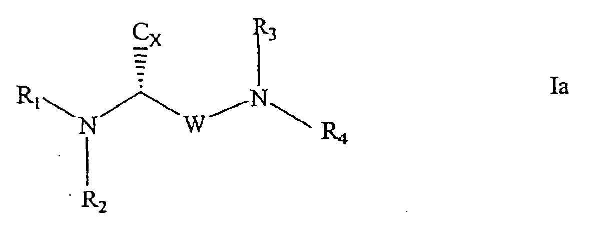 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin C_{X} ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und 1 bis 3 Kohlenstoffatomen in dem Alkylteil davon, einer Arylalkylgruppe der Formel (2) 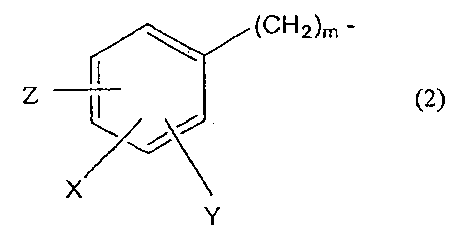 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 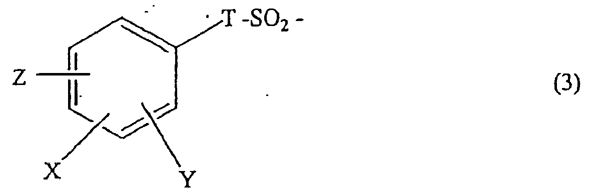 einer Heterozyklus-Alkylsulfonylgruppe der Formel Heterozyklus-(CH₂)ₘ-SO₂- und einer Carbonylgruppe der Formel (4) 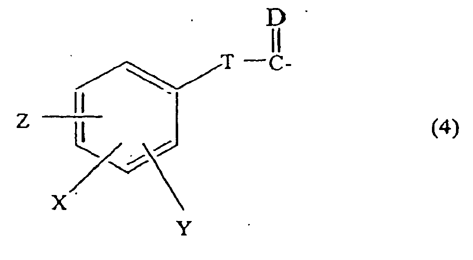 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind,
zur Herstellung eines Medikaments zur Behandlung oder Prävention von Alzheimer-Krankheit bei einem Patienten, der eine solche Behandlung benötigt.

8. Verwendung einer Verbindung der Formel Ia zur Herstellung eines Medikaments zur Behandlung oder Prävention von Zuständen, ausgewählt aus der Gruppe bestehend aus Alzheimer-Krankheit, geringer kognitiver Beeinträchtigung (MCI), Down-Syndrom, hereditärer zerebraler Hämorrhagie mit Amyloidose des Dutch-Typs, zerebraler amyloider Angiopathie, degenerativer Demenz, einschließlich Demenz gemischten vaskulären und degenerativen Ursprungs, Demenz im Zusammenhang mit Parkinson, frontotemporaler Demenz mit Parkinsonismus (FTDP), Demenz im Zusammenhang mit progressiver supranukleärer Lähmung, Demenz im Zusammenhang mit kortikaler Basaldegeneration, oder Alzheimer-Krankheit des diffusen Lewy-Body-Typs: 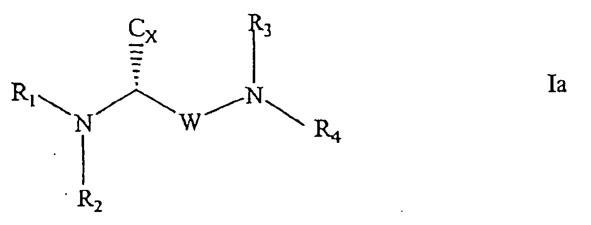 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin Cₓ ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 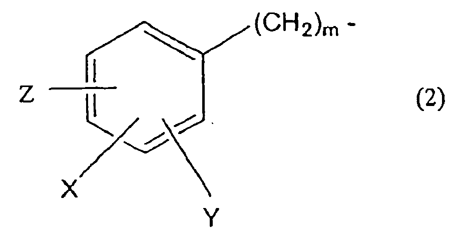 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 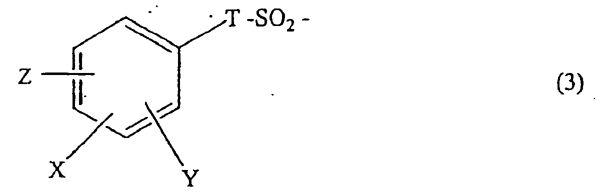 einer Heterozyklus-Alkylsulfonylgruppe der Formel Heterozyklus-(CH₂)ₘ-SO₂- und einer Carbonylgruppe der Formel (4) 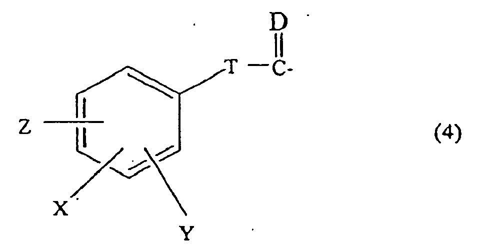 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind.

9. Ein in vitro-Verfahren zur Hemmung der Beta-Secretase-Aktivität, umfassend das In-Kontakt-Bringen einer wirksamen Menge zur Hemmung einer Verbindung der Formel Ia: 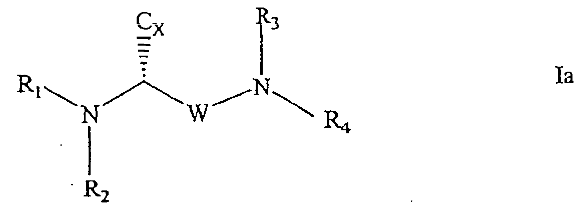 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin Cₓ ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 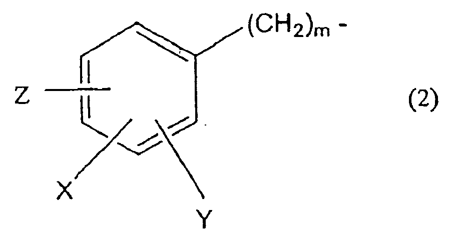 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 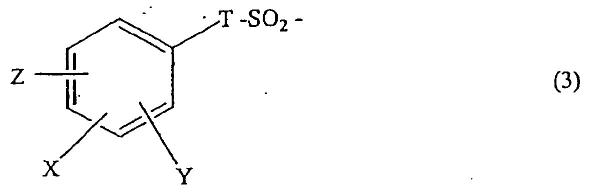 einer Heterozyklus-Alkylsulfonylgruppe der Formel Heterozyklus-(CH₂)ₘ-SO₂- und einer Carbonylgruppe der Formel (4) 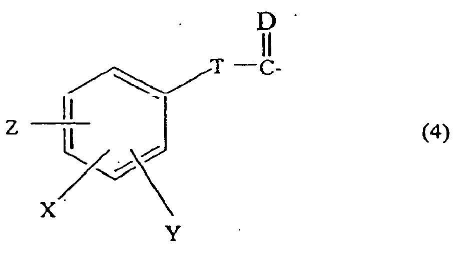 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind.

10. Ein in vitro-Verfahren zur Hemmung der Spaltung eines Amyloidprecursorprotein (APP)-Isotyps an einer Stelle in dem APP-Isotyp, die einer Spaltung zugänglich ist, umfassend das In-Kontakt-Bringen des APP-Isotyps mit einer wirksamen spaltungshemmenden Menge einer Verbindung der Formel Ia: 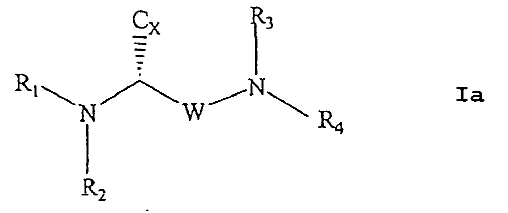 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin Cₓ ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 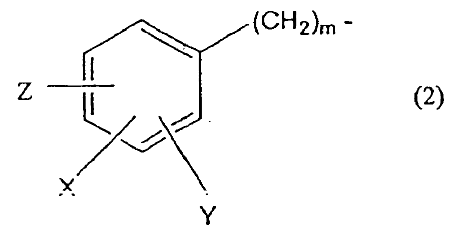 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 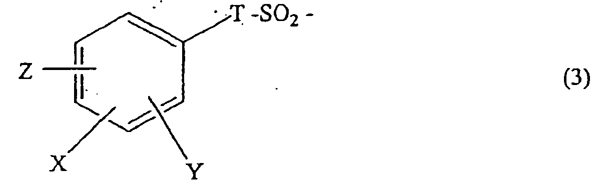 einer Heterozyklus-Alkylsulfonylgruppe der Formel Heterozyklus-(CH₂)ₘ-SO₂- und einer Carbonylgruppe der Formel (4) 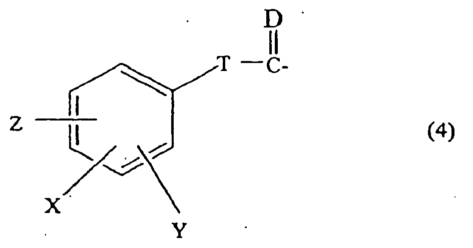 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind.

11. Ein in vitro-Verfahren zur Hemmung der Bildung von Amyloidbetapeptid (A beta) in einer Zelle, umfassend das Verabreichen einer Zelle einer wirksamen hemmenden Menge einer Verbindung der Formel Ia: 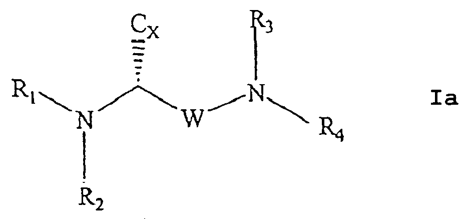 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin C_{X} ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 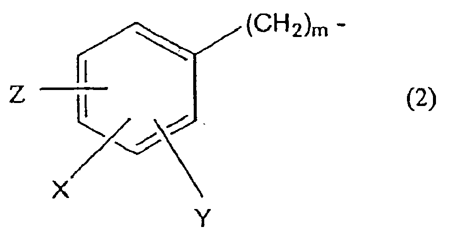 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 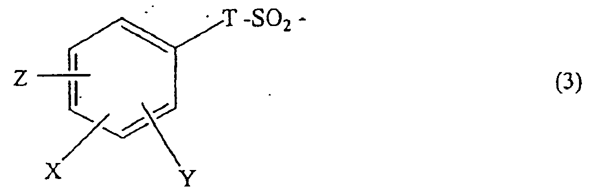 einer Heterozyklus-Alkylsulfonylgruppe der Formel Heterozyklus-(CH₂)ₘ-SO₂- und einer Carbonylgruppe der Formel (4) 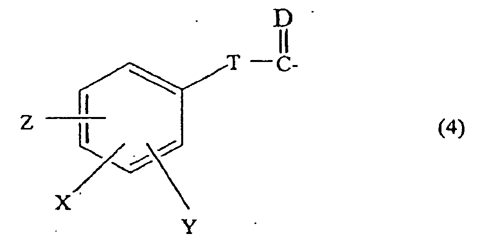 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind.

12. Verfahren nach Anspruch 11, wobei die Zelle eine tierische Zelle ist.

13. Verfahren nach Anspruch 12, wobei die tierische Zelle eine Säugetierzelle ist.

14. Verfahren nach Anspruch 13, wobei die Säugetierzelle eine menschliche Zelle ist.

15. Zusammensetzung, umfassend Beta-Secretase komplexiert mit einer Verbindung der Formel Ia: 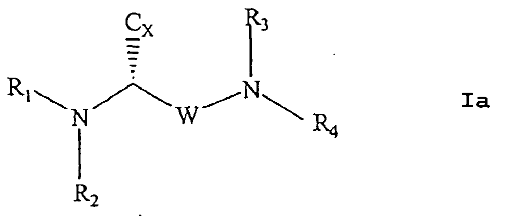 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin C_{X} ausgewählt ist aus der Gruppe bestehend aus -COOM, -COOR₅, -CH₂OH, -CONR₅R₆, -CONHOH, 9-Fluorenylmethoxycarbonyllysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 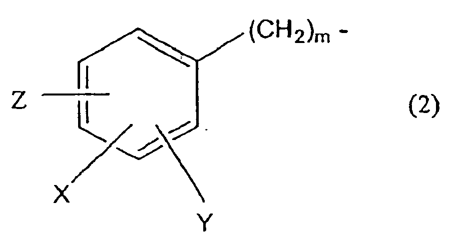 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthrace- nyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 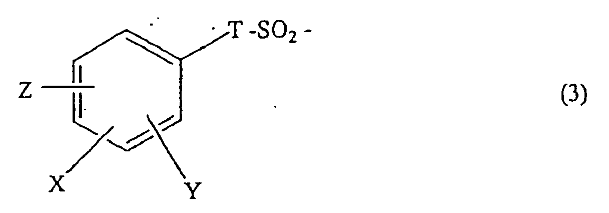 einer Heterozyklus-Alkylsulfonylgruppe der Formel Heterozyklus-(CH₂)ₘ-SO₂- und einer Carbonylgruppe der Formel (4) 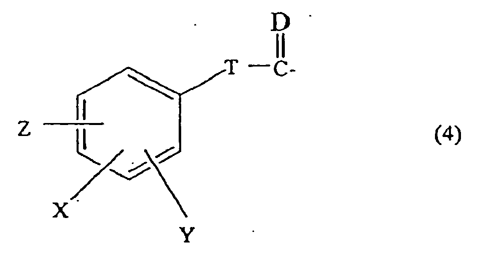 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind.

16. Verfahren zur Herstellung eines Beta-Secretase-Komplexes, umfassend die Zusammensetzung nach Anspruch 15.

17. Verwendung einer Verbindung der Formel Ia: 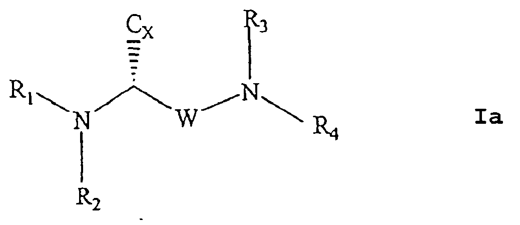 und wobei die Verbindung der Formel Ia eine Aminogruppe oder pharmazeutisch verträgliche Ammoniumsalze davon umfasst,
worin W ausgewählt ist aus der Gruppe bestehend aus -(CH₂)ₙ- und -CH₂-XX-CH₂-CH₂-,
worin n gleich 1, 2, 3, 4 oder 5 ist,
worin XX ausgewählt ist aus der Gruppe bestehend aus O, NR₅, S, SO und SO₂,
worin Cₓ ausgewählt ist aus der Gruppe bestehend aus -COOM, -COOR₅, -CH₂OH, -CONR₅R₆, -CONHOH, 9-Fluorenylmethoxycarbonyl-lysyl-NH-CO-, Benzyloxycarbonyl und Tetrazolyl,
worin M ein Alkali- oder Erdalkalimetall ist,
worin R₁ und R₃ gleich oder verschieden sind und ausgewählt sind aus der Gruppe bestehend aus H, tert-Butoxycarbonyl, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und mit 1 bis 3 Kohlenstoffatomen in dem Alkylteil davon, einer Arylalkylgruppe der Formel (2) 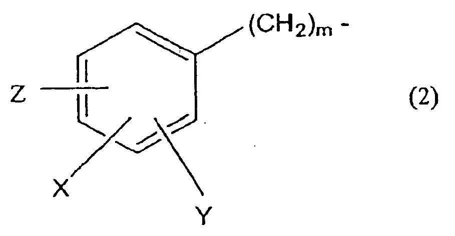 und einer Heterozyklus-Alkylgruppe der Formel Heterozyklus-(CH₂)ₘ-, worin R₂ und R₄ gleich oder verschieden sind und unabhängig ausgewählt aus der Gruppe bestehend aus H, CHO-, CF₃-, CH₃CO-, Benzoyl, 9-Fluorenylmethoxycarbonyl, tert-Butoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅OCH₂CO-, (C₆H₅)₂COHCO-, C₆H₅SCH₂CO-, C₆H₅CH₂CH₂CS-, Cholesteryl-OCO-, 2-Chinolin-CO-, Xanthen-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, Fluoren-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄-SO₂-, C₆H₅CHCH-SO₂-, 4-NO₂C₆H₄-SO₂-, einer Arylalkylgruppe der vorstehend definierten Formel (2), einer Sulfonylgruppe der Formel (3) 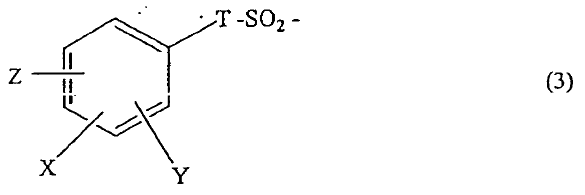 einer Heterozyklus-Alkylsulfonylgruppe der Formel Heterozyklus-(CH₂)ₘ-SO₂- und einer Carbonylgruppe der Formel (4) 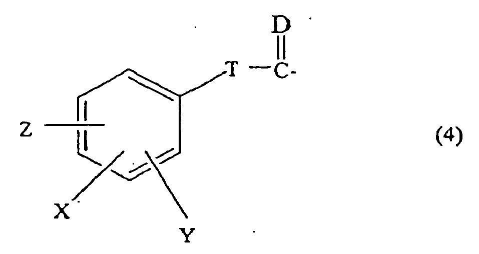 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine substituierte Phenylgruppe ist, substituiert mit einem oder mehreren Substituenten, ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome umfassen, ausgewählt aus der Gruppe bestehend aus Stickstoff, Sauerstoff und Schwefel, wobei die heterozyklischen Gruppen monozyklisch, bizyklisch oder monozyklisch kondensiert mit einem oder zwei Benzolringen sind,
zur Herstellung eines Medikaments zur Behandlung oder Prävention einer Krankheit, **gekennzeichnet durch** Beta-Amyloid-Abscheidungen auf oder in dem Gehirn.

18. Verwendung nach einem der Ansprüche 1 bis 5, des Weiteren umfassend die Verwendung von einem oder mehreren therapeutischen Mitteln, ausgewählt aus der Gruppe bestehend aus einem Antioxidans, einem entzündungshemmenden Mittel, einem Gamma-Secretase-Hemmer, einem neurotrophen Mittel, einem Acetylcholesterinase-Hemmer, einem Statin, P-gp-Hemmern, einem A Beta-Peptid und einem anti-A Beta-Peptid.

19. Verwendung nach Anspruch 1, wobei die Verbindung der Formel Ia eine Aminogruppe oder pharmazeutisch verträgliche Ammoniumsalze davon umfasst, worin W gleich -(CH₂)ₙ-, n gleich 3 oder 4 und D gleich O ist.

20. Verwendung nach Anspruch 1, wobei die Verbindung der Formel Ia eine Aminogruppe oder pharmazeutisch verträgliche Ammoniumsalze davon umfasst, worin der Heterozyklus ausgewählt ist aus der Gruppe, bestehend aus Benzimidazolyl, Imidazolyl, Imidazolinyl, Imidazolidinyl, Chinolyl, Isochinolyl, Indolyl, Pyridyl, Pyrrolyl, Pyrrolinyl, Pyrrolidinyl, Pyrazolyl, Pyrazinyl, Chinoxolyl, Piperidinyl, Morpholinyl, β-Carbolinyl, Tetrazolyl, Thiazolidinyl, Benzofuranyl, Thiamorpholinyl, Benzoxazolyl, Oxopiperidinyl, Oxopyrrolidinyl, Oxoazepinyl, Azepinyl, Isoxazolyl, Tetrahydropyranyl, Tetrahydrofuranyl, Thiadiazolyl, Thiadiazinyl, Benzodioxolyl, Thiophenyl, Tetrahydrothiophenyl, Nicoticoyl, Morpholincarbodithioyl und Sulfolanyl.

21. Verwendung nach Anspruch 1, wobei die Verbindung der Formel Ia eine Aminogruppe oder pharmazeutisch verträgliche Ammoniumsalze umfasst, worin n gleich 4 ist.

22. Verwendung nach Anspruch 1, wobei die Verbindung der Formel Ia eine Aminogruppe oder pharmazeutisch verträgliche Ammoniumsalze davon umfasst, worin Cₓ ausgewählt ist aus der Gruppe bestehend aus -COOM, -COOR₅, -CH₂OH, -CONHOH und Benzyloxycarbonyl, worin M ein Alkalimetall ist und R wie in Anspruch 1 definiert ist, worin R₁ und R₃ gleich oder verschieden sind und ausgewählt sind aus der Gruppe bestehend aus H, einer gerad- oder verzweigtkettigen Alkylgruppe mit 1 bis 6 Kohlenstoffatomen, einer Cycloalkylalkylgruppe mit 3 bis 7 Kohlenstoffatomen in dem Cycloalkylteil davon und 1 bis 3 Kohlenstoffatomen in dem Alkylteil davon und einer Arylalkylgruppe der Formel (2), wie in Anspruch 1 definiert, worin Z und Y jeweils H sind, m gleich 1 ist und X gleich H, Br oder F ist, worin R₂ und R₄ gleich oder verschieden sind und ausgewählt sind aus der Gruppe bestehend aus H, 9-Fluorenylmethoxycarbonyl, Benzyloxycarbonyl, 2-Chlorbenzyloxycarbonyl, 4-OH-7-CF₃-Chinolin-3-CO-, 3-Indol-CH₂CH₂CO-, 3-Indol-CH₂CO-, 3-Indol-CO-, 2-Indol-CO-, C₆H₅CHCHCO-, C₆H₅CH₂CH₂CO-, C₆H₅CH₂CH₂CH₂CO-, C₆H₅CH₂CHCHCO-, C₆H₅OCH₂CO-, (C₆H₅)₂COHCO-, C₆H₅CHSCH₂CO-, C₆H₅CH₂CH₂CS-, 4-HOC₆H₄CH₂CH₂CO-, Cholesteryl-OCO-, 2-Chinolin-CO-, Fluoren-CO-, Xanthen-9-CO-, 4-C₆H₅CH₂CH₂CONHC₆H₄SO₂-, 4-NO₂C₆H₄CHCHCO-, 3-NO₂C₆H₄CHCHCO-, 2-NO₂C₆H₄CHCHCO-, 2,3-(CH₃O)₂C₆H₃CHCHCO-, 3, 4- (CH₃O)₂C₆H₃CHCHCO-, 2, 5- (CH₃O)₂C₆H₃CHCHCO-, 2, 5- (CH₃O)₂C₆H₃CH₂CH₂CO-, 3, 5- (CH₃O)₂C₆H₃CH₂CH₂CO-, 3, 4- (CH₃O)₂C₆H₃CH₂CH₂CO-, 2,4- (CH₃O)₂C₆H₃CHCHCO-, 2, 4- (CH₃O)₂C₆H₃CH₂CH₂CO-, 3,4(CH₃O)₂C₆H₃CHCHCO-, 2, 3- (CH₃O)₂C₆H₃CH₂CH₂CO-, 4-CH₃OC₆H₄CHCHCO-, 4-CH₃OC₆H₄CH₂CH₂CO-, 2-CH₃OC₆H₄CHCHCO-, 3-CH₃OC₆H₄CHCHCO-, 3-CH₃OC₆H₄CH₂CH₂CO-, 2-CH₃OC₆H₄CH₂CH₂CO-, 4-CH₃OC₆H₄CHCHCO-, 4-HOC₆H₄CHCHCO-, 3-NH₂C₆H₄CH₂CH₂CO-, 3-C₅H₄NCHCHCO-, 3-C₅H₄NCH₂CH₂CO-, Fluoren-CH₂CO-, Campher-10-CH₂-SO₂-, (C₆H₅)₂CH-CO-, 1-Naphthyl-SO₂-, 2-Naphthyl-SO₂-, Fluorenyl-SO₂-, Phenanthryl-SO₂-, Anthracenyl-SO₂-, Chinolin-SO₂-, 4-CH₃COONHC₆H₄SO₂-, C₆H₅CHCH-SO₂-, 4-NO₂C₆H₄-SO₂- und einer Sulfonylgruppe der Formel (3) 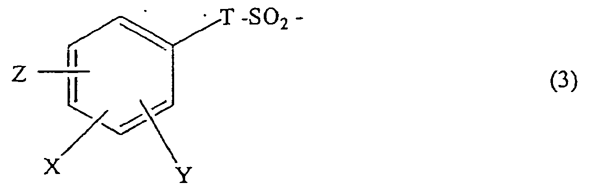 worin T gleich -(CH₂)ₘₘ- ist, worin mm gleich 0 ist und worin X, Y und Z unabhängig voneinander ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂ und -COR₅, worin R₅ ausgewählt ist aus der Gruppe bestehend aus H und einer gerad- oder verzweigtkettigen Alkylgruppe mit 1 bis 6 Kohlenstoffatomen.

23. Verwendung nach Anspruch 1, wobei die Verbindung der Formel Ia eine Aminogruppe oder pharmazeutisch verträgliche Ammoniumsalze davon umfasst, worin R₂ eine Sulfonylgruppe der Formel (3) ist 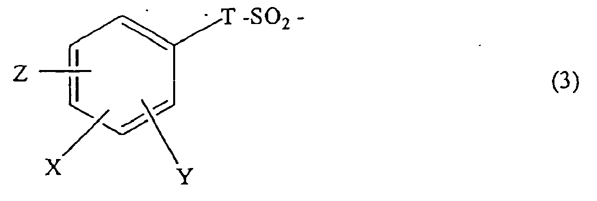 worin T ausgewählt ist aus der Gruppe, bestehend aus -(CH₂)ₘₘ-, -CH=CH- und -CH₂-CH=CH-;
worin mm gleich U, 1, 2, 3 oder 4 ist;
worin X, Y und Z gleich oder verschieden sind und unabhängig voneinander ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅-, -COOR₅, -CH₂OH, -COR₅ und -NHCOAryl, wobei Aryl eine unsubstituierte Phenylgruppe oder eine mit einem oder mehreren Substituenten aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COR₅, -CH₂OH, -COR₅, substituierte Phenylgruppe ist, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe, bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und 1 bis 4 Heteroatome, ausgewählt aus der Gruppe bestehend aus Stickstoff, Sauerstoff und Schwefel umfassen, wobei die heterozyklischen Gruppen monozyklische, bizyklische oder monozyklische mit einem oder zwei Benzolringen kondensierte Gruppen sind.

24. Verwendung nach Anspruch 23, wobei die Verbindung der Formel Ia eine Aminogruppe oder pharmazeutisch verträgliche Ammoniumsalze davon umfasst, worin W gleich -(CH₂)ₙ- ist und worin n gleich 4 ist.

25. Verwendung nach Anspruch 1, wobei die Verbindung der Formel Ia eine Aminogruppe oder pharmazeutisch verträgliche Ammoniumsalze davon umfasst, worin R₂ eine Sulfonylgruppe der Formel (3) ist 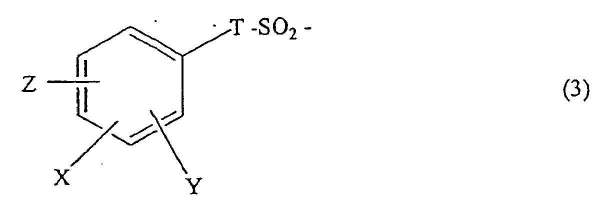 worin T ausgewählt ist aus der Gruppe bestehend aus -(CH₂)ₘₘ-, -CH=CH- und -CH₂-CH=CH-;
worin D ausgewählt ist aus der Gruppe bestehend aus O, NR₇ und S,
worin m gleich 1, 2, 3 oder 4 ist;
worin mm gleich 0, 1, 2, 3 oder 4 ist;
worin X, Y und Z gleich oder verschieden sind und unabhängig voneinander ausgewählt sind aus der Gruppe bestehend aus H, einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -NHCOHeterozyklus, wobei der Heterozyklus wie vorstehend definiert ist, -OR₅, -SR₅, -SOR₅, -SO₂R₅, -COOR₅, -CH₂OH, -COR₅ und -NHCOAryl, wobei Aryl eine unsubstituierte Phenylgruppe oder eine mit einem oder mehreren Substituenten ausgewählt aus der Gruppe bestehend aus einer gerad- oder verzweigtkettigen Alkylgruppe mit 1 bis 6 Kohlenstoffatomen, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ substituierte Phenylgruppe ist, worin R₅ und R₆ unabhängig voneinander ausgewählt sind aus der Gruppe bestehend aus H und einer gerad- oder verzweigtkettigen Alkylgruppe mit 1 bis 6 Kohlenstoffatomen, worin R₇ ausgewählt ist aus der Gruppe bestehend aus HO-, CH₃O-, NC-, Benzyloxy und H₂N- und worin der Heterozyklus ausgewählt ist aus der Gruppe bestehend aus heterozyklischen Gruppen mit 5 bis 7 Ringatomen, wobei die Ringatome Kohlenstoffatome und von 1 bis 4 Heteroatome, ausgewählt aus der Gruppe bestehend aus Stickstoff, Sauerstoff und Schwefel, umfassen, wobei die heterozyklischen Gruppen monozyklische, bizyklische oder monozyklische mit 1 oder 2 Benzolringen kondensierte Gruppen sind; und worin R₃ gleich H ist.

26. Verwendung nach Anspruch 25, wobei die Verbindung der Formel Ia eine Aminogruppe oder pharmazeutisch verträgliche Ammoniumsalze davon umfasst, worin W gleich -(CH₂)ₙ- ist und worin n gleich 4 ist.

27. Verwendung nach Anspruch 2, wobei die Verbindung der Formel Ia eine Aminogruppe oder pharmazeutisch verträgliche Ammoniumsalze davon umfasst, worin R₁ ausgewählt ist aus der Gruppe bestehend aus Isobutyl, Cyclopropylmethyl und Benzyl, worin R₂ eine Sulfonylgruppe der Formel (3), wie in Anspruch 1 definiert, ist, worin R₃ gleich H ist und worin Cₓ ausgewählt ist aus der Gruppe bestehend aus -COOM und -COOR₅, wobei M ein Alkalimetall ist und R₅ wie in Anspruch 1 definiert ist.

28. Verwendung nach Anspruch 2, wobei die Verbindung der Formel Ia eine Aminogruppe oder pharmazeutisch verträgliche Ammoniumsalze davon umfasst, worin n gleich 4 ist, worin R₁ ausgewählt ist aus der Gruppe bestehend aus Isobutyl, Cyclopropylmethyl und Benzyl, worin R₂ eine Sulfonylgruppe der Formel (3), wie in Anspruch 1 definiert, ist, worin T gleich -(CH₂)ₘₘ- ist, worin mm gleich 0 ist, worin X, Y und Z gleich oder verschieden sind und ausgewählt sind aus der Gruppe bestehend aus H, einer gerad- oder verzweigtkettigen Alkylgruppe mit 1 bis 6 Kohlenstoffatomen, Br, NO₂, NH₂ und OR₅, worin R₃ gleich H ist, worin Cₓ ausgewählt ist aus der Gruppe bestehend aus -COOM und COOR₅, worin M ein Alkalimetall ist, worin R₅ wie in Anspruch 1 definiert ist und worin R₄ ausgewählt ist aus der Gruppe bestehend aus 9-Fluorenylmethoxycarbonyl, 2, 3- (CH₃O)₂C₆H₃CH₂CH₂CO-, 2,4-(CH₃O)₂C₆H₃CH₂CH₂CO-, 3-Indol-CH₂CH₂CO-, C₆H₅CH₂CH₂CO-, C₆H₅SCH₂CO-, C₆H₅OCH₂CO-, Xanthen-9-CO-, 4-CH₃OC₆H₄CH₂CH₂CO-, 3-CH₃OC₆H₄CH₂CH₂CO-, 2-CH₃OC₆H₄CH₂CH₂CO-, 3-NH₂C₆H₄CH₂CH₂CO- und 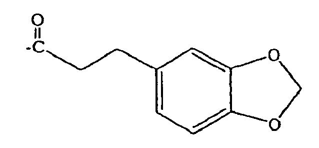 ist.

## Revendications

1. Utilisation d'un composé de formule la ou d'un sel de celui-ci acceptable du point de vue pharmaceutique : 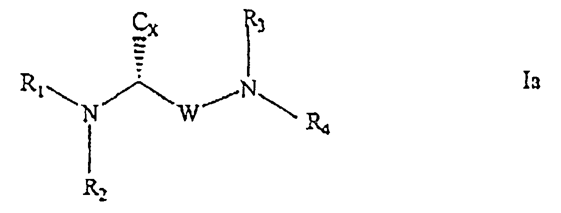 et dans laquelle le composé de formule Ia comprend un groupe amino
ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 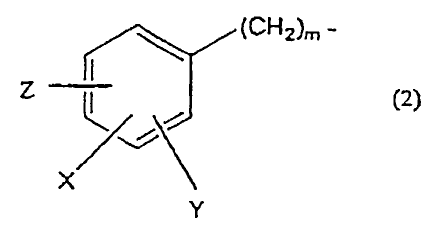 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 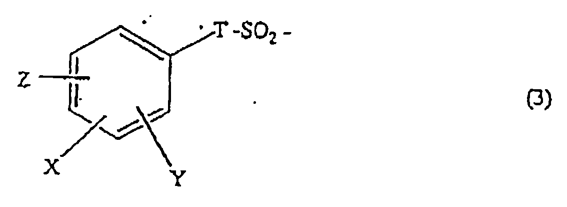 un groupe hétérocycle-alkylsulfonyle de formule hétérocycle-(CH₂)ₘ-SO₂- et un groupe carbonyle de formule (4) : 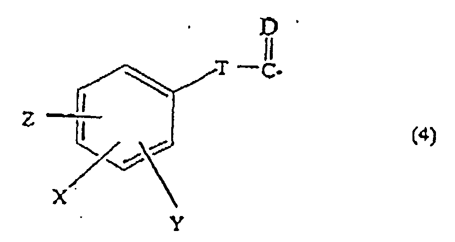 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1, 2, 3 ou 4 ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, Cl, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène, pour la préparation d'un médicament destiné au traitement ou à la prévention de la maladie d'Alzheimer chez un sujet ayant besoin d'un tel traitement.

2. Utilisation selon la revendication 1 pour le traitement de la maladie d'Alzheimer chez un sujet ayant besoin d'un tel traitement.

3. Utilisation selon la revendication 1 ou 2 pour le traitement de la maladie d'Alzheimer par modulation de l'activité de l'enzyme convertissant l'amyloïde bêta.

4. Utilisation selon la revendication 1, comprenant de plus l'utilisation d'un inhibiteur de P-gp, ou d'un sel de celui-ci acceptable du point de vue pharmaceutique.

5. Utilisation d'un composé de formule (Ia) ou d'un sel de celui-ci acceptable du point de vue pharmaceutique : 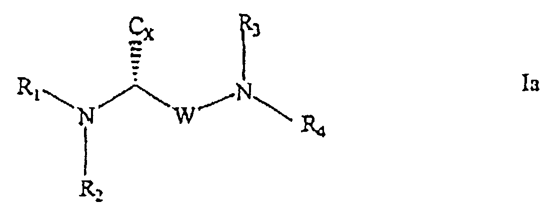 et dans laquelle le composé de formule la comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 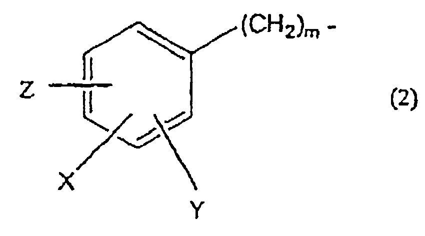 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 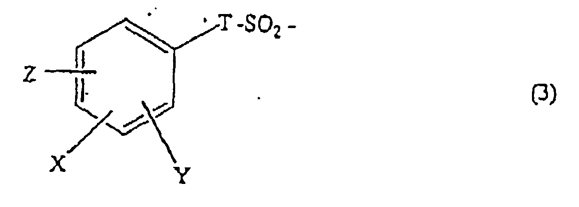 un groupe hétérocycle-alkylsulfonyle de formule hétérocycle-(CH₂)ₘ-SO₂- et un groupe carbonyle de formule (4) : 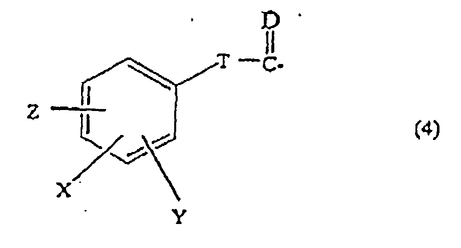 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1, 2, 3 ou 4 ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, Cl, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène, pour la préparation d'un médicament pour traiter un sujet qui a, ou pour empêcher un sujet d'avoir une maladie ou un état choisi dans le groupe comprenant la maladie d'Alzheimer, pour contribuer à prévenir ou à retarder l'apparition de la maladie d'Alzheimer, pour traiter des sujets souffrant d'une légère altération cognitive (MCI) et pour prévenir ou retarder l'apparition de la maladie d'Alzheimer chez des sujets qui vont progresser de MCI à AD, pour traiter le syndrome de Down, pour traiter des humains qui ont une hémorragie cérébrale héréditaire avec amyloïdose du type Dutch, pour traiter une angiopathie amyloïde cérébrale et prévenir ses conséquences potentielles, à savoir des hémorragies lobaires isolées et récurrentes, pour traiter d'autres démences dégénératives, y compris les démences d'origine vasculaire et dégénérative mixte, les démences associées à la maladie de Parkinson, les démences frontotemporales avec parkinsonisme (FTDP), les démences associées à une paralysie supranucléaire progressive, les démences associées à une dégénérescence basale corticale, ou un type diffus à corps de Lewy de la maladie d'Alzheimer.

6. Utilisation selon l'une quelconque des revendications 1 à 5, dans laquelle le composé de formule Ia est choisi dans le groupe consistant en : 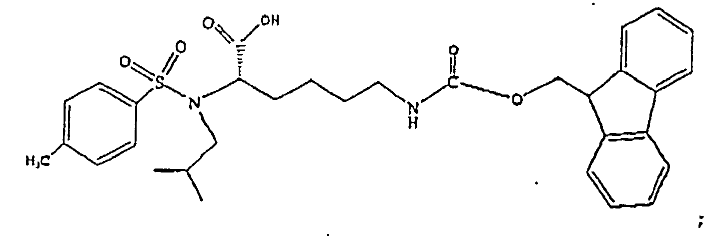 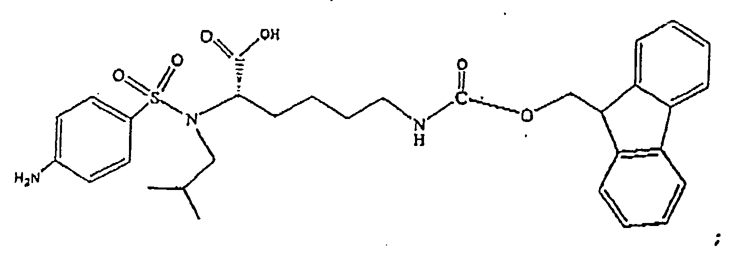 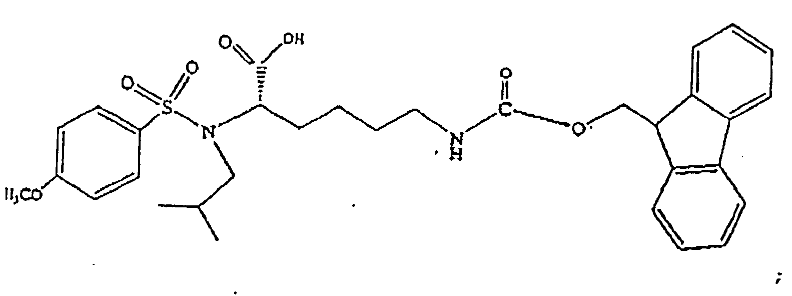 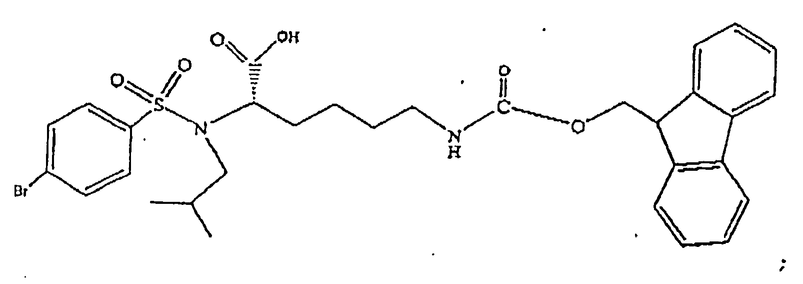 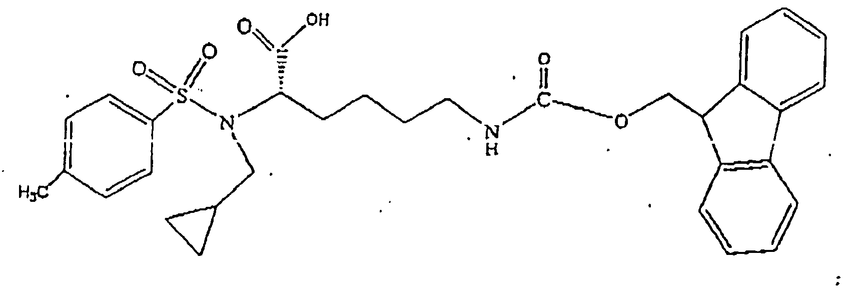 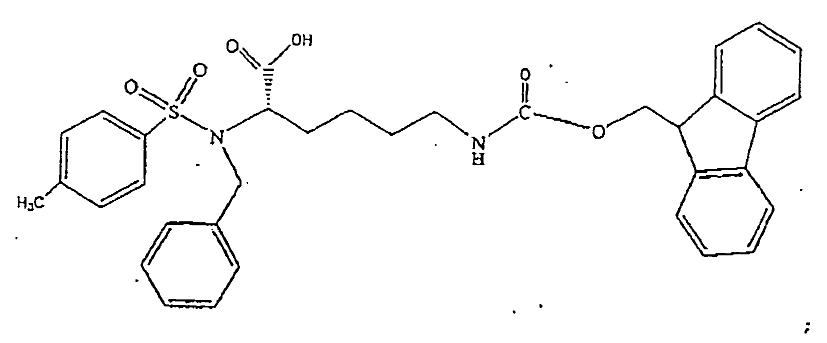 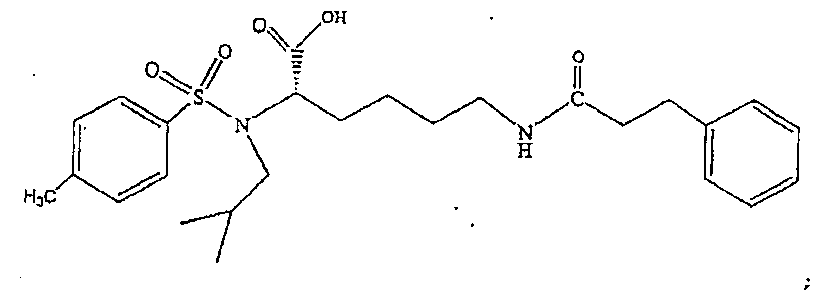 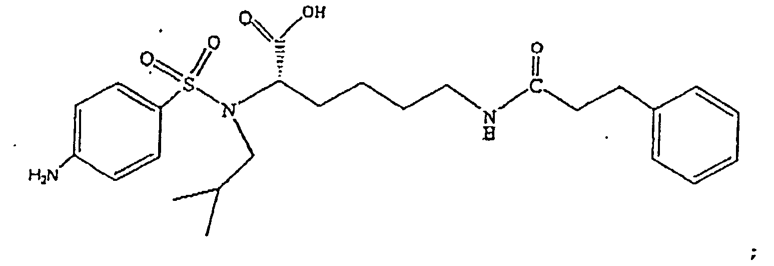 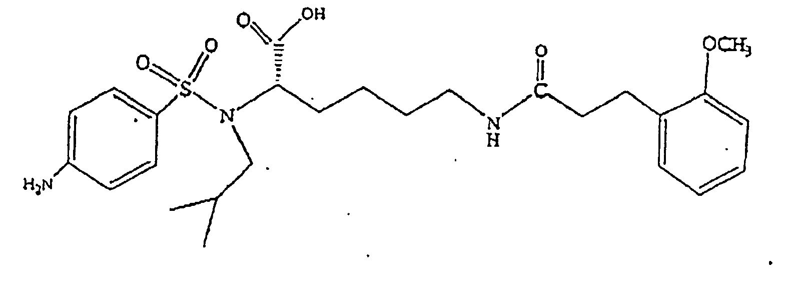 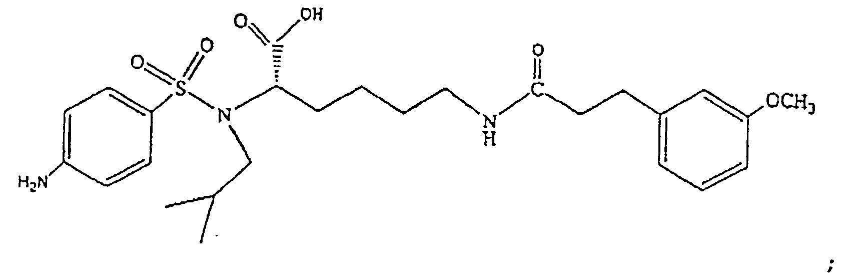 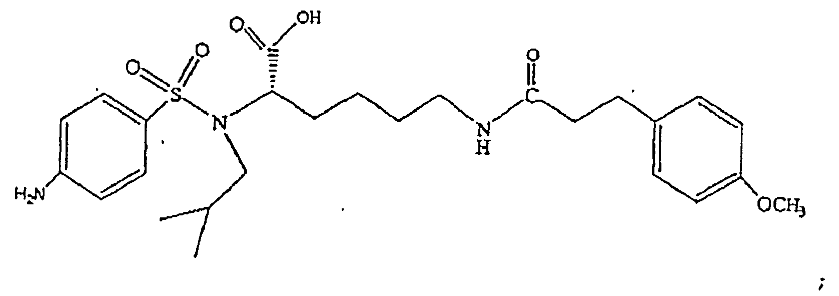 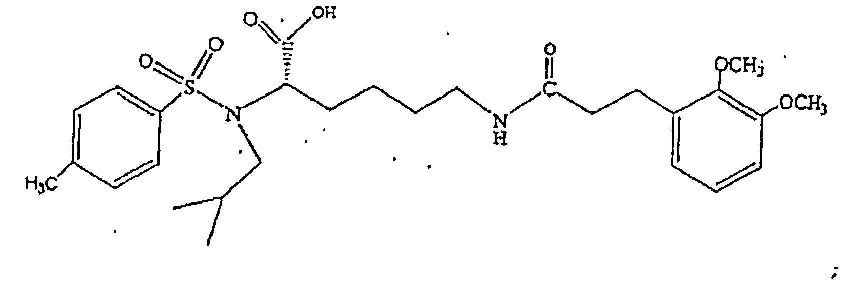 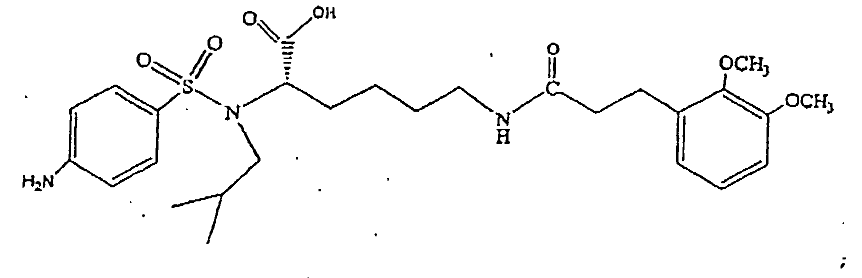 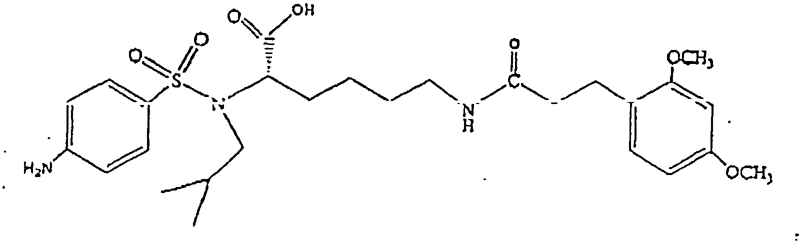 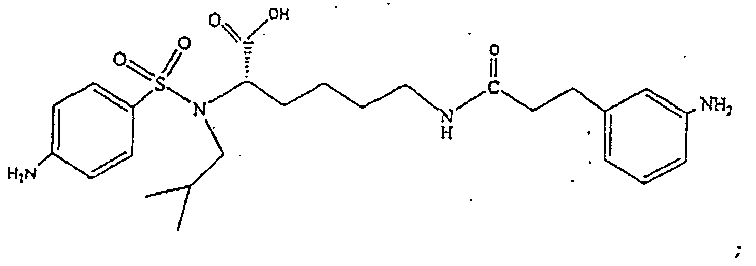 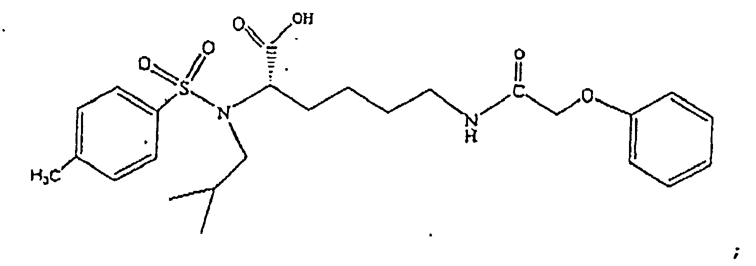 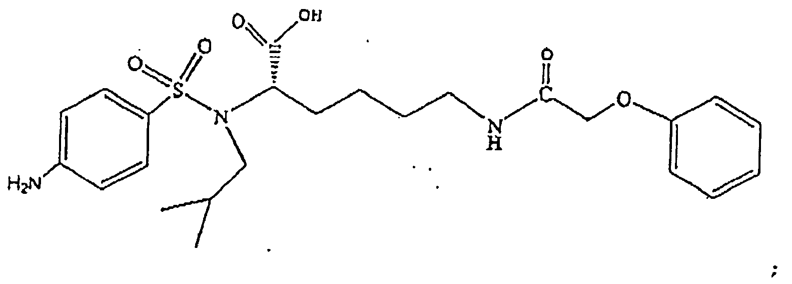 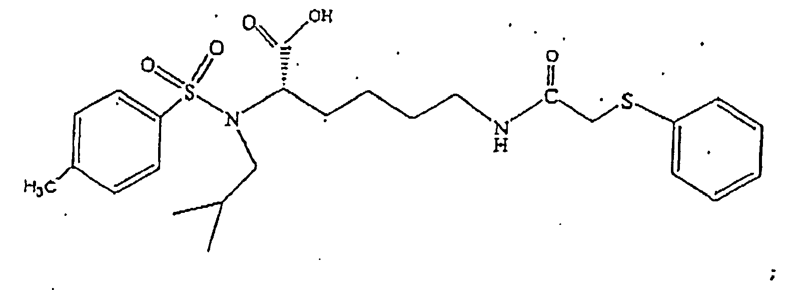 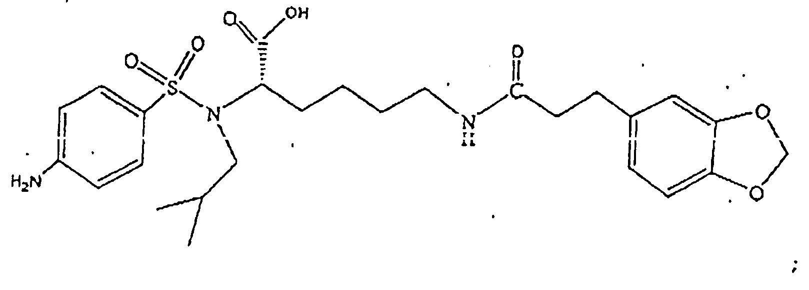 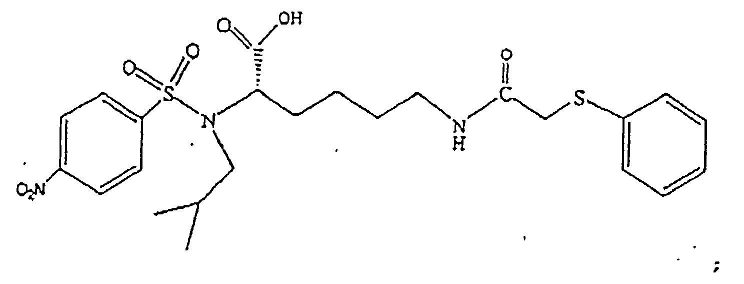 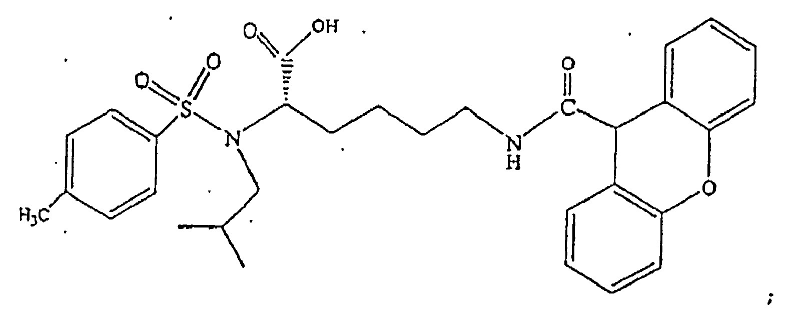 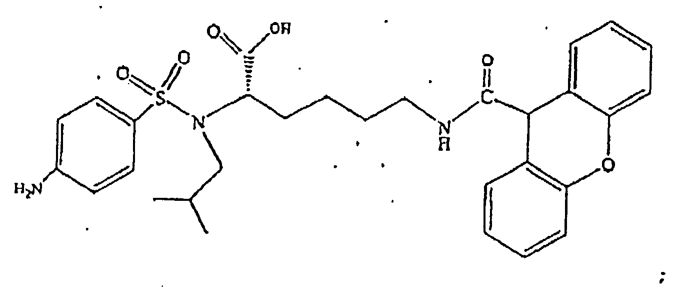 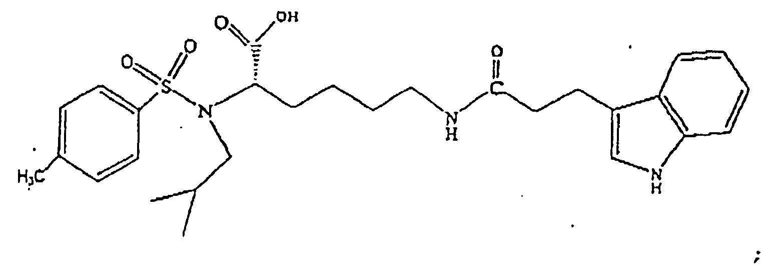 et 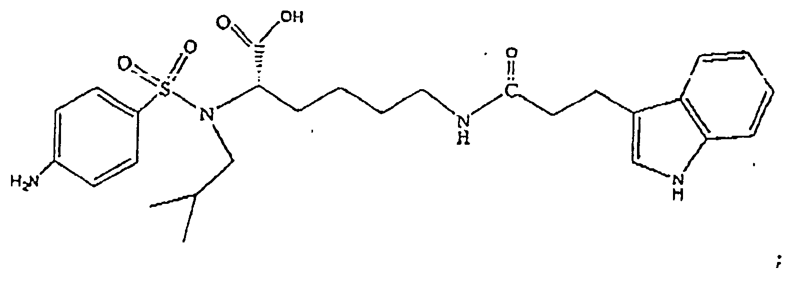 ou les sels d'ammonium, K, Na et Cs de ceux-ci acceptables du point de vue pharmaceutique.

7. Utilisation d'une composition comprenant un ou plusieurs supports acceptables du point de vue pharmaceutique et un composé de formule Ia ou un sel de celui-ci acceptable du point de vue pharmaceutique : 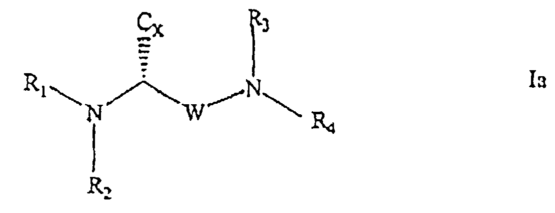 et dans laquelle le composé de formule Ia comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 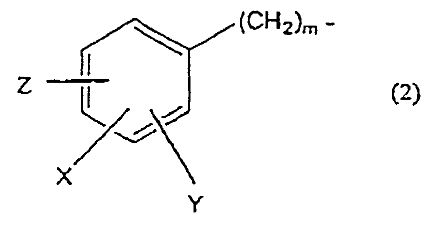 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 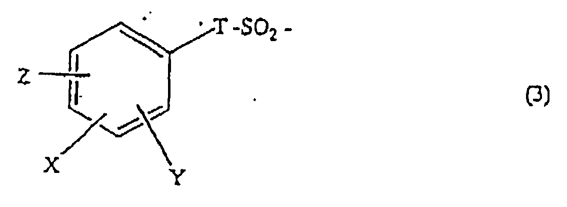 un groupe hétérocycle-alkylsulfonyle de formule hétérocycle-(CH₂)ₘ-SO₂- et un groupe carbonyle de formule (4) : 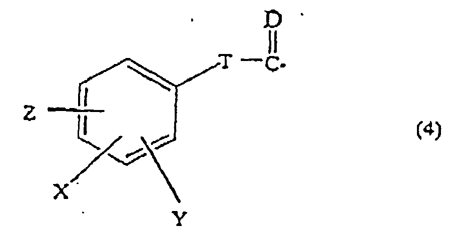 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1, 2, 3 ou 4 ;
où mm est 0, 1, 2, 3 ou 4 ;
où X, Y et Z, identiques ou différents, sont choisis indépendamment dans le groupe consistant en H, un groupe alkyle linéaire ou ramifié de 1 à 6 atomes de carbone, F, Cl, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène, pour la préparation d'un médicament destiné à traiter ou prévenir la maladie d'Alzheimer chez un sujet ayant besoin d'un tel traitement.

8. Utilisation d'un composé de formule la dans la fabrication d'un médicament destiné au traitement ou à la prévention d'états choisis dans le groupe consistant en maladie d'Alzheimer, légère altération cognitive (MCI), syndrome de Down, hémorragie cérébrale héréditaire avec amyloïdose du type Dutch, angiopathie amyloïde cérébrale, démences dégénératives, y compris les démences d'origine vasculaire et dégénérative mixte, les démences associées à la maladie de Parkinson, les démences frontotemporales avec parkinsonisme (FTDP), les démences associées à une paralysie supranucléaire progressive, les démences associées à une dégénérescence basale corticale, ou un type diffus à corps de Lewy de la maladie d'Alzheimer : 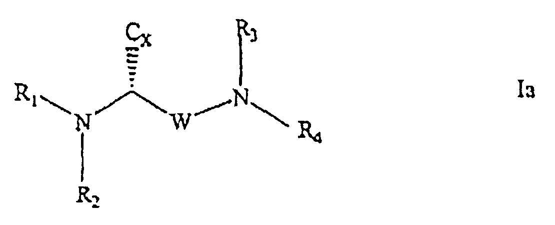 et dans laquelle le composé de formule Ia comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 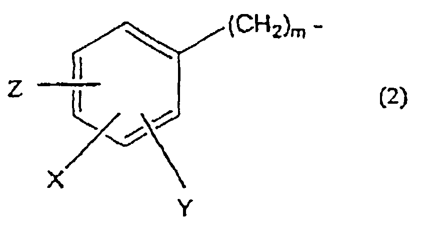 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 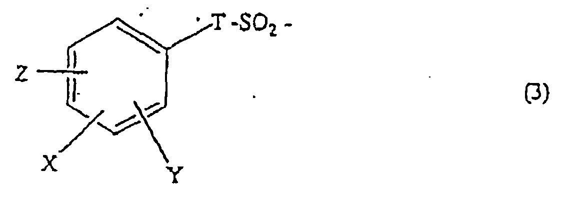 un groupe hétérocycle-alkylsulfonyle de formule hétérocycle-(CH₂)ₘ-SO₂- et un groupe carbonyle de formule (4) : 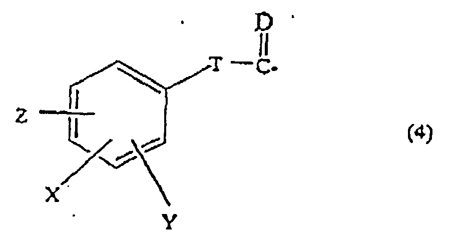 dans lesquelles T est choisi dans le groupe consistant en - (CH₂)ₘₘ,-, -CH=CH- et -CH₂-CH=CH- ;
où D est choisi dans le groupe consistant en O, NR₇ et S,
où m est 1, 2, 3 ou 4 ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, Cl, Br, 1, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, Cl, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène.

9. Procédé *in vitro* pour inhiber l'activité bêta-sécrétase, comprenant la mise en contact d'une quantité efficace pour l'inhibition d'un composé de formule la : 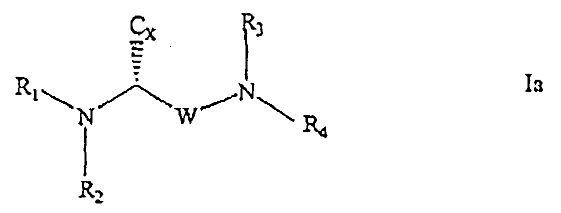 et dans laquelle le composé de formule la comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 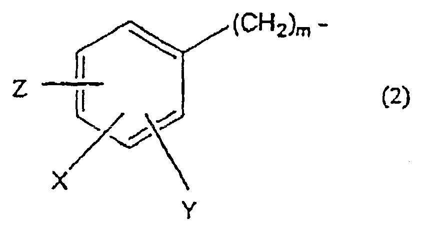 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 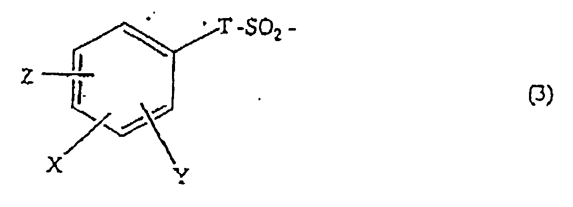 un groupe hétérocycle-alkylsulfonyle de formule hétérocycle-(CH₂)ₘ-SO₂- et un groupe carbonyle de formule (4) : 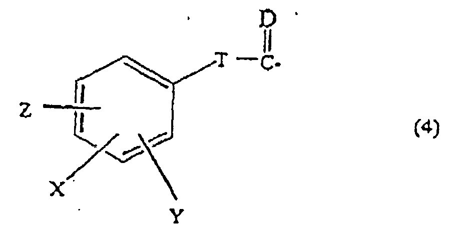 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1, 2, 3 ou 4 ;
où mm est 0, 1 , 2, 3 ou 4 ;
où X, Y et Z, identiques ou différents, sont indépendamment choisis dans le groupe consistant en H, un groupe alkyle linéaire ou ramifié de 1 à 6 atomes de carbone, F, CI, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène.

10. Procédé *in vitro* pour inhiber le clivage d'un isotype de protéine précurseur d'amyloïde (APP) au niveau d'un site dans l'isotype de APP qui est susceptible d'être clivé, comprenant la mise en contact dudit isotype de APP avec une quantité inhibitrice efficace du clivage d'un composé de formule Ia : 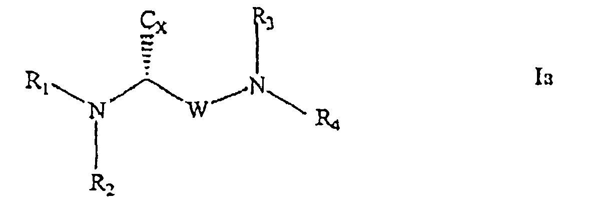 et dans laquelle le composé de formule Ia comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 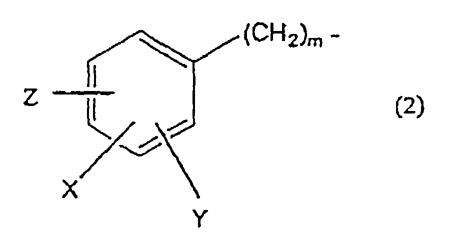 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 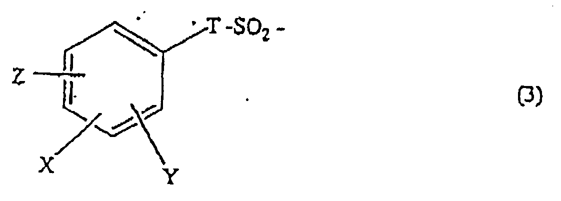 un groupe hétérocycle-alkylsulfonyle de formule hétérocycle-(CH₂)ₘ-SO₂- et un groupe carbonyle de formule (4) : 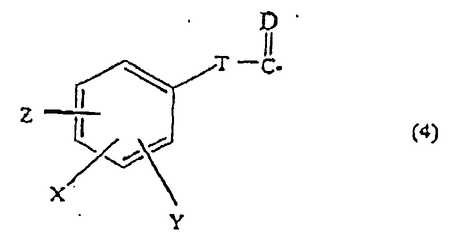 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1, 2, 3 ou 4 ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, Cl, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, 1, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène.

11. Procédé *in vitro* pour inhiber la production du peptide amyloïde bêta (A bêta) dans une cellule, comprenant l'administration à ladite cellule d'une quantité inhibitrice efficace d'un composé de formule Ia : 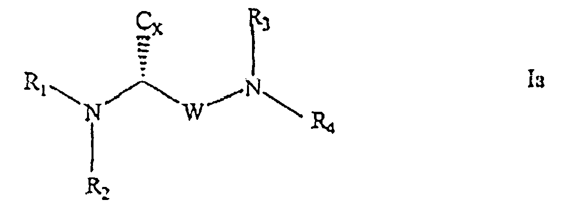 et dans laquelle le composé de formule la comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 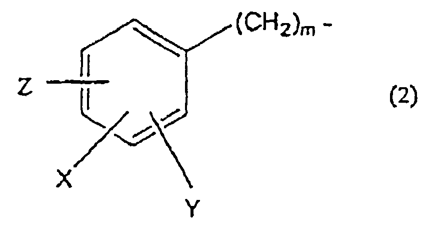 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 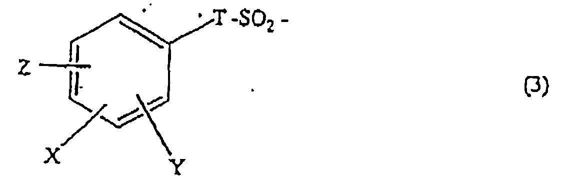 un groupe hétérocycle-alkylsulfonyle de formule hétérocycle-(CH₂)ₘ-SO₂- et un groupe carbonyle de formule (4) : 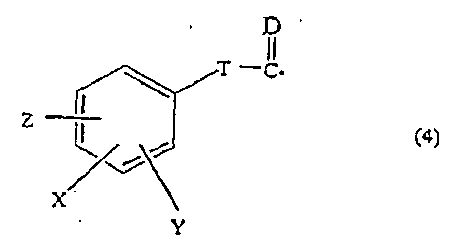 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1, 2, 3 ou 4 ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, Cl, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène.

12. Procédé selon la revendication 11 , dans lequel la cellule est une cellule animale.

13. Procédé selon la revendication 12, dans lequel la cellule animale est une cellule de mammifère.

14. Procédé selon la revendication 13, dans lequel la cellule de mammifère est humaine.

15. Composition comprenant une bêta-sécrétase complexée avec un composé de formule la : 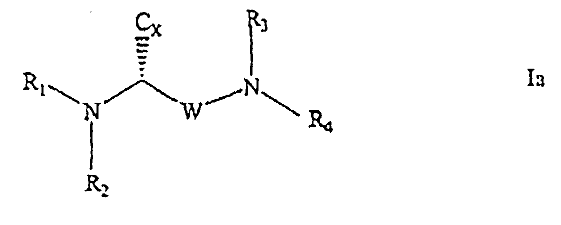 et dans laquelle le composé de formule Ia comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 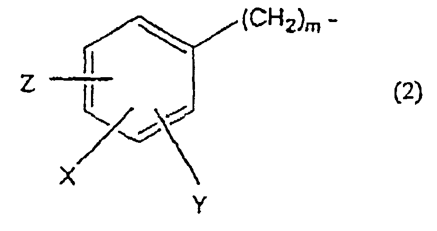 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 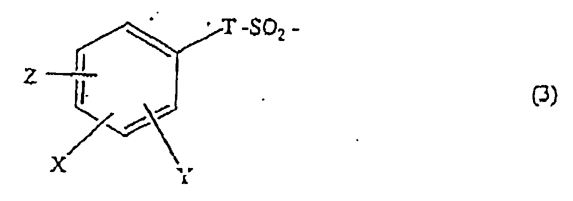 un groupe hétérocycle-alkylsulfonyle de formule hétérocycle-(CH₂)ₘ-SO₂- et un groupe carbonyle de formule (4) : 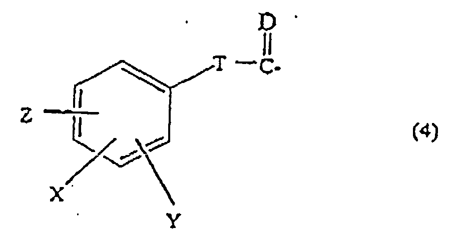 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1 , 2, 3 ou 4 ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, Cl, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène.

16. Procédé de production d'un complexe de bêta-sécrétase comprenant la composition selon la revendication 15.

17. Utilisation d'un composé de formule Ia : 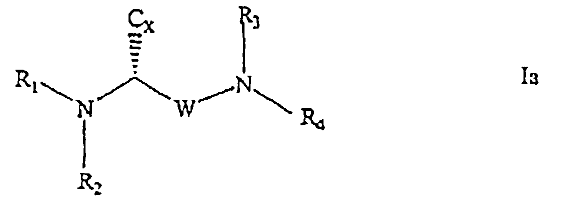 et dans laquelle le composé de formule la comprend un groupe amino ou des sels d'ammonium de celui-ci acceptables du point de vue pharmaceutique,
dans laquelle W est choisi dans le groupe consistant en -(CH₂)ₙ- et -CH₂-XX-CH₂-CH₂-
où n est 1, 2, 3, 4 ou 5,
où XX est choisi dans le groupe consistant en O, NR₅, S, SO et SO₂,
dans laquelle Cx est choisi dans le groupe consistant en - COOM, -COOR₅, -CH₂OH, -CONR₅R₆, -CONHOH, 9-fluorénylméthoxycarbonyl-lysyl-NH-CO-, benzyloxycarbonyle et tétrazolyle,
où M est un métal alcalin ou un métal alcalino-terreux,
dans laquelle R₁ et R₃, identiques ou différents, sont choisis dans le groupe consistant en H, t-butoxycarbonyle, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone, un groupe arylalkyle de formule (2) : 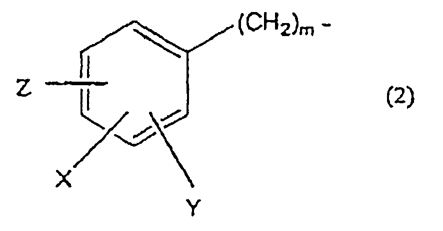 et un groupe hétérocycle-alkyle de formule hétérocycle-(CH₂)ₘ-
dans laquelle R₂ et R₄, identiques ou différents, sont indépendamment choisis dans le groupe consistant en H, CHO-, CF₃-, CH₃CO-, benzoyle, 9-fluorénylméthoxycarbonyle, t-butoxycarbonyle, benzyloxycarbonyle, 2-chlorobenzyloxy-carbonyle, 4-OH-7-CF₃₋quinoléine-3-CO-, 3-indole-CH₂CH₂CO-, 3-indole-CH₂CO-, 3-indole-CO-, 2-indole-CO-, C₆H₅OCH₂CO-, (C₆H₅)₂COHCO-, C₆H₅SCH₂CO-, C₆H₅CH₂CH₂CS-, cholestéryl-OCO-, 2-quinoléine-CO-, xanthène-9-CO-, 4-C₆H₅CH₂CH₂CONHC₆H₄SO₂-, 2-NO₂C₆H₄CHCHCO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, fluorène-CO-, 1-naphtyl-SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂-, un groupe arylalkyle de formule (2) telle que définie plus haut, un groupe sulfonyle de formule (3) : 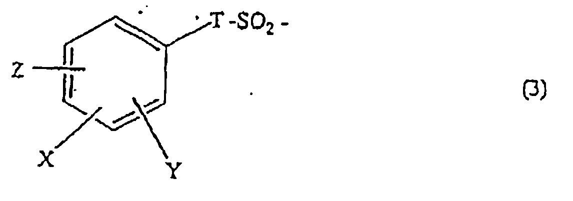 un groupe hétérocycle-alkylsulfonyle de formule hétérocycle-(CH₂)ₘ-SO₂- et un groupe carbonyle de formule (4) : 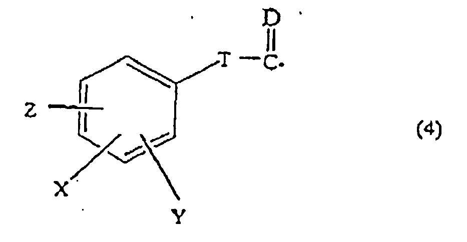 dans lesquelles T est choisi dans le groupe consistant en - (CH₂)ₘₘ-, -CH=CH- et -CH₂-CH=CH- ;
où D est choisi dans le groupe consistant en O, NR₇ et S,
où m est 1, 2, 3 ou 4 ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, CI, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène pour la préparation d'un médicament destiné au traitement ou à la prévention d'une maladie **caractérisée par** des dépôts d'amyloïde bêta sur ou dans le cerveau.

18. Utilisation selon l'une quelconque des revendications 1 à 5, comprenant de plus l'utilisation d'un ou de plusieurs agents thérapeutiques choisis dans le groupe consistant en un antioxydant, un anti-inflammatoire, un inhibiteur de gamma sécrétase, un agent neurotrophique, un inhibiteur d'acétyl cholinestérase, une statine, des inhibiteurs de P-gp, un peptide A bêta et un peptide anti-A bêta.

19. Utilisation selon la revendication 1, dans laquelle le composé de formule Ia comprend un groupe amino ou des sels d'ammonium de celui-ci acceptables du point de vue pharmaceutique, dans laquelle W est -(CH₂)ₙ-, n est 3 ou 4 et D est O.

20. Utilisation selon la revendication 1, dans laquelle le composé de formule Ia comprend un groupe amino ou des sels d'ammonium de celui-ci acceptables du point de vue pharmaceutique, où l'hétérocycle est choisi dans le groupe consistant en benzimidazolyle, imidazolyle, imidazolinyle, imidazolidinyle, quinoléyle, isoquinoléyle, indolyle, pyridyle, pyrrolyle, pyrrolinyle, pyrrolidinyle, pyrazolyle, pyrazinyle, quinoxolyle, pipéridinyle, morpholinyle, β-carbolinyle, tétrazolyle, thiazolidinyle, benzofuranyle, thiamorpholinyle, benzoxazolyle, oxopipéridinyle, oxopyrrolidinyle, oxoazépinyle, azépinyle, isoxazolyle, tétrahydropyranyle, tétrahydrofuranyle, thiadiazolyle, thiadiazinyle, benzodioxolyle, thiophényle, tétrahydrothiophényle, nicoticoyle, morpholinecarbodithioyle et sulfolanyle.

21. Utilisation selon la revendication 1, dans laquelle le composé de formule Ia comprend un groupe amino ou des sels d'ammonium de celui-ci acceptables du point de vue pharmaceutique, dans laquelle n est 4.

22. Utilisation selon la revendication 1, dans laquelle le composé de formule la comprend un groupe amino ou des sels d'ammonium de celui-ci acceptables du point de vue pharmaceutique, dans laquelle Cx est Cx est choisi dans le groupe consistant en - COOM, -COOR₅, -CH₂OH, -CONHOH et benzyloxycarbonyle, où M est un métal alcalin et R₇ est tel que défini dans la revendication 1, dans laquelle R₁ et R₃, identiques ou différents, sont choisis dans le groupe consistant en H, un groupe alkyle linéaire ou ramifié de 1 à 6 atomes de carbone, un groupe cycloalkylalkyle dont la partie cycloalkyle a 3 à 7 atomes de carbone et la partie alkyle a 1 à 3 atomes de carbone et un groupe arylalkyle de formule (2) tel que défini dans la revendication 1, dans laquelle Z et Y sont chacun H, m est 1 et X est H, Br ou F, dans laquelle R₂ et R₄, identiques ou différents, sont choisis dans le groupe consistant en H, 9-fluorénylméthoxycarbonyle, benzyloxycarbonyle, 2-chlorobenzyloxycarbonyle, 4-OH-7-CF₃- quinoléine-3-CO-, 3-indole-CH₂CH₂CO-, 3-indole-CH₂CO-, 3-indole-CO-, 2-indole-CO-, C₆H₅CHCHCO-, C₆H₅CH₂CH₂CO-, C₆H₅CH₂CH₂CH₂CO-, C₆H₅CH₂CHCHCO-, C₆H₅OCH₂CO-, (C₆H₅)₂COHCO-, C₆H₅SCH₂CO-, C₆H₅CH₂CH₂CS-, 4-HOC₆H₄CH₂CH₂CO-, cholestéryl-OCO-, 2-quinoléine-CO-, fluorène-CO-, xanthène-9-CO-, 4-C₆H₅CH₂CH₂CONHC₆H₄SO₂-, 4-NO₂C₆H₄CHCHCO-, 3-NO₂C₆H₄CHCHCO-, 2-NO₂C₆H₄CHCHCO-, 2,3-(CH₃O)₂C₆H₃CHCHCO-, 3,4-(CH₃O)₂C₆H₃CHCHCO-, 2,5-(CH₃O)₂C₆H₃CHCHCO-, 2,5-(CH₃O)₂C₆H₃CH₂CH₂CO-, 3,5-(CH₃O)₂C₆H₃CH₂CH₂CO-, 3,4-(CH₃O)₂C₆H₃CH₂CH₂CO-, 2,4-(CH₃O)₂C₆H₃CHCHCO-, 2,4-(CH₃O)₂C₆H₃CH₂CH₂CO-, 3,4-(CH₃O)₂C₆H₃CHCHCO-, 2,3-(CH₃O)₂C₆H₃CH₂CH₂CO-, 4-CH₃OC₆H₄CHCHCO-, 4-CH₃OC₆H₄CH₂CH₂CO-, 2-CH₃OC₆H₄CHCHCO-, 3-CH₃OC₆H₄CHCHCO-, 3-CH₃OC₆H₄CH₂CH₂CO-, 2-CH₃OC₆H₄CH₂CH₂CO-, 4-CH₃OC₆H₄CHCHCO-, 4-HOC₆H₄CHCHCO-, 3-NH₂C₆H₄CH₂CH₂CO-, 3-C₅H₄NCHCHCO-, 3-C₅H₄NCH₂CH₂CO-, fluorène-CH₂CO-, camphre-10-CH₂-SO₂-, (C₆H₅)₂CH-CO-, 1 naphtyl SO₂-, 2-naphtyl-SO₂-, fluorényl-SO₂-, phénanthryl-SO₂-, anthracényl-SO₂-, quinoléine-SO₂-, 4-CH₃COONHC₆H₄-SO₂-, C₆H₅CHCH-SO₂-, 4-NO₂-C₆H₄-SO₂- et un groupe sulfonyle de formule (3) : 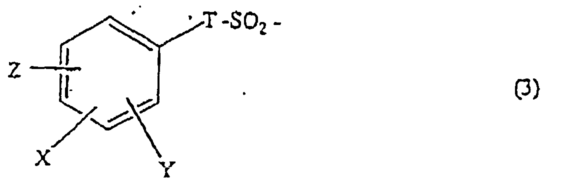 dans laquelle T est -(CH₂)ₘₘ- où mm est 0 et où X, Y et Z sont choisis indépendamment dans le groupe consistant en H, un groupe alkyle linéaire ou ramifié de 1 à 6 atomes de carbone, F, CI, Br, I, -CF₃, -NO₂, -NH₂ et -COR₅, où R₅ est choisi dans le groupe consistant en H et un groupe alkyle linéaire ou ramifié de 1 à 6 atomes de carbone.

23. Utilisation selon la revendication 1, dans laquelle le composé de formule Ia comprend un groupe amino ou des sels d'ammonium de celui-ci acceptables du point de vue pharmaceutique, dans laquelle R₂ est un groupe sulfonyle de formule (3) : 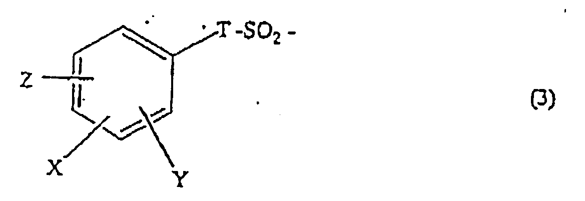 dans laquelle T est choisi dans le groupe consistant en -(CH₂)ₘₘ-, -CH=CH- et -CH₂-CH=CH- ;
où mm est 0, 1, 2, 3 ou 4 ;
où X, Y et Z, identiques ou différents, sont indépendamment choisis dans le groupe consistant en H, un groupe alkyle linéaire ou ramifié de 1 à 6 atomes de carbone, F, CI, Br, I, -CF₃, -NO₂, -NH₂, -NHR₅, -NR₅R₆, -NHCOR₅, -NHCOhéterocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyioxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène.

24. Utilisation selon la revendication 23, dans laquelle le composé de formule Ia comprend un groupe amino ou des sels d'ammonium de celui-ci acceptables du point de vue pharmaceutique, dans laquelle W est -(CH₂)ₙ-, et dans laquelle n est 4.

25. Utilisation selon la revendication 1, dans laquelle le composé de formule la comprend un groupe amino ou des sels d'ammonium de celui-ci acceptables du point de vue pharmaceutique, dans laquelle R₂ est un groupe sulfonyle de formule (3) : 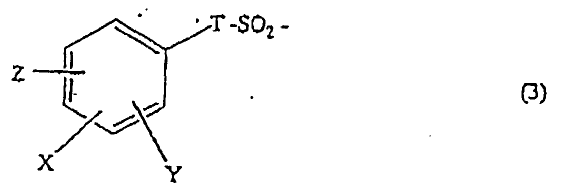 dans laquelle T est choisi dans le groupe consistant en -(CH₂)ₘₘ-, -CH=CH- et -CH₂-CH=CH- ;
où D est choisi dans le groupe consistant en U, NR₇ et S,
où m est 1, 2, 3 ou 4 ;
où mm est 0, 1 , 2, 3 ou 4 ;
où X, Y et Z, identiques ou différents, sont indépendamment choisis dans le groupe consistant en H, un groupe alkyle linéaire ou ramifié de 1 à 6 atomes de carbone, F, Cl, Br, I, -CF₃, -NO₂, -NH₂, -NHR₅, -NR₅R₆, -NHCOR₅, -NHCOhétérocycle, hétérocycle étant tel que défini plus haut, -OR₅, -SR₅, -SOR₅, -SO₂R₅, -COOR₅, -CH₂OH, -COR₅ et -NHCOaryle, aryle étant un groupe phényle non substitué ou un groupe phényle substitué par un ou plusieurs membres du groupe consistant en un groupe alkyle linéaire ou ramifié de 1 à 6 atomes de carbone, F, CI, Br, I, -CF₃, -NO₂, -NH₂, -NHR₅, -NR₅R₆, -NHCOR₅, -OR₅, -SR₅, -SOR₅, -SO₂R₅, -COOR₅, -CH₂OH, -COR₅ où R₅ et R₆ sont choisis indépendamment dans le groupe consistant en H, et un groupe alkyle linéaire ou ramifié de 1 à 6 atomes de carbone, où R₇ est choisi dans le groupe consistant en HO-, CH₃O-, NC-, benzyloxy et H₂N- et où le groupe hétérocycle est choisi dans le groupe consistant en groupes hétérocycliques comprenant 5 à 7 atomes de cycle, lesdits atomes de cycle comprenant des atomes de carbone et de un à quatre hétéroatomes choisis dans le groupe consistant en atomes d'azote, d'oxygène et de soufre, lesdits groupes hétérocycliques étant monocycliques, bicycliques ou monocycliques condensés à un ou deux noyaux benzène ; et dans laquelle R₃ est H.

26. Utilisation selon la revendication 25, dans laquelle le composé de formule Ia comprend un groupe amino ou des sels d'ammonium de celui-ci acceptables du point de vue pharmaceutique, dans laquelle W est -(CH₂)ₙ- et dans laquelle n est 4.

27. Utilisation selon la revendication 2, dans laquelle le composé de formule Ia comprend un groupe amino ou des sels d'ammonium de celui-ci acceptables du point de vue pharmaceutique, dans laquelle R₁ est choisi dans le groupe consistant en isobutyle, cyclopropylméthyle et benzyle, dans laquelle R₂ est un groupe sulfonyle de formule (3) tel que défini dans la revendication 1, dans laquelle R₃ est H et dans laquelle Cx est choisi dans le groupe consistant en -COOM et -COOR₅, M étant un métal alcalin et R₅ étant tel que défini dans la revendication 1.

28. Utilisation selon la revendication 2, dans laquelle le composé de formule la comprend un groupe amino ou des sels d'ammonium de celui-ci acceptables du point de vue pharmaceutique, dans laquelle n est 4, dans laquelle R₁ est choisi dans le groupe consistant en isobutyle, cyclopropylméthyle et benzyle, dans laquelle R₂ est un groupe sulfonyle de formule (3) tel que défini dans la revendication 1, dans laquelle T est -(CH₂)ₘₘ₋, dans laquelle mm est 0, dans laquelle X, Y et Z, identiques ou différents, sont choisis dans le groupe consistant en H, un groupe alkyle linéaire ou ramifié de 1 à 6 atomes de carbone, Br, NO₂, NH₂ et OR₅ dans laquelle R₃ est H, où Cx est choisi dans le groupe consistant en -COOM et -COOR₅, où M est un métal alcalin, où R₅ est tel que défini dans la revendication 1 et dans laquelle R₄ est choisi dans le groupe consistant en 9-fluorénylméthoxycarbonyle, 2,3-(CH₃O)₂C₆H₃CH₂CH₂CO-, 2,4-(CH₃O)₂C₆H₃CH₂CH₂CO-, 3-indole-CH₂CH₂CO-, C₆H₅CH₂CH₂CO-, C₆H₅SCH₂CO-, C₆H₅OCH₂CO-, xanthène-9-CO-, 4-CH₃OC₆H₄CH₂CH₂CO-, 3-CH₃OC₆H₄CH₂CH₂CO-, 2-CH₃OC₆H₄CH₂CH₂CO-, 3-NH₂C₆H₄CH₂CH₂CO- et
